# EUROPEAN PATENT APPLICATION

(11) **EP 4 582 076 A1**
(43) Date of publication of application: **09.07.2025**
(21) Application number: 24222845.0
(22) Date of filing: 23.12.2024
(51) Int. Cl.: A61K 8/36, A61K 8/63, A61K 8/68, A61K 8/92, A61Q 5/00, A61Q 17/00, A61Q 19/08

(54) **COMPOSITION COMPRISING CERAMIDE AND MONO-RHAMNOLIPID**

(30) Priority: 05.01.2024 EP 24150530
(71) Applicant: Evonik Operations GmbH, 45128 Essen (DE)
(72) Inventor: Liebig, Stefan Julian, 40627 Düsseldorf (DE); Truchan, Nadine, 40235 Düsseldorf (DE); Blaschek, Svenja, 45897 Gelsenkirchen (DE); Yalcinkaya-Demirel, Hacer, 45130 Essen (DE); Klostermann, Kristin, 45141 Essen (DE); Pyko, Cornelia, 61118 Bad Vilbel (DE); van Logchem, Monica Desiree, 4762 PA Zevenbergen (NL); Tuchscherer, Benjamin, 44379 Dortmund (DE); Maczkiewitz, Ursula, 45219 Essen (DE)
(74) Representative: Evonik Patent Association

(57) **Abstract**

The invention relates to a composition comprising ceramide and mono-rhamnolipid.

## Description

### Field of the invention

The invention relates to a composition comprising ceramide and mono-rhamnolipid.

### Prior art

Ceramides are a class of lipophilic amides which form the lipid matrix of the skin together with fatty acids and cholesterol. These Amides play an important role in the cosmetics and are established ingredients in various formulations for beneficial effects on skin barrier, trans epidermal water loss and further functions. Many formulations are based on emulsions with higher oil content. Only a few examples of water-based formulations are known as ceramides are hardly soluble substances in aqueous environments.

WO2023161179 discloses liposomal composition comprising biosurfactants and the use of the liposomal composition for encapsulation of at least one cosmetic, pharmaceutical and/or nutraceutical active ingredient. With this it provides liposomal compositions which allows solubilization of hardly soluble substances with outstanding long-time stability in terms of reprecipitation.

KR20190080060 discloses transparent formulations containing ceramides. One disadvantage of these formulation is the usage of ethoxylated surfactants. Another disadvantage is the need for an oily compound, which might be not desired in an aqueous application. US8313755B2 discloses clear aqueous ceramide compositions with polyhydric alcohols.

it is an object of the instant invention to provide compositions comprising ceramides with a clear appearance.

### Description of the invention

it was found, surprisingly, that a combination of mono-rhamnolipids and ceramides give clear aqueous compositions.

The present invention therefore provides compositions comprising mono-rhamnolipids and ceramides.

One advantage of the compositions according to the invention is their enhanced deposition of ceramides on the skin.

A further advantage is the improved penetration of the ceramides in the presence of mono-rhamnolipids.

Another advantage is that the compatibility with organic acids is very high. Another advantage is the improved foam quality of the compositions according to the invention. A further advantage is the foam boosting effect of the compositions according to the invention. Another advantage is the good compatibility of the compositions according to the invention with other ingredients.

A further advantage is the enhanced skin barrier protection.

A further advantage is the enhanced deposition of ceramides on the hair in the composition according to the invention.

A further advantage is the improved skin feel of the composition according to the invention. A further advantage is the improved conditioning effect on hair by the composition according to the invention.

A further advantage is the improved thickenability of formulations.

A further advantage is that the composition according to the invention can be easily incorporated into different formulations.

Another advantage is that the composition according to the invention can be obtaines without any heating step.

Another advantage is the high stability of the composition according to the invention. A further advantage is the mildness of the composition composition according to the invention.

The present invention therefore provides a composition comprising
A) at least one ceramide, and
B) at least one rhamnolipid,
characterized in that component B) comprises at least 50 wt.-% mono-rhamnolipids, wherein the weight percentages refer to all rhamnolipids comprised in the total composition.

in the context of the present invention, the term "ceramide" is understood to mean acylated sphingoid bases, where the sphingoid bases are preferably selected from sphingosine, sphinganine, 6-hydroxysphingosine and phytosphingosine, also in glycosylated form, for example as glucosylceramides.

When determining the amount of rhamnolipid only the mass of the rhamnolipid is taken into account while disregarding the counter ion of the salt, in case the rhamnolipid is present as a salt. Where average values are stated hereinbelow, then, unless stated otherwise, these are number-averaged average values.

Unless stated otherwise, percentages are data in per cent by weight. The same is true for parts per million (ppm).

Wherever measurement values are stated hereinbelow, then, unless stated otherwise, these have been determined at a temperature of 25°C and a pressure of 1013 mbar.

A preferred composition according to the instant invention is characterized in that said at least one ceramide is selected from the group comprising, preferably consisting of, ceramide NP, ceramide AP, ceramide EOP, ceramide NG (also known as ceramide NDS and ceramide 2), ceramide ADS, ceramide EODS, ceramide NS, ceramide AS, ceramide EOS, ceramide NH, ceramide AH and ceramide EOH, preferably selected from the group comprising ceramide NP, ceramide NG, ceramide AP and ceramide EOP, most preferably ceramide NP.

A preferred composition according to the instant invention is characterized in that component A) is comprised in an amount of from 0.0005 wt.-% to 5.0 wt.-%, more preferably from 0.001 wt.-% to 2.0 wt.-%, more preferably from 0.002 wt.-% to 1.5 wt.-%, even more preferably from 0.05 wt.-% to 1.0 wt.-%, where the percentages by weight refer to the total composition.

A preferred composition according to the invention is characterized in that said composition comprises at least two ceramides, preferably at least three ceramides, particularly preferably precisely three ceramides.

The term "rhamnolipids" in the context of the present invention preferably is understood to mean particularly compounds of the general formula (I) and salts thereof, 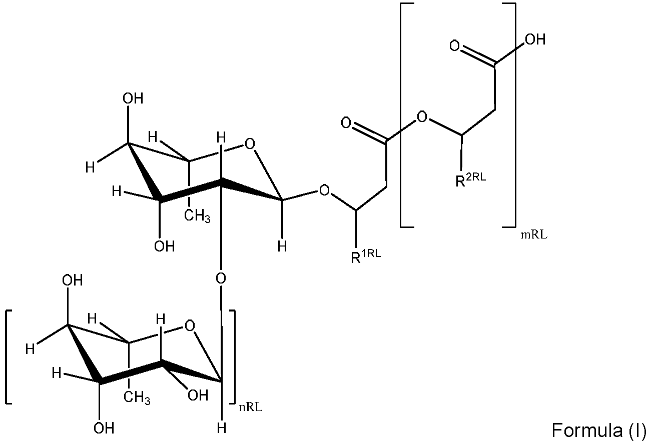 where
mRL = 2, 1 or 0, preferably 1 or 0,
nRL = 1 or 0,
R^{1RL} and R^{2RL} = mutually independently, identical or different, organic residues having 2 to 24, preferably 5 to 13 carbon atoms, in particular optionally branched, optionally substituted, particularly hydroxy-substituted, optionally unsaturated, in particular optionally mono-, bi- or tri-unsaturated alkyl residues, preferably those selected from the group consisting of pentenyl, heptenyl, nonenyl, undecenyl and tridecenyl and (CH₂)ₒ-CH₃ where o = 1 to 23, preferably 4 to 12. If nRL = 1, the glycosidic bond between the two rhamnose units is preferably in the α-configuration. The optically active carbon atoms of the fatty acids are preferably present as R-enantiomers (e.g. (R)-3-{(R)-3-[2-O-(α-L-rhamnopyranosyl)-α-L-rhamnopyranosyl]oxydecanoyl}oxydecanoate).

The term "di-rhamnolipid" in the context of the present invention is understood to mean compounds of the general formula (I) or salts thereof, where nRL = 1.

The term "mono-rhamnolipid" in the context of the present invention is understood to mean compounds of the general formula (I) or salts thereof, where nRL = 0.

Distinct rhamnolipids are abbreviated according to the following nomenclature:
"diRL-CXCY" are understood to mean di-rhamnolipids of the general formula (I), in which one of the residues R^{1RL} and R^{2RL} = (CH₂)ₒ-CH₃ where o = X-4 and the remaining residue R¹ or R² = (CH₂)ₒ-CH₃ where o = Y-4.
"monoRL-CXCY" are understood to mean mono-rhamnolipids of the general formula (I), in which one of the residues R^{1RL} and R^{2RL} = (CH₂)ₒ-CH₃ where o = X-4 and the remaining residue R^{1RL} or R^{2RL} = (CH₂)ₒ-CH₃ where o = Y-4.

The nomenclature used therefore does not distinguish between "CXCY" and "CYCX". For rhamnolipids where mRL=0, monoRL-CX or diRL-CX is used accordingly.

If one of the abovementioned indices X and/or Y is provided with ":Z", this signifies that the respective residue R^{1RL} and/or R^{2RL} is equal to an unbranched, unsubstituted hydrocarbon residue having X-3 or Y-3 carbon atoms having Z double bonds.

The following listed rhamnolipids, given that they comprise di-rhamnolipids, can be adjusted to the desired high content of mono-rhamnolipids by for example rhamnosidases. Rhamnolipids applicable in the context of the instant invention can also be produced by fermentation of *Pseudomonas,* especially *Pseudomonas aeruginosa,* which are preferably non genetically modified cells, a technology already disclosed in the eighties, as documented e.g. in EP0282942 and DE4127908. Rhamnolipids produced in *Pseudomonas aeruginosa* cells which have been improved for higher rhamnolipid titres by genetical modification can also be used in the context of the instant invention; such cells have for example been disclosed by Lei et al. in Biotechnol Lett. 2020 Jun;42(6):997-1002.

Rhamnolipids produced by *Pseudomonas aeruginosa* are commercially available from Jeneil Biotech Inc., e.g. under the tradename Zonix,from Logos Technologies (technology acquired by Stepan), e.g. under the tradename NatSurFact, from Biotensidion GmbH, e.g. under the tradename Rhapynal, from AGAE technologies, e.g. under the name R90, R95, R95Md, R95Dd, from Locus Bio-Energy Solutions and from Shanghai Yusheng Industry Co. Ltd., e.g. under the tradename Bio-201 Glycolipids.

The composition according to the instant invention is preferably characterized in that component B) comprises
12 wt.-% to 32 wt.-% monoRL-C8C10,
51 wt.-% to 81 wt.-% monoRL-C10C10,
1 wt.-% to 9 wt.-% monoRL-C10C12,
1 wt.-% to 9 wt.-% monoRL-C10C12:1,
wherein the weight percentages refer to all mono-rhamnolipids comprised in the composition.

A preferred composition according to the instant invention is characterized in that it is a non-liposomal composition.

The term "non-liposomal composition" means, that the composition does not comprise liposomes.

A preferred composition according to the instant invention is characterized in that it is does not comprise phospholipids.

A preferred composition according to the instant invention is characterized in that it comprises particles with a mean particle size of 13 nm or less, preferably in the range of from 3 nm to 12 nm. Photon correlation spectroscopy is employed in order to determine the mean particle size preferably at a total concentration of component A) of 0,5 wt.% with the composition comprising water. The measurement is performed using a Zetasizer Nano ZS90, Malvern Instruments Ltd.,

UK, according to the manufacturer's instruction. The Z-average is the intensity weighted mean hydrodynamic size of the ensemble collection of particles measured by dynamic light scattering (DLS). The Z-average is derived from a Cumulants analysis of the measured correlation curve, wherein a single particle size is assumed and a single exponential fit is applied to the autocorrelation function (see Zetasizer Nano ZS90 User Manual MAN0485-1-1 09 June 2017).

A preferred composition according to the instant invention is characterized in that component B) is comprised in an amount of from 0.1 wt.-% to 15.0 wt.-%, preferably from 0.5 wt.-% to 10.0 wt.-%, more preferably from 1.0 wt.-% to 8.0 wt.-%, wherein the percentages by weight refer to the total composition.

According to the invention, the ratio by weight of component A) to component B) in the composition according to the invention is preferably from 0.0005: 15 to 0.1: 1, preferably from 0.005: 10 to 0.5: 5, particularly preferably from 0.05: 7 to 0.1: 5.

A preferred composition according to the instant invention is characterized in that it comprises
C) cholesterol and/or at least one cholesterol derivative selected from the group comprising cholesterol sulfate, cholesterol hydrogen succinate and 7-dehydrocholesterol.

Component c) preferably is comprised in an amount of from 0.01 wt.-% to 3.0 wt.-%, preferably from 0.02 wt.-% to 2.0 wt.-%, more preferably from 1.0 wt.-% to 0.05 wt.-%, where the percentages by weight refer to the total composition.

A preferred composition according to the instant invention is characterized in that it comprises
D) at least one sphingoid base, preferably selected from the group comprising sphingosine, sphinganine, 6-hydroxysphingosine, N-acetylphytosphingosine and phytosphingosine, especially phytosphingosine.

Component D) preferably is comprised in an amount of from 0.001 wt.-% to 2.0 wt.-%, preferably from 0.002 wt.-% to 1.5 wt.-%, more preferably from 0.05 wt.-% to 1.0 wt.-%, where the percentages by weight refer to the total composition.

A preferred composition according to the instant invention is characterized in that it comprises instead of or additionally to component D)
E) at least one fatty acid, preferably selected from the group of fatty acids comprising 12 to 28 carbon atoms, especially palmitic acid, stearic acid, octadecanoic acid, arachidic acid, behenic acid, docosanoic acid and lignoceric acid.

Component E) preferably is comprised in an amount of from 0.01 wt.-% to 3.0 wt.-%, preferably from 0.02 wt.-% to 2.0 wt.-%, more preferably from 1.0 wt.-% to 0.05 wt.-%, where the percentages by weight refer to the total composition.

A preferred composition according to the instant invention is characterized in that it has a pH in the range of 4.0 to 8.0, preferably 4.5 to 7.4 particularly preferably 5.0 to 7.2. The "pH" in connection with the present invention is defined as the value which is measured for the relevant composition at 22°C after stirring for five minutes using a pH electrode calibrated in accordance with ISO 4319 (1977).

A preferred composition according to the instant invention is characterized in that it comprises water, preferably in the range of 75.0 wt.-% to 99.5 wt.-%, preferably from 80.0 wt.-% to 98.5 wt.-%, more preferably from 85.0 wt.-% to 98.0 wt.-%, based on the total composition.

The present invention further relates to a process for producing a cosmetic or pharmaceutical formulation comprising at least one ceramide, comprising the process steps of:
a) providing a composition according to the instant invention;
b) adding a cosmetic or pharmaceutical acceptable carrier, preferable water, and preferably one further cosmetical and pharmaceutical formulation ingredient.

Said one further cosmetical and pharmaceutical formulation ingredient of step b) is, of course different from any component A) to E) comprised in the composition of the instant invention. Preferred compositions according to the instant invention are preferably used in the context of the process according to the instant invention.

Said further cosmetical and pharmaceutical formulation ingredient preferably added in step b) of the process of the instant invention is preferably selected from the group of surfactants, emollients, emulsifiers, thickeners, UV light protection filters, antioxidants, hydrotropes, solids and fillers, film formers, pearlescence additives, opacifiers, deodorant and antiperspirant active ingredients, insect repellents, self-tanning agents, preservatives, conditioning agents, perfumes, dyes, odour absorbers, superfatting agents and solvents, preferably perfumes, conditioning agents and thickeners,

A preferred process according to the instant invention is characterized in that said process comprises
c) solubilization of the formulation.

In consequence, cosmetic or pharmaceutical formulation produced by the process according to the instant invention preferably is a water based surfactant formulation.

To facilitate the solubilization step c) of the process according to the instant invention it is preferred in accordance with the invention that the at least one additional surfactant is present in process step c).

Said at least one additional surfactant present in process step c) of the instant invention is preferably selected from the group of anionic, cationic, non-ionic, semi-polar, amphoteric and zwitterionic surfactants.

The nonionic surfactants used are preferably alkoxylated, advantageously ethoxylated, in particular primary alcohols having preferably 8 to 18 carbon atoms and on average 1 to 12 mol of ethylene oxide (EO) per mol of alcohol, in which the alcohol radical can be linear or preferably 2-position methyl-branched or can contain linear and methyl-branched radicals in a mixture, as are customarily present in oxo alcohol radicals. In particular, however, alcohol ethoxylates with linear radicals from alcohols of native origin having 12 to 18 carbon atoms, for example from coconut, palm, tallow fat or oleyl alcohol, and on average 2 to 8 EO per mol of alcohol are preferred. The preferred ethoxylated alcohols include, for example, C12-C14-alcohols with 3 EO, 4 EO or 7 EO, C9-C11-alcohol with 7 EO, C13-C15-alcohols with 3 EO, 5 EO, 7 EO or 8 EO, - 9 -C12-C18-alcohols with 3 EO, 5 EO or 7 EO and mixtures of these, such as mixtures of C12-C14-alcohol with 3 EO and C12-C18-alcohol with 7 EO. The stated degrees of ethoxylation are statistical average values which can be an integer or a fraction for a specific product. Preferred alcohol ethoxylates have a narrowed homolog distribution.

In addition to these nonionic surfactants, it is also possible to use fatty alcohols with more than 12 EO. Examples thereof are tallow fatty alcohol with 14 EO, 25 EO, or 40 EO. Nonionic surfactants which contain EO and PO (propylene oxide) groups together in the molecule can also be used. In this connection, it is possible to use block copolymers with EO-P0 block units or PO-E0 block units, but also EO-PO-E0 copolymers or P0-E0-P0 copolymers.

It is of course also possible to use mixed alkoxylated nonionic surfactants in which EO and PO units are not distributed blockwise, but randomly. Such products are obtainable as a result of the simultaneous action of ethylene oxide and propylene oxide on fatty alcohols. Furthermore, alkyl glycosides can also be used as further nonionic surfactants. A further class of preferably used nonionic surfactants, which are used either as the sole nonionic surfactant or in combination with other nonionic surfactants, are alkoxylated, preferably ethoxylated or ethoxylated and propoxylated fatty acid alkyl esters, preferably having 1 to 4 carbon atoms in the alkyl chain, in particular fatty acid methyl esters, as are described for example in the Japanese patent application JP 58/217598 or which are preferably prepared by the process described in the international patent application WO-A-90/13533.

Nonionic surfactants of the amine oxide type, for example N-cocoalkyl-N,N-dimethylamine oxide and N-tallowalkyl-N,N-dihydroxyethylamine oxide, and of the fatty acid alkanolamide type may also be suitable. The amount of these nonionic surfactants is preferably not more than that of the ethoxylated fatty alcohols, in particular not more than half thereof.

Further suitable surfactants are polyhydroxy fatty acid amides; the polyhydroxy fatty acid amides are substances which can usually be obtained by reductive amination of a reducing sugar with ammonia, an alkylamine or an alkanolamine and subsequent acylation with a fatty acid, a fatty acid alkyl ester or a fatty acid chloride.

Further suitable nonionic surfactants are polyglycerol partial esters based on mono- and dicarboxylic acids and crosslinked polyglycerol partial esters based on mono- and dicarboxylic acids.

The anionic surfactants used are, for example, those of the sulphonate and sulphate type. Suitable surfactants of the sulphonate type here are preferably C9-C13-alkylbenzenesulphonates, olefinsulphonates, i.e. mixtures of alkene- and hydroxyalkanesulphonates, and also disulphonates, as are obtained, for example, from C12-C18-monoolefins with a terminal or internal double bond by sulphonation with- 10 -gaseous sulphur trioxide and subsequent alkaline or acidic hydrolysis of the sulphonation products. Also of suitability are alkanesulphonates which are obtained from C12-C18-alkanes, for example by sulphochlorination or sulphoxidation with subsequent hydrolysis or neutralization. Similarly, the esters of a-sulpho fatty acids (ester sulphonates), for example the a-sulphonated methyl esters of hydrogenated coconut, palm kernel or tallow fatty acids, are also suitable.

Further suitable anionic surfactants are sulphated fatty acid glycerol esters. Fatty acid glycerol esters are to be understood as meaning the mono-, di- and triesters, and also mixtures thereof, as are obtained in the preparation by esterification of a monoglycerol with 1 to 3 mol of fatty acid or in the transesterification of triglycerides with 0.3 to 2 mol of glycerol. Preferred sulphated fatty acid glycerol esters here are the sulphation products of saturated fatty acids having 6 to 22 carbon atoms, for example of caproic acid, caprylic acid, capric acid, myristic acid, lauric acid, palmitic acid, stearic acid or behenic acid.

Preferred alk(en)yl sulphates are the alkali metal and in particular the sodium salts of the sulphuric acid half-esters of the C12-C18-fatty alcohols, for example from coconut fatty alcohol, tallow fatty alcohol, lauryl, myristyl, cetyl or stearyl alcohol or the C10-C20-oxo alcohols and those half-esters of secondary alcohols of these chain lengths. Furthermore, preference is given to alk(en)yl sulphates of the specified chain length which contain a synthetic straight-chain alkyl radical prepared on a petrochemical basis, and which have an analogous degradation behaviour to the suitable compounds based on fatty chemical raw materials.

The sulphuric acid monoesters of the straight-chain or branched C7-C20-alcohols ethoxylated with 1 to 6 mol of ethylene oxide, such as 2-methyl-branched C9-alcohols having on average 3.5 mol of ethylene oxide (EO) or C12-C18-fatty alcohols with 1 to 4 EO, are also suitable. On account of their high foaming behaviour, they are used in cleaning compositions only in relatively small amounts, for example in amounts of from 1 to 5% by weight.

Further suitable anionic surfactants are also the salts of alkylsulphosuccinic acid, which are also referred to as sulphosuccinates or as sulphosuccinic acid esters and constitute the monoesters and/or diesters of sulphosuccinic acid with alcohols, preferably fatty- 11 -alcohols and in particular ethoxylated fatty alcohols. Preferred sulphosuccinates contain C8-C18-fatty alcohol radicals or mixtures of these. Particularly preferred sulphosuccinates contain a fatty alcohol radical which is derived from ethoxylated fatty alcohols. In this connection, sulphosuccinates whose fatty alcohol radicals are derived from ethoxylated fatty alcohols with a narrow homolog distribution are particularly preferred in turn. It is likewise also possible to use alk(en)ylsuccinic acid having preferably 8 to 18 carbon atoms in the alk(en)yl chain or salts thereof.

Particularly preferred anionic surfactants are soaps. Also of suitability are saturated and unsaturated fatty acid soaps, such as the salts of lauric acid, myristic acid, palmitic acid, stearic acid, (hydrogenated) erucic acid and behenic acid, and also soap mixtures derived in particular from natural fatty acids, for example coconut, palm kernel, olive oil or tallow fatty acid. The anionic surfactants including the soaps can be in the form of their sodium, potassium or ammonium salts, as well as soluble salts of organic bases, such as mono-, di- or triethanolamine. Preferably, the anionic surfactants are in the form of their sodium or potassium salts, in particular in the form of the sodium salts.

Amphoteric surfactants which can be used according to the invention are those surface-active compounds which carry at least one quaternary ammonium group and at least one -CO₂- or -SO₃-group in the molecule. Particularly preferred amphoteric surfactants in this connection are betaine surfactants such as alkyl- or alkylamidopropylbetaines. In particular, betaines such as the N-alkyl-N,N-dimethylammonium glycinates, e.g. the cocoalkyldimethylammonium glycinate, N-acylaminopropyl-N, N-dimethylammonium glycinates, e.g. the cocoacylaminopropyldimethylammonium glycinate, the C12-C18-alkyldimethylacetobetaine, the cocoamidopropyldimethylacetobetaine, 2-alky1-3-carboxymethyl-3-hydroxyethylimidazolines and sulphobetaines having in each case 8 to 18 carbon atoms in the alkyl or acyl group, and also the cocoacylaminoethylhydroxyethylcarboxymethyl glycinate are preferred here. A particularly preferred zwitterionic surfactant is the N,N-dimethyl-N-(lauroylamidopropyl)ammoniumacetobetaine known under the INCl name Cocamidopropyl Betaine.

Further suitable amphoteric surfactants are formed by the group of amphoacetates and amphodiacetates, in particular, for example, coco- or laurylamphoacetates or -diacetates, the group of amphopropionates and amphodipropionates, and the group of amino acid-based surfactants such as acyl glutamates, in particular disodium cocoyl- 12 -glutamate and sodium cocoyl glutamate, acyl glycinates, in particular cocoyl glycinates, and acyl sarcosinates, in particular ammonium lauroyl sarcosinate and sodium cocoyl sarcosinate.

Said at least one additional surfactant present in process step c) of the instant invention is preferably selected from biosurfactants different from rhamnolipids, preferably glycolipids, more preferably selected from sophorolipids and glucolipids.

A preferred process according to the instant invention is characterized in that said process comprises
d) adjusting the pH of the formulation to a range of 4.0 to 8.0, preferably 4.5 to 7.4 particularly preferably 5.0 to 7.2.

The formulations prepared by the process according to the invention are in particular physically stable formulations. In the context of the present invention, the term "physically stable formulations containing at least one ceramide" is understood in particular to mean formulations which in particular do not exhibit any crystallization of the ceramides and the sphingoid base and no separation or inhomogeneity after six months of storage at 25°C.

The present invention further relates to the use of mono-rhamnolipid, preferably in an amount of from 0.1 wt.-% to 15.0 wt.-%, preferably from 0.5 wt.-% to 10.0 wt.-%, more preferably from 1.0 wt.-% to 8.0 wt.-%, wherein the percentages refer to the total composition, for solubilizing and/or physical stabilization of a ceramide-containing composition, in particular with regard to homogeneity, and/or for preventing crystallization of at least one ceramide in a composition.

In the context of the use according to the instant invent the ceramide-containing composition are preferably equal to the preferred compositions according to the instant invention, especially regarding components A) and B).

The examples adduced hereinafter describe the present invention by way of example, without any intention that the invention, the scope of application of which is apparent from the entirety of the description and the claims, be restricted to the embodiments specified in the examples.

### Examples:

### Example 1: Preparation of highly concentrated mono-rhamnolipids

Mono-rhamnolipids were produced by a fermentation of a *Pseudomonas putida* strain pBBR1MCS2-Plac-rhlAB, which was created just like the *Pseudomonas putida* strain pBBR1MCS2-Plac-rhlABC-T-Ptac-rhlC-T described in EP2786743, yet the Bsu36l restriction site was inserted directly behind the stop codon of the rhlB gene, thereby omitting rhIC. The preculture in a shake flask was carried out as described in EP2598646. For the main culture, a mineral medium (M9) was likewise employed. The fermentation was conducted in a 2-litre fermenter in a carbon-limited manner via a glucose feed input. The glucose feed input takes place by reference to the dissolved oxygen signal. The dissolved oxygen was regulated at 20% saturation via the stirrer speed. The pH is regulated to 7 via a pH electrode and addition of 2M sulphuric acid or a 20% by weight ammonia solution. To prevent excessive foaming of the fermentation broth, the defoamer Dow Corning 1500 was added as required. The fermentation was conducted over 4 days to a dry biomass of 16 g/L. The mono-rhamnolipid concentration was determined by HPLC and was 8.5 g/L.

After separating off the cells by means of centrifugation at 10,000 g, the fermentation broth was adjusted to a pH of 3.1 by adding concentrated H₂SO₄.

A multiphase composition was obtained, which was separated by centrifugation at 10,000 g and the upper aqueous phase was discarded.

The remnant was treated further.

The compositions listed in table1 were obtained by elevating the pH using KOH (aq) and diluting with water to the given mono-rhamnolipid concentration; percentage by weight referring to the total composition.

### Example 2: Preparation of clear aqueous solutions of ceramides

The dissolution capacity of the mono-rhamnolipid was investigated by mixing these with ceramides. Ceramides were added to a mono-rhamnolipid solution and heating to a temperature of 50°C and afterwards water is added in the ratios given in table 1 and the pH was adjusted to the given value. The solutions were cooled to room temperature and optical evaluation was performed directly and 24 h after preparation of the solutions.

The evaluation of the maximum amount ceramide solubilized is determined by the mixture resulting in a clear formulation. The sample was assessed visually and additionally by microscopic analysis using polarized light. When no illumination of the sample is observed the solution is free from crystals and according to the definition clear.

As shown in table 1 surprisingly the combinations of mono-rhamnolipids and ceramides gave clear solutions, whereas combination of ceramides and di-rhamnolipid or PEG-40 Hydrogenated Castor Oil resulted in turbid solutions.

**Table 1: Examples of combinations of mono-rhamnolipids, di-rhamnolipids, PEG-40 Hydrogenated Castor Oil and ceramides**

| | Example 2a | Example 2b | Example 2c | Example 2d | Example 2e |
|---|---|---|---|---|---|
| | according to the invention | according to the invention | according to the invention | not according to the invention | not according to the invention |
| Appearance | Clear solution | Clear solution | Clear solution | Turbid solution | Turbid solution |
| Appearance with polarized light | No crystals visible | No crystals visible | No crystals visible | Turbid Solution | Turbid Solution |
| Content mono-RL active in % | 3,7 | 3,7 | 3,9 | | |
| Content di-RL active in % | | | | 3.9 | |
| Content PEG-40 Hydrogenated Castor Oil in % | | | | | 3.9 |
| Content Ceramide NP(Ceramide 3B) in % | 0,15 | 0,20 | 0,19 | 0.19 | 0.19 |
| Content Water in % | ad 100 | ad 100 | ad 100 | ad 100 | ad 100 |
| pH | 7 | 7 | 7 | 7 | 7 |

### Example 3

The dissolution capacity of the mono-rhamnolipid was investigated by mixing these with ceramides. Ceramides were added to a mono-rhamnolipid solution and heating to a temperature of 70°C and afterwards water is added in the ratios given in table 1 and the pH was adjusted to the given value. The solutions were cooled to room temperature and optical evaluation was performed directly and 24 h after preparation of the solutions.

The evaluation of the maximum amount ceramide solubilized is determined by the mixture resulting in a clear formulation. The sample was assessed visually and additionally by microscopic analysis using polarized light. When no illumination of the sample is observed the solution is free from crystals and according to the definition clear.

As shown in table 2 surprisingly the combinations of mono-rhamnolipids and ceramides gave clear solutions, whereas combination of ceramides and di-rhamnolipid or PEG-40 Hydrogenated Castor Oil resulted in turbid solutions.

**Table 2: Examples of combinations of mono-rhamnolipids, di-rhamnolipids, PEG-40 Hydrogenated Castor Oil and ceramides**

| | Example 3a | Example 3b | Example 3c | Example 3d | Example 3e |
|---|---|---|---|---|---|
| | according to the invention | according to the invention | according to the invention | not according to the invention | not according to the invention |
| Appearance | Clear solution | Clear solution | Clear solution | Turbid solution | Turbid solution |
| Appearance with polarized light | No crystals visible | No crystals visible | No crystals visible | Turbid Solution | Turbid Solution |
| Content mono-RL active in % | 3,7 | 3,7 | 3,9 | | |
| Content di-RL active in % | | | | 3.9 | |
| Content PEG-40 Hydrogenated Castor Oil in % | | | | | 3.9 |
| Content Ceramide NG in % | 0,15 | 0,20 | 0,19 | 0.19 | 0.19 |
| Content Water in % | ad 100 | ad 100 | ad 100 | ad 100 | ad 100 |
| pH | 7 | 7 | 7 | 7 | 7 |

### Example formulations

In the below "Composition A" refers to the following two compositions A1 and A2 given figures in wt.-%:

| | Composition A1 | Composition A2 |
|---|---|---|
| L-(+)-lactic acid | 3 | 3 |
| Ceramide NP | 3 | - |
| Ceramide NG | - | 3 |
| mono-Rhamnolipid from example 1 | 75 | 75 |
| Water | ad 100 | ad 100 |
| | pH 3 | pH 3 |

Thus, each formulations listed below is disclosed in two versions.

### Example formulation 1: Volume and Body Shampoo

| *Ingredient* | *%* |
|---|---|
| Aqua | ad 100 |
| Sodium Lauryl Sulfate | 7.0 |
| Sodium Laureth Sulfate | 5.0 |
| Cocamidopropyl Betaine | 2.0 |
| Composition A | 2.0 |
| Sodium Citrate | 1.5 |
| Sodium Xylenesulfonate | 1.5 |
| Sodium Chloride | 1.4 |
| Citric Acid | ad pH 5.5 |
| Sodium Levulinate, Sodium Benzoate | 1.0 |
| Hydroxypropyl Methylcellulose | 1.0 |
| Tetrasodium EDTA | 0.8 |
| Butylphenyl Methylpropional | 0.5 |
| Panthenol | 0.5 |
| Panthenyl Ethyl Ether | 0.3 |
| Starch Hydroxypropltrimonium chloride | 0.2 |
| Linalool | 0.1 |
| Hexyl Cinnamal | 0.1 |
| Limonene | 0.1 |
| Benzyl Salicylate | 0.2 |
| Magnesium Nitrate | 0.1 |
| Magnesium Chloride | 0.1 |
| Methylchloroisothiazolinone | 0.1 |
| Dyes | q. s. |

### Example formulation 2: Repair Shampoo

| *Ingredient* | *%* |
|---|---|
| Aqua | ad 100 |
| Sodium Laureth Sulfate | 7.0 |
| Glycolipids | 3.0 |
| Composition A | 3.0 |
| Glycol Distearate | 2.5 |
| Cocamidopropyl Betaine | 2.0 |
| Sodium Citrate | 1.8 |
| Cocamide MEA | 1.8 |
| Sodium Xylenesulfonate | 1.5 |
| Dimethicone | 1.5 |
| Citric Acid | ad pH 5.0 |
| Sodium Benzoate | 0.7 |
| Polyquaternium-76 | 0.5 |
| Sodium Chloride | 0.9 |
| Glycerin | 0.5 |
| Tetrasodium EDTA | 0.3 |
| Butylphenyl Methylpropional | 0.2 |
| Hexyl Cinnamal | 0.1 |
| Linalool | 0.1 |
| alpha-lsomethyl lonone | 0.1 |
| Geraniol | 0.2 |
| Hydrochloric Acid | 0.1 |
| Magnesium Nitrate | 0.1 |
| Methylchloroisothiazolinone | 0.1 |
| Magnesium Chloride | 0.1 |
| Methylisothiazolinone | 0.1 |
| Dyes | q. s. |

### Example formulation 3: Anti-Dandruff Shampoo

| *Ingredient* | *%* |
|---|---|
| Aqua | ad 100 |
| Sodium Laureth Sulfate | 6.0 |
| Sodium Lauryl Sulfate | 5.0 |
| Composition A | 4.0 |
| Cocamide MEA | 4.0 |
| Zinc Carbonate | 3.0 |
| Glycol Distearate | 2.5 |
| Dimethicone | 1.0 |
| Zinc Pyrithione | 1.0 |
| Sodium Chloride | 0.6 |
| Cetyl Alcohol | 0.8 |
| Guar Hydroxypropyltrimonium Chloride | 0.3 |
| Sodium Xylenesulfonate | 0.5 |
| Magnesium Sulfate | 0.7 |
| Sodium Benzoate | 0.7 |
| Ammonium Laureth Sulfate | 0.3 |
| Citric Acid | ad pH 4.5 |
| Benzyl Alcohol | 0.2 |
| Methylchloroisothiazolinone | 0.1 |
| Methylisothiazolinone | 0.1 |
| Parfum, Dyes | q. s. |

### Example formulation 4: Strengthening Shampoo

| *Ingredient* | *%* |
|---|---|
| Water | ad 100 |
| Sodium Lauryl Sulfate | 6.0 |
| Sodium Laureth Sulfate | 4.0 |
| Cocamidopropyl Betaine | 3.0 |
| Composition A | 3.0 |
| Glycol Distearate | 2.5 |
| Dimethicone | 2.0 |
| Sodium Citrate | 1.5 |
| Cocamide MEA | 1.0 |
| Sodium Xylenesulfonate | 1.0 |
| Citric Acid | ad pH 5.0 |
| Sodium Benzoate | 0.7 |
| Sodium Chloride | 0.8 |
| Tetrasodium EDTA | 0.3 |
| Polyquaternium-6 | 0.5 |
| Honey (Mel) | 0.5 |
| Prunus Armeniaca (Apricot) Fruit Extract | 0.5 |
| Methylchloroisothiazolinone | 0.1 |
| Methylisothiazolinone | 0.1 |
| Parfum, Dyes | q. s. |

### Example formulation 5: Shampoo

| *Ingredient* | % |
|---|---|
| Aqua | ad 100 |
| Sodium Laureth Sulfate | 9.0 |
| Cocamidopropyl Betaine | 3.0 |
| Composition A | 3.0 |
| Glycerin | 2.5 |
| Sodium Chloride | 1.7 |
| Glycol Distearate | 1.5 |
| Panthenol | 0.2 |
| Propylene Glycol | 0.3 |
| Hydroxypropyl Guar Hydroxypropyltrimonium Chloride | 0.3 |
| Sodium Benzoate | 0.7 |
| PEG-55 Propylene Glycol Oleate | 0.5 |
| Citric Acid | ad pH 5.5 |
| Hydrolyzed Wheat Protein | 0.3 |
| Argania Spinosa Kernel Oil | 0.1 |
| Laureth-4 | 0.5 |
| Potassium Sorbate | 0.2 |
| Parfum, Dyes | q. s. |

### Example formulation 6: Moisturising Shampoo

| *Ingredient* | % |
|---|---|
| Aqua | ad 100 |
| Sodium Laureth Sulfate | 8.0 |
| Cocamidopropyl Betaine | 3.0 |
| Sodium Chloride | 2.2 |
| Composition A | 2.0 |
| Hydrolyzed Keratin | 2.0 |
| Water Lily (Nymphaea Odorata) Root Extract | 1.8 |
| Aloe Barbadensis Leaf Juice | 1.5 |
| Glyceryl Glucoside | 1.0 |
| Panthenol | 1.0 |
| Decyl Glucoside | 1.0 |
| Polyquaternium-10 | 0.2 |
| Guar Hydroxypropyltrimonium Chloride | 0.3 |
| PEG-40 Hydrogenated Castor Oil | 0.7 |
| Glycerin | 0.5 |
| Citric Acid | ad pH 5.0 |
| Sodium Benzoate | 1.2 |
| Propylene Glycol | 1.0 |
| Citronellol | 0.1 |
| Limonene | 0.1 |
| alpha-lsomethyl lonone | 0.1 |
| Benzyl Alcohol | 0.2 |
| Dyes | q. s. |

### Example formulation 7: Shampoo

| *Ingredient* | % |
|---|---|
| Aqua | ad 100 |
| Decyl Glucoside | 6.0 |
| Sodium Myreth Sulfate | 5.5 |
| Composition A | 3.0 |
| PEG-200 Hydrogenated Glyceryl Palmate | 2.5 |
| PEG-80 Sorbitan Laurate | 2.0 |
| PEG-90 Glycol Isostearate | 2.0 |
| Disodium PEG-5 Laurylcitrate Sulfosuccinate | 1.5 |
| Polyquaternium-10 | 1.2 |
| Hydrolyzed Silk | 1.2 |
| Laureth-2 | 1.2 |
| Citric Acid | ad pH 5.5 |
| Sodium Laureth Sulfate | 1.0 |
| PEG-40 Hydrogenated Castor Oil | 0.9 |
| Sodium Benzoate | 0.5 |
| Parfum, Dyes | q. s. |

### Example formulation 8: Cleansing Oil Shampoo

| *Ingredient* | % |
|---|---|
| Aqua | ad 100 |
| Sodium Laureth Sulfate | 7.0 |
| MIPA-Laureth Sulfate | 4.0 |
| Sodium Chloride | 3.2 |
| Cocamidopropyl Betaine | 3.0 |
| Composition A | 3.0 |
| Glycerin | 2.5 |
| PEG-18 Castor Oil Dioleate | 2.0 |
| Propylene Glycol | 2.0 |
| PEG-55 Propylene Glycol Oleate | 1.7 |
| Behenamidopropyl Dimethylamine | 1.2 |
| Laureth-5 Carboxylic Acid | 1.0 |
| Persea Gratissima (Avocado) Oil | 1.0 |
| PPG-5-Ceteth-20 | 1.0 |
| Sodium Benzoate | 0.7 |
| Laureth-2 | 0.5 |
| Salicylic Acid | 0.3 |
| Linalool | 0.2 |
| alpha-lsomethyl lonone | 0.1 |
| Limonene | 0.1 |
| Zea Mays (Corn) Germ Oil | 0.2 |
| Argania Spinosa Oil Kernel Oil | 0.1 |
| Camellia Oleifera Seed Oil | 0.1 |
| Sodium Hydroxide | 0.3 |
| Citric Acid | ad pH 5.0 |
| Parfum, Dyes | q. s. |

### Example formulation 9: Shine Shampoo

| *Ingredient* | % |
|---|---|
| Aqua | ad 100 |
| Ammonium Lauryl Sulfate | 5.5 |
| Composition A | 2.0 |
| Cocamidopropyl Betaine | 2.0 |
| Glycolipids | 2.0 |
| Sodium Chloride | 2.0 |
| Cocamide MEA | 2.0 |
| Nacre Powder | 1.0 |
| Sodium Benzoate | 0.7 |
| Hydroxypropyl Guar Hydroxypropyltrimonium Chloride | 0.7 |
| Hydroxypropyltrimonium Hydrolyzed Wheat Protein | 0.7 |
| Sodium Hydroxide | 0.2 |
| Salicylic Acid | 0.5 |
| Limonene | 0.1 |
| Benzoic Acid | 0.5 |
| Linalool | 0.3 |
| Benzyl Alcohol | 0.5 |
| Tin Oxide | 0.2 |
| alpha-lsomethyl lonone | 0.1 |
| Acrylates Copolymer | 0.1 |
| Citric Acid | ad pH 5.5 |
| Citronellol | 0.1 |
| Hexyl Cinnamal | 0.1 |
| Dyes | q. s. |

### Example formulation 10: Shampoo

| *Ingredient* | % |
|---|---|
| Aqua | ad 100 |
| Sodium Cocoyl Isethionate | 3.5 |
| Sodium Lauryl Sulfoacetate | 3.5 |
| Disodium Laureth Sulfosuccinate | 3.5 |
| Cocamidopropyl Betaine | 3.0 |
| Composition A | 3.0 |
| Behenamidopropyl Dimethylamine | 0.9 |
| Sodium Lauroyl Sarcosinate | 1.0 |
| Glycol Distearate | 1.0 |
| C11-15 Pareth-7 | 1.0 |
| C12-13 Pareth-23 | 1.0 |
| C12-13 Pareth-3 | 1.0 |
| Decyl Glucoside | 1.0 |
| Tocopherol | 1.0 |
| Hydrogenated Coconut Acid | 1.0 |
| Sodium Chloride | 0.8 |
| Sodium Isethionate | 0.7 |
| Sodium Benzoate | 0.7 |
| Sodium Hydroxide | 0.3 |
| Phenoxyethanol | 0.6 |
| PPG-5 Ceteth-20 | 0.5 |
| PEG-55 Propylene Glycol Oleate | 0.5 |
| Trideceth-12 | 0.5 |
| Polyquaternium-7 | 0.5 |
| Polyquaternium-10 | 0.3 |
| Limonene | 0.1 |
| Benzoic Acid | 0.3 |
| Benzophenone-4 | 0.3 |
| Benzyl Salicylate | 0.2 |
| Linalool | 0.6 |
| Benzyl Alcohol | 0.6 |
| Amodimethicone | 0.5 |
| Propylene Glycol | 0.5 |
| Carbomer | 0.7 |
| Rosmarinus Officinalis (Rosemary) Leaf Oil | 0.7 |
| Methylparaben | 0.3 |
| Methylchloroisothiazolinone | 0.1 |
| Methylisothiazolinone | 0.1 |
| Citric Acid | ad pH 5.0 |
| Laureth-9 | 0.5 |
| Divinyldimethicone/Dimethicone Copolymer | 0.2 |
| Glycerin | 0.2 |
| Parfum, Dyes | q. s. |

### Example formulation 11: Reinforcing Shampoo

| *Ingredient* | % |
|---|---|
| Aqua | ad 100 |
| Sodium Laureth Sulfate | 6.0 |
| Dimethicone | 3.0 |
| Sodium Chloride | 3.0 |
| Coco-Betaine | 3.0 |
| Composition A | 2.0 |
| Glycol Distearate | 1.0 |
| Guar Hydroxypropyltrimonium Chloride | 0.3 |
| Cocamide MIPA | 0.5 |
| Hydrolyzed keratin | 0.7 |
| Sodium Benzoate | 0.7 |
| Hydroxypropyltrimonium Hydrolyzed Wheat Protein | 0.3 |
| Sodium Cocoate | 0.7 |
| Sodium Hydroxide | 0.6 |
| Arginine | 0.7 |
| Salicylic Acid | 0.5 |
| Limonene | 0.2 |
| Linalool | 0.2 |
| 2-Oleamido-1.3-Octadecanediol | 0.1 |
| Carbomer | 0.5 |
| Methyl Cocoate | 0.5 |
| Citric Acid | ad pH 5.0 |
| Hexylene Glycol | 0.2 |
| Hexyl Cinnamal | 0.1 |
| Glyceryl Linoleate | 0.1 |
| Glyceryl Oleate | 0.2 |
| Glyceryl Linolenate | 0.2 |
| Dyes | q. s. |

### Example formulation 12: Shampoo

| *Ingredient* | % |
|---|---|
| Aqua | ad 100 |
| Sodium Laureth Sulfate | 9.0 |
| Dimethicone | 3.5 |
| Sodium Chloride | 3.2 |
| *Coco*-Betaine | 3.0 |
| Glycol Distearate | 3.0 |
| Composition A | 2.0 |
| Guar Hydroxypropyltrimonium Chloride | 0.3 |
| Niacinamide | 0.1 |
| Cocamide MIPA | 2.0 |
| Saccharum Officinarum (Sugar Cane) Extract | 2.0 |
| Sodium Benzoate | 1.0 |
| Sodium Cocoate | 1.5 |
| Sodium Hydroxide | 0.6 |
| Salicylic Acid | 0.5 |
| Camellia Sinensis Extract/Camellia Sinensis Leaf Extract | 0.5 |
| Linalool | 0.2 |
| Benzyl Salicylate | 0.2 |
| Pyrus Malus (Apple) Fruit Extract | 0.3 |
| Carbomer | 0.5 |
| Pyridoxine HCl | 0.3 |
| Persea Gratissima (Avocado) Oil | 0.2 |
| Citric Acid | ad pH 5.5 |
| Butylphenyl Methylpropional | 0.1 |
| Methyl Cocoate | 0.3 |
| Hexylene Glycol | 0.1 |
| Hexyl Cinnamal | 0.1 |
| Dyes | q. s. |

### Example formulation 13: Fortifying Shampoo

| *Ingredient* | % |
|---|---|
| Aqua | ad 100 |
| Ammonium Lauryl Sulfate | 6.0 |
| Cocamidopropyl Betaine | 3.0 |
| Sodium Chloride | 2.7 |
| Composition A | 2.0 |
| Niacinamide | 0.1 |
| Saccharum Officinarum (Sugar Cane) Extract | 0.9 |
| Cocos Nucifera (Coconut) Oil | 0.9 |
| Sodium Benzoate | 0.9 |
| Hydroxypropyl Guar Hydroxypropyltrimonium Chloride | 0.1 |
| Sodium Hydroxide | ad pH |
| Salicylic Acid | 0.4 |
| Limonene | 0.1 |
| Benzoic Acid | 0.4 |
| Linalool | 0.3 |
| Cinnamyl Alcohol | 0.2 |
| Pyrus Malus (Apple) Fruit Extract | 0.5 |
| Pyridoxine HCl | 0.2 |
| Bisabolol | 0.2 |
| Citric Acid | ad pH 5.0 |
| Citronellol | 0.1 |
| Hexylene Glycol | 0.2 |
| Dyes | q. s. |

### Example formulation 14: Shampoo

| *Ingredient* | % |
|---|---|
| Aqua | ad 100 |
| Sodium Laureth Sulfate | 9.0 |
| Cocamidopropyl Betaine | 2.5 |
| Composition A | 2.5 |
| Sodium Chloride | 2.5 |
| Camellia Sinensis Leaf Extract | 2.0 |
| Guar Hydroxypropyltrimonium Chloride | 0.3 |
| Maltodextrin | 1.2 |
| Disodium EDTA | 1.0 |
| PPG-12 | 0.7 |
| Citric Acid | ad pH 5.0 |
| Sodium Hydroxide | 0.3 |
| Sodium Benzoate | 0.7 |
| Butylphenyl Methylpropional | 0.2 |
| Geraniol | 0.2 |
| Hexyl Cinnamal | 0.1 |
| Limonene | 0.1 |
| Linalool | 0.1 |
| Dyes | q. s. |

### Example formulation 15: Brunette Shampoo

| *Ingredient* | % |
|---|---|
| Aqua | ad 100 |
| Sodium Laureth Sulfate | 8.5 |
| Cocamidopropyl Betaine | 4.0 |
| Sodium Chloride | 3.5 |
| Composition A | 2.0 |
| Selaginella Lepidophylla Aerial Extract | 2.0 |
| Gluconolactone | 1.8 |
| Glycerin | 1.8 |
| Dimethiconol | 1.0 |
| Guar Hydroxypropyltrimonium Chloride | 0.5 |
| TEA-Dodecylbenzenesulfonate | 1.0 |
| Maltodextrin | 1.0 |
| Disodium EDTA | 0.7 |
| Carbomer | 0.9 |
| PPG-12 | 0.7 |
| Citric Acid | ad pH 5.5 |
| Sodium Hydroxide | 0.9 |
| TEA-Sulfate | 0.7 |
| Triethanolamine | 0.5 |
| DMDM Hydantoin | 0.1 |
| Sodium Benzoate | 0.7 |
| Methylchloroisothiazolinone | 0.1 |
| Methylisothiazolinone | 0.1 |
| alpha-lsomethyl lonone | 0.1 |
| Butylphenyl Methylpropional | 0.2 |
| Hexyl Cinnamal | 0.1 |
| Limonene | 0.1 |
| Linalool | 0.1 |
| Dyes | q. s. |

### Example formulation 16: 2 in 1 Shampoo

| *Ingredient* | *%* |
|---|---|
| Aqua | ad 100 |
| Sodium Laureth Sulfate | 8.0 |
| PEG-200 Hydrogenated Glyceryl Palmate | 3.0 |
| Composition A | 3.0 |
| Disodium Cocoamphodiacetate | 3.0 |
| Polysorbate 20 | 2.5 |
| Glycerin | 2.5 |
| Sodium Chloride | 2.0 |
| PEG-7 Glyceryl Cocoate | 2.0 |
| Sodium Benzoate | 1.0 |
| Sodium Laureth-8 Sulfate | 1.0 |
| Sodium Oleth Sulfate | 1.0 |
| Sodium Hydroxide | 0.3 |
| PPG-5 Ceteth-20 | 1.0 |
| PEG-55 Propylene Glycol Oleate | 0.5 |
| Polyquaternium-10 | 0.2 |
| Salicylic Acid | 0.3 |
| Maltodextrin | 0.2 |
| Propylene Glycol | 0.2 |
| Disodium Ricinoleamido MEA-Sulfosuccinate | 0.1 |
| Citric Acid | ad pH 5.5 |
| Prunus Armeniaca (Apricot) Kernel Oil | 0.1 |
| Parfum, Dyes | q. s. |

### Example formulation 17: Shampoo

| *Ingredient* | *%* |
|---|---|
| Aqua | ad 100 |
| Sodium Laureth Sulfate | 9.0 |
| Cocamidopropyl Betaine | 3.0 |
| Sodium Chloride | 2.5 |
| Glycol Distearate | 2.5 |
| Hydrolyzed Keratin | 2.0 |
| Simmondsia Chinensis (Jojoba) Seed Oil | 2.0 |
| Composition A | 2.0 |
| Argania Spinosa Kernel Oil | 0.5 |
| Persea Gratissima (Avocado) Oil | 0.5 |
| Rosmarinus Officinalis (Rosemary) Leaf Extract | 0.3 |
| Helianthus Annuus (Sunflower) Seed Oil | 0.3 |
| Cocamide MEA | 0.4 |
| Guar Hydroxypropyltrimonium Chloride | 0.2 |
| Polyquaternium-47 | 0.2 |
| PEG-20 Castor Oil | 0.5 |
| Phenethyl Benzoate | 0.7 |
| Disodium EDTA | 0.2 |
| Methylchloroisothiazolinone | 0.3 |
| Methylisothiazolinone | 0.3 |
| Benzyl Salicylate | 0.1 |
| Hexyl Cinnamal | 0.1 |
| Citronellol | 0.1 |
| Dyes | q. s. |

### Example formulation 18: Foam Shampoo

| *Ingredient* | *%* |
|---|---|
| Aqua | ad 100 |
| Sodium Laureth Sulfate | 5.0 |
| Isobutane | 5.0 |
| Decyl Glucoside | 5.0 |
| Propane | 3.5 |
| Sodium Cocoamphoacetate | 3.5 |
| Composition A | 3.5 |
| Glycolipids | 1.0 |
| Panthenol | 0.2 |
| Polyquaternium-10 | 0.2 |
| Hydroxypropyl Guar Hydroxypropyltrimonium Chloride | 0.1 |
| Sodium PCA | 0.5 |
| Octyldodecyl PCA | 0.5 |
| Maltodextrin | 0.5 |
| Tilia Cordata Flower Extract | 0.7 |
| Sodium Chloride | 0.9 |
| Citric Acid | ad pH 5.0 |
| Glycerin | 0.9 |
| Propylene Glycol | 0.3 |
| Butane | 0.7 |
| Sodium Acetate | 0.7 |
| Sorbitol | 0.5 |
| Isopropyl Alcohol | 0.5 |
| Dyes | q. s. |

### Example formulation 19: Anti-Dandruff Shampoo

| *Ingredient* | *%* |
|---|---|
| Aqua | ad 100 |
| Sodium Laureth Sulfate | 10.0 |
| Cocamidopropyl Betaine | 3.0 |
| Acrylates Copolymer | 2.5 |
| Sodium Lauroyl Glutamate | 2.5 |
| Zinc Pyrithione | 2.0 |
| Composition A | 2.0 |
| Stearamidopropyl Dimethylamine | 1.0 |
| Piroctone Olamine | 0.7 |
| Hydroxypropyl Guar Hydroxypropyltrimonium Chloride | 0.2 |
| Propylene Glycol | 0.5 |
| Sodium PCA | 0.2 |
| Citrus Medica Limonum (Lemon) Peel Extract | 0.5 |
| Citric Acid | ad pH 5.0 |
| Glycol Distearate | 0.8 |
| PPG-9 | 0.2 |
| Laureth-4 | 0.2 |
| Sodium Chloride | 0.9 |
| Sodium Hydroxide | 0.3 |
| Sodium Naphthalenesulfonate | 0.7 |
| Sorbitol | 0.7 |
| DMDM Hydantoin | 0.1 |
| Sodium Benzoate | 0.8 |
| Limonene | 0.1 |
| Linalool | 0.1 |
| Butylphenyl Methylpropional | 0.1 |
| alpha-Isomethyl lonone | 0.1 |
| Parfum, Dyes | q. s. |

### Example formulation 20: Shampoo

| *Ingredient* | *%* |
|---|---|
| Aqua | ad 100 |
| Sodium Laureth Sulfate | 5.0 |
| Disodium Cocoamphodiacetate | 2.0 |
| Composition A | 2.0 |
| Laureth-2 | 0.3 |
| Panthenol | 0.2 |
| Niacinamide | 0.1 |
| Sodium Chloride | 0.7 |
| Citric Acid | ad pH 5.0 |
| PEG-7 Glyceryl Cocoate | 0.5 |
| Sodium Benzoate | 0.5 |
| Polyquaternium-10 | 0.7 |
| Salicylic Acid | 0.5 |
| Hexyl Salicylate | 0.2 |
| Benzyl Salicylate | 0.1 |
| Hexyl Cinnamal | 0.1 |
| Linalool | 0.1 |
| Limonene | 0.1 |
| Propylene Glycol | 0.2 |
| Dyes | q. s. |

### Example formulation 21: Intensive Care Oil-Shampoo

| *Ingredient* | *%* |
|---|---|
| Aqua | ad 100 |
| Sodium Laureth Sulfate | 8.0 |
| Disodium Cocoamphodiacetate | 3.0 |
| Composition A | 0.5 |
| Prunus Armeniaca (Apricot) Kernel Oil | 2.5 |
| Hydrolyzed Keratin | 2.5 |
| Panthenol | 2.4 |
| Sodium Chloride | 2.2 |
| Citric Acid | ad pH 5.5 |
| Stearamidopropyl Dimethylamine | 1.2 |
| Polyquaternium-10 | 0.5 |
| Sodium Benzoate | 0.5 |
| PEG-7 Glyceryl Cocoate | 0.7 |
| Laureth-2 | 0.4 |
| PEG-40 Hydrogenated Castor Oil | 0.4 |
| Propylene Glycol | 0.8 |
| PEG-55 Propylene Glycol Oleate | 0.7 |
| Hexyl Cinnamal | 0.1 |
| Butylphenyl Methylpropional | 0.2 |
| Benzyl Salicylate | 0.1 |
| Linalool | 0.2 |
| Benzyl Alcohol | 0.5 |
| Limonene | 0.1 |
| Sodium Acetate | 0.2 |
| Dyes | q. s. |

### Example formulation 22: Shampoo

| *Ingredient* | % |
|---|---|
| Aqua | ad 100 |
| Sodium Laureth Sulfate | 8.5 |
| Disodium Cocoamphodiacetate | 3.5 |
| Sodium Chloride | 3.5 |
| Composition A | 3.0 |
| Hydrolyzed Collagen | 1.0 |
| Panthenol | 0.4 |
| Cocodimonium Hydroxypropyl Hydrolyzed Keratin | 0.3 |
| Hydrolyzed Keratin | 0.3 |
| Citric Acid | ad pH 5.5 |
| Sodium Benzoate | 0.7 |
| Polyquaternium-10 | 0.2 |
| Hexyl Cinnamal | 0.1 |
| Limonene | 0.1 |
| Linalool | 0.1 |
| Benzyl Salicylate | 0.1 |
| Benzyl Alcohol | 0.2 |
| Propylene Glycol | 0.2 |
| Dyes | q. s. |

### Example formulation 23: Shampoo

| *Ingredient* | % |
|---|---|
| Aqua | ad 100 |
| Sodium Laureth Sulfate | 8.0 |
| Cocamidopropyl Betaine | 3.0 |
| Composition A | 3.0 |
| PEG-7 Glyceryl Cocoate | 2.5 |
| Dimethicone | 2.5 |
| Prunus Armeniaca (Apricot) Kernel Oil | 2.5 |
| Panthenol | 0.3 |
| Cocodimonium Hydroxypropyl Hydrolyzed Keratin | 0.7 |
| Hydrolyzed Keratin | 0.7 |
| Sodium Chloride | 0.8 |
| Cocamide MEA | 0.5 |
| Glycol Distearate | 0.2 |
| Sodium Benzoate | 0.7 |
| Citric Acid | ad pH 5.5 |
| Polyquaternium-10 | 0.5 |
| Laureth-4 | 0.4 |
| PEG-40 Hydrogenated Castor Oil | 0.2 |
| Hydrogenated Castor Oil | 0.7 |
| Laureth-7 | 0.5 |
| Hexyl Cinnamal | 0.1 |
| Propylene Glycol | 0.8 |
| Hexyl Salicylate | 0.3 |
| Glycerin | 0.5 |
| Linalool | 0.2 |
| Limonene | 0.1 |
| Dyes | q. s. |

### Example formulation 24: Shampoo

| *Ingredient* | % |
|---|---|
| Aqua | ad 100 |
| Sodium Coco Sulfate | 6.0 |
| Lauryl Glucoside | 4.0 |
| Glycerin | 3.5 |
| Sodium Lactate | 2.5 |
| Composition A | 2.0 |
| Stearamidopropyl Dimethylamine | 1.0 |
| Betaine | 0.8 |
| Sodium Chloride | 0.9 |
| Simmondsia Chinensis Seed Oil | 0.8 |
| Sodium Cocoyl Glutamate | 0.7 |
| Disodium Cocoyl Glutamate | 0.5 |
| Xanthan Gum | 0.5 |
| Hydrated Silica | 0.5 |
| Parfum, Dyes | q. s. |

### Example formulation 25: Shampoo for Children

| *Ingredient* | % |
|---|---|
| Aqua | ad 100 |
| Sodium Coco Sulfate | 7.0 |
| Decyl Glucoside | 5.0 |
| Lactis Proteinum | 3.5 |
| Sorbitan Caprylate | 3.0 |
| Composition A | 3.0 |
| Glycerin | 2.5 |
| Sodium Lactate | 2.5 |
| Alcohol | 2.0 |
| Hydrolyzed Wheat Protein | 0.7 |
| Hydrolyzed Wheat Starch | 0.7 |
| Sodium Chloride | 0.9 |
| Limonene | 0.1 |
| Citral | 0.1 |
| Phenethyl Alcohol | 0.1 |
| Dyes | q. s. |

### Example formulation 26: Intensive Cream Shampoo

| *Ingredient* | % |
|---|---|
| Aqua | ad 100 |
| Sodium Laureth Sulfate | 8.5 |
| Cocamidopropyl Betaine | 3.0 |
| Composition A | 2.5 |
| Sodium Chloride | 2.5 |
| Panthenol | 0.5 |
| Hydrolyzed Keratin | 0.8 |
| Argania Spinosa Kernel Oil | 0.5 |
| Cocamide MEA | 0.5 |
| Glycol Distearate | 0.5 |
| PEG-7 Glyceryl Cocoate | 0.4 |
| Sodium Benzoate | 0.7 |
| Citric Acid | ad pH 5.0 |
| Laureth-4 | 0.3 |
| PEG-40 Hydrogenated Castor Oil | 0.4 |
| Hydrogenated Castor Oil | 0.4 |
| Styrene/Acrylates Copolymer | 0.5 |
| Dimethicone | 0.8 |
| Polyquaternium-7 | 0.9 |
| PEG-12 Dimethicone | 0.5 |
| Guar Hydroxypropyltrimonium Chloride | 0.3 |
| Benzyl Salicylate | 0.1 |
| Propylene Glycol | 0.8 |
| Glycerin | 0.5 |
| Limonene | 0.1 |
| Linalool | 0.1 |
| PEG-14m | 0.1 |
| Dyes | q. s. |

### Example formulation 27: Shampoo

| *Ingredient* | % |
|---|---|
| Aqua | ad 100 |
| Sodium Laureth Sulfate | 7.5 |
| Cocamidopropyl Betaine | 3.0 |
| Composition A | 2.5 |
| Sodium Chloride | 2.0 |
| Sodium Xylenesulfonate | 1.7 |
| Cocamide MEA | 1.5 |
| Sodium Citrate | 1.5 |
| Citric Acid | ad pH 5.0 |
| Dimethiconol | 1.0 |
| Cassia Hydroxypropyltrimonium Chloride | 0.3 |
| Sodium Benzoate | 0.9 |
| Disodium EDTA | 0.7 |
| Panthenol | 0.7 |
| Panthenyl Ethyl Ether | 0.3 |
| Persea Gratissima (Avocado) Oil | 0.2 |
| Methylchloroisothiazolinone | 0.1 |
| Methylisothiazolinone | 0.1 |
| Parfum, Dyes | q. s. |

### Example formulation 28: Caffeine Shampoo

| *Ingredient* | % |
|---|---|
| Aqua | ad 100 |
| Sodium Laureth Sulfate | 6.0 |
| Laureth-2 | 4.0 |
| Disodium Laureth Sulfosuccinate | 2.5 |
| Sodium Lauroyl Glutamate | 2.5 |
| Composition A | 2.0 |
| Sodium Chloride | 2.0 |
| Caffeine | 1.5 |
| Panthenol | 0.2 |
| PEG-120 Methyl Glucose Dioleate | 0.5 |
| Hydrolysed Wheat Protein | 0.7 |
| Citric Acid | ad pH 5.5 |
| Sodium Citrate | 0.5 |
| Menthol | 0.7 |
| PEG-40 Hydrogenated Castor Oil | 0.4 |
| Potassium Sorbate | 0.3 |
| Polyquaternium-7 | 0.4 |
| Disodium EDTA | 0.5 |
| Sodium Benzoate | 0.7 |
| Zinc PCA | 0.3 |
| Niacinamide | 0.1 |
| Limonene | 0.1 |
| Tocopherol | 0.1 |
| Phenoxyethanol | 0.5 |
| Methylparaben | 0.3 |
| Propylparaben | 0.3 |
| Dyes | q. s. |

### Example formulation 29: Power Grey Shampoo

| *Ingredient* | % |
|---|---|
| Aqua | ad 100 |
| Sodium Laureth Sulfate | 6.5 |
| Sodium Cocoamphoacetate | 4.5 |
| PEG-3 Distearate | 3.0 |
| Cocamidopropyl Betaine | 3.0 |
| Composition A | 2.5 |
| Sodium Chloride | 2.5 |
| Caffeine | 1.5 |
| Panthenol | 0.2 |
| Phenoxyethanol | 0.7 |
| Coco Glucoside | 0.9 |
| Glyceryl Oleate | 0.4 |
| Phosphoric Acid | 0.2 |
| PEG-120 Methyl Glucose Dioleate | 0.9 |
| Menthol | 0.9 |
| Polyquaternium-7 | 0.7 |
| Sodium Hydroxide | 0.3 |
| Citric Acid | ad pH 5.5 |
| Niacinamide | 0.1 |
| Zinc PCA | 0.5 |
| Limonene | 0.1 |
| Sodium Benzoate | 0.2 |
| Methylparaben | 0.3 |
| Propylparaben | 0.3 |
| Dyes | q. s. |

### Example formulation 30: Shampoo

| *Ingredient* | % |
|---|---|
| Aqua | ad 100 |
| Lauryl Glucoside | 6.5 |
| Cocamidopropyl Betaine | 3.0 |
| Coco Glucoside | 2.5 |
| Composition A | 2.0 |
| Glyceryl Oleate | 1.5 |
| Sodium Cocoyl Glutamate | 1.0 |
| Panthenol | 0.5 |
| Polyquaternium-10 | 0.3 |
| Chamomilla Recutita Extract | 0.5 |
| Glyceryl Caprylate | 0.3 |
| p-Anisic Acid | 0.05 |
| Citric Acid | ad pH 5.0 |
| Parfum, Dyes | q. s. |

### Example formulation 31: Caffeine Shampoo

| *Ingredient* | % |
|---|---|
| Aqua | ad 100 |
| Sodium Coco Sulfate | 6.0 |
| Glycerin | 5.5 |
| Lauryl Glucoside | 4.5 |
| Cocamidopropyl Betaine | 3.0 |
| Composition A | 2.5 |
| Lauroyl Sarcosine | 2.0 |
| Caffeine | 1.0 |
| Xanthan Gum | 1.0 |
| Hydroxypropyl Guar Hydroxypropyltrimonium Chloride | 0.3 |
| Alcohol | 0.8 |
| Coffea Arabica Bean Extract | 0.3 |
| Panthenyl Ethyl Ether | 0.4 |
| Panthenol | 0.2 |
| Limonene | 0.1 |
| Linalool | 0.1 |
| Dyes | q. s. |

### Example formulation 32: Shampoo

| *Ingredient* | % |
|---|---|
| Aqua | ad 100 |
| Sodium Laureth Sulfate | 6.5 |
| Cocamidopropyl Betaine | 3.0 |
| Sodium Chloride | 3.0 |
| Composition A | 2.0 |
| Panthenol | 0.2 |
| Benzophenone-4 | 0.9 |
| Glycol Distearate | 0.7 |
| Laureth-4 | 0.8 |
| Glycerin | 0.9 |
| Formic Acid | 0.2 |
| Decyl Glucoside | 0.9 |
| Polyquaternium-10 | 0.4 |
| Niacinamide | 0.1 |
| Sodium Benzoate | 0.1 |
| Potassium Sorbate | 0.2 |
| Citric Acid | ad pH 5.0 |
| Parfum, Dyes | q. s. |

### Example formulation 33: Shampoo

| *Ingredient* | % |
|---|---|
| Aqua | ad 100 |
| Sodium Laureth Sulfate | 8.5 |
| Cocamidopropyl Betaine | 3.0 |
| Sodium Chloride | 2.0 |
| Disodium Cocoamphodiacetate | 2.0 |
| Composition A | 2.0 |
| Sodium Benzoate | 0.7 |
| Panthenol | 0.2 |
| Sodium Laureth-8 Sulfate | 0.7 |
| Citric Acid | ad pH 5.0 |
| Sodium Cocoyl Glutamate | 0.8 |
| Glycol Distearate | 0.9 |
| Styrene/Acrylates Copolymer | 0.5 |
| Sodium Oleth Sulfate | 0.7 |
| Coco Glucoside | 0.9 |
| Cocamide MEA | 0.7 |
| Isopropyl Alcohol | 0.2 |
| Parfum, Dyes | q. s. |

### Example formulation 34: Volume Shampoo

| *Ingredient* | *%* |
|---|---|
| Aqua | ad 100 |
| Sodium Laureth Sulfate | 7.0 |
| Cocamidopropyl Betaine | 3.0 |
| Coco Glucoside | 3.0 |
| Composition A | 2.0 |
| Nymphaea Coerulea Flower Extract | 2.0 |
| Hydrolyzed Rice Protein | 2.0 |
| Cocamide MEA | 2.0 |
| Polyquaternium-10 | 0.5 |
| Malic Acid | ad pH 5.5 |
| Disodium EDTA | 0.5 |
| Propylene Glycol | 0.5 |
| Benzophenone-4 | 0.3 |
| Ethylhexyl Methoxycinnamate | 0.3 |
| Sodium Chloride | 0.9 |
| Benzyl Alcohol | 0.9 |
| PPG-9 | 0.2 |
| Methylisothiazolinone | 0.1 |
| Methylparaben | 0.1 |
| Propylparaben | 0.1 |
| Parfum, Dyes | q. s. |

### Example formulation 35: Shampoo

| *Ingredient* | *%* |
|---|---|
| Aqua | ad 100 |
| Sodium Laureth Sulfate | 6.5 |
| Coco Glucoside | 5.0 |
| Cocamidopropyl Betaine | 3.0 |
| Composition A | 3.0 |
| Glycol Distearate | 2.8 |
| Dimethicone | 1.7 |
| Laureth-23 | 1.5 |
| Laureth-4 | 1.5 |
| Polyquaternium-7 | 0.5 |
| Cocamide MEA | 0.7 |
| Cetyl Alcohol | 0.4 |
| Simmondsia Chinensis Seed Oil | 0.8 |
| Panthenol | 0.3 |
| Malic Acid | ad pH 5.5 |
| Glycine | 0.7 |
| Benzyl Alcohol | 0.7 |
| Disodium EDTA | 0.5 |
| Sodium Hydroxide | 0.2 |
| Guar Hydroxypropyltrimonium Chloride | 0.5 |
| Ethylhexyl Methoxycinnamate | 0.7 |
| Methylparaben | 0.3 |
| Propylparaben | 0.3 |
| PPG-9 | 0.2 |
| Benzophenone-4 | 0.3 |
| Sodium Chloride | 0.8 |
| DMDM Hydantoin | 0.1 |
| Hexyl Cinnamal | 0.1 |
| Limonene | 0.1 |
| Dyes | q. s. |

### Example formulation 36: Shampoo

| *Ingredient* | *%* |
|---|---|
| Aqua | ad 100 |
| Sodium Laureth Sulfate | 5.0 |
| Sodium Lauryl Sulfate | 5.0 |
| Cocamidopropyl Betaine | 3.0 |
| Composition A | 2.5 |
| Olea Europaea Fruit Oil | 2.0 |
| Cocamide MEA | 2.0 |
| Sodium Chloride | 1.9 |
| Glycol Distearate | 1.7 |
| Glycolipids | 1.0 |
| Benzyl Alcohol | 1.0 |
| Polyquaternium-10 | 0.5 |
| Hydroxypropyl Methylcellulose | 0.5 |
| Silicone Quaternium-18 | 0.9 |
| Malic Acid | ad pH 5.5 |
| Disodium EDTA | 0.5 |
| Sodium Xylenesulfonate | 0.7 |
| Trideceth-6 | 0.7 |
| Trideceth-12 | 0.5 |
| DMDM Hydantoin | 0.2 |
| Methylparaben | 0.3 |
| Propylparaben | 0.3 |
| Linalool | 0.1 |
| Hexyl Cinnamal | 0.1 |
| Butylphenyl Methylpropional | 0.1 |
| Limonene | 0.1 |
| Dyes | q. s. |

### Example formulation 37: Shampoo

| *Ingredient* | *%* |
|---|---|
| Aqua | ad 100 |
| Sodium Laureth Sulfate | 3.0 |
| Sodium Lauryl Sulfate | 3.5 |
| Glycol Distearate | 3.3 |
| Dimethicone | 3.0 |
| Cocamidopropyl Betaine | 2.5 |
| Composition A | 2.0 |
| Benzyl Alcohol | 0.7 |
| Betaine | 0.4 |
| Cetyl Alcohol | 0.7 |
| Cocamide MEA | 0.7 |
| Glyceryl Oleate | 0.5 |
| Propylene Glycol | 0.6 |
| Menthol | 0.7 |
| Guar Hydroxypropyltrimonium Chloride | 0.4 |
| Butylene Glycol | 0.5 |
| Menthyl Lactate | 0.2 |
| Disodium EDTA | 0.5 |
| Tocopheryl Acetate | 0.1 |
| Laureth-4 | 0.7 |
| Niacinamide | 0.1 |
| Maltodextrin | 0.2 |
| Sodium Ascorbyl Phosphate | 0.1 |
| PPG-9 | 0.2 |
| Salicylic Acid | 0.7 |
| Pyridoxine HCl | 0.1 |
| Malic Acid | ad pH 5.0 |
| Laureth-23 | 0.5 |
| Silica | 0.7 |
| Sodium Chloride | 0.7 |
| Sodium Hydroxide | 0.3 |
| Methylchloroisothiazolinone | 0.1 |
| Methylisothiazolinone | 0.1 |
| Parfum, Dyes | q. s. |

### Example formulation 38: Shampoo

| *Ingredient* | *%* |
|---|---|
| Aqua | ad 100 |
| Cocamidopropyl Betaine | 7.0 |
| Sodium Methyl Cocoyl Taurate | 4.0 |
| Glycolipids | 2.0 |
| Composition A | 1.0 |
| Sodium Chloride | 2.0 |
| Sodium C14-16 Olefin Sulfonate | 1.0 |
| PEG-120 Methyl Glucose Dioleate | 1.0 |
| Glycol Distearate | 1.0 |
| Sodium Benzoate | 1.0 |
| Caprylyl/Capryl Glucoside | 0.7 |
| PEG-7 Glyceryl Cocoate | 0.7 |
| Citric Acid | ad pH 5.3 |
| Parfum | 0.2 |
| Polyquaternium-10 | 0.2 |
| Coconut Acid | 0.1 |
| Laureth-4 | 0.1 |
| Hydrogenated Castor Oil | 0.1 |
| Glycerin | 0.1 |
| Lactic Acid | 0.1 |
| Propylene Glycol | 0.1 |
| Hydrolyzed Keratin | 0.1 |
| Ceramide NP | 0.05 |
| Panthenol | 0.05 |

### Example formulation 39: Conditioning Shampoo

| *Ingredient* | % |
|---|---|
| Aqua | ad 100 |
| Sodium Cocoamphoacetate | 5.0 |
| Cocamidopropyl Betaine | 4.0 |
| Glycolipids | 1.5 |
| Composition A | 1.5 |
| Starch Hydroxypropltrimonium chloride | 1.2 |
| Isostearamide MIPA; Glyceryl Laurate | 1.2 |
| Isoamyl Laurate | 1.0 |
| Bis-(Isostearoyl/Oleoyl Isopropyl) Dimonium Methosulfate | 0.5 |
| Citric Acid | 3.0 |
| Perfume, Preservative, Dyes | q. s. |

### Example formulation 40: Conditioning Shampoo

| *Ingredient* | % |
|---|---|
| Aqua | ad 100 |
| Sodium Laureth Sulfate | 7.0 |
| Cocamidopropyl Betaine | 1.5 |
| Composition As | 1.5 |
| Palmitamidopropyltrimonium Chloride | 1.0 |
| Polysilicone-19 | 1.0 |
| PEG-18 Glyceryl Oleate/Cocoate | 2.5 |
| Citric Acid | ad pH 5.5 |
| Perfume, Preservative | q. s. |

### Example formulation 41: Conditioning Shampoo

| *Ingredient* | % |
|---|---|
| Aqua | ad 100 |
| Sodium Cocoamphoacetate | 5.0 |
| Cocamidopropyl Betaine | 3.0 |
| Disodium Lauryl Sulfosuccinate | 3.0 |
| Glycolipids | 1.5 |
| Composition As | 1.5 |
| Palmitamidopropyltrimonium Chloride | 2.0 |
| Isostearamide MIPA, Glyceryl Laurate | 1.0 |
| PEG-200 Hydrogenated Glyceryl Palmate; PEG-7 Glyceryl Cocoate | 1.0 |
| Methoxy PEG/PPG-7/3 Aminopropyl Dimethicone | 0.5 |
| Starch Hydroxypropltrimonium chloride | 0.3 |
| Polyquaternium-10 | 0.2 |
| Citric Acid | ad pH 5.5 |
| Perfume, Preservative | q. s. |

### Example formulation 42: Conditioning Shampoo

| *Ingredient* | % |
|---|---|
| Aqua | ad 100 |
| Sodium Laureth Sulfate | 6.0 |
| Cocamidopropyl Betaine | 3.0 |
| Composition A | 3.0 |
| Glycol Distearate, Laureth-4, Cocamidopropyl Betaine | 2.0 |
| PEG-120 Methyl Glucose Dioleate | 1.7 |
| Composition A | 1.5 |
| Sorbitan Sesquicaprylate | 1.0 |
| Isoamyl Laurate | 1.0 |
| Sodium Chloride | 1.0 |
| Silicone-Quaternium-22 | 0.8 |
| Glycolipids | 0.5 |
| Hydroxypropyl Guar Hydroxypropyltrimonium Chloride | 0.5 |
| Creatine | 0.5 |
| Citric Acid | ad pH 5.5 |
| Perfume, Preservative | q. s. |

### Example formulation 43: Natural Shampoo

| *Ingredient* | % |
|---|---|
| Aqua | ad 100 |
| Sodium Coco-Sulfate | 9.0 |
| Glycerin | 5.0 |
| Lauryl Glucoside | 2.5 |
| Composition A | 2.5 |
| Sodium Lactate | 1.0 |
| Betaine | 1.0 |
| Hamamelis Virginiana Leaf Extract | 1.0 |
| Alcohol Denat. | 1.0 |
| Sodium/Disodium Cocoyl Glutamate | 0.8 |
| Maris Sal | 0.5 |
| Dipotassium Glycyrrhizate | 0.5 |
| Glycerophosphocholine | 0.1 |
| Parfum, Preservative | q. s. |

### Example formulation 44: Natural Shampoo

| *Ingredient* | % |
|---|---|
| Aqua | ad 100 |
| Sodium Coco-sulfate | 6.0 |
| Glycerin | 5.0 |
| Coco-glucoside | 4.0 |
| Lauryl Glucoside | 3.0 |
| Composition A | 2.0 |
| Glyceryl Oleate | 1.5 |
| Sodium Chloride | 1.0 |
| Calendula Officinalis Flower Extract | 0.5 |
| Parfum | 0.5 |
| Starch Hydroxypropyltrimonium chloride | 0.3 |
| Glyceryl Undecylenate | 0.3sarch |
| Polyglyceryl-4 Caprate | 0.3 |
| Xanthan Gum | 0.3 |
| Locust bean Gum | |
| Lysolecithin | 0.2 |
| Coco-Caprylate | 0.1 |
| Magnolia Officinalis Bark Extract | 0.1 |
| Tocopherol | 0.1 |
| Squalene | 0.1 |

### Example formulation 45: Natural Shampoo

| *Ingredient* | % |
|---|---|
| Aqua | ad 100 |
| Sodium Lauroyl Methyl Isethionate | 5.0 |
| Disodium Cocoamphodipropionate | 4.0 |
| Acrylates Copolymer | 2.0 |
| Hydroxyethylcellulose | 2.0 |
| Composition A | 2.0 |
| Cocos Nucifera Oil | 0.8 |
| Panthenol | 0.4 |
| Tocopherol | 0.4 |
| Tocopheryl Acetate | 0.2 |
| Cetyl Triethylmonium Dimethicone PEG-8 Succinate | 0.2 |
| Glycol Distearate | 0.2 |
| Laureth-4 | 0.2 |
| Cocamidopropyl Betaine | 0.2 |
| Ethylhexyl Salicylate | 0.1 |
| Disodium EDTA | 0.1 |
| Ethylhexylglycerin | 0.1 |
| Glycerin | 0.1 |
| Hexylene Glycol | 0.1 |
| Sodium Coco PG-dimonium Chloride Phosphate | 0.1 |
| BHT | 0.1 |
| Citric Acid | 0.1 |
| Phenoxyethanol | 0.1 |
| Formic Acid | 0.1 |
| Parfum | q.s. |

### Example formulation 46: Natural Shampoo

| *Ingredient* | % |
|---|---|
| Aqua | ad 100 |
| Moroccan Lava Clay | 5.0 |
| Behenyl Alcohol | 3.0 |
| Glycerin | 2.5 |
| Glyceryl Stearate Citrate | 2.0 |
| Lauryl Glucoside | 2.0 |
| Coco-glucoside | 2.0 |
| Helianthus Annuus Hybrid Oil | 1.5 |
| Prunus Armeniaca Kernel Oil | 1.5 |
| Composition A | 1.0 |
| Glycolipids | 1.0 |
| Argania Spinosa Kernel Oil | 1.0 |
| Magnolia Officinalis Bark Extract | 1.0 |
| Triethyl Citrate, Glyceryl Caprylate Benzoic Acid | 1.0 |
| Xanthan Gum | 0.8 |
| Sodium Lactate | 0.5 |
| Benzyl Alcohol | 0.3 |
| Lactic Acid | 0.2 |
| Parfum | q.s. |

### Example formulation 47: Natural Shampoo

| *Ingredient* | % |
|---|---|
| Aqua | ad 100 |
| Cocamidopropyl Betaine | 7.0 |
| Cetrimonium Chloride | 5.0 |
| Decyl Glucoside | 5.0 |
| Sodium Chloride | 2.0 |
| Composition A | 1.0 |
| Sorbitan Caprylate | 0.8 |
| Cocos Nucifera Oil | 0.8 |
| Inulin | 0.5 |
| Benzyl Alcohol | 0.2 |
| Cetearyl Alcohol | 0.2 |
| Xanthan Gum | 0.2 |
| Sodium Cocoyl Glutamate | 0.1 |
| Parfum | 0.1 |
| Glycerin | 0.2 |
| Argania Spinosa Kernel Oil | 0.1 |
| Titanium Dioxide | 0.1 |
| Hydroxypropyl Guar Hydroxypropyltrimonium Chloride | 0.1 |
| Lactic Acid | 0.1 |
| Hexyl Cinnamal | 0.1 |
| Sodium Hydroxide | 0.1 |
| Ascorbyl Palmitate | 0.1 |
| Tocopherol | 0.1 |

### Example formulation 48: Natural Shampoo

| *Ingredient* | % |
|---|---|
| Aqua | ad 100 |
| Glycerin | 5.0 |
| Sodium Coco-sulfate | 5.0 |
| Lauryl Glucoside | 4.0 |
| Composition A | 4.0 |
| Betaine | 3.0 |
| Maris Sal | 2.0 |
| Camellia Oleifera Leaf Extract | 1.5 |
| Pisum Sativum Peptide | 1.5 |
| Oryza Sativa Extract | 1.0 |
| Pisum Sativum Seed Extract | 0.5 |
| Disodium Cocoyl Glutamate | 0.4 |
| Sodium Cocoyl Glutamate | 0.4 |
| PCA Glyceryl Oleate | 0.2 |
| Alcohols | 0.1 |
| Parfum | q.s. |

### Example formulation 49: Silk Shampoo

| *Ingredient* | % |
|---|---|
| Aqua | ad 100 |
| Composition A | 3,0 |
| Sodium Laureth sulfate | 4,0 |
| Sodium Chloride | 1,2 |
| Cocamidopropyl Betaine | 2,3 |
| Glycosphingolipids | 1,0 |
| Hydrolyzed Silk | 0,8 |
| Sericin | 0,7 |
| Hydrolyzed Keratin | 0,3 |
| Parfum (Fragrance) | 0,1 |
| PEG-7 Glyceryl Cocoate | 0,4 |
| Polyquaternium-10 | 0,2 |
| Sodium Benzoate | 0,2 |
| PEG-120 Methyl Glucose Dioleate | 0,3 |
| Coco-Glucoside | 0,5 |
| Glyceryl Oleate | 0,4 |
| Citric Acid | 0,6 |
| PEG-40 Hydrogenated Castor Oil | 0,7 |
| Glycol Distearate | 1,0 |
| Hydrogenated Castor Oil | 0,4 |
| Butylene Glycol | 0,7 |
| Laureth-4 | 0,8 |
| Prunus Armeniaca (Apricot) Kernel Oil | 0,4 |
| Propylene Glycol | 0,5 |
| Sodium Hydroxide | 0,5 |
| Benzyl Alcohol | 0,8 |
| Phenoxyethanol | 0,2 |
| Potassium Sorbate | 0,2 |
| Methylparaben | 0,1 |
| Propylparaben | 0,1 |

### Example formulation 50: Ultra Sensitive Shampoo

| *Ingredient* | % |
|---|---|
| Aqua | ad 100 |
| Composition A | 5,0 |
| Coco-Glucoside | 5,0 |
| Glycerin | 3,0 |
| Sodium Coco-Sulfate | 2,0 |
| Sodium Cocoamphoacetate | 2,0 |
| Citric Acid | 1,0 |
| Panthenol | 1,0 |
| Niacinamid | 0,5 |
| Tocopherol | 0,3 |
| Ascorbyl Palmitate | 0,25 |
| Glyceryl Oleate | 0,25 |
| Hydroxypropyl Guar Hydroxypropyltrimonium Chloride | 0,15 |
| Hydrogenated Palm Glycerides Citrate | 0,15 |
| Lecithin | 0,1 |
| Sodium Benzoate | 0,1 |
| Potassium Sorbate | 0,1 |
| Sodium Chloride | 0,1 |
| Sodium Hydroxide | 0,1 |

### Example formulation 51: Anti-Hair Loss Tonic

| *Ingredient* | *%* |
|---|---|
| Aqua | ad 100 |
| Alcohol Denat. | 50.0 |
| PEG-40 Hydrogenated Castor Oil | 2.5 |
| Composition A | 1.5 |
| PEG-7 Glyceryl Cocoate | 2.0 |
| Sphinganine | 0.1 |
| Parfum, Preservative | q. s. |

### Example formulation 52: Shower Oil

| *Ingredient* | *%* |
|---|---|
| Aqua | ad 100 |
| Magnesium Laureth Sulfate | 6.0 |
| PEG-7 Glyceryl Cocoate | 5.0 |
| Hydrogenated Starch Hydrolysate | 3.0 |
| Cocamidopropyl Betaine | 3.0 |
| Composition A | 3.0 |
| Decyl Glucoside | 1.0 |
| Prunus Amygdalus Dulcis (Sweet Almond) Oil | 1.0 |
| Olive Oil | 0.5 |
| PEG-7 Esters | 0.2 |
| Parfum | 0.1 |
| Polysorbate 20 | 0.1 |
| Sodium Benzoate | 0.2 |
| Citric Acid | 0.1 |
| PEG-6 Caprylic/Capric Glycerides | 0.1 |
| PEG-120 Methyl Glucose Dioleate | 0.1 |
| Disodium EDTA | 0.1 |

### Example formulation 53: Shower Mousse

| *Ingredient* | *%* |
|---|---|
| Aqua | ad 100 |
| Sodium Lauroyl Glutamate | 15.0 |
| Isobutane | 6.0 |
| Cocamidopropyl Betaine | 4.0 |
| Glycerin | 3.0 |
| Composition A | 3.0 |
| Sodium Chloride | 2.6 |
| Sodium Lauroyl Glycinate | 1.5 |
| Propane | 1.0 |
| Butane | 0.6 |
| Citric Acid | 0.4 |
| lodopropynyl Butylcarbamate | 0.3 |
| Lauric Acid | 0.3 |
| Parfum | 0.1 |
| Polysorbate 20 | 0.1 |
| Sodium Benzoate | 0.1 |
| Sodium Hydroxide | 0.1 |
| Stearic Acid | 0.1 |
| Tetrasodium EDTA | 0.1 |

### Example formulation 54: Shower Jelly

| *Ingredient* | *%* |
|---|---|
| Glycerin | 3.5 |
| Sodium Laureth Sulfate | 3.5 |
| Composition A | 3.0 |
| Propylene Glycol | 2.0 |
| Chondrus Crispus Extract | 2.0 |
| Pyrus Malus Juice | 1.0 |
| Vitis Vinifera Juice | 0.5 |
| Parfum | 0.2 |
| Butylphenyl Methylpropional | 0.1 |

### Example formulation 55: Shower Jelly

| *Ingredient* | *%* |
|---|---|
| Aqua | ad 100 |
| Glycerin | 9.0 |
| Coco-Glucoside | 5.0 |
| Decyl Glucoside | 5.0 |
| Composition A | 2.0 |
| Chondrus crispus extract | 1.0 |
| Parfum | 0.2 |
| Citric Acid | 0.2 |
| Sodium Anisate | 0.1 |
| Sodium Levulinate | 0.1 |

### Example formulation 56: Shower Scrub

| *Ingredient* | *%* |
|---|---|
| Aqua | ad 100 |
| Maris Sal | 15.0 |
| Sodium Laureth Sulfate | 9.0 |
| Composition A | 2.0 |
| Sodium Cocoamphoacetate | 1.0 |
| Betaine | 1.0 |
| Citrus Limon (Lemon) Peel Powder | 0.5 |
| Lactic Acid | ad pH 5.5 |
| Parfum, Dyes | q s. |

### Example formulation 57: Cellulose Scrub

| *Ingredient* | *%* |
|---|---|
| Aqua | ad 100 |
| Composition A | 7.0 |
| Coco Glucoside | 6.0 |
| Glycerin | 5.0 |
| Glyceryl Stearate | 5.0 |
| Citrus Aurantium Dulcis Fruit Water | 4.5 |
| Helianthus Annuus Hybrid Oil | 4.0 |
| Cellulose | 2.0 |
| Jojoba Esters | 2.0 |
| Cetyl Alcohol | 2.0 |
| Simmondsia Chinensis (Jojoba) Seed Oil | 2.0 |
| Olea Europaea (Olive) Fruit Oil | 2.0 |
| Hydrogenated Castor Oil | 0.8 |
| Prunus Armeniaca (Apricot) Kernel Oil | 0.5 |
| Leontopodium Alpinum Extract | 0.1 |
| Xanthan Gum | 0.3 |
| Benzyl Alcohol | 0.2 |
| Parfum | 0.1 |
| Dehydroacetic Acid | 0.1 |
| Citric acid | ad pH 5.0 |
| Lactic acid | 0.1 |
| Sodium Hydroxide | 0.1 |
| Parfum, Dyes | q. s. |

### Example formulation 58: Exfoliating Body Scrub

| *Ingredient* | *%* |
|---|---|
| Aqua | ad 100 |
| Coco-Glucoside | 7.0 |
| Alcohol | 6.0 |
| Glycerin | 5.0 |
| Glycine Soja Oil | 5.0 |
| Hydrated Silica | 4.0 |
| Xanthan Gum | 3.0 |
| Composition A | 3.0 |
| Disodium/Sodium Cocoyl Glutamate | 1.0 |
| Coffea Arabica (Coffee) Seed Powder | 1.0 |
| Cocos Nucifera (Coconut) Shell Powder | 1.0 |
| Citric Acid | ad pH 5.2 |
| Glyceryl Oleate | 0.5 |
| Parfum | 0.1 |
| Preservative, Dyes | q. s. |

### Example formulation 59: Dry Shampoo

| *Ingredient* | % |
|---|---|
| Butane, Propane | 53.0 |
| Alcohol Denat. | 36.0 |
| Aluminum Starch Octenylsuccinate | 3.0 |
| Panthenol | 1.5 |
| Tocopherol | 1.0 |
| Cocos Nucifera Oil | 1.0 |
| Curcuma Longa (Turmeric) Root Extract | 0.6 |
| Silica | 0.5 |
| Sodium Bicarbonate | 0.5 |
| Charcoal Powder | 0.4 |
| Hydrolyzed Rice Protein | 0.4 |
| Aminopropyl Phenyl Trimethicone | 0.4 |
| Benzophenone-4 | 0.3 |
| Polysorbate 20 | 0.3 |
| Composition A | 0.3 |
| Cyclopentasiloxane | 0.2 |
| Cyclohexasiloxane | 0.2 |
| Glycerin | 0.2 |
| Aqua | 0.2 |
| Parfum | q.s. |

### Example formulation 60: Dry Shampoo

| *Ingredient* | % |
|---|---|
| Butane, Isobutane, Propane | 55.0 |
| Oryza Sativa (Rice) Starch | 25.0 |
| Alcohol denat. | 18.0 |
| Parfum | 1.0 |
| Composition A | 0.5 |
| Distearyldimonium Chloride | 0.3 |
| Cetrimonium Chloride | 0.2 |

### Shower

### Example formulation 61: Shower Gel

| *Ingredient* | % |
|---|---|
| Aqua | ad 100 |
| Sodium Laureth Sulfate | 8.5 |
| Cocamidopropyl Betaine | 3.0 |
| Sodium Chloride | 2.7 |
| Composition A | 2.5 |
| Guar Gum | 0.5 |
| Polyquaternium-7 | 0.5 |
| Bisabolol | 0.2 |
| Trideceth-9 | 0.5 |
| Sodium Sulfate | 0.9 |
| Tetrasodium EDTA | 0.2 |
| Citric Acid | ad pH 5.5 |
| Sodium Benzoate | 0.7 |
| Benzyl Alcohol | 0.7 |
| Benzyl Salicylate | 0.1 |
| Geraniol | 0.2 |
| Hexyl Cinnamal | 0.1 |
| Limonene | 0.1 |
| Linalool | 0.1 |
| Dyes | q. s. |

### Example formulation 62: Body Wash

| *Ingredient* | % |
|---|---|
| Water | ad 100 |
| Sodium Laureth Sulfate | 4.0 |
| Sodium Lauryl Sulfate | 4.0 |
| Sodium Chloride | 3.3 |
| Cocamidopropyl Betaine | 3.0 |
| Composition A | 3.0 |
| Sodium Benzoate | 1.5 |
| Citric Acid | ad pH 5.5 |
| Disodium EDTA | 0.5 |
| Zea Mays (Corn) Silk Extract | 0.5 |
| Methylchloroisothiazolinone | 0.1 |
| Methylisothiazolinone | 0.1 |
| Potassium Sorbate | 0.2 |
| Parfum, Dyes | q. s. |

### Example formulation 63: Cool Down Shower Gel

| *Ingredient* | % |
|---|---|
| Aqua | ad 100 |
| Sodium Laureth Sulfate | 8.0 |
| Cocamidopropyl Betaine | 3.0 |
| Composition A | 3.0 |
| Glycerin | 2.7 |
| Acrylates Copolymer | 2.5 |
| Sodium Chloride | 2.2 |
| Phenoxyethanol | 1.5 |
| Taurine | 1.5 |
| Benzyl Alcohol | 1.0 |
| Sodium Hydroxide | 0.5 |
| PEG-40 Hydrogenated Castor Oil | 0.7 |
| Citric Acid | ad pH 5.0 |
| Mannitol | 0.7 |
| Benzophenone-4 | 0.3 |
| Ethylhexyl Salicylate | 0.5 |
| Cellulose | 0.5 |
| Linalool | 0.1 |
| Butyl Methoxydibenzoylmethane | 0.2 |
| Hydroxypropyl Methylcellulose | 0.2 |
| Dyes | q. s. |

### Example formulation 64: Shower Gel

| *Ingredient* | % |
|---|---|
| Aqua | ad 100 |
| Cocamidopropyl Betaine | 5.0 |
| Sodium Myreth Sulfate | 4.5 |
| Sodium Laureth Sulfate | 4.0 |
| Composition A | 3.5 |
| PEG-7 Glyceryl Cocoate | 3.2 |
| Glycerin | 3.0 |
| Glyceryl Glucoside | 2.5 |
| Bisabolol | 2.0 |
| Chamomilla Recutita (Matricaria) Flower Extract | 2.2 |
| Sodium Chloride | 2.2 |
| PEG-40 Hydrogenated Castor Oil | 2.0 |
| Citric Acid | ad pH 5.0 |
| Glycolipids | 1.5 |
| PEG-90 Glyceryl Isostearate | 1.2 |
| PEG-200 Hydrogenated Glyceryl Palmate | 1.2 |
| Benzophenone-4 | 1.0 |
| Laureth-2 | 0.7 |
| Polyquaternium-7 | 0.5 |
| BHT | 0.2 |
| Sodium Benzoate | 0.2 |
| Methylparaben | 0.2 |
| Benzyl Alcohol | 0.2 |
| Citronellol | 0.1 |
| Limonene | 0.1 |
| Dyes | q. s. |

### Example formulation 65: Shower Gel

| *Ingredient* | % |
|---|---|
| Aqua | ad 100 |
| Sodium Laureth Sulfate | 8.0 |
| Cocamidopropyl Betaine | 3.0 |
| Acrylates Copolymer | 3.0 |
| PEG-7 Glyceryl Cocoate | 2.7 |
| Composition A | 2.5 |
| Helianthus Annuus Seed Oil | 1.5 |
| Glycerin | 1.5 |
| Glycolipids | 1.5 |
| Sodium Chloride | 1.0 |
| PEG-40 Hydrogenated Castor Oil | 1.0 |
| Disodium Cocoyl Glutamate | 1.0 |
| Trisodium EDTA | 1.0 |
| Propylene Glycol | 1.0 |
| Microcrystalline Cellulose | 0.9 |
| Benzophenone-4 | 0.5 |
| Sodium Sulfate | 0.7 |
| Phenoxyethanol | 0.7 |
| Acacia Gum | 0,3 |
| Propylparaben | 0.1 |
| Methylparaben | 0.1 |
| Benzyl Alcohol | 0.2 |
| Parfum, Dyes | q. s. |

### Example formulation 66: Body Wash

| *Ingredient* | % |
|---|---|
| Aqua | ad 100 |
| Sodium Hydroxypropyl Starch Phosphate | 6.0 |
| Sodium Laureth Sulfate | 5.5 |
| Cocamidopropyl Betaine | 3.0 |
| Composition A | 3.0 |
| Lauric Acid | 2.5 |
| Petrolatum | 2.0 |
| Sodium Cocoyl Glycinate | 2.0 |
| Glycerin | 1.7 |
| Sodium Lauroyl Isethionate | 1.5 |
| Glycine Soja (Soybean) Oil | 1.0 |
| Helianthus Annuus (Sunflower) Oil | 1.0 |
| Sodium Chloride | 1.0 |
| Stearic Acid | 0.7 |
| Guar Hydroxypropyltrimonium Chloride | 0.3 |
| DMDM Hydantoin | 0.1 |
| Tallow Acid | 0.2 |
| Palmitic Acid | 0.1 |
| Sodium Isethionate | 0.2 |
| BHT | 0.1 |
| Tetrasodium EDTA | 0.2 |
| Methylisothiazolinone | 0.3 |
| lodopropynyl Butylcarbamate | 0.1 |
| Titanium Dioxide | 0.1 |
| Parfum, Dyes | q. s. |

### Example formulation 67: Anti-Aging Body Wash

| *Ingredient* | % |
|---|---|
| Aqua | ad 100 |
| Petrolatum | 5.0 |
| Mineral (Paraffinum Liquidum) Oil | 4.0 |
| Sodium Trideceth Sulfate | 3.5 |
| Sodium Lauryl Sulfate | 3.0 |
| Sodium Lauroamphoacetate | 3.0 |
| Sodium Chloride | 2.3 |
| Composition A | 2.0 |
| Trideceth-3 | 1.0 |
| Simmondsia Chinensis (Jojoba) Butter | 0.7 |
| Niacinamide | 0.1 |
| Panthenol | 0.3 |
| Soluble Collagen | 0.5 |
| Citric Acid | ad pH 5.5 |
| Disodium EDTA | 0.3 |
| Guar Hydroxypropyltrimonium Chloride | 0.3 |
| Methylchloroisothiazolinone | 0.1 |
| Methylisothiazolinone | 0.1 |
| PEG-90m | 0.7 |
| Sodium Benzoate | 0.3 |
| Sodium Hydroxide | 0.1 |
| Xanthan Gum | 0.5 |
| Calcium Alginate | 0.1 |
| Parfum, Dyes | q. s. |

### Example formulation 68: Shower Gel

| *Ingredient* | % |
|---|---|
| Aqua | ad 100 |
| Sodium Laureth Sulfate | 9.0 |
| Cocamidopropyl Betaine | 3.0 |
| Composition A | 3.0 |
| Glycerin | 3.0 |
| Sodium Chloride | 2.8 |
| PEG-7 Glyceryl Cocoate | 2.5 |
| PEG-40 Hydrogenated Castor Oil | 2.5 |
| Citric Acid | ad pH 5.5 |
| Sodium Benzoate | 1.0 |
| Potassium Sorbate | 0.7 |
| Alcohol | 0.5 |
| Citronellol | 0.2 |
| Benzyl Alcohol | 0.7 |
| Limonene | 0.1 |
| Linalool | 0.2 |
| Geraniol | 0.2 |
| Hexyl Cinnamal | 0.1 |
| Ethylhexyl Salicylate | 0.1 |
| Octocrylene | 0.3 |
| Butyl Methoxydibenzoylmethane | 0.2 |
| Dyes | q. s. |

### Example formulation 69: Shower Gel

| *Ingredient* | % |
|---|---|
| Aqua | ad 100 |
| Alcohol | 6.0 |
| Coco Glucoside | 6.0 |
| Glycerin | 3.0 |
| Composition A | 3.0 |
| Xanthan Gum | 1.5 |
| Disodium Cocoyl Glutamate | 2.0 |
| Citric Acid | ad pH 5.5 |
| Citrus Aurantifolia (Lime) Oil | 0.5 |
| Sodium Cocoyl Glutamate | 0.7 |
| Glyceryl Oleate | 0.3 |
| Sodium Lauroyl Lactylate | 0.8 |
| Limonene | 0.1 |
| Linalool | 0.1 |
| Dyes | q. s. |

### Example formulation 70: Shower Gel for Men

| *Ingredient* | % |
|---|---|
| Aqua | ad 100 |
| Sodium Laureth Sulfate | 9.5 |
| Cocamidopropyl Betaine | 3.0 |
| Cocamide MEA | 3.0 |
| Composition A | 3.0 |
| Glycerin | 2.5 |
| PPG-12 | 2.0 |
| Sodium Chloride | 2.0 |
| Citric Acid | ad pH 5.5 |
| Sodium Benzoate | 1.0 |
| Phenoxyethanol | 0.7 |
| Benzyl Alcohol | 0.7 |
| Butylphenyl Methylpropional | 0.1 |
| Citronellol | 0.1 |
| Geraniol | 0.2 |
| Hexyl Cinnamal | 0.1 |
| Limonene | 0.1 |
| Linalool | 0.1 |
| Dyes | q. s. |

### Example formulation 71: Moisturising Shower Cream

| *Ingredient* | *%* |
|---|---|
| Aqua | ad 100 |
| Sodium Laureth Sulfate | 8.5 |
| Cocamidopropyl Betaine | 3.0 |
| Glycerin | 2.5 |
| Composition A | 2.0 |
| Sodium Chloride | 1.7 |
| Cocamide MEA | 1.5 |
| Styrene/Acrylates Copolymer | 1.5 |
| Sodium Salicylate | 1.0 |
| Sodium Benzoate | 1.0 |
| Polyquaternium-7 | 1.0 |
| Citric Acid | ad pH 5.0 |
| Tetrasodium EDTA | 1.0 |
| Glycol Distearate | 0.9 |
| Laureth-4 | 0.5 |
| Magnolia Officinalis Flower Extract | 0.2 |
| Butyrospermum Parkii Butter Extract | 0.2 |
| Prunus Amygdalus Dulcis (Sweet Almond) Extract | 0.1 |
| Tapioca Starch polymethylsilsesquioxane | 0.5 |
| Benzyl Benzoate | 0.1 |
| Cinnamyl Alcohol | 0.1 |
| Hexyl Cinnamal | 0.1 |
| Parfum, Dyes | q. s. |

### Example formulation 72: Shower Cream

| | |
|---|---|
| | |
| Aqua | ad 100 |
| Ammonium Lauryl Sulfate | 6.0 |
| Coco Glucoside | 3.5 |
| Cocamidopropyl Betaine | 2.0 |
| Sodium Cocoamphoacetate | 2.0 |
| Composition A | 1.5 |
| Argania Spinosa Kernel Oil | 1.0 |
| Butyrospermum Parkii Butter | 1.0 |
| Glycerin | 1.0 |
| Polyquaternium-7 | 1.0 |
| Styrene/Acrylates Copolymer | 1.0 |
| Gelatin Crosspolymer | 0.5 |
| Sodium Chloride | 0.5 |
| Citric Acid | ad pH 5.0 |
| Sodium Benzoate | 0.3 |
| Sodium Salicylate | 0.1 |
| Linalool | 0.1 |
| Dyes | q. s. |

### Example formulation 73: Shower Cream

| *Ingredient* | *%* |
|---|---|
| Aqua | ad 100 |
| Sodium Laureth Sulfate | 7.0 |
| Cocamidopropyl Betaine | 3.0 |
| Composition A | 2.0 |
| Sodium Chloride | 1.9 |
| Polyquaternium-7 | 1.5 |
| Cocamide MEA | 1.5 |
| PEG-7 Glyceryl Cocoate | 1.0 |
| Laureth-10 | 1.0 |
| Glycol Distearate | 0.8 |
| Citric Acid | ad pH 5.0 |
| Linalool | 0.1 |
| Limonene | 0.1 |
| Benzyl Salicylate | 0.1 |
| Butylphenyl Methylpropional | 0.1 |
| alpha-Isomethyl lonone | 0.1 |
| Hexyl Cinnamal | 0.1 |
| Citronellol | 0.1 |
| Sodium Benzoate | 0.7 |
| Sodium Salicylate | 0.1 |
| Dyes | q. s. |

### Example formulation 74: Shower Cream

| *Ingredient* | *%* |
|---|---|
| Aqua | ad 100 |
| Glycerin | 7.5 |
| Glycine Soja Oil | 3.0 |
| Lauryl Glucoside | 3.0 |
| Sodium Coco Sulfate | 3.0 |
| Composition A | 2.5 |
| Alcohol | 1.5 |
| Xanthan Gum | 1.5 |
| Butyrospermum Parkii Butter | 1.2 |
| Sodium Cetearyl Sulfate | 1.0 |
| Sodium Cocoyl Glutamate | 1.0 |
| Disodium Cocoyl Glutamate | 1.0 |
| Tocopherol | 0.1 |
| Helianthus Annuus Seed Oil | 0.3 |
| Limonene | 0.1 |
| Benzyl Salicylate | 0.1 |
| Linalool | 0.1 |
| Dyes | q. s. |

### Example formulation 75: Shower Gel

| *Ingredient* | *%* |
|---|---|
| Aqua | ad 100 |
| Sodium Coco Sulfate | 5.0 |
| Glycerin | 5.0 |
| Lauryl Glucoside | 4.0 |
| Sodium Lactate | 2.5 |
| Composition A | 2.0 |
| Sodium Cocoyl Glutamate | 2.0 |
| Disodium Cocoyl Glutamate | 2.0 |
| Alcohol | 1.0 |
| Prunus Cerasus Fruit Extract | 1.0 |
| Limonene | 0.1 |
| Coumarin | 0.2 |
| Linalool | 0.1 |
| Citral | 0.1 |
| Dyes | q. s. |

### Example formulation 76: Shower Gel

| *Ingredient* | *%* |
|---|---|
| Aqua | ad 100 |
| Alcohol | 5.0 |
| Coco-Glucoside | 5.0 |
| Glycerin | 5.0 |
| Composition A | 2.5 |
| Xanthan Gum | 2.5 |
| Disodium Cocoyl Glutamate | 2.0 |
| Citric Acid | ad pH 5.0 |
| Citrus Aurantifolia (Lime) Oil | 1.0 |
| Sodium Cocoyl Glutamate | 1.0 |
| Glyceryl Oleate | 1.0 |
| Sodium Lauroyl Lactylate | 0.7 |
| Limonene | 0.1 |
| Citral | 0.1 |
| Linalool | 0.1 |
| Dyes | q. s. |

### Example formulation 77: Oil Shower

| *Ingredient* | *%* |
|---|---|
| Aqua | ad 100 |
| Sodium Laureth Sulfate | 8.0 |
| Cocamidopropyl Betaine | 3.0 |
| Composition A | 2.0 |
| Acrylates Copolymer | 2.0 |
| Glycolipids | 2.0 |
| Tocopheryl Acetate | 0.5 |
| PEG-7 Glyceryl Cocoate | 0.5 |
| Disodium EDTA | 0.4 |
| Benzophenone-4 | 0.3 |
| PEG-40 Hydrogenated Castor Oil | 0.7 |
| Cellulose | 0.7 |
| Hydroxypropyl Methylcellulose | 0.7 |
| Sodium Chloride | 0.9 |
| Ethylhexylglycerin | 0.2 |
| Phenoxyethanol | 0.7 |
| Methylisothiazolinone | 0.3 |
| Sodium Benzoate | 0.3 |
| Sodium Hydroxide | 0.3 |
| Citric Acid | ad pH 5.5 |
| Parfum, Dyes | q. s. |

### Example formulation 78: Shower Gel

| *Ingredient* | *%* |
|---|---|
| Aqua | ad 100 |
| Ammonium Lauryl Sulfate | 6.0 |
| Coco-Betaine | 3.0 |
| Composition A | 3.0 |
| Lauroyl/Myristoyl Methyl Glucamide | 1.5 |
| Citric Acid | ad pH 5.0 |
| Benzyl Alcohol, Glycerin, Benzoic Acid, Sorbic Acid | 1.0 |
| Parfum | 0.8 |
| Aloe Vera Leaf Extract | 0.1 |

### Example formulation 79: Showergel

| *Ingredient* | % |
|---|---|
| Aqua | ad 100 |
| Composition A | 7.0 |
| Sesamum Indicum (Sesame) Seed Oil | 4.0 |
| Glycolipid | 3.0 |
| Sucrose. Alcohol | 2.0 |
| Amino Acids | 1.0 |
| Glycerin | 1.0 |
| Carrageenan | 0.3 |
| Lavandula Officinalis Flower Oil | 0.3 |
| Xanthan Gum | 0.3 |
| Lactic Acid | 0.1 |

### Example formulation 80: Shower Scrub

| *Ingredient* | % |
|---|---|
| Aqua | ad 100 |
| Maris Sal | 15.0 |
| Sodium Laureth Sulfate | 9.0 |
| Composition A | 2.0 |
| Sodium Cocoamphoacetate | 1.0 |
| Betaine | 1.0 |
| Citrus Limon (Lemon) Peel Powder | 0.5 |
| Lactic Acid | ad pH 5.5 |
| Parfum, Dyes | q s. |

### Example formulation 81: Cellulose Scrub

| *Ingredient* | % |
|---|---|
| Aqua | ad 100 |
| Composition A | 7.0 |
| Coco Glucoside | 6.0 |
| Glycerin | 5.0 |
| Glyceryl Stearate | 5.0 |
| Citrus Aurantium Dulcis Fruit Water | 4.5 |
| Helianthus Annuus Hybrid Oil | 4.0 |
| Cellulose | 2.0 |
| Jojoba Esters | 2.0 |
| Cetyl Alcohol | 2.0 |
| Simmondsia Chinensis (Jojoba) Seed Oil | 2.0 |
| Olea Europaea (Olive) Fruit Oil | 2.0 |
| Hydrogenated Castor Oil | 0.8 |
| Prunus Armeniaca (Apricot) Kernel Oil | 0.5 |
| Leontopodium Alpinum Extract | 0.1 |
| Xanthan Gum | 0.3 |
| Benzyl Alcohol | 0.2 |
| Parfum | 0.1 |
| Dehydroacetic Acid | 0.1 |
| Citric acid | ad pH 5.0 |
| Lactic acid | 0.1 |
| Sodium Hydroxide | 0.1 |
| Parfum, Dyes | q. s. |

### Example formulation 82: Exfoliating Body Scrub

| *Ingredient* | % |
|---|---|
| Aqua | ad 100 |
| Coco-Glucoside | 7.0 |
| Alcohol | 6.0 |
| Glycerin | 5.0 |
| Glycine Soja Oil | 5.0 |
| Hydrated Silica | 4.0 |
| Xanthan Gum | 3.0 |
| Composition A | 3.0 |
| Disodium/Sodium Cocoyl Glutamate | 1.0 |
| Coffea Arabica (Coffee) Seed Powder | 1.0 |
| Cocos Nucifera (Coconut) Shell Powder | 1.0 |
| Citric Acid | ad pH 5.2 |
| Glyceryl Oleate | 0.5 |
| Parfum | 0.1 |
| Preservative, Dyes | q. s. |

### Example formulation 83: Washing Powder

| *Ingredient* | % |
|---|---|
| Talc | 60.0 |
| Sodium Cocoyl Isethionate | 15.0 |
| Sodium C14-16 Olefin Sulfonate | 10.0 |
| Sodium Lauroyl Glutamate | 4.0 |
| Composition A | 3.0 |
| Potassium Laurate | 2.0 |
| Sodium Myristoyl Glutamate | 1.5 |
| Chondrus Crispus | 1.5 |
| Isostearyl Alcohol | 1.0 |
| Glycerin | 1.0 |
| BHT | 0.4 |
| Butylene Glycol | 0.3 |
| Protease | 0.2 |
| Salicylic Acid | 0.1 |

### Example formulation 84: Washing Powder

| *Ingredient* | % |
|---|---|
| Hydrates Silica | ad 100 |
| Zea Mays Starch | 30.0 |
| Sodium Cocoyl Isethionate | 6.0 |
| Amylopektin | 5.0 |
| Sodium Lauryl Sulfate | 2.5 |
| Sodium Bicarbonate | 2.0 |
| Citric Acid | 1.5 |
| Sodium Lauroyl Glutamate | 1.0 |
| Sodium Palmitate | 1.0 |
| Composition A | 1.0 |
| Diglycerin | 0.9 |
| Sodium Lauroyl Aspartate | 0.8 |
| Titanium Dioxide | 0.5 |
| Maltodextrin | 0.5 |
| Allantoin | 0.4 |
| Aqua | 0.3 |
| Butylene Glycol | 0.3 |
| Aloe Vera Leaf Extract | 0.1 |
| Citrus Limon (Lemon) Peel Powder | 0,1 |
| Parfum, Preservatives, Dyes | q. s. |

### Example formulation 85: Powder Cleanser

| *Ingredient* | *%* |
|---|---|
| Hydrated Silica | ad 100 |
| Tapioca Flour | 10.0 |
| Capryl/Capramidopropyl Betaine | 5.0 |
| Sodium Cocoamphoacetate | 5.0 |
| Helianthus Annuus (Sunflower) Seed Oil | 4.0 |
| Oleyl Erucate | 2.0 |
| Sucrose Cocoate | 2.0 |
| Composition A | 2.0 |
| Coconut Acid | 1.0 |
| Citric Acid | ad pH 6.0 |
| Preservatives, parfum | q. s. |

### Example formulation 86: Powder Cleanser

| *Ingredient* | *%* |
|---|---|
| Talc | ad 100 |
| Distarch Phosphate | 10.0 |
| Disodium Lauryl Sulfosuccinate | 8.0 |
| Magnesium Aluminum Silicate | 5.0 |
| Oryza Sativa Powder | 5.0 |
| Sodium Lauroyl Glutamate | 2.0 |
| Composition A | 2.0 |
| Glucose | 2.0 |
| Magnesium Oxide | 2.0 |
| Maltodextrin | 2.0 |
| Titanium Dioxide | 1.0 |
| Pumice | 0.5 |
| Chlorella Vulgaris Powder | 0.1 |

### Soap

### Example formulation 87: Gentle Liquid Soap

| *Ingredient* | *%* |
|---|---|
| Aqua | ad 100 |
| Sodium Laureth Sulfate | 8.5 |
| Cocamidopropyl Betaine | 3.0 |
| Composition A | 2.5 |
| PEG-7 Glyceryl Cocoate | 2.5 |
| Cocamide DEA | 2.5 |
| Glycerin | 2.0 |
| Sorbitan Sesquicaprylate | 1.5 |
| C12-15 Pareth-12 | 1.2 |
| Sodium Benzoate | 1.0 |
| Potassium Sorbate | 1.0 |
| Citric Acid | ad pH 5.5 |
| PEG-200 Hydrogenated Glyceryl Palmate | 1.0 |
| Sodium Lactate | 1.0 |
| Sodium Chloride | 0.7 |
| Tetrasodium Glutamate Diacetate | 0.2 |
| Linalool | 0.1 |
| Limonene | 0.1 |
| Citronellol | 0.1 |
| Dyes | q. s. |

### Example formulation 88: Soap

| *Ingredient* | *%* |
|---|---|
| Aqua | ad 100 |
| Cocamidopropyl Betaine | 5.0 |
| Glycolipids | 2.5 |
| Composition A | 4.0 |
| Glycerin | 2.7 |
| PEG-7 Glyceryl Cocoate | 2.5 |
| Simmondsia Chinensis (Jojoba) Seed Oil | 2.5 |
| Glyceryl Glucoside | 2.3 |
| Mel | 2.0 |
| Sodium Laureth Sulfate | 3.0 |
| Sodium Chloride | 2.1 |
| Citric Acid | ad pH 5.0 |
| PEG-40 Hydrogenated Castor Oil | 1.8 |
| PEG-120 Methyl Glucose Dioleate | 1.7 |
| Trisodium EDTA | 1.0 |
| Glycol Distearate | 1.0 |
| Polyquaternium-10 | 0.3 |
| Laureth-10 | 0.7 |
| Benzophenone-4 | 0.5 |
| Cocamide MEA | 0.8 |
| Maltodextrin | 0.5 |
| Sodium Benzoate | 0.7 |
| Sodium Salicylate | 0.6 |
| Benzoic Acid | 0.4 |
| Linalool | 0.1 |
| Parfum, Dyes | q. s. |

### Example formulation 89: Caring Liquid Soap

| *Ingredient* | *%* |
|---|---|
| Aqua | ad 100 |
| Aloe Barbadensis Leaf Juice | 5.0 |
| Ammonium Lauryl Sulfate | 5.0 |
| Coco Glucoside | 4.0 |
| Cocamidopropyl Betaine | 4.0 |
| Sodium Cocoamphoacetate | 1.5 |
| Composition A | 1.5 |
| Glycerin | 1.5 |
| Sodium Chloride | 1.5 |
| Citric Acid | ad pH 5.5 |
| Sodium Benzoate | 1.0 |
| Sodium Salicylate | 0.5 |
| Methylisothiazolinone | 0.3 |
| Linalool | 0.1 |
| Butylphenyl Methylpropional | 0.2 |
| Limonene | 0.1 |
| Dyes | q. s. |

### Example formulation 90: Cream Soap

| *Ingredient* | *%* |
|---|---|
| Aqua | ad 100 |
| Alcohol | 5.0 |
| Coco Glucoside | 5.0 |
| Glycerin | 5.0 |
| Composition A | 2.5 |
| Disodium Cocoyl Glutamate | 2.0 |
| Xanthan Gum | 1.5 |
| Citric Acid | ad pH 5.5 |
| Malva Sylvestris Leaf Extract | 1.0 |
| Sodium Lauroyl Lactylate | 1.0 |
| Glyceryl Oleate | 1.0 |
| Sodium Cocoyl Glutamate | 0.7 |
| Linalool | 0.1 |
| Limonene | 0.1 |
| Dyes | q. s. |

### Example formulation 91: Liquid Soap

| *Ingredient* | *%* |
|---|---|
| Aqua | ad 100 |
| Sodium Laureth Sulfate | 5.0 |
| Sodium Chloride | 3.5 |
| Cocamidopropyl Betaine | 3.5 |
| Coco Glucoside | 3.0 |
| Composition A | 2.0 |
| Glyceryl Oleate | 1.0 |
| Hibiscus Sabdariffa Flower Extract | 1.0 |
| Alcohol | 1.0 |
| Sodium Lactate | 1.0 |
| Citric Acid | ad pH 5.0 |
| Sodium Benzoate | 0.7 |
| Potassium Sorbate | 0.1 |
| Parfum, Dyes | q. s. |

### Example formulation 92: Liquid Soap

| *Ingredient* | *%* |
|---|---|
| Aqua | ad 100 |
| Glycerin | 7.0 |
| Alcohol | 4.0 |
| Sodium Coco Sulfate | 3.0 |
| Lauryl Glucoside | 2.5 |
| Composition A | 2.0 |
| Xanthan Gum | 1.5 |
| Mangifera Indica (Mango) Fruit Extract | 0.7 |
| Limonene | 0.1 |
| Linalool | 0.1 |
| Dyes | q. s. |

### Example formulation 93: Liquid Soap

| *Ingredient* | *%* |
|---|---|
| Aqua | ad 100 |
| Sodium Lauryl Sulfate | 6.0 |
| Cocamidopropyl Betaine | 3.0 |
| Composition A | 3.0 |
| Triethylcitrate; Caprylyl Glycol; Benzoic Acid | 1.0 |
| Lactic Acid | ad pH 5.0 |
| Chamomilla Recutita Extract | 0.5 |
| Sodium Chloride | 0.9 |
| Sodium Lactate | 0.7 |
| Styrene/Acrylates Copolymer | 0.5 |
| Glycerin | 0.5 |
| Polyquaternium-7 | 0.3 |
| Hexyl Cinnamal | 0.1 |
| Sorbitol | 0.2 |
| Trisodium Ethylenediamine Disuccinate | 0.2 |
| Propylene Glycol | 0.3 |
| PEG-40 Hydrogenated Castor Oil | 0.9 |
| 1,2-Hexandiol | 0.5 |
| Parfum, Dyes | q. s. |

### Example formulation 94: Cream Soap

| *Ingredient* | *%* |
|---|---|
| Aqua | ad 100 |
| Sodium Laureth Sulfate | 8.0 |
| Cocamidopropyl Betaine | 3.0 |
| Sodium Chloride | 2.6 |
| Composition A | 2.5 |
| Polyquaternium-7 | 0.4 |
| Glycerin | 0.9 |
| Glycol Distearate | 0.7 |
| Laureth-4 | 0.5 |
| Laureth-2 | 0.8 |
| PEG-55 Propylene Glycol Oleate | 0.4 |
| Propylene Glycol | 0.5 |
| Citric Acid | ad pH 5.5 |
| Hexyl Cinnamal | 0.1 |
| Benzyl Salicylate | 0.1 |
| Linalool | 0.1 |
| Limonene | 0.1 |
| Butylphenyl Methylpropional | 0.1 |
| Sodium Benzoate | 0.1 |
| Dyes | q. s. |

### Example formulation 95: Jelly Soap

| *Ingredient* | *%* |
|---|---|
| Glycerin | 50.0 |
| Decyl Glucoside | 5.0 |
| Glycolipids | 3.0 |
| Composition A | 2.5 |
| Chondrus Crispus Powder | 2.0 |
| Methylpropanediol | 1.0 |
| Caprylyl Glycol | 1.0 |
| Phenylpropanol | 0.5 |
| Parfum | 0.2 |
| Helianthus Annuus (Sunflower) Seed Oil | 0.2 |
| Hydrogenated Coconut Oil | 0.2 |
| Olea Europea (Olive) Fruti Oil | 0.2 |
| Oryza Sativa (Rice) Bran Oil | 0.2 |
| Vitis Vinifera (Grape) Seed Oil | 0.1 |
| Prunus Amygdalus Dulcis (Sweet Almond) Oil | 0.1 |
| Aqua | ad 100 |
| Sodium Benzoate | 0.1 |
| Citric Acid | 0.1 |

### Example formulation 96: Soap

| *Ingredient* | *%* |
|---|---|
| Talc | 65.0 |
| Composition A | 10.0 |
| Corn (Zea Mays) Flour | 9.0 |
| Glycerin | 7.0 |
| Sodium Laureth Sulfate | 4.5 |
| Citrus Aurantium Bergamia Fruit Oil | 3.5 |
| Benzyl Benzoate | 0.5 |
| Butylphenyl Methylpropional | 0.2 |
| Parfum | 0.2 |

### Example formulation 97: Soap

| *Ingredient* | % |
|---|---|
| Aqua | ad 100 |
| Composition A | 6.0 |
| Glycolipids | 5.0 |
| Propylene Glycol | 5.0 |
| Helianthus Annuus Seed Oil | 4.0 |
| Cocos Nucifera Oil | 4.0 |
| Titanium Dioxide | 0.2 |
| Sodium Hydroxide | 0.2 |
| Sodium Coco-sulfate | 0.2 |
| Parfum | 0.1 |
| Sodium Chloride | 0.1 |
| L-limonene | 0.1 |

### Example formulation 98: Painting Soap

| *Ingredient* | % |
|---|---|
| Aqua | ad 100 |
| Mica | 7.0 |
| Glycine Soja Oil | 5.0 |
| Glycerin | 4.0 |
| Alcohol | 3.0 |
| Cocamidopropyl Betaine | 3.0 |
| Composition A | 2.0 |
| Cetearyl Alcohol | 1.5 |
| Myristyl Alcohol | 1.0 |
| Glyceryl Stearate Citrate | 1.0 |
| Parfum | 0.4 |
| Xanthan Gum | 0.3 |
| Helianthus Annuus Seed Oil | 0.2 |
| Simmondsia Chinensis Seed Oil | 0.2 |
| Rubus Idaeus Fruit Extract | 0.2 |
| Tocopherol | 0.1 |

### Example formulation 99: Painting Soap

| *Ingredient* | % |
|---|---|
| Aqua | ad 100 |
| Glycerin | 10.0 |
| Zea Starch | 9.0 |
| Lauryl Glucoside | 4.0 |
| Methylpropanediol | 3.0 |
| Stearyl Alcohol | 3.0 |
| Cocamidopropyl Betaine | 2.0 |
| Composition A | 2.0 |
| Glycine Soja Oil | 1.0 |
| Tocopherol | 0.5 |
| Coco-Glucoside | 0.4 |
| Panthenol | 0.2 |
| Glycerin Oleate | 0.2 |
| Caprylyl Glycol | 0.2 |
| Phenlypropanol | 0.2 |
| Sodium Hydroxide | 0.1 |
| Sodium Chloride | 0.1 |
| Xanthan Gum | 0.1 |
| Citric Acid | 0.1 |
| Parfum | 0.1 |
| Beta Carotene | 0.1 |
| Dyes | q. s. |

### Bath

### Example formulation 100: Bath

| *Ingredient* | % |
|---|---|
| Aqua | ad 100 |
| PEG-80 Sorbitan Laurate | 4.0 |
| Cocamidopropyl Betaine | 4.0 |
| Sodium Laureth Sulfate | 4.0 |
| Composition A | 2.0 |
| Polysorbate 20 | 2.0 |
| Sodium Chloride | 1.5 |
| Citric Acid | ad pH 5.0 |
| Sodium Lauroamphoacetate | 1.0 |
| PEG-150 Distearate | 1.0 |
| Disodium Lauroamphodiacetate | 1.0 |
| Sodium Benzoate | 0.5 |
| Parfum, Dyes | q. s. |

### Example formulation 101: Creme Oil Bath

| *Ingredient* | % |
|---|---|
| Aqua | ad 100 |
| Sodium Laureth Sulfate | 5.0 |
| Cocamidopropyl Betaine | 3.0 |
| Decyl Glucoside | 2.8 |
| Composition A | 2.5 |
| Glycerin | 2.5 |
| Glyceryl Glucoside | 2.0 |
| Diamond Powder | 2.0 |
| Helianthus Annuus (Sunflower) Seed Oil | 1.7 |
| Sodium Chloride | 1.5 |
| Glycol Distearate | 1.5 |
| PEG-40 Hydrogenated Castor Oil | 1.5 |
| Citric Acid | ad pH 5.5 |
| PEG-90 Glyceryl Isostearate | 1.2 |
| Laureth-4 | 1.0 |
| PEG-200 Hydrogenated Glyceryl Palmate | 1.0 |
| PEG-7 Glyceryl Cocoate | 1.0 |
| Benzophenone-4 | 0.9 |
| Laureth-2 | 0.7 |
| Sodium Benzoate | 0.7 |
| Butylphenyl Methylpropional | 0.1 |
| Linalool | 0.1 |
| Limonene | 0.1 |
| alpha-lsomethyl lonone | 0.1 |
| Dyes | q. s. |

### Example formulation 102: Relaxing Good Sleep Bath

| *Ingredient* | % |
|---|---|
| Aqua | ad 100 |
| Cocamidopropyl Betaine | 3.0 |
| Coco Glucoside | 2.5 |
| Sodium Chloride | 2.5 |
| Disodium Lauryl Sulfosuccinate | 2.0 |
| Composition A | 2.0 |
| Glycerin | 1.7 |
| Sodium PCA | 0.5 |
| Glyceryl Oleate | 0.4 |
| Sodium Hydroxide | 0.3 |
| Citric Acid | ad pH 5.5 |
| Linalool | 0.1 |
| Limonene | 0.1 |
| Geraniol | 0.1 |
| Dyes | q. s. |

### Example formulation 103: Pampering Oil Bath

| *Ingredient* | % |
|---|---|
| Aqua | ad 100 |
| Glycine Soja Oil | 20.0 |
| Composition A | 5.0 |
| Polyglyceryl-3 Palmitate | 4.5 |
| Glyceryl Caprylate | 1.5 |
| Simmondsia Chinensis Oil | 1.5 |
| Prunus Amygdalus Dulcis (Sweet Almond) Oil | 1.0 |
| Triticum Vulgare Germ Oil | 1.0 |
| Tocopherol | 0.2 |
| Limonene | 0.1 |
| Linalool | 0.1 |
| p-Anisic Acid | 0.1 |
| Dyes | q. s. |

### Example formulation 104: Aroma Bath

| *Ingredient* | % |
|---|---|
| Aqua | ad 100 |
| Alcohol | 5.0 |
| Coco Glucoside | 5.0 |
| Sodium Coco Sulfate | 4.0 |
| Composition A | 2.5 |
| Xanthan Gum | 1.9 |
| Glycerin | 1.8 |
| Sodium Cocoyl Glutamate | 1.5 |
| Disodium Cocoyl Glutamate | 1.5 |
| Melissa Officinalis Extract | 1.0 |
| Lavandula Angustifolia Oil | 1.0 |
| Linalool | 0.1 |
| Limonene | 0.1 |
| Geraniol | 0.1 |
| Dyes | q. s. |

### Example formulation 105: Bath Bomb

| *Ingredient* | % |
|---|---|
| Sodium Bicarbonate | 70.0 |
| Citric Acid | 8.0 |
| Citrus Nobilis Oil | 6.0 |
| Potassium Tartrate | 6.0 |
| Sodium Laureth Sulfate | 4.0 |
| Composition A | 4.0 |
| Betaine | 1.5 |
| Parfum | 0.5 |
| Dyes | q. s. |

### Example formulation 106: Bath Bomb

| *Ingredient* | % |
|---|---|
| Sodium Bicarbonate | 67.0 |
| Citric Acid | 9.0 |
| Zea Mays Starch | 8.0 |
| Butyrospermum Parkii Butter | 5.0 |
| Sodium Lauryl Sulfoacetate | 4.0 |
| Composition A | 4.0 |
| Theobroma Cacao Seed Butter | 2.0 |
| Theobroma Cacao (Cocoa) Fruit Powder | 1.0 |
| Parfum, Dyes | q.s. |

### Example formulation 107: Bath Salt

| *Ingredient* | % |
|---|---|
| Maris Sal | ad 100 |
| Parfum | 2.0 |
| Glycolipids | 1.0 |
| Composition A | 1.0 |
| Silica | 0.5 |
| Simmondsia Chinensis Seed Oil | 0.1 |
| Preservative, Dyes | q. s. |

### Example formulation 108: Bath Salt

| *Ingredient* | % |
|---|---|
| Maris Sal | ad 100 |
| Rosmarinus Officinalis Leaf Oil | 3.0 |
| Parfum | 1.5 |
| Polysorbate 20 | 1.5 |
| Composition A | 1.5 |
| Preservative, Dyes | q. s. |

### x-in-1

### Example formulation 109: Shower Gel and Shampoo

| *Ingredient* | % |
|---|---|
| Aqua | ad 100 |
| Coco Glucoside | 5.0 |
| Sodium Coco Sulfate | 3.0 |
| Cocamidopropyl Hydroxysultaine | 2.5 |
| Composition A | 2.0 |
| Lauryl Glucoside | 2.0 |
| Glyceryl Oleate | 1.8 |
| Inulin | 1.5 |
| Lecithin | 1.5 |
| Polyquaternium-39 | 1.5 |
| Citric Acid | ad pH 5.0 |
| Styrene/Acrylates Copolymer | 1.0 |
| Sodium Chloride | 1.0 |
| Denatonium Benzoate | 0.5 |
| Hydrogenated Palm Glycerides Citrate | 0.7 |
| Tocopherol | 0.3 |
| Sodium Benzoate | 0.7 |
| Phenoxyethanol | 0.3 |
| Parfum, Dyes | q. s. |

### Example formulation 110: Shower, Shampoo and Shave

| *Ingredient* | *%* |
|---|---|
| Aqua | ad 100 |
| Cocamidopropyl Betaine | 5.0 |
| Sodium Laureth Sulfate | 4.0 |
| PEG-7 Glyceryl Cocoate | 4.0 |
| Sodium Chloride | 3.5 |
| Composition A | 2.0 |
| PEG-3 Distearate | 3.0 |
| Glycerin | 2.5 |
| Glyceryl Glucoside | 1.2 |
| Helianthus Annuus (Sunflower) Seed Oil | 1.0 |
| Persea Gratissima (Avocado) Oil | 1.0 |
| Polyquaternium-7 | 1.0 |
| PEG-90m | 1.0 |
| PEG-40 Hydrogenated Castor Oil | 1.0 |
| Citric Acid | ad pH 5.0 |
| Benzophenone-4 | 0.5 |
| PEG-200 Hydrogenated Glyceryl Palmate | 0.5 |
| Silica | 0.7 |
| Sodium Benzoate | 0.6 |
| Methylparaben | 0.4 |
| Propylparaben | 0.4 |
| Limonene | 0.1 |
| Butylphenyl Methylpropional | 0.1 |
| Benzyl Alcohol | 0.1 |
| Dyes | q. s. |

### Example formulation 111: 2-in-1 Body Wash and Shampoo

| *Ingredient* | *%* |
|---|---|
| Aqua | ad 100 |
| Sodium Laureth Sulfate | 8.5 |
| Glycerin | 2.5 |
| Sodium Cocoyl Isethionate | 2.5 |
| Ammonium Lauryl Sulfate | 2.5 |
| Lauryl Betaine | 2.5 |
| Lauryl Glucoside | 1.5 |
| Sodium Chloride | 1.0 |
| Composition A | 1.0 |
| Polyquaternium-39 | 0.5 |
| Polyquaternium-7 | 0.5 |
| Panthenol | 0.1 |
| Citric Acid | ad pH 5.5 |
| Sodium Citrate | 0.2 |
| Propylene Glycol | 0.3 |
| PEG-55 Propylene Glycol Oleate | 0.7 |
| Styrene/Acrylates Copolymer | 0.2 |
| Phenoxyethanol | 0.7 |
| Sodium Benzoate | 0.3 |
| Tetrasodium EDTA | 0.1 |
| Parfum, Dyes | q. s. |

### Example formulation 112: 2-in-1 Bubble Bath and Wash

| *Ingredient* | *%* |
|---|---|
| Aqua | ad 100 |
| Cocamidopropyl Betaine | 4.0 |
| Glycerin | 4.0 |
| Sodium Lauroamphodiacetate | 2.0 |
| Sodium Chloride | 2.0 |
| Coco Glucoside | 1.5 |
| Hydroxypropyl Starch Phosphate | 1.0 |
| Disodium Lauroamphodiacetate | 1.0 |
| Composition A | 1.0 |
| Lauric Acid | 1.0 |
| Acrylates/C10-30 Alkyl Acrylate Crosspolymer | 1.0 |
| Polyquaternium-7 | 1.0 |
| Styrene/Acrylates Copolymer | 0.5 |
| Citric Acid | ad pH 5.5 |
| Sodium Hydroxide | 0.3 |
| Sodium Benzoate | 0.2 |
| Benzoic Acid | 0.1 |
| Parfum, Dyes | q. s. |

### Example formulation 113: Body and Face Wash

| *Ingredient* | *%* |
|---|---|
| Aqua | ad 100 |
| Sodium Laureth Sulfate | 4.5 |
| Petrolatum | 3.0 |
| Acrylates Copolymer | 2.5 |
| Cocamide MEA | 2.5 |
| Composition A | 2.0 |
| PPG-9 | 2.0 |
| Guar hydroxypropyltrimonium chloride | 0.2 |
| Menthol | 0.2 |
| DMDM Hydantoin | 0.1 |
| Tetrasodium EDTA | 0.2 |
| Methylisothiazolinone | 0.1 |
| BHT | 0.1 |
| Parfum, Dyes | q. s. |

### Example formulation 114: Hair and Body Shampoo

| *Ingredient* | *%* |
|---|---|
| Aqua | ad 100 |
| Sodium Laureth Sulfate | 10.0 |
| Sodium Chloride | 4.0 |
| Glycerin | 3.5 |
| Coco-Betaine | 2.0 |
| Composition A | 2.0 |
| Niacinamide | 0.1 |
| Saccharum Officinarum (Sugar Cane) Extract | 1.0 |
| Sodium Benzoate | 1.0 |
| Sodium Hydroxide | 0.3 |
| PPG-5-Ceteth-20 | 1.0 |
| Polyquaternium-10 | 0.7 |
| Salicylic Acid | 0.5 |
| Limonene | 0.1 |
| Linalool | 0.2 |
| Benzyl Salicylate | 0.1 |
| Pyrus Malus (Apple Fruit) Extract | 0.3 |
| Pyridoxine HCl | 0.2 |
| Citric Acid | ad pH 5.0 |
| Taurine | 0.1 |
| Dyes | q. s. |

### Example formulation 115: After-Sun Hair and Body Shampoo

| *Ingredient* | *%* |
|---|---|
| Aqua | ad 100 |
| Sodium Laureth Sulfate | 7.0 |
| Cocamidopropyl Betaine | 5.0 |
| PEG-18 Glyceryl Oleate/Cocoate | 2.5 |
| Composition A | 2.0 |
| Glycol Distearate | 2.0 |
| Coco Glucoside | 2.0 |
| Laureth-4 | 1.5 |
| Sodium Chloride | 0.9 |
| Panthenol | 0.1 |
| Sodium PCA | 0.7 |
| Polyquaternium-10 | 0.3 |
| Octyldodecyl PCA | 0.2 |
| Glycerin | 0.9 |
| PPG-9 | 0.7 |
| Citric Acid | ad pH 5.5 |
| Tetrasodium EDTA | 0.3 |
| Quaternium-80 | 0.2 |
| Propylene Glycol | 0.3 |
| Isopropyl Alcohol | 0.3 |
| Sodium Benzoate | 0.7 |
| Limonene | 0.1 |
| Linalool | 0.1 |
| Benzyl Salicylate | 0.1 |
| Butylphenyl Methylpropional | 0.1 |
| Ceramide NP | 0.05 |
| Dyes | q. s. |

### Example formulation 116: Hair and Body Shampoo & Shower Gel

| *Ingredient* | % |
|---|---|
| Aqua | ad 100 |
| Sodium Laureth Sulfate | 7.0 |
| Sodium Chloride | 3.8 |
| Cocamidopropyl Betaine | 3.5 |
| Composition A | 3.0 |
| Panthenol | 0.3 |
| Hydrolyzed Keratin | 0.7 |
| Taurine | 0.5 |
| Simmondsia Chinensis Seed Oil | 0.5 |
| Disodium Cocoamphodiacetate | 0.5 |
| Citric Acid | ad pH 5.0 |
| Sodium Benzoate | 0.7 |
| Cocamide MEA | 0.4 |
| PEG-7 Glyceryl Cocoate | 0.3 |
| Polyquaternium-10 | 0.2 |
| Propylene Glycol | 0.3 |
| PEG-40 Hydrogenated Castor Oil | 0.4 |
| Linalool | 0.2 |
| Geraniol | 0.1 |
| Butylphenyl Methylpropional | 0.1 |
| Dyes | q. s. |

### Facial Cleansing-Example formulationen

### Example formulation 117: Eye Make-Up Remover

| *Ingredient* | % |
|---|---|
| Aqua | ad 100 |
| Butylene Glycol | 6.0 |
| Propanediol | 2.0 |
| Decyl Glucoside | 1.0 |
| Composition A | 1.0 |
| Glycerin | 1.0 |
| Aloe Barbadensis Leaf Extract | 0.5 |
| Citric Acid | ad pH 7 |
| Bioflavonoids | 0.1 |
| Pantolactone | 0.1 |

### Example formulation 118: Eye Make-Up Remover

| *Ingredient* | % |
|---|---|
| Aqua | ad 100 |
| Cyclopentasiloxane | 8.0 |
| Glycerin | 6.0 |
| Isohexadecane | 6.0 |
| Butylene Glycol | 4.0 |
| Panthenol | 3.0 |
| Composition A | 3.0 |
| Sodium Chloride | 2.0 |
| Disodium EDTA | 1.0 |
| Sodium Hydroxide | 0.2 |
| Citric Acid | ad pH 7 |
| Phenoxyethanol | 0.2 |
| Methylparaben | 0.1 |

### Example formulation 119: Micellar Lotion

| *Ingredient* | % |
|---|---|
| Aqua | ad 100 |
| Poloxamer 124 | 2.0 |
| Composition A | 2.0 |
| Glyceryl Glucoside | 1.5 |
| Glycerin | 1.0 |
| Polyglyceryl-10 Laurate | 1.0 |
| 1,2-Hexandiol | 1.0 |
| Decyl Glucoside | 0.5 |
| Arginine HCl | 0.3 |
| Sodium Cocoamphoacetate | 0.5 |
| Trisodium EDTA | 0.2 |
| Citric Acid | ad pH 6.5 |
| Phenoxyethanol | 0.1 |
| Sodium Hyaluronate | 0.05 |

### Example formulation 120: Waterproof Eye Make-Up Removal

| *Ingredient* | % |
|---|---|
| Aqua | ad 100 |
| Aloe Barbadensis Leaf Juice | 5.0 |
| Sorbitol | 3.0 |
| Glycerin | 3.0 |
| Panthenol | 2.2 |
| Sodium Lauryl Glucose Carboxylate | 2.0 |
| Biosaccharide Gum-1 | 1.5 |
| Lauryl Glucoside | 1.0 |
| Composition A | 0.5 |
| Allantoin | 0.2 |
| Phenoxyethanol | 0.2 |
| Sodium Benzoate | 0.2 |
| Sorbic Acid | q.s. |
| Citric Acid | ad pH 6 |

### Example formulation 121: Waterproof Eye Make-Up Removal

| *Ingredient* | % |
|---|---|
| Aqua | ad 100 |
| Isododecane | 6.0 |
| Isopropyl Palmitate | 4.0 |
| Dimethicone | 3.0 |
| Sodium Ascorbyl Phosphate | 2.0 |
| Sodium Chloride | 2.0 |
| Composition A | 1.5 |
| Caprylyl/Capryl Glucoside | 1.5 |
| Trisodium EDTA | 1.0 |
| Citric Acid | ad pH 7 |
| Sodium Hydroxide | 0.2 |
| Phenoxyethanol | 0.2 |
| Benzethonium Chloride | 0.2 |

### Example formulation 122: Micellar Gel

| *Ingredient* | % |
|---|---|
| Aqua | ad 100 |
| Propanediol | 5.0 |
| Composition A | 3.0 |
| Glycerin, Aqua, Sodium Levulinate, Sodium Anisate | 3.0 |
| Polyglyceryl-4 Caprate | 2.0 |
| Xanthan Gum | 1.5 |
| Lactobacillus Ferment | 0.05 |
| Lactic Acid | ad pH 5.5 |

### Example formulation 123: Micellar Gel

| *Ingredient* | % |
|---|---|
| Aqua | ad 100 |
| Propylene Glycol | 4.0 |
| Glycerin | 4.0 |
| Poloxamer 124 | 2.5 |
| Composition A | 2.5 |
| 1,2-Hexandiol | 2.0 |
| Pentylene Glycol | 1.0 |
| Acrylates/C10-30 Alkyl Acrylate Crosspolymer | 0.5 |
| Sodium Hyaluronate | 0.2 |
| Trisodium EDTA | 0.1 |
| Sodium Hydroxide | ad pH 6 |
| Cetrimonium Chloride | 0.1 |
| Phenoxyethanol | 0.1 |

### Example formulation 124: Micellar Make-Up Remover

| *Ingredient* | *%* |
|---|---|
| Aqua | ad 100 |
| Aloe Barbadensis Leaf Juice | 4.0 |
| Caprylyl/Capryl Glucoside | 2.0 |
| Glycolipids | 1.5 |
| Composition A | 1.5 |
| Betaine | 1.0 |
| Glycerin | 1.0 |
| Benzyl Alcohol, Caprylyl Glycol, Benzoic Acid | 1.0 |
| Decyl Glucoside | 0.5 |
| Parfum | q. s. |
| Citric Acid | ad pH 7.0 |

### Example formulation 125: Facial Cleansing Foam

| *Ingredient* | *%* |
|---|---|
| Aqua | ad 100 |
| Glycine Soja Oil | 5.0 |
| Coco-Glucoside | 4.0 |
| Glycerin | 3.5 |
| Composition A | 3.0 |
| Alcohol Denat. | 2.0 |
| Sodium Coco-Sulfate | 1.5 |
| Sodium/Disodium Cocoyl Glutamate | 1.0 |
| Glycolipids | 0.8 |
| Glyceryl Oleate | 0.5 |
| Hamamelis Virginiana Leaf Extract | 0.5 |
| Tocopherol | 0.1 |
| Helianthus Annuus Seed Oil | 0.1 |
| Lactic Acid | ad pH 6.5 |
| Preservative | q. s. |

### Example formulation 126: Facial cleansing Foam

| *Ingredient* | *%* |
|---|---|
| Aqua | ad 100 |
| Sodium/Disodium Cocoyl Glutamate | 4.0 |
| Composition A | 4.0 |
| Sodium Lauryl Sulfate | 3.0 |
| Parfum | 1.0 |
| Benzyl Alcohol, Caprylyl Glycol, Benzoic Acid | 1.0 |
| Glycerin | 0.8 |
| Sodium Lauryl Glucose Carboxylate | 0.7 |
| Sodium Cottonseedamphoacetate | 0.7 |
| Lauryl Glucoside | 0.5 |
| Citric Acid | ad pH 7.0 |
| Capryloyl Glycine | 0.2 |
| Sodium Hydroxide | 0.2 |
| 1,2-Hexandiol | 0.2 |
| Allantoin | 0.1 |
| Hydroxypropyl Guar Hydroxypropyltrimonium Chloride | 0.1 |
| Tetrasodium EDTA | 0.1 |
| Dehydroacetic Acid | 0.1 |
| Sorbic Acid | 0.1 |
| Tropolone | 0.1 |

### Example formulation 127: Cleansing Foam

| *Ingredient* | *%* |
|---|---|
| Aqua | ad 100 |
| Coco-Glucoside | 2.0 |
| Glycerin | 1.5 |
| Sodium Coco-Sulfate | 1.5 |
| Glyceryl Oleate | 1.0 |
| Decyl Glucoside | 0.5 |
| Sodium Benzoate | 0.5 |
| Citric Acid | ad pH 5.0 |
| Panthenol | 0.4 |
| Composition A | 0.2 |
| Albumen | 0.1 |
| Potassium Sorbate | 0.1 |
| Tocopherol | 0.1 |
| Hydrogenated Palm Glycerides Citrate | 0.1 |

### Example formulation 128: Cleansing Foam

| *Ingredient* | *%* |
|---|---|
| Aqua | ad 100 |
| Aloe Barbadensis Leaf Juice | 28.0 |
| Glycerin | 7.0 |
| Decyl Glucoside | 5.0 |
| Ammonium Lauryl Sulfate | 2.0 |
| Benzyl Alcohol, Caprylyl Glocol, Benzoic Acid | 2.0 |
| Parfum | 0.8 |
| Composition A | 0.1 |
| Phytic Acid | 0.1 |
| Sodium Hydroxide | ad pH 7.0 |
| Gluconolactone | 0.1 |

### Example formulation 129: Cleansing Foam

| *Ingredient* | *%* |
|---|---|
| Aqua | ad 100 |
| Glycerin | 3.0 |
| Decyl Glucoside | 3.0 |
| Glycolipids | 2.0 |
| Pentylene Glycol | 2.0 |
| Composition A | 1.0 |
| Citric Acid | ad pH 6.0 |
| Papain | 0.1 |

### Example formulation 130: Pump Foam for Facial Cleansing

| *Ingredient* | *%* |
|---|---|
| Aqua | ad 100 |
| Sorbitol | 9.0 |
| Glycerin | 5.0 |
| Decyl Glucoside | 4.0 |
| Disodium Cocoyl Glutamate | 4.0 |
| Glyceryl Glucoside | 2.0 |
| Composition A | 2.0 |
| Propylene Glycol | 2.0 |
| Prunus Amygdalus Dulcis Oil | 2.0 |
| PEG-40 Hydrogenated Castor Oil | 1.5 |
| Sodium Sulfate | 0.5 |
| Citric Acid | ad pH |
| Polyquaternium-10 | 0.5 |
| Sodium Benzoate | 0.2 |
| Aminopeptidase | 0.1 |
| Panthenol | 0.1 |

### Example formulation 131: Pump Foam for Facial Cleansing

| *Ingredient* | *%* |
|---|---|
| Aqua | ad 100 |
| Sucrose | 12.0 |
| Glycerin | 6.0 |
| Rosa Damascena Flower Water | 5.0 |
| Coco-Glucoside | 4.5 |
| Composition A | 2.5 |
| Aqua, Sodium Levulinate, Potassium Sorbate | 2.0 |
| Sodium Cocoyl Glutamate | 3.0 |
| Polyglyceryl-4 Caprate | 3.0 |
| Betaine | 1.0 |
| Citric Acid | ad pH 5.0 |
| Parfum | 0.8 |
| Sodium Chloride | 0.5 |

### Example formulation 132: Micellar Water

| *Ingredient* | *%* |
|---|---|
| Aqua | ad 100 |
| PEG-6 Caprylic/capric Glycerides | 3.0 |
| Polysorbate 20 | 2.0 |
| Composition A | 0.8 |
| Butylene Glycol | 0.5 |
| PEG-8 | 0.4 |
| Poloxamer 407 | 0.4 |
| Disodium EDTA | 0.3 |
| Citric Acid | ad pH 6.5 |
| BHT | 0.2 |
| Sodium Benzoate | 0.2 |

### Example formulation 133: Refreshing Micellar Water

| *Ingredient* | *%* |
|---|---|
| Aqua | ad 100 |
| Methylpropanediol | 3.0 |
| Propylene Glycol | 2.5 |
| Glycerin | 2.5 |
| Sodium Cocoamphoacetate | 2.0 |
| Glycolipids | 1.5 |
| Parfum | 0.8 |
| Composition A | 0.5 |
| Sodium Salicylate | 0.5 |
| Sodium Benzoate | 0.5 |
| Citric Acid | ad pH |
| Sodium Chloride | 0.2 |
| Tetrasodium EDTA | 0.1 |

### Example formulation 134: Oil-infused Micellar Water

| *Ingredient* | *%* |
|---|---|
| Aqua | ad 100 |
| Isododecane | 7.5 |
| Dimethicone | 7.5 |
| Dicaprylyl Ether | 5.0 |
| Hexylene Glycol | 1.4 |
| Propanediol | 1.0 |
| Composition A | 1.0 |
| Glycolipids | 1.0 |
| Sodium Chloride | 1.0 |
| Trisiloxane | 0.8 |
| Alcohol Denat. | 0.8 |
| Glycerin | 0.8 |
| Propylene Glycol Dicaprylate/Dicaprate | 0.8 |
| Disodium EDTA | 0.5 |
| Hydroxyethylcellulose | 0.5 |
| Phenoxyethanol, Benzoic Acid | 0.5 |
| Decyl Glucoside | 0.5 |
| Caprylyl Glycol | 0.5 |
| Sodium Citrate | 0.3 |
| Citric Acid | ad pH 6 |
| Panthenol | 0.2 |
| Benzophenone-3 | 0.1 |
| Tocopheryl Acetate | 0.1 |
| Tocopherol | 0.1 |
| Zea Mays Oil | 0.1 |
| Chlorphenesin | 0.1 |

### Example formulation 135: Facial Tonic

| *Ingredient* | *%* |
|---|---|
| Aqua | ad 100 |
| Glycerin. | 2.5 |
| Propanediol | 2.0 |
| Arginine | 1.0 |
| Composition A | 1.0 |
| Salicylic Acid | 1.0 |
| Coco-Betaine | 1.0 |
| Caprylyl/Capryl Glucoside | 0.8 |
| Disodium Cocoyl Glutamate | 0.8 |
| Xanthan Gum | 0.5 |
| Phytic Acid | 0.5 |
| Sodium Chloride | 0.5 |
| Sodium Cocoyl Glutamate | 0.4 |
| Citric Acid | ad pH 5.5 |

### Example formulation 136: Facial Tonic

| *Ingredient* | *%* |
|---|---|
| Aqua | ad 100 |
| Glycerin | 5.0 |
| Caprylyl/Capryl Glucoside | 2.0 |
| Composition A | 1.5 |
| Glyceryl Caprylate | 1.5 |
| Parfum | 1.0 |
| Sodium Levulinate | 1.0 |
| Sodium Anisate | 1.0 |
| Sodium Salicylate | 1.0 |
| Sodium Cocoyl Glutamate | 0.1 |
| Polyglyceryl-6 Oleate | 0.1 |
| Disodium Cocoyl Glutamate | 0.1 |
| Citric Acid | ad pH 5.5 |
| Sodium Surfactin | 0.05 |

### Example formulation 137: Facial Tonic

| *Ingredient* | *%* |
|---|---|
| Aqua | ad 100 |
| PEG-6 Caprylic/Capric Glycerides | 4.0 |
| Composition A | 3.0 |
| Fructooligosaccharides | 1.0 |
| Mannitol | 1.0 |
| Xylitol | 1.0 |
| Propylene Glycol | 1.0 |
| Cetrimonium Bromide | 1.0 |
| Disodium EDTA | 0.4 |
| Rhamnose | 0.1 |

### Example formulation 138: Micellar Water

| *Ingredient* | *%* |
|---|---|
| Aqua | ad 100 |
| Glycerin | 5.0 |
| Polyglyceryl-4 Caprate | 5.0 |
| Betaine | 3.0 |
| Composition A | 2.0 |
| Sodium Levulinate | 2.0 |
| Lactobacillus Ferment | 0.2 |
| Sodium Benzoate | 0.2 |
| Sodium Hydroxide | 0.2 |
| Parfum | 0.1 |
| Lactic Acid | ad pH 5.8 |

### Example formulation 139: Micellar Water

| *Ingredient* | *%* |
|---|---|
| Aqua | ad 100 |
| Glycerin | 6.0 |
| Poloxamer 184 | 2.5 |
| Phenoxyethanol | 1.0 |
| PEG-40 Hydrogenated Castor Oil | 1.0 |
| Lactobacillus Extract, Propylene Glycol | 0.8 |
| Composition A | 0.5 |
| Parfum | 0.5 |
| Citric Acid | ad pH 6.4 |
| Sodium Hydroxide | 0.2 |
| Disodium EDTA | 0.1 |

### Example formulation 140: Micellar Water

| *Ingredient* | *%* |
|---|---|
| Aqua | ad 100 |
| Composition A | 5.0 |
| PEG-40 Hydrogenated Castor Oil | 2.5 |
| Glycerin | 2.5 |
| Prunus Amygdalus Dulcis Oil | 1.0 |
| Sorbitol | 1.0 |
| Decyl Glucoside | 1.0 |
| 1,2-Hexandiol | 0.1 |
| Glyceryl Glucoside | 0.5 |
| Poloxamer 124 | 0.5 |
| Propylene Glycol | 0.5 |
| Disodium Cocoyl Glutamate | 0.5 |
| Sodium Chloride | 0.2 |
| Trisodium EDTA | 0.2 |
| Polyquaternium-10 | 0.2 |
| Citric Acid | ad pH 6.2 |
| Sodium Acetate | 0.1 |
| Phenoxyethanol | 0.1 |

### Example formulation 141: Micellar Water

| *Ingredient* | *%* |
|---|---|
| Aqua | ad 100 |
| Composition A | 5.0 |
| Glycerin | 4.0 |
| Coco-Glucoside | 2.0 |
| Polysorbate 80 | 0.2 |
| Calendula Officinalis Flower Extract | 2.0 |
| Caprylyl Glycol | 1.5 |
| Citric Acid | ad pH |
| Sodium Benzoate | 0.9 |
| Parfum | 0.6 |
| Isoleucine | 0.2 |
| Panthenol | 0.2 |
| PPG-1-PEG-9 Lauryl Glycol Ether | 0.2 |
| Propylene Glycol | 0.2 |
| Phytosphingosine | 0.1 |
| Sodium Chloride | 0.2 |

### Example formulation 142: Micellar Water

| *Ingredient* | *%* |
|---|---|
| Aqua | ad 100 |
| Hexylene Glycol | 1.4 |
| Glycerin | 1.2 |
| Composition A | 1.0 |
| Poloxamer 184 | 1.0 |
| Disodium Cocoamphodiacetate | 1.0 |
| Disodium EDTA | 0.5 |
| Polyaminopropyl Biguanide | 0.2 |
| Lactic Acid | ad pH 5.4 |

### Example formulation 143: Micellar Water

| *Ingredient* | *%* |
|---|---|
| Aqua | ad 100 |
| Glycerin | 5.0 |
| Alcohol Denat. | 2.0 |
| Sodium Lactate | 2.0 |
| Sodium PCA | 1.0 |
| Composition A | 0.5 |
| Parfum | 0.3 |
| Aloe Barbadensis Leaf Juice | 0.3 |
| Cocoyl Glutamic Acid | 0.2 |
| Sodium Cocoyl Glutamate | 0.2 |
| Glycolipids | 0.1 |
| Citric Acid | 0.1 |
| Lactic Acid | 0.1 |
| Sodium Hyaluronate | 0.1 |
| Phytosphingosine HCl | 0.1 |
| Benzyl Alcohol | 0.1 |

### Example formulation 144: Face Cleanser and Scrub

| *Ingredient* | *%* |
|---|---|
| Aqua | ad 100 |
| Sodium Coco-Sulfate | 4.0 |
| Composition A | 2.0 |
| Glycerin | 2.0 |
| Sodium Cocoyl Glycinate | 1.5 |
| Silica | 1.5 |
| Acrylates Copolymer | 0.8 |
| PEG-7 Glyceryl Cocoate | 0.7 |
| Sodium Chloride | 0.5 |
| Polysorbate 20 | 0.5 |
| Cocamidopropyl Betaine | 0.5 |
| Panthenol | 0.2 |
| Parfum | 0.2 |
| Charcoal Powder | 0.2 |
| Salix Alba (Willow) Bark Extract | 0.2 |
| Glycol Distearate | 0.1 |
| Lecithin | 0.1 |
| Laureth-4 | 0.1 |
| Citric Acid | ad pH 5.6 |
| Lactic Acid | 0.1 |
| Tocopherol | 0.1 |
| Ascorbyl Palmitate | 0.1 |
| Disodium EDTA | 0.1 |
| Phenoxyethanol | 0.1 |
| Ethylhexylglycerin | 0.1 |
| Sodium Benzoate | 0.1 |
| Benzyl Alcohol | 0.1 |
| Potassium Sorbate | 0.1 |

### Microemulsions

### Example formulation 145: Microemulsion for Make-Up Removal

| *Ingredient* | *%* |
|---|---|
| Water | ad 100 |
| 1,3-Butanediol | 15.0 |
| Polyglyceryl-4 Caprate | 6.0 |
| Polyglyceryl-3 Caprate | 6.0 |
| Composition A | 6.0 |
| Glycerin | 6.0 |
| Isohexadecane | 3.0 |
| Sodium Benzoate | 0.8 |
| Citric Acid | ad pH 5.0 |

### Example formulation 146: Microemulsion for Make-Up Removal

| *Ingredient* | *%* |
|---|---|
| Water | ad 100 |
| 1,3-Butanediol | 7.5 |
| Polyglyceryl-4 Caprate | 5.5 |
| Glycolipids | 3.0 |
| Composition A | 1.5 |
| Glycerin | 5.0 |
| Sodium Benzoate | 0.8 |
| Citric Acid | Ad pH 5.0 |

### Toothpaste

### Example formulation 147: Toothpaste

| *Ingredient* | *%* |
|---|---|
| Aqua | ad 100 |
| Glycerin | 29.0 |
| Sodium Metaphosphate | 12.0 |
| Hydrated Silica (Hydrated) | 10.0 |
| Xylitol | 10.0 |
| Composition A | 1.0 |
| Olaflur | 0.13 |
| Cocamidopropyl Betaine | 0.5 |
| Propylene Glycol | 1.0 |
| Chamomilla Recutita Flower Extract | 0.4 |
| Alcohol Denat | 0.5 |
| Panthenol | 0.1 |
| Sodium Fluoride | 0.1 |
| Hydroxyethylcellulose | 1.0 |
| Titanium Dioxide | 0.4 |
| Aroma | 1.0 |
| Citronellol | 0.0 |
| Eucalyptol | 0.2 |
| Eugenol | 0.1 |
| Menthol | 0.15 |
| Dyes, pH adjuster, preservative | q. s. |

### Example formulation 148: Toothpaste

| *Ingredient* | *%* |
|---|---|
| Aqua | ad 100 |
| Hydrated Silica (Hydrated) | 22.0 |
| Sorbitol | 30.0 |
| Hydroxyethylcellulose | 1.5 |
| Composition A | 1.0 |
| Olaflur | 0.19 |
| Aroma | 1.0 |
| Limonene | 0.1 |
| Titanium Dioxide | 0.3 |
| Saccharin | 0.2 |
| Dyes, pH adjuster, preservative | q. s. |

### Example formulation 149: Toothpaste

| *Ingredient* | *%* |
|---|---|
| Sorbitol | 45.0 |
| Aqua | ad 100 |
| Hydrated Silica (Hydrated) | 18.0 |
| Sodium Lauryl Sulfate | 1.05 |
| Composition A | 0.75 |
| PEG-32 | 1.0 |
| Flavor | 1.0 |
| Cellulose Gum | 0.8 |
| Cocamidopropyl Betaine | 0.35 |
| Sodium Saccharin | 0.2 |
| Sodium Fluoride | 0.23 |
| Zinc Sulfate | 0.3 |
| Mica | 0.5 |
| Dyes solution (1% FD&C Blue No.1) | 0.02 |
| Titanium Dioxide | 0.3 |
| Eugenol | 0.2 |
| pH adjuster, preservative | q. s. |

### Example formulation 150: Toothpaste

| *Ingredient* | *%* |
|---|---|
| Glycerin | 35.0 |
| Hydrated Silica (Hydrated) | 22.0 |
| Aqua | ad 100 |
| Sodium Bicarbonate | 20.0 |
| PEG-12 | 3.0 |
| Composition A | 1.5 |
| Sodium Lauryl Sulfate | 1.0 |
| Flavor | 1.0 |
| Sodium Hydroxide | 0.9 |
| Cellulose Gum | 0.9 |
| Chondrus Crispus | 0.5 |
| Sodium Saccharin | 0.1 |
| Calcium Peroxide | 0.1 |
| Titanium Dioxide | 0.4 |
| Sodium monofluorophosphate | 0.76 |
| PH adjuster, preservative | q. s. |

### Example formulation 151: Toothpaste

| *Ingredient* | *%* |
|---|---|
| Aqua | ad 100 |
| Glycerin | 33.0 |
| Hydrated Silica (Hydrated) | 24.0 |
| Composition A | 1.5 |
| Cellulose Gum | 0.8 |
| Aroma | 0.9 |
| Sodium Fluoride | 0.23 |
| Sodium Saccharin | 0.2 |
| Commiphora Myrrha Oil | 0.05 |
| Salvia Officinalis Oil | 0.05 |
| Mentha Piperita Oil | 0.2 |
| Chamomilla Recutita Flower Extract | 0.2 |
| Limonene | 0.1 |
| Titanium Dioxide | 0.3 |
| Dyes solution (1% FD&C Blue No.1) | 0.05 |
| pH adjuster, preservative | q. s. |

### Example formulation 152: Toothpaste

| *Ingredient* | *%* |
|---|---|
| Glycerin | 27.0 |
| Hydrated Silica (Hydrated) | 21.0 |
| Sodium Hexametaphosphate | 13.0 |
| Aqua | ad 100 |
| PEG-6 | 5.0 |
| Flavor | 1.0 |
| Trisodium Phosphate | 1.0 |
| Sodium Lauryl Sulfate | 1.05 |
| Composition A | 0.35 |
| Chondrus Crispus | 0.7 |
| Cocamidopropyl Betaine | 0.1 |
| Sodium Saccharin | 0.2 |
| Polyethylene Glycol | 0.5 |
| Xanthan Gum | 0.7 |
| Sucralose | 0.5 |
| Mica | 0.3 |
| Titanium Dioxide | 0.5 |
| Sodium Fluoride | 0.32 |
| Dyes, pH adjuster, preservative | q. s. |

### Example formulation 153: Toothpaste

| *Ingredient* | *%* |
|---|---|
| Aqua | ad 100 |
| Sorbitol | 29.0 |
| Hydrated Silica (Hydrated) | 22.0 |
| Disodium Pyrophosphate | 5.0 |
| Composition A | 1.0 |
| Glycolipids | 1.0 |
| Cellulose Gum | 1.0 |
| Aroma | 0.9 |
| Sodium Bicarbonate | 0.8 |
| Carbomer | 0.8 |
| Sodium Fluoride | 0.32 |
| Sodium Saccharin | 0.1 |
| Xanthan Gum | 0.5 |
| Titanium Dioxide | 0.5 |
| Limonene | 0.2 |
| Dyes, pH adjuster, preservative | q. s. |

### Example formulation 154: Toothpaste

| *Ingredient* | *%* |
|---|---|
| Aqua | ad 100 |
| Hydrated Silica (Hydrated) | 21.0 |
| Sorbitol | 19.0 |
| Glycerin | 15.0 |
| Propylene Glycol | 5.0 |
| Tetrapotassium Pyrophosphate | 5.0 |
| Pentasodium Triphosphate | 5.0 |
| Sodium C14-16 Olefin Sulfonate | 0.5 |
| Glycolipids | 0.5 |
| Composition A | 0.5 |
| Disodium Pyrophosphate | 0.7 |
| Aroma | 0.3 |
| Titanium Dioxide | 0.4 |
| Xanthan Gum | 0.7 |
| Sodium Fluoride | 0.32 |
| Sodium Saccharin | 0.15 |
| Allantoin | 0.2 |
| Chamomilla Recutita Flower Extract | 0.1 |
| Salvia Officinalis Leaf Extract | 0.2 |
| Zinc Chloride | 0.3 |
| Dyes solution (1% FD&C Blue No.1) | 0.05 |
| pH adjuster, preservative | q. s. |

### Example formulation 155: Toothpaste

| *Ingredient* | *%* |
|---|---|
| Aqua | ad 100 |
| Hydrated Silica (Hydrated) | 21.0 |
| Xylitol | 10.0 |
| Sorbitol | 10.0 |
| Composition A | 1.5 |
| Betaine | 0.5 |
| Aloe Barbadensis Leaf Juice Powder | 0.3 |
| Xanthan Gum | 0.5 |
| Zinc Gluconate | 0.5 |
| Sodium Coco-sulfate | 0.5 |
| Mentha Spicata Crispa Herb Oil | 0.1 |
| Calendula Officinalis Flower Extract | 0.2 |
| Chamomilla Recutita Flower Extract | 0.2 |
| L-limonene | 0.1 |
| Mentha Arvensis Leaf Oil | 0.1 |
| Sodium Fluoride | 0.32 |
| Titanium Dioxide | 0.4 |
| PH adjuster, preservative | q. s. |

### Example formulation 156: Toothpaste

| *Ingredient* | *%* |
|---|---|
| Aqua | ad 100 |
| Hydroxyapatite | 20.0 |
| Propylene Glycol | 10.0 |
| Glycerin | 10.0 |
| Sorbitol | 10.0 |
| Tetrapotassium Pyrophosphate | 7.0 |
| Silica | 7.0 |
| Composition A | 1.0 |
| Aroma | 1.0 |
| Cellulose Gum | 1.0 |
| Sodium C14-16 Olefin Sulfonate | 1.0 |
| Sodium Fluoride | 0.32 |
| Sodium Cocoyl Isethionate | 0.5 |
| Sodium Saccharin | 0.15 |
| Limonene | 0.2 |
| Titanium Dioxide | 0.4 |
| Dyes, pH adjuster, preservative | q. s. |

### Example formulation 157: Toothpaste

| *Ingredient* | *%* |
|---|---|
| Aqua | ad 100 |
| Hydrogenated Starch Hydrolysate (Hydrogenated, Hydrolysed) | 20.0 |
| Hydrated Silica (Hydrated), | 15.0 |
| Glycerin | 15.0 |
| Alumina | 10.0 |
| Sodium Lauryl Sulfate | 1.05 |
| PEG-12 | 1.0 |
| Composition A | 0.75 |
| Perlite | 0.5 |
| Pentapotassium Triphosphate | 0.8 |
| Pentasodium Triphosphate | 0.8 |
| Sodium Monofluorophosphate | 0.76 |
| Cellulose Gum | 0.9 |
| Sodium Saccharin | 0.2 |
| Cocamidopropyl Betaine | 0.35 |
| Aroma | 1.0 |
| Limonene | 0.1 |
| Dyes solution (1% FD&C Blue No.1) | 0.05 |
| Titanium Dioxide | 0.3 |
| pH adjuster, preservative | q. s. |

### Example formulation 158: Toothpaste

| *Ingredient* | *%* |
|---|---|
| Dicalcium Phosphate Dihydrate (Dihydrate) | 25.0 |
| Aqua | ad 100 |
| Sorbitol | 20.0 |
| Glycerin | 20.0 |
| Methyl Salicylate | 1.0 |
| Sodium Lauryl Sulfate | 0.9 |
| Composition A | 0.9 |
| Titanium Dioxide | 0.5 |
| Chondrus Crispus | 0.8 |
| Cellulose Gum | 0.5 |
| Sodium Silicate | 0.3 |
| Hydrated Silica (Hydrated) | 1.0 |
| Thymol | 0.1 |
| Sodium Saccharin | 0.2 |
| Sodium Hydroxide | 0.5 |
| Menthol | 0.3 |
| Dyes solution (1% FD&C Blue No.1) | 0.02 |
| pH adjuster, preservative | q. s. |

### Example formulation 159: Toothpaste

| *Ingredient* | *%* |
|---|---|
| Aqua | ad 100 |
| Hydrated Silica (Hydrated) | 21.0 |
| Sorbitol | 21.0 |
| Glycerin | 18.0 |
| PEG-6 | 5.0 |
| Composition A | 1.5 |
| Xanthan Gum | 0.8 |
| Flavor | 0.7 |
| Titanium Dioxide | 0.5 |
| Sodium Citrate | 0.4 |
| Sodium Fluoride | 0.32 |
| Zinc Chloride | 0.5 |
| Sodium Saccharin | 0.2 |
| Chondrus Crispus | 0.7 |
| Limonene | 0.1 |
| Dyes solution (1% FD&C Blue No.1) | 0.04 |
| pH adjuster, preservative | q. s. |

### Example formulation 160: Toothpaste

| *Ingredient* | *%* |
|---|---|
| Aqua | ad 100 |
| Sorbitol | 36.0 |
| Hydrated Silica (Hydrated), | 18.0 |
| Glycerin | 18.0 |
| Potassium Nitrate | 1.0 |
| Aroma | 1.0 |
| Cocamidopropyl Betaine | 1.0 |
| Composition A | 1.0 |
| Xanthan Gum | 0.7 |
| Sodium Saccharin | 0.2 |
| Sodium Fluoride | 0.32 |
| Mica | 0.4 |
| Titanium Dioxide | 0.4 |
| Sodium Hydroxide | 0.5 |
| Limonene | 0.1 |
| Eugenol | 0.1 |
| Dyes, pH adjuster, preservative | q. s. |

### Example formulation 161: Toothpaste

| *Ingredient* | *%* |
|---|---|
| Glycerin | 33.0 |
| PEG-8 | 5.0 |
| Hydrated Silica (Hydrated), | 5.0 |
| Calcium Sodium Phosphosilicate | 3.0 |
| Cocamidopropyl Betaine | 1.0 |
| Composition A | 1.0 |
| Sodium Methyl Cocoyl Taurate | 0.3 |
| Aroma | 0.8 |
| Titanium Dioxide | 0.5 |
| Carbomer | 0.5 |
| Sodium Saccharin | 0.2 |
| Sodium Fluoride | 0.32 |
| Limonene | 0.2 |
| Dyes, pH adjuster, preservative | q. s. |

### Example formulation 162: Toothpaste

| *Ingredient* | *%* |
|---|---|
| Aqua | ad 100 |
| Sorbitol | 63.0 |
| Hydrated Silica (Hydrated) | 14.0 |
| Silica | 7.0 |
| Composition As | 1.0 |
| Titanium Dioxide | 0.6 |
| Maris Sal | 0.5 |
| Glycolipdis | 0.5 |
| Sodium Cocoyl Glutamate | 0.5 |
| Disodium Cocoyl Glutamate | 0.5 |
| Xanthan Gum | 0.7 |
| Sodium Fluoride | 0.32 |
| Echinacea Purpurea Extract (Extract) | 0.1 |
| Arnica Montana Flower Extract | 0.1 |
| Mentha Piperita Leaf Water | 0.1 |
| Myrtus Communis Leaf Water | 0.1 |
| Glycerin | 1.0 |
| Menthol | 0.2 |
| Aroma | 1.0 |
| L-limonene | 0.2 |
| Dyes, pH adjuster, preservative | q. s. |

### Example formulation 163: Toothpaste

| *Ingredient* | *%* |
|---|---|
| Sorbitol | 33.0 |
| Aqua | ad 100 |
| Hydrated Silica (Hydrated) | 18.0 |
| Glycerin | 15.0 |
| PEG-32 | 4.0 |
| Sodium Lauryl Sulfate | 1.5 |
| Cellulose Gum | 1.0 |
| Composition A | 1.0 |
| Sodium Saccharin | 0.3 |
| Eucalyptol | 0.2 |
| Methyl Salicylate | 0.5 |
| Thymol | 0.1 |
| Phosphoric Acid | 0.3 |
| Menthol | 0.4 |
| Zinc Citrate | 0.5 |
| Sodium Phosphate | 0.2 |
| Xanthan Gum | 0.5 |
| Benzoic Acid | 0.2 |
| Sodium Monofluorophosphate | 0.76 |
| Flavor | 1.0 |
| Dyes solution (1% FD&C Blue No.1) | 0.05 |
| pH adjuster, preservative | q. s. |

### Example formulation 164: Toothpaste

| *Ingredient* | *%* |
|---|---|
| Hydrogenated Starch Hydrolysate (Hydrogenated, Hydrolysed) | 30.0 |
| Aqua | ad 100 |
| Hydrated Silica (Hydrated) | 22.0 |
| PEG-32 | 4.0 |
| Composition A | 1.0 |
| Aroma | 1.0 |
| Cellulose Gum | 1.0 |
| Sodium Fluoride | 0.32 |
| Sodium Saccharin | 0.25 |
| PVM/MA Copolymer | 0.7 |
| Trisodium Phosphate | 0.8 |
| Calcium Aluminum Borosilicate | 0.4 |
| Glycerin | 0.9 |
| Lecithin | 0.2 |
| Tin Oxide | 0.2 |
| Limonene | 0.1 |
| Dyes solution (1% FD&C Blue No.1) | 0.04 |
| Titanium Dioxide | 0.3 |
| pH adjuster, preservative | q. s. |

### Example formulation 165: Toothpaste

| *Ingredient* | *%* |
|---|---|
| Aqua | ad 100 |
| Sorbitol | 50.0 |
| Hydrated Silica (Hydrated), | 14.0 |
| Silica Dimethyl Silylate | 3.5 |
| Hydroxyethylcellulose | 3.5 |
| Composition A | 1.05 |
| PEG-40 Hydrogenated Castor Oil (Hydrogenated) | 0.8 |
| Aroma | 0.9 |
| Sodium Gluconate | 0.4 |
| Limonene | 0.1 |
| PEG-3 tallow aminopropyl amine | 0.5 |
| Olaflur | 0.06 |
| Stannous Fluoride | 0.4 |
| Sodium Saccharin | 0.2 |
| Potassium Hydroxide | 0.4 |
| Hydrochloric Acid | 0.2 |
| Dyes solution (1% FD&C Blue No.1) | 0.04 |
| pH adjuster, preservative | q. s. |

### Example formulation 166: Toothpaste

| *Ingredient* | % |
|---|---|
| Aqua | ad 100 |
| Sorbitol | 45.0 |
| Hydrated Silica (Hydrated) | 21.0 |
| Sodium Lauryl Sulfate | 1.5 |
| Composition A | xx |
| Cellulose Gum | 1.0 |
| Aroma | 1.0 |
| Zinc Citrate | 0.5 |
| Chondrus Crispus Powder (Powdered) | 0.8 |
| Sodium Saccharin | 0.2 |
| Sodium Fluoride | 0.1 |
| Titanium Dioxide | 0.5 |
| Hydroxyethylcellulose | 0.3 |
| Sodium Citrate | 0.2 |
| Stannous Fluoride | 0.4 |
| Dyes, pH adjuster, preservative | q. s. |

### Example formulation 167: Toothpaste

| *Ingredient* | % |
|---|---|
| Sodium Bicarbonate | 35.00 |
| PEG-8 | 5.0 |
| Hydrated Silica (Hydrated | 18.0 |
| Glycerin | 18.0 |
| Composition A | 1.0 |
| Sodium Lauryl Sulfate | 0.5 |
| Sodium Saccharin | 0.5 |
| Aroma | 0.9 |
| Dipotassium Phosphate | 0.8 |
| Sodium Carbonate | 0.5 |
| Sodium Fluoride | 0.32 |
| PEG/PPG-116/66 Copolymer | 0.8 |
| Calcium Peroxide | 0.5 |
| Calcium Hydroxide | 0.4 |
| Dyes solution (1% FD&C Blue No.1) | 0.04 |
| Sodium Silicate | 0.5 |
| Limonene | 0.1 |
| Silica | 0.9 |
| Titanium Dioxide | 0.3 |
| Dyes, pH adjuster, preservative | q. s. |

### Example formulation 168: Toothpaste

| *Ingredient* | % |
|---|---|
| Glycerin | 33.0 |
| Hydrated Silica | 22.0 |
| Aqua | ad 100 |
| Sorbitol | 15.0 |
| Tetrapotassium Pyrophosphate | 3.5 |
| Composition A | 1.5 |
| Titanium Dioxide | 0.4 |
| Sodium Lauroyl Sarcosinate | 0.5 |
| Cellulose Gum | 0.8 |
| Lauryl Glucoside | 0.5 |
| PVP | 0.5 |
| Cocamidopropyl Betaine | 0.5 |
| Xanthan Gum | 0.5 |
| Stannous Fluoride | 0.4 |
| Dyes, pH adjuster, preservative | q. s. |

### Example formulation 169: Toothpaste

| *Ingredient* | % |
|---|---|
| Aqua | ad 100 |
| Hydrated Silica (Hydrated) | 20.0 |
| Sorbitol | 18.0 |
| Xylitol | 14.0 |
| Composition A | 1.2 |
| Propylene Glycol | 1.0 |
| Glycerin | 1.0 |
| Sodium C14-16 Olefin Sulfonate | 0.3 |
| Aroma | 1.0 |
| Xanthan Gum | 0.5 |
| Disodium Phosphate | 0.4 |
| Sodium Fluoride | 0.32 |
| Zinc Chloride | 0.5 |
| Sodium Saccharin | 0.2 |
| Citric Acid | 0.2 |
| Titanium Dioxide | 0.4 |
| Dyes, pH adjuster, preservative | q. s. |

### Example formulation 170: Toothpaste

| *Ingredient* | % |
|---|---|
| Aqua | ad 100 |
| Hydrogenated Starch Hydrolysate (Hydrogenated, Hydrolysed) | 25.0 |
| Hydrated Silica (Hydrated) | 21.0 |
| Hydroxyapatite | 4.5 |
| PEG-32 | 4.5 |
| Composition A | 1.0 |
| Sodium Lauryl Sulfate | 0.5 |
| Aroma | 0.9 |
| Sodium Monofluorophosphate | 0.76 |
| Trisodium Phosphate | 0.5 |
| Cellulose Gum | 1.0 |
| Sodium Saccharin | 0.2 |
| Sodium Hydroxide | 0.3 |
| Limonene | 0.1 |
| Dyes solution (1% FD&C Blue No.1) | 0.05 |
| Titanium Dioxide | 0.4 |
| pH adjuster, preservative | q. s. |

### Example formulation 171: Toothpaste

| *Ingredient* | % |
|---|---|
| Glycerin | 32.0 |
| Hydrated Silica (Hydrated) | 21.0 |
| Aqua | ad 100 |
| Sodium Bicarbonate | 30.0 |
| PEG-12 | 3.0 |
| Composition A | 1.5 |
| Sodium Lauryl Sulfate | 1.0 |
| Flavor | 1.0 |
| Sodium Hydroxide | 1.0 |
| Cellulose Gum | 1.0 |
| Chondrus Crispus | 0.7 |
| Sodium Saccharin | 0.15 |
| Calcium Peroxide | 0.5 |
| Titanium Dioxide | 0.5 |
| Sodium Monofluorophosphate | 0.76 |
| Dyes, pH adjuster, preservative | q. s. |

### Example formulation 172: Toothpaste

| *Ingredient* | % |
|---|---|
| Aqua | ad 100 |
| Sorbitol | 63.0 |
| Hydrated Silica (Hydrated) | 22.0 |
| Propylene Glycol | 1.5 |
| Cellulose Gum | 1.5 |
| Composition A | 1.0 |
| Sodium C 14-16 Olefin Sulfonate | 0.5 |
| Aroma | 0.8 |
| Calcium Glycerophosphate | 0.4 |
| Sodium Fluoride | 0.11 |
| Sodium Saccharin | 0.4 |
| Cocamidopropyl Betaine | 0.5 |
| Citral | 0.1 |
| Geraniol | 0.1 |
| Limonene | 0.1 |
| Linalool | 0.1 |
| Titanium Dioxide | 0.2 |
| Dyes, pH adjuster, preservative | q. s. |

### Example formulation 173: Toothpaste

| *Ingredient* | % |
|---|---|
| Aqua | ad 100 |
| Calcium Carbonate | 30.0 |
| Silica | 7.0 |
| Glycerin | 5.0 |
| Hydrated Silica | 2.5 |
| Composition A | 1.5 |
| Aroma | 1.0 |
| Chondrus Crispus Powder | 0.5 |
| Cellulose Gum | 0.5 |
| Chlorhexidine Gluconate | 0.1 |
| Limonene | 0.15 |
| Sodium Saccharin | 0.15 |
| Titanium Dioxide | 0.1 |
| Sodium Fluoride | 0.32 |
| Dyes, pH adjuster, preservative | q. s. |

### Example formulation 174: Toothpaste

| *Ingredient* | % |
|---|---|
| Aqua | ad 100 |
| Hydrogenated Starch Hydrolysate | 20.0 |
| Hydrated Silica | 15.0 |
| PEG-32 | 4.0 |
| Glycolipids | 1.5 |
| Composition A | 1.0 |
| Propylene Glycol | 0.9 |
| Aroma | 0.9 |
| Sodium Lauryl Sulfat | 0.5 |
| Hydroxyethylcellulose | 0.5 |
| Olaflur | 0.13 |
| Titanium Dioxide | 0.1 |
| Sodium Fluoride | 0.1 |
| Sodium Saccharin | 0.2 |
| Chamomilla Recutita Flower Extract | 0.2 |
| Chlorhexidine Digluconate | 0.1 |
| Limonene | 0.1 |
| Citral | 0.1 |
| Dyes, pH adjuster, preservative | q. s. |

### Example formulation 175: Toothpaste

| *Ingredient* | *%* |
|---|---|
| Aqua | ad 100 |
| Sorbitol | 20.0 |
| Hydrated Silica | 15.0 |
| Silica | 2.0 |
| Composition A | 1.0 |
| Cocamidopropyl Betaine | 0.75 |
| Titanium Dioxide | 0.15 |
| Flavor | 0.8 |
| Linalool | 0.2 |
| Sodium Monofluorophosphate | 0.76 |
| Sodium Fluoride | 0.1 |
| Sodium Saccharin | 0.25 |
| Cellulose Gum | 0.5 |
| Limonene | 0.1 |
| Chlorhexidine Digluconate | 0.12 |
| Dyes, pH adjuster, preservative | q. s. |

### Example formulation 176: Toothpaste

| *Ingredient* | *%* |
|---|---|
| Aqua | ad 100 |
| Hydrated Silica | 15.0 |
| Glycerin | 15.0 |
| Potassium Nitrate | 5.0 |
| Sorbitol | 4.5 |
| Sodium Cocoamphoacetate | 0.5 |
| Composition A | 0.5 |
| PEG-40 Hydrogenated Castor Oil | 0.5 |
| Carboxymethyl Cellulose | 0.5 |
| Titanium Dioxide | 0.1 |
| Sodium Fluoride | 0.32 |
| Sodium Saccharin | 0.2 |
| Aroma | 0.2 |
| Chlorhexidine Digluconate | 0.12 |
| Dyes, pH adjuster, preservative | q. s. |

### Example formulation 177: Toothpaste for Kids

| *Ingredient* | *%* |
|---|---|
| Aqua | ad 100 |
| Sorbitol | 28.0 |
| Hydrated Silica (Hydrated) | 18.0 |
| Hydroxyethylcellulose | 1.5 |
| Titanium Dioxide | 0.5 |
| Cocamidopropyl Betaine | 0.5 |
| Composition A | 0.5 |
| Olaflur | 0.07 |
| Aroma | 0.8 |
| Limonene | 0.1 |
| Sodium Saccharin | 0.2 |
| Hydrochloric Acid | 0.7 |
| Dyes, pH adjuster, preservative | q. s. |

### Example formulation 178: Toothpaste for Children

| *Ingredient* | *%* |
|---|---|
| Sorbitol | 33.0 |
| Aqua | ad 100 |
| Hydrated Silica (Hydrated) | 22.0 |
| Composition A | 1.0 |
| Sodium C14-16 Olefin Sulfonate | 1.0 |
| Aroma | 0.8 |
| Cellulose Gum | 0.8 |
| Calcium Glycerophosphate | 0.5 |
| Olaflur | 0.07 |
| Sodium Fluoride | 0.1 |
| Propylene Glycol | 0.5 |
| Sodium Saccharin | 0.2 |
| Cocamidopropyl Betaine | 0.35 |
| Mica | 0.5 |
| Citral | 0.1 |
| Limonene | 0.1 |
| Linalool | 0.1 |
| Titanium Dioxide | 0.4 |
| Dyes solution (1% FD&C Blue No.1) | 0.05 |
| Olaflur | 0.07 |
| pH adjuster, preservative | q. s. |

### Example formulation 179: Baby Toothpaste

| *Ingredient* | *%* |
|---|---|
| Aqua | ad 100 |
| Hydrogenated Starch Hydrolysate (Hydrogenated, Hydrolysed) | 14.0 |
| Hydrated Silica (Hydrated) | 14.0 |
| PEG-32 | 3.5 |
| Silica | 7.0 |
| Composition A | 1.5 |
| Cellulose Gum | 1.0 |
| Aroma | 1.0 |
| Propylene Glycol | 0.7 |
| PEG-40 Hydrogenated Castor Oil (Hydrogenated), | 0.8 |
| Olaflur | 0.06 |
| Sodium Chloride | 0.2 |
| Sodium Saccharin | 0.15 |
| Tocopheryl Acetate | 0.1 |
| Glycerin | 1.0 |
| Retinyl Palmitate | 0.1 |
| Dyes solution (1% FD&C Blue No.1) | 0.02 |
| pH adjuster, preservative | q. s. |

### Example formulation 180: Toothpaste for Children

| *Ingredient* | *%* |
|---|---|
| Aqua | ad 100 |
| Hydrated Silica (Hydrated) | 22.0 |
| Glycerin | 20.0 |
| Xylitol | 10.0 |
| Composition A | 1.5 |
| Propylene Glycol | 0.7 |
| Xanthan Gum | 0.5 |
| Titanium Dioxide | 0.5 |
| Flavor | 0.9 |
| Sodium Lauroyl Sarcosinate | 0.5 |
| Disodium EDTA | 0.3 |
| Sodium Monofluorophosphate | 0.76 |
| Sodium Chloride | 0.2 |
| Dyes, pH adjuster, preservative | q. s. |

### Example formulation 181: Kids Toothpaste

| *Ingredient* | % |
|---|---|
| Aqua | ad 100 |
| Sorbitol | 40.0 |
| Hydrated Silica (Hydrated) | 17.0 |
| Glycerin | 26.0 |
| Composition A | 1.5 |
| Xanthan Gum | 1.0 |
| Steareth-30 | 1.0 |
| Chondrus Crispus | 1.0 |
| Aroma | 1.0 |
| Disodium Phosphate | 0.5 |
| Sodium Benzoate | 0.2 |
| Amyloglucosidase | 0.1 |
| Citric Acid | 0.3 |
| Zinc Gluconate | 0.5 |
| Glucose Oxidase | 0.1 |
| Sodium Fluoride | 0.22 |
| Sodium Saccharin | 0.15 |
| Potassium Thiocyanate | 0.1 |
| Lactoperoxidase | 0.1 |
| Titanium Dioxide | 0.3 |
| Dyes, pH adjuster, preservative | q. s. |

### Example formulation 182: 2 in 1 Toothpaste + Mouthwash

| *Ingredient* | % |
|---|---|
| Sorbitol | 38.0 |
| Aqua | ad 100 |
| Hydrated Silica (Hydrated) | 21.0 |
| Pentasodium Triphosphate | 5.0 |
| Sodium Lauryl Sulfate | 0.35 |
| Composition A | 1.05 |
| PEG-32 | 1.0 |
| Aroma | 1.0 |
| Alumina | 0.5 |
| Alcohols | 0.7 |
| Xanthan Gum | 0.8 |
| Sodium Fluoride | 0.23 |
| Sodium Saccharin | 0.2 |
| Disodium Phosphate | 0.5 |
| Cocamidopropyl Betaine | 0.35 |
| Zinc Sulfate | 0.5 |
| Trisodium Phosphate | 0.3 |
| Sodium Chloride | 0.4 |
| Sodium Sulfate | 0.2 |
| Limonene | 0.1 |
| Titanium Dioxide | 0.5 |
| Dyes, pH adjuster, preservative | q. s. |

### Example formulation 183: 2 in 1 Toothpaste + Mouthwash

| *Ingredient* | % |
|---|---|
| Sorbitol | 29.0 |
| Aqua | ad 100 |
| Hydrated Silica | 15.0 |
| PEG-32 | 3.0 |
| Sodium Lauryl Sulfate | 0.5 |
| Composition A | 0.5 |
| Glycolipids | 0.5 |
| Aroma | 0.9 |
| Alcohols | 0.7 |
| Xanthan Gum | 0.5 |
| PEG-30 Glyceryl Stearate | 0.5 |
| Sodium Fluoride | 0.05 |
| Sodium Saccharin | 0.7 |
| Disodium Phosphate | 0.3 |
| Cocamidopropyl Betaine | 0.5 |
| Zinc Sulfate | 0.3 |
| Trisodium Phosphate | 0.5 |
| Sodium Chloride | 0.1 |
| Sodium Sulfate | 0.2 |
| Limonene | 0.02 |
| Dyes, pH adjuster, Preservative | q. s. |

### Mouthwash

### Example formulation 184: Mouthrinse without Alcohol

| *Ingredient* | % |
|---|---|
| Aqua | ad 100 |
| Glycerin | 11.0 |
| Propylene Glycol | 7.0 |
| Composition A | 1.0 |
| Poloxamer 407 | 0.75 |
| Aroma | 0.9 |
| Cetylpyridinium Chloride | 0.04 |
| Sodium Fluoride | 0.05 |
| Sodium Saccharin | 0.07 |
| Menthol | 0.1 |
| Dyes, pH adjuster, Preservative | q. s. |

### Example formulation 185: Mouthrinse without Alcohol

| *Ingredient* | % |
|---|---|
| Aqua | ad 100 |
| Sorbitol | 15.0 |
| Glycerin | 7.5 |
| Composition A | 1.0 |
| Xylitol | 1.0 |
| Cellulose Gum | 0.5 |
| Zinc PCA | 0.5 |
| Zinc Hydroxyapatite | 0.05 |
| Aroma | 0.9 |
| Silica | 0.05 |
| Ricinus Communis Seed Oil | 0.1 |
| Ammonium Acryloyldimethyltaurate/VP Copolymer | 0.05 |
| Mentha Arvensis Leaf Oil | 0.01 |
| Sodium Myristoyl Sarcosinate | 0.2 |
| Sodium Methyl Cocoyl Taurate | 0.2 |
| Sodium Saccharin | 0.5 |
| Sodium Fluoride | 0.05 |
| Limonene | 0.1 |
| Dyes, pH adjuster, Preservative | q. s. |

### Example formulation 186: Mouthrinse without Alcohol

| *Ingredient* | % |
|---|---|
| Aqua | ad 100 |
| Sorbitol | 20.0 |
| PEG-40 | 0.5 |
| Composition A | 0.5 |
| Flavor | 0.05 |
| Xanthan Gum | 0.1 |
| Zinc Sulfate | 0.15 |
| Sodium Saccharin | 0.05 |
| Sodium Fluoride | 0.05 |
| Gellan Gum | 0.2 |
| Calcium Aluminum Borosilicate | 0.1 |
| Silica | 0.05 |
| Titanium Dioxide | 0.05 |
| Tin Oxide | 0.07 |
| Eugenol | 0.15 |
| Limonene | 0.05 |
| Linalool | 0.05 |
| Dyes, pH adjuster, Preservative | q. s. |

### Example formulation 187: Mouthrinse without Alcohol

| *Ingredient* | % |
|---|---|
| Aqua | ad 100 |
| Glycerin | 7.5 |
| Composition A | 0.5 |
| Glycolipids | 0.5 |
| Flavor | 0.3 |
| Cetylpyridinium Chloride | 0.04 |
| Sodium Saccharin | 0.05 |
| Sucralose | 0.05 |
| Sodium Fluoride | 0.02 |
| Disodium Phosphate | 0.25 |
| Dyes, pH adjuster, Preservative | q. s. |

### Example formulation 188: Mouthrinse

| *Ingredient* | % |
|---|---|
| Aqua | ad 100 |
| Glycerin | 10.0 |
| Sorbitol | 10.0 |
| Cocamidopropyl Betaine | 1.0 |
| Composition A | 0.5 |
| Potassium Chloride | 0.1 |
| Propylene Glycol | 0.5 |
| Aroma | 0.25 |
| Sodium Fluoride | 0.02 |
| Olaflur | 0.16 |
| Potassium Acesulfame | 0.1 |
| Sodium Chloride | 0.1 |
| Limonene | 0.03 |
| Sodium Sulfate | 0.05 |
| Dyes, pH adjuster, Preservative | q. s. |

### Example Example formulation 189: Mouthrinse without Alcohol

| *Ingredient* | % |
|---|---|
| Aqua | ad 100 |
| Sorbitol | 15.0 |
| Composition A | 1.0 |
| Aloe Barbadensis Leaf Extract | 0.15 |
| Calendula Officinalis Flower Extract | 0.2 |
| Chamomilla Recutita Flower Extract | 0.2 |
| Sodium Fluoride | 0.02 |
| Calcium Glycerophosphate | 0.05 |
| Zinc Gluconate | 0.8 |
| Mentha Spicata Crispa Herb Oil | 0.05 |
| Mentha Arvensis Leaf Oil | 0.05 |
| Menthol | 0.04 |
| Dyes, pH adjuster, Preservative | q. s. |

### Example formulation 190: Mouthrinse without Alcohol

| *Ingredient* | % |
|---|---|
| Aqua | ad 100 |
| Glycerin | 15.0 |
| Sodium Gluconate | 1.5 |
| Composition A | 0.5 |
| PEG-40 Hydrogenated Castor Oil | 0.5 |
| Olaflur | 0.16 |
| Aroma | 0.25 |
| Stannous Chloride | 0.05 |
| Sodium Fluoride | 0.02 |
| Cocamidopropyl Betaine | 0.35 |
| Sodium Saccharin | 0.07 |
| Dyes, pH adjuster, Preservative | q. s. |

### Example formulation 191: Mouthrinse without Alcohol

| *Ingredient* | % |
|---|---|
| Aqua | ad 100 |
| Glycerin | 10.0 |
| Sorbitol | 8.0 |
| Composition A | 1.0 |
| Glycolipids | 0.5 |
| Tetrasodium Pyrophosphate | 0.25 |
| Aroma | 0.04 |
| Limonene | 0.1 |
| Sodium Fluoride | 0.02 |
| Sodium Saccharin | 0.08 |
| Dyes, pH adjuster, Preservative | q. s. |

### Example formulation 192: Mouthrinse without Alcohol

| *Ingredient* | % |
|---|---|
| Aqua | ad 100 |
| Glycerin | 7.5 |
| PEG-60 Hydrogenated Castor Oil | 1.0 |
| Composition A | 0.5 |
| Sodium Citrate | 0.45 |
| Aroma | 0.3 |
| Zinc Chloride | 0.15 |
| Cetylpyridinium Chloride | 0.05 |
| Sodium Saccharin | 0.08 |
| Sodium Fluoride | 0.02 |
| Dyes, pH adjuster, Preservative | q. s. |

### Example formulation 193: Mouthrinse without Alcohol

| *Ingredient* | % |
|---|---|
| Aqua | ad 100 |
| Glycerin | 11.0 |
| Composition A | 2.0 |
| Flavor | 1.0 |
| Cetylpyridinium Chloride | 0.5 |
| Poloxamer 407 | 0.1 |
| Sodium Saccharin | 0.1 |
| Sodium Fluoride | 0.02 |
| Sucralose | 0.05 |
| Mentha Viridis Leaf Oil | 0.1 |
| Dyes, pH adjuster, Preservative | q. s. |

### Example formulation 194: Mouthrinse without Alcohol

| *Ingredient* | % |
|---|---|
| Aqua | ad 100 |
| Glycerin | 12.0 |
| Sorbitol | 8.0 |
| Potassium Nitrate | 0.15 |
| PEG-60 Hydrogenated Castor Oil | 0.5 |
| Composition A | 0.25 |
| Poloxamer 407 | 0.25 |
| Aroma | 0.2 |
| Disodium Phosphate, Sodium Phosphate | 0.25 |
| Sodium Fluoride | 0.05 |
| Sodium Saccharin | 0.08 |
| Dyes, pH adjuster, Preservative | q. s. |

### Example formulation 195: Mouthrinse without Alcohol

| *Ingredient* | % |
|---|---|
| Aqua | ad 100 |
| Sorbitol | 10.0 |
| PEG-40 Hydrogenated Castor Oil | 1.0 |
| Potassium Citrate | 1.0 |
| Composition A | 0.25 |
| Glycerin | 0.9 |
| Aroma | 0.4 |
| Zinc Sulfate | 0.15 |
| Sodium Saccharin | 0.1 |
| Propylene Glycol | 0.3 |
| Citrus Limon Juice | 0.05 |
| Aloe Barbadensis Leaf Extract | 0.1 |
| Limonene | 0.05 |
| Sodium Fluoride | 0.02 |
| Dyes, pH adjuster, Preservative | q. s. |

### Example formulation 196: Mouthrinse without Alcohol

| *Ingredient* | % |
|---|---|
| Aqua | ad 100 |
| Glycerin | 12.0 |
| Composition A | 1.2 |
| Gycolipids | 1.0 |
| Hydrogenated Starch Hydrolysate | 1.0 |
| Xylitol | 1.0 |
| Steareth-30 | 0.2 |
| Amyloglucosidase | 0.5 |
| Allantoin | 0.1 |
| Glucose Oxidase | 0.5 |
| Sodium Fluoride | 0.05 |
| Limonene | 0.1 |
| Dyes, pH adjuster, Preservative | q. s. |

### Example formulation 197: Mouthrinse

| *Ingredient* | % |
|---|---|
| Aqua | ad 100 |
| Glycerin | 11.0 |
| Propylene Glycol | 7.5 |
| Sorbitol | 7.5 |
| Tetrapotassium Pyrophosphate | 1.5 |
| Polysorbate 20 | 0.5 |
| Composition A | 0.5 |
| Tetrasodium Pyrophosphate | 0.75 |
| Zinc Citrate | 0.15 |
| PVM/MA Copolymer | 0.1 |
| Flavor | 0.2 |
| Sodium Fluoride | 0.05 |
| Sodium Saccharin | 0.07 |
| Dyes, pH adjuster, Preservative | q. s. |

### Example formulation 198: Mouthrinse

| *Ingredient* | % |
|---|---|
| Aqua | ad 100 |
| Glycerin | 11.0 |
| Alcohol | 7.5 |
| Sorbitol | 7.5 |
| Composition A | 1.0 |
| Poloxamer 407 | 0.5 |
| Sodium Phosphate, Disodium Phosphate | 0.15 |
| Flavor | 0.9 |
| Cetylpyridinium Chloride | 0.05 |
| Sodium Saccharin | 0.08 |
| Sodium Fluoride | 0.05 |
| Dyes, pH adjuster, Preservative | q. s. |

### Example formulation 199: Mouthrinse

| *Ingredient* | % |
|---|---|
| Aqua | ad 100 |
| Alcohols | 22.0 |
| Sorbitol | 10.0 |
| Flavor | 1.0 |
| Composition A | 1.0 |
| Sodium Saccharin | 0.09 |
| Eucalyptol | 0.1 |
| Thymol | 0.06 |
| Sucralose | 0.06 |
| Sodium Fluoride | 0.05 |
| Menthol | 0.04 |
| Dyes, pH adjuster, Preservative | q. s. |

### Example formulation 200: Mouthrinse

| *Ingredient* | % |
|---|---|
| Aqua | ad 100 |
| Glycerin | 12.0 |
| Xylitol | 7.0 |
| Disodium Phosphate | 1.5 |
| Composition A | 1.0 |
| Cocamidopropyl Betaine | 0.5 |
| Propylene Glycol | 0.5 |
| Stannous Fluoride | 0.09 |
| Aroma | 0.3 |
| Mentha Piperita Leaf Extract | 0.1 |
| Chamomilla Recutita Flower Extract | 0.2 |
| Salvia Officinalis Leaf Extract | 0.25 |
| Commiphora Myrrha Resin Extract | 0.15 |
| Sodium Saccharin | 0.1 |
| Citral | 0.05 |
| Limonene | 0.02 |
| Linalool | 0.02 |
| Dyes, pH adjuster, Preservative | q. s. |

### Example formulation 201: Mouthrinse

| *Ingredient* | *%* |
|---|---|
| Aqua | ad 100 |
| Glycerin | 11.0 |
| Sorbitol | 11.0 |
| Propylene Glycol | 3.0 |
| Disodium Pyrophosphate | 1.5 |
| PEG-40 Hydrogenated Castor Oil | 1.0 |
| Composition A | 0.5 |
| PVM/MA Copolymer | 0.5 |
| Tetrapotassium Pyrophosphate | 0.25 |
| Sodium Levulinate | 0.25 |
| Sodium Anisate | 0.25 |
| Olaflur | 0.16 |
| Aroma | 0.8 |
| Arginine | 0.8 |
| Sodium Saccharin | 0.07 |
| Sodium Fluoride | 0.02 |
| Dyes, pH adjuster, Preservative | q. s. |

### Example formulation 202: Mouthrinse

| *Ingredient* | *%* |
|---|---|
| Aqua | ad 100 |
| Alcohol Denat. | 33.0 |
| Glycerin | 10.0 |
| Sorbitol | 8.0 |
| PEG-40 Hydrogenated Castor Oil | 1.0 |
| Composition A | 1.0 |
| Glycolipids | 0.5 |
| Aroma | 0.6 |
| Menthol | 0.05 |
| Sodium Saccharin | 0.07 |
| Aluminum Lactate | 0.2 |
| Thymol | 0.06 |
| Curcuma Xanthorrhiza Root Extract | 0.05 |
| Sodium Fluoride | 0.02 |
| Dyes, pH adjuster, Preservative | q. s. |

### Example formulation 203: Mouthrinse

| *Ingredient* | *%* |
|---|---|
| Aqua | ad 100 |
| Sorbitol | 12.5 |
| Propylene Glycol | 3.5 |
| Composition A | 0.5 |
| Sodium Lauryl Sulfate | 0.5 |
| Poloxamer 407 | 0.5 |
| Flavor | 0.3 |
| Eucalyptol | 0.08 |
| Methyl Salicylate | 0.1 |
| Thymol | 0.05 |
| Stannous Fluoride | 0.09 |
| Menthol | 0.1 |
| Sodium Saccharin | 0.06 |
| Sucralose | 0.7 |
| Camellia Sinensis Leaf Extract | 0.05 |
| Dyes, pH adjuster, Preservative | q. s. |

### Example formulation 204: Mouthrinse

| *Ingredient* | *%* |
|---|---|
| Aqua | ad 100 |
| Composition A | 1.0 |
| Eucalyptol | 0.9 |
| Zinc Chloride | 0.25 |
| Methyl Salicylate | 0.05 |
| Thymol | 0.05 |
| Levomenthol | 0.07 |
| Stannous Fluoride | 0.09 |
| Dyes, pH adjuster, Preservative | q. s. |

### Example formulation 205: Mouthrinse

| *Ingredient* | *%* |
|---|---|
| Aqua | ad 100 |
| Alcohol Denat. | 15.0 |
| Sorbitol | 5.0 |
| Glycine | 1.0 |
| Composition A | 1.0 |
| Aroma | 0.2 |
| Zinc Sulfate | 0.2 |
| Sodium Fluoride | 0.02 |
| Sodium Saccharin | 0.07 |
| Dyes, pH adjuster, Preservative | q. s. |

### Example formulation 206: Mouthrinse

| *Ingredient* | *%* |
|---|---|
| Aqua | ad 100 |
| Sorbitol | 11.0 |
| Alcohol Denat. | 11.0 |
| Xylitol | 5.0 |
| PEG-40 Hydrogenated Castor Oil | 0.75 |
| Composition A | 0.5 |
| Isopropyl Alcohol | 0.5 |
| Menthol | 0.05 |
| Sodium Citrate | 0.15 |
| Mentha Piperita Herb Oil | 0.25 |
| Sodium Saccharin | 0.08 |
| Methyl Diisopropyl Propionamide | 0.05 |
| Thymol | 0.07 |
| Glycolipids | 0.1 |
| Sodium Fluoride | 0.05 |
| O-cymen-5-ol | 0.05 |
| Cyclodextrin | 0.07 |
| Eugenia Caryophyllus Leaf Oil | 0.1 |
| Propylene Glycol | 0.25 |
| Butylene Glycol | 0.25 |
| Psidium Guajava Leaf Extract | 0.08 |
| Phyllanthus Emblica Fruit Extract | 0.08 |
| Echinacea Purpurea Root Extract | 0.08 |
| Salvia Officinalis Leaf Extract | 0.2 |
| Dyes, pH adjuster, Preservative | q. s. |

### Example formulation 207: Mouthrinse

| *Ingredient* | *%* |
|---|---|
| Aqua | ad 100 |
| Alcohols | 15.0 |
| Sorbitol | 7.0 |
| Sodium Lauroyl Sarcosinate | 1.0 |
| Composition A | 0.25 |
| PEG-40 Hydrogenated Castor Oil | 0.25 |
| Aroma | 0.4 |
| Cetylpyridinium Chloride | 0.04 |
| Sodium Citrate | 0.15 |
| Sodium Saccharin | 0.08 |
| Sodium Fluoride | 0.05 |
| Limonene | 0.05 |
| Dyes, pH adjuster, Preservative | q. s. |

### Example formulation 208: Mouthrinse

| *Ingredient* | % |
|---|---|
| Aqua | ad 100 |
| Alcohol denat. | 18.0 |
| Sorbitol | 8.0 |
| Aroma | 1.0 |
| Poloxamer 407 | 0.9 |
| Methyl salicylate | 0.2 |
| Sodium Saccharin | 0.2 |
| Sodium Fluoride | 0.05 |
| Composition A | 0.9 |
| Chlorhexidine Digluconate | 0.2 |
| PEG-40 Hydrogenated Castor Oil | 0.2 |
| Dyes, pH adjuster, Preservative | q. s. |

### Example formulation 209: Mouthrinse

| *Ingredient* | % |
|---|---|
| Aqua | ad 100 |
| Glycerin | 10.0 |
| Hydrogenated Starch Hydrolysate | 1.0 |
| PVP | 0.5 |
| Composition A | 0.5 |
| Aroma | 0.4 |
| Zinc Acetate | 0.2 |
| Sodium Fluoride | 0.32 |
| Potassium Acesulfame | 0.15 |
| Chlorhexidine Digluconate | 0.2 |
| Dyes, pH adjuster, Preservative | q. s. |

### Example formulation 210: Mouthrinse

| *Ingredient* | % |
|---|---|
| Aqua | ad 100 |
| Glycerin | 12.0 |
| Sorbitol | 7.0 |
| Composition A | 0.75 |
| PEG-40 Hydrogenated Castor Oil | 0.5 |
| Chlorhexidine Gluconate | 0.2 |
| Mentha Piperita Oil | 0.2 |
| Sodium Fluoride | 0.05 |
| Sodium Saccharin | 0.2 |
| Dyes, pH adjuster, Preservative | q. s. |

### Example formulation 211: Mouthrinse

| *Ingredient* | % |
|---|---|
| Aqua | ad 100 |
| Glycerin | 15.0 |
| Macrogolglycerol Hydroxystearate | 7.0 |
| Sorbitol | 7.0 |
| Composition A | 0.5 |
| Cocamidopropyl Betaine | 0.2 |
| Sodium Fluoride | 0.02 |
| Chlorhexidine Digluconate | 0.2 |
| Flavor | 0.5 |
| Dyes, pH adjuster, Preservative | q. s. |

### Example formulation 212: Mouthrinse Concentrate

| *Ingredient* | % |
|---|---|
| Propylene Glycol | 55.0 |
| Aqua | ad 100 |
| Aroma | 2.0 |
| Sodium Saccharin | 0.4 |
| Composition A | 0.75 |
| Polysorbate 20 | 0.25 |
| Commiphora Myrrha Oil | 2.0 |
| Salvia Officinalis Oil | 0.2 |
| Mentha Piperita Oil | 0.2 |
| Chamomilla Recutita Flower Extract | 0.2 |
| Limonene | 0.1 |
| Sodium Fluoride | 0.02 |
| Dyes, pH adjuster, Preservative | q. s. |

### Example formulation 213: Mouthrinse Concentrate

| *Ingredient* | % |
|---|---|
| Propylene Glycol | 45.0 |
| PEG-6 | 5.0 |
| Aqua | ad 100 |
| Composition A | 1.0 |
| Bisabolol | 0.2 |
| Zinc Chloride | 0.25 |
| Cetylpyridinium Chloride | 0.1 |
| Sodium Saccharin | 0.5 |
| Sodium Fluoride | 0.05 |
| Salvia Officinalis Oil | 2.5 |
| Eugenol | 0.2 |
| Limonene | 0.1 |
| Linalool | 0.1 |
| Citronellol | 0.1 |
| Dyes, pH adjuster, Preservative | q. s. |

### Example formulation 214: Mouthrinse Concentrate

| *Ingredient* | % |
|---|---|
| Alcohols | 55.0 |
| Aqua | ad 100 |
| Aroma | 1.5 |
| Composition A | 0.7 |
| Sodium C14-17 Alkyl Sulfonate | 0.7 |
| Mentha Arvensis Leaf Oil | 2.0 |
| Sodium Saccharin | 0.5 |
| Chamomilla Recutita Flower Extract | 0.5 |
| Commiphora Myrrha Resin Extract | 0.2 |
| Salvia Officinalis Leaf Extract | 0.2 |
| Sodium Fluoride | 0.02 |
| Limonene | 0.1 |
| Dyes, pH adjuster, Preservative | q. s. |

### Example formulation 215: Peroxide Mouthrinse

| *Ingredient* | % |
|---|---|
| Aqua | ad 100 |
| Glycerin | 11.0 |
| Alcohol | 5.0 |
| H₂O₂ 35% | 4.30 |
| Polyphosphate | 1.0 |
| Menthol | 0.05 |
| Coolant | 0.02 |
| Flavor | 0.15 |
| Calcium Chloride | 0.03 |
| Composition A | 0.50 |
| Sodium Saccharin | 0.1 |
| Sodium Fluoride | 0.22 |
| Dyes, pH adjuster, Preservative | q. s. |

### Example formulation 216: Fluoride Gel

| *Ingredient* | *%* |
|---|---|
| Aqua | ad 100 |
| Propylene Glycol | 30.0 |
| Hydroxyethylcellulose | 3.0 |
| Saccharin | 1.0 |
| Composition A | 0.5 |
| Pyrus Malus Fruit | 0.2 |
| Mentha Piperita Oil | 0.2 |
| Flavor | 0.5 |
| Aminofluoride Dectafluor | 0.29 |
| Olaflur | 3.03 |
| Sodium Fluoride | 2.21 |
| Dyes, pH adjuster, Preservative | q. s. |

### Example formulation 217: Fluoride Gel

| *Ingredient* | *%* |
|---|---|
| Aqua | ad 100 |
| Sorbitol | 15.00 |
| Silica | 5.0 |
| PEG 600 | 3.5 |
| Tetrapotassium Pyrophosphate | 1.5 |
| Xanthan Gum | 0.5 |
| Sodium Lauryl Sulfate | 0.75 |
| Composition A | 0.75 |
| Aroma | 0.9 |
| Methol | 0.05 |
| Anethol | 0.05 |
| Citronellol | 0.2 |
| Sodium Fluoride | 5.0 |
| Dyes, pH adjuster, Preservative | q. s. |

### Example formulation 218: Dental Floss

| *Ingredient* | *%* |
|---|---|
| PTFE | ad 100 |
| Aroma | 1.0 |
| Acacia Senegal Gum | 0.7 |
| Cera Alba | 0.7 |
| Potassium Acesulfame | 0.5 |
| Sodium Lauryl Sulfate | 0.25 |
| Composition A | 0.25 |
| Glycerin | 0.7 |
| Silica | 0.5 |
| Sodium Fluoride | 0.22 |

### Example formulation 219: Teeth Wipe

| *Ingredient* | *%* |
|---|---|
| Aqua | q. s. 100 |
| Alcohol Denat. | 30.00 |
| Glycerin | 20.00 |
| Stevia | 1.0 |
| Polysorbate 20 | 0.5 |
| Composition A | 0.5 |
| Aroma | 1.0 |
| Dyes, pH adjuster, Preservatives | q. s. |

### Example formulation 220: Chewing Gum

| *Ingredient* | *%* |
|---|---|
| Gum Base | ad 100 |
| Sorbit | 40.0 |
| Isomalt | 9.0 |
| Xylitol | 2.5 |
| Mannit D | 2.5 |
| Aspartame | 0.1 |
| Acesulfam K | 0.05 |
| Emulgum | 0.2 |
| Sorbitol | 12.00 |
| Glycerin | 1.0 |
| Composition A | 1.0 |
| Flavor | 1.0 |
| Sodium Fluoride | 0.05 |
| Dyes, pH adjuster, Preservative | q. s. |

### Example formulation 221: Mouthwash Tabs

| *Ingredient* | *%* |
|---|---|
| Dicalcium Phosphate Anhydrous | ad 100 |
| Sodium Bicarbonate | 35.0 |
| Silica | 7.0 |
| Citric Acid | 4.0 |
| Composition A | 3.0 |
| Sorbitol | 1.0 |
| Glycerin | 1.0 |
| Stevia Rebaudiana Extract (Stevia) | 0.2 |
| Xylitol | 0.5 |
| Aroma | 0.1 |
| Mentha Piperita Oil | 0.1 |
| Dyes, pH adjuster, Preservative | q. s. |

### Example formulation 222: Tooth Tab

| *Ingredient* | *%* |
|---|---|
| Dicalcium Phosphate Anhydrous | ad 100 |
| Potassium Bitartrate | 15.0 |
| Sorbitol | 15.0 |
| Composition A | 6.0 |
| Lauroyl Sarcosine | 0.8 |
| Kaolin | 0.5 |
| Sodium Saccharin | 0.5 |
| Aroma | 0.2 |
| Dyes, pH adjuster, Preservative | q. s. |

### Example formulation 223: Powder Toothpaste

| *Ingredient* | *%* |
|---|---|
| Calcium Carbonate | ad 100 |
| Sorbitol | 15.0 |
| Kaolin | 15.0 |
| Sodium Bicarbonate | 15.0 |
| Composition A | 1.5 |
| Aroma | 1.0 |
| Citrus Limon Peel Oil | 1.0 |
| Stevia Rebaudiana Extract | 0.5 |
| Xylitol | 0.5 |
| Citral | 0.1 |
| Geraniol | 0.1 |
| Limonene | 0.1 |
| Dyes, pH adjuster, Preservative | q. s. |

### Example formulation 224: Cleansing Gel

| Ingredients | % |
|---|---|
| Composition A | 3.0 |
| Capryl/Capramidopropyl Betaine | 6.6 |
| Sodium Cocoamphoacetate | 8.0 |
| Xanthan Gum | 1.5 |
| Water | ad 100 |
| Glycerin (and) Heptylglycerin (and) Sodium Anisate | 3.3 |
| pH | 6.0 |

### Example formulation 225: Clear Conditioning Hair and Body Shampoo

| Ingredients | % |
|---|---|
| Composition A | 5 |
| Cocamidopropyl Betaine | 25 |
| Sodium Cocoyl Isethionate | 4 |
| Sodium Levulinate (and) Potassium Sorbate | 1.5 |
| Aqua | ad 100 |
| pH Value | 6 |

### Example formulation 226: Cleansing Foam

| Ingredients | % |
|---|---|
| Composition A | 3,0 |
| Capryl/Capramidopropyl Betaine | 6.6 |
| Sodium Cocoamphoacetate | 8.0 |
| Aqua | ad 100 |
| pH | 6.0 |

### Example formulation 227: Body Wash

| Ingredients | % |
|---|---|
| Composition A | 5.0 |
| Sodium Cocoyl Glutamate (and) Disodium Cocoyl Glutamate | 5.0 |
| Lauryl Glucoside | 11.0 |
| Cocamidopropyl Betaine | 13.3 |
| Glycerin | 5.0 |
| Sorbityl/Xylityl Pelargonate | 1.0 |
| Water | ad 100 |
| pH-value | 5.5 |

### Example formulation 228: Micellar Water

| Ingredients | % |
|---|---|
| Composition A | 3 |
| Polyglyceryl-6 Caprylate (and) Polyglyceryl-3 Cocoate (and) Polyglyceryl-4 Caprate (and) Polyglyceryl-6 Ricinoleate | 1.5 |
| Polyglyceryl-6 Caprylate (and) Polyglyceryl-4 Caprate | 2 |
| Natural Betaine | 2 |
| Glycerin | 1 |
| Aqua | ad 100 |
| Benzyl Alcohol (and) Caprylyl Glycol (and) Benzoic Acid | 1 |
| Appearance | clear |
| pH vlaue | 6.5 |

### Example formulation 229:: Micellar Water 2.

| Ingredients | % |
|---|---|
| Composition A | 1.0 |
| Polyglyceryl-6 Caprylate (and) Polyglyceryl-3 Cocoate (and) Polyglyceryl-4 Caprate (and) Polyglyceryl-6 Ricinoleate | 1.50 |
| Polyglyceryl-6 Caprylate (and) Polyglyceryl-4 Caprate | 2 |
| Betaine | 2 |
| Glycerin | 1 |
| Aqua | ad 100 |
| Hyaluronic Acid | 0.1 |
| Benzyl Alcohol (and) Caprylyl Glycol (and) Benzoic Acid | 1 |
| Appearance | clear |
| pH vlaue | 6.5 |

### Example formulation 230: Liquid Serum or Toner.

| Ingredients % | % |
|---|---|
| Composition A | 2.6 |
| Pentylene Glycol | 4.0 |
| Glycerin | 4.0 |
| Aqua | ad 100 |
| pH-Wert | 6 |
| Appearance | clear |

### Example formulation 231: Repairing Conditioner.

| Ingredients | % |
|---|---|
| Composition A | 3 |
| Distearoylethyl Dimonium Chloride (and) Cetearyl Alcohol | 3.1 |
| Isoamyl Laurate | 1 |
| Cetearyl Alcohol | 4.4 |
| Glycerin | 2 |
| Aqua | ad 100 |
| Pentylene Glycol | 4 |

### Example formulation 232: Hair Conditioner.

| Ingredients | % |
|---|---|
| Composition A | 6 |
| Bis-(lsostearoyl/Oleoyl Isopropyl) Dimonium Methosulfate | 3.1 |
| Isoamyl Laurate | 1 |
| Cetearyl Alcohol | 4.4 |
| Glycerin | 2 |
| Aqua | ad 100 |
| Pentylene Glycol | 4 |

### Example formulation 233: Milky Creamy Cleanser.

| Ingredients | % |
|---|---|
| Glycerin | 2 |
| Pentylene Glycol | 3 |
| Aqua | ad 100 |
| Xanthan Gum | 0.3 |
| Cetearyl Alcohol | 5 |
| Polyglyceryl-3 Distearate (and) Glyceryl Stearate Citrate | 5 |
| Composition A | 10 |
| pH value | 4.7 |

### Example formulation 234: Creamy Cleanser.

| Ingredients | % |
|---|---|
| Glycerin | 2 |
| Pentylene Glycol | 3 |
| Aqua | ad 100 |
| Xanthan Gum | 0.3 |
| Cetearyl Alcohol | 5 |
| Polyglyceryl-3 Distearate (and) Glyceryl Stearate Citrate | 5 |
| Composition A | 2 |
| pH value | 4.7 |

### Example formulation 235: Body Cleanser.

| Ingredients | % |
|---|---|
| Composition A | 5.0 |
| Sodium Cocoyl Glutamate (and) Disodium Cocoyl Glutamate | 5.0 |
| Lauryl Glucoside | 11.0 |
| Cocamidopropyl Betaine | 13.3 |
| Glycerin | 5.0 |
| Hydroxypropyl Guar (and) Hydroxypropyl Guar Hydroxypropyltrimonium Chloride | 0.2 |
| Aqua | ad 100 |
| pH-value | 6.0 |

### Example formulation 236: Shampoo Formulation.

| Ingredients | % |
|---|---|
| Composition A | 5.0 |
| Capryl/Capramidopropyl Betaine | 5.3 |
| Sodium Coco-Sulfate | 3.9 |
| Coco-Glucoside | 2.2 |
| Lauryl Glucoside | 6.6 |
| Aqua | ad 100 |
| Sodium Levulinate (and) Potassium Sorbate | 1.5 |
| pH-Wert | 5.8 |

### Example formulation 237: Conditioning Shampoo.

| Ingredients | % |
|---|---|
| Composition A | 5.0 |
| Capryl/Capramidopropyl Betaine | 5.3 |
| Sodium Coco-Sulfate | 3.9 |
| Coco-Glucoside | 2.2 |
| Lauryl Glucoside | 6.6 |
| Aqua | ad 100 |
| Sodium Benzoate | 1.5 |
| pH-Wert | 5.5 |

### Example formulation 238: Clear Hydrating Shampoo

| Ingredients | % |
|---|---|
| Composition A | 8.0 |
| Sodium Cocoyl Glutamate (and) Disodium Cocoyl Glutamate | 5.0 |
| Lauryl Glucoside | 11.0 |
| Cocamidopropyl Betaine | 13.3 |
| Glycerin | 5.0 |
| Sorbityl/Xylityl Pelargonate | 2.0 |
| Aqua | ad 100 |
| Hydroxypropyl Guar (and) Hydroxypropyl Guar Hydroxypropyltrimonium Chloride | 0.2 |
| Benzyl Alcohol (and) Caprylyl Glycol (and) Benzoic Acid | 1.0 |
| pH-Wert | 6.0 |

### Example formulation 239: Conditioning Ceramide Shampoo

| Ingredients | % |
|---|---|
| Composition A | 5.0 |
| Sodium Cocoyl Glutamate (and) Disodium Cocoyl Glutamate | 5.0 |
| Lauryl Glucoside | 11.0 |
| Cocamidopropyl Betaine | 13.3 |
| Glycerin | 5.0 |
| Sorbityl/Xylityl Pelargonate | 1.0 |
| Aqua | ad 100 |
| Hydroxypropyl Guar (and) Hydroxypropyl Guar Hydroxypropyltrimonium Chloride | 0.2 |
| Benzyl Alcohol (and) Caprylyl Glycol (and) Benzoic Acid | 0.8 |
| pH-Wert | 5.0 |

### Example formulation 240: Ceramide Cleansing Gel

| Ingredients | % |
|---|---|
| Capryl/Capramidopropyl Betaine | 6.6 |
| Sodium Cocoamphoacetate | 8.0 |
| Composition A | 0.5 |
| Xanthan Gum | 1.5 |
| Aqua | ad 100 |
| pH-Wert | 6 |

### Example formulation 241: Sensitive Shampoo

| Ingredients | % |
|---|---|
| Aqua | ad 100 |
| Coco-Glucoside | 5 |
| Glycerin | 3 |
| Sodium Coco-Sulfate | 3 |
| Sodium Cocoamphoacetate | 3 |
| Citric Acid | 3 |
| Panthenol | 0.5 |
| Composition A | 0.5 |
| Niacinamide | 0.2 |
| Tocopherol | 0.2 |
| Ascorbyl Palmitate | 0.2 |
| Glyceryl Oleate | 0.2 |
| Hydroxypropyl Guar Hydroxypropyltrimonium Chloride | 0.2 |
| Hydrogenated Palm Glycerides Citrate | 0.2 |
| Lecithin | 0.1 |
| Sodium Benzoate | 0.1 |
| Potassium Sorbate | 0.1 |
| Sodium Chloride | 0.1 |
| Sodium Hydroxide | 0.1 |

### Example formulation 242: Anti Dandruff Shampoo

| Ingredients | % |
|---|---|
| Aqua | ad 100 |
| Sodium Laureth Sulfate | 4.5 |
| Sodium Lauryl Sulfate | 4.5 |
| Sodium Chloride | 3 |
| Cocamidopropyl Betaine | 3 |
| Sodium Xylenesulfonate | 3 |
| Glycol Distearate | 3 |
| Sodium Citrate | 1.5 |
| Parfum | 1 |
| Composition A | 0.3 |
| Piroctone Olamine | 0.5 |
| Sodium Benzoate | 0.5 |
| Dimethiconol | 0.5 |
| Citric Acid | 0.5 |
| Sodium Salicylate | 0.5 |
| Guar Hydroxypropyltrimonium Chloride | 0.5 |
| Dimethicone | 0.5 |
| Hexyl Cinnamal | 0.2 |
| Linalool | 0.2 |
| Tetrasodium EDTA | 0.2 |
| Sodium Hydroxide | 0.2 |
| TEA-Dodecylbenzenesulfonate | 0.2 |
| Trideceth-10 | 0.2 |
| Niacinamide | 0.2 |
| Panthenol | 0.1 |
| Tocopheryl Acetate | 0.1 |
| Benzyl Alcohol | 0.1 |
| Triethylene Glycol | 0.1 |
| Propylene Glycol | 0.1 |
| Cl 42090 | 0.1 |
| Cl 17200 | 0.1 |

### Example formulation 243: Classic Shampoo

| Ingredients | % |
|---|---|
| Aqua | ad 100 |
| Sodium Laureth Sulfate | 6 |
| Cocamidopropyl Betaine | 3 |
| Decyl Glucoside | 1.5 |
| Coco-Glucoside | 1.5 |
| Sodium Chloride | 1 |
| Citric Acid | 1 |
| Gossypium Hirsutum Seed Oil | 1 |
| Glycerin | 1 |
| Composition A | 3.0 |
| Guar Hydroxypropyltrimonium Chloride | 0.2 |
| Glyceryl Oleate | 0.2 |
| Glycol Distearate | 0.2 |
| Dicaprylyl Ether | 0.2 |
| Hydrogenated Castor Oil | 0.2 |
| Sodium Benzoate | 0.2 |
| Benzoic Acid | 0.2 |
| Parfum | 0.2 |

### Example formulation 244: Shampoo for Sensitive Scalp

| Ingredients | % |
|---|---|
| Aqua | ad 100 |
| Sodium Laureth Sulfate | 9 |
| Cocamidopropyl Betaine | 1.5 |
| Coco-Glucoside | 1.5 |
| Betaine | 1 |
| Panthenol | 0.5 |
| Erythritol | 0.5 |
| Glyceryl Oleate | 0.5 |
| Sorbitan Caprylate | 0.5 |
| Lactic Acid | 0.5 |
| Piroctone Olamine | 0.5 |
| Composition A | 5.0 |
| Polyquaternium-10 | 0.2 |
| Benzyl Alcohol | 0.2 |
| Sodium Chloride | 0.2 |
| Propylene Glycol | 0.2 |
| Tetrasodium Glutamate Diacetate | 0.2 |
| Glycerin | 0.2 |
| Mentha Aquatica Leaf Extract | 0.1 |
| Sorbitol | 0.1 |
| Tilia Cordata Flower Extract | 0.1 |
| Sodium Benzoate | 0.1 |
| Parfum | 0.1 |
| Cl 15985 | 0.1 |
| Cl 42090. | 0.1 |

### Example formulation 245: Shampoo for Curls

| Ingredients | % |
|---|---|
| Aqua | ad 100 |
| Sodium C14-16 Olefin Sulfonate | 6 |
| Cocamidopropyl Betaine | 3 |
| Decyl Glucoside | 1.5 |
| Coco-Glucoside | 1.5 |
| Glycol Distearate | 1 |
| Cocamide Mea | 1 |
| Parfum | 0.5 |
| Citric Acid | 0.5 |
| Hydroxypropyl Methylcellulose | 0.5 |
| Stearoxypropyl Dimethylamine | 0.5 |
| Composition A | 6.0 |
| Guar Hydroxypropyltrimonium Chloride | 0.2 |
| Polyquaternium-7 | 0.2 |
| Lactic Acid | 0.2 |
| Laureth-16 | 0.2 |
| Propylene Glycol | 0.2 |
| Quaternium-80 | 0.2 |
| Polyquaternium-10 | 0.2 |
| Polyquaternium-52 | 0.2 |
| Sodium Hydroxide | 0.2 |
| Sodium Chloride | 0.2 |
| Stearyl Alcohol | 0.2 |
| Rosa Canina Fruit Oil | 0.1 |
| BHT | 0.1 |
| Tocopherol | 0.1 |
| Sodium Benzoate | 0.1 |
| Methylchloroisothiazolinone | 0.1 |
| Methylisothiazolinone | 0.1 |
| Benzyl Salicylate | 0.1 |
| Linalool | 0.1 |

### Example formulation 246: Repair Shampoo

| Ingredients | % |
|---|---|
| Aqua / Water | ad 100 |
| Sodium Laureth Sulfate | 9 |
| Sodium Chloride | 3 |
| Cocamidopropyl Betaine | 3 |
| Coco-Betaine | 1.5 |
| Dimethicone | 1 |
| Glycol Distearate (F.I.L. C269048/1). | 1.0 |
| Composition A | 7.0 |
| Parfum / Fragrance | 0.2 |
| Ci 77891 / Titanium Dioxide | 0.2 |
| Guar Hydroxypropyltrimonium Chloride | 0.2 |
| Mica | 0.2 |
| Sodium Benzoate | 0.2 |
| Sodium Hydroxide | 0.2 |
| Phenoxyethanol | 0.2 |
| Steareth-6 | 0.2 |
| Acetic Acid | 0.2 |
| PEG-100 Stearate | 0.2 |
| Arginine | 0.2 |
| Trideceth-10 | 0.2 |
| Trideceth-3 | 0.2 |
| Salicylic Acid | 0.2 |
| Limonene | 0.2 |
| Fumaric Acid | 0.2 |
| Linalool | 0.2 |
| Benzyl Alcohol | 0.2 |
| Amodimethicone | 0.2 |
| 2-Oleamido-1 | 0.2 |
| 3-Octadecanediol | 0.2 |
| Alpha-Isomethyl lonone | 0.2 |
| Carbomer | 0.2 |
| Serine | 0.2 |
| Citric Acid | 0.2 |
| Citronellol | 0.2 |
| Hexylene Glycol | 0.2 |
| Hexyl Cinnamal | 0.2 |

### Example formulation 247: Repair Shampoo

| Ingredients | % |
|---|---|
| Aqua | ad 100 |
| Disodium Laureth Sulfosuccinate | 4.5 |
| Cocamidopropyl Hydroxysultaine | 4.5 |
| Cocamidopropyl Betaine | 3 |
| PEG-18 Glyceryl Oleate/Cocoate | 2 |
| Glycol Distearate | 1 |
| 1.2-Hexanediol | 1 |
| Cocamide MEA | 1 |
| C13-15 Alkane | 1 |
| Composition A | 2.0 |
| Parfum [Fragrance] | 0.25 |
| Soy Amino Acids | 0.2 |
| Wheat Amino Acids | 0.2 |
| Avena Sativa (Oat) Peptide | 0.2 |
| Gardenia Taitensis Flower Extract | 0.2 |
| Lycium Barbarum Fruit Extract | 0.2 |
| Malva Sylvestris (Mallow) Extract | 0.2 |
| Juniperus Virginiana Oil | 0.2 |
| Citrus Aurantium Peel Oil | 0.2 |
| Citric Acid | 0.2 |
| Tetramethyl Acetyloctahydronaphthalenes | 0.2 |
| Laureth-10 | 0.2 |
| Guar Hydroxypropyltrimonium Chloride | 0.2 |
| Propylene Glycol | 0.2 |
| Glycerin | 0.2 |
| Ethylhexyl Methoxycinnamate | 0.2 |
| Diethylamino Hydroxybenzoyl Hexyl Benzoate | 0.2 |
| Caramel | 0.2 |
| Linalyl Acetate | 0.2 |
| Methylchloroisothiazolinone | 0.2 |
| Arginine HCl | 0.2 |
| Serine | 0.2 |
| Threonine | 0.2 |
| Methylisothiazolinone | 0.2 |
| Caprylyl Glycol | 0.2 |
| Hexyl Cinnamal | 0.1 |
| Limonene | 0.1 |
| Linalool | 0.1 |

### Example formulation 248: Shampoo for Glossy Hair

| Ingredients | % |
|---|---|
| Water | ad 100 |
| Sodium Coco-Sulfate | 6 |
| Cocamidopropyl Betaine | 3 |
| Decyl Glucoside | 1.5 |
| PCA Glyceryl Oleate | 1 |
| Coco-Glucoside | 1 |
| Sea Salt (Maris Sal) | 0.5 |
| Citric Acid | 0.5 |
| Vitis Vinifera (Grape) Seed Oil* | 0.5 |
| Cetearyl Alcohol | 0.5 |
| Cetearyl Alcohol | 0.5 |
| Glycerin | 0.5 |
| Composition A | 10 |
| Punica Granatum Fruit Extract* | 0.2 |
| Aloe Barbadensis Leaf Juice Powder* | 0.2 |
| Lycium Barbarum Fruit Extract* | 0.2 |
| Vaccinium Macrocarpon (Cranberry) Fruit Extract* | 0.2 |
| Glyceryl Undecylenate | 0.2 |
| Levulinic Acid | 0.2 |
| Guar Hydroxypropyltrimonium Chloride | 0.2 |
| Sodium Levulinate | 0.2 |
| Sodium Cetearyl Sulfate | 0.2 |
| Alcohol** denat. | 0.2 |
| Pisum Sativum (Pea) Peptide | 0.2 |
| Tridecane | 0.2 |
| Lactic Acid | 0.2 |
| Undecane | 0.2 |
| Sodium Sulfate | 0.2 |
| Lactobacillus Ferment | 0.2 |
| Sodium Chloride | 0.2 |
| Tocopherol | 0.2 |
| Helianthus Annuus (Sunflower) Seed Oil | 0.2 |
| Fragrance (Parfum)*** | 0.2 |
| Limonene*** | 0.1 |
| Linalool*** | 0.1 |
| Benzyl Salicylate*** | 0.1 |

### Example formulation 249: Bond Repair Shampoo

| Ingredients | % |
|---|---|
| Wasser | ad 100 |
| Natrium-Lauroylmethyl-lsethionate | 4 |
| Cocamidopropyl-Hydroxysultaine | 3 |
| Decylglucoside | 1.5 |
| Potassium-Cocoyl-Glycinate | 1 |
| Di Sodium Cocoyl Glutamate | 1 |
| Potassium-Cocoate | 1 |
| Sodium-Lauroyl-Sarcosinate | 1 |
| Sodium-Cocoyl-Glutamate | 1 |
| Glycereth-26 | 1 |
| Glycol-Distearate | 1 |
| Acrylat-Copolymer | 1 |
| Fragranc | 0.5 |
| Phenoxyethanol | 0.5 |
| Cocamidopropylamin-Oxide | 0.5 |
| Dinatrium-Laureth-Sulfosuccinate | 0.5 |
| Methyl-Gluceth-20 | 0.5 |
| PEG-120 Methylglucoside-Dioleate | 0.5 |
| Amodimethicone | 0.5 |
| Panthenol | 0.5 |
| Glycerin | 0.5 |
| Helianthus Annuus(Sonnenblumen)-Öl | 0.5 |
| Composition A | 2.5 |
| Polyquaternium-10 | 0.2 |
| Citric Acid | 0.2 |
| Ethylhexylglycerine | 0.2 |
| Sodium-Lauryl-Sulfoacetate | 0.2 |
| Divinyldimethicon/Dimethicon-Copolymer | 0.2 |
| Chlorphenesin | 0.2 |
| Polyquaternium-11 | 0.2 |
| Guar-Hydroxypropyltrimoniumchloride | 0.2 |
| C11-15 Pareth-7 | 0.2 |
| Trinatrium-Ethylendiamin-Disuccinate | 0.2 |
| Laureth-9 | 0.2 |
| Trideceth-12 | 0.2 |
| Hydrolyzed Vegetable Protein PG-Propyl Silanetriol | 0.2 |
| C12-13 Pareth-3 | 0.2 |
| C12-13 Pareth-23 | 0.2 |
| Tocopherol | 0.2 |
| Pseudozyma Epicola Argania Spinosa Kernel Oil Ferment Filtrate | 0.2 |
| Pseudozyma Epicola/Argania Spinosa Kernel Oil | 0.2 |
| Ferment-Filtrat | 0.2 |
| Prunus Armeniaca(Apricot)-Kernel Oil | 0.2 |
| Quaternium-95 | 0.2 |
| Propanediol | 0.2 |
| Helianthus Annuus(Sunflower)-Seed extrakt | 0.2 |
| PEG-8 | 0.1 |
| Bis-Aminopropyl Diglycol Dimaleate | 0.1 |
| Euterpe Oleracea Fruit-Extract | 0.1 |
| Rosmarinus Officinalis (Rosemary)-Leafextract | 0.1 |
| Punica Granatum-Extrakt | 0.1 |
| Origanum Vulgare-Leafextract | 0.1 |
| Musa Sapientum(Banana)-Extract | 0.1 |
| Morinda Citrifolia-Fruit extract | 0.1 |
| Sodium-Hyaluronate | 0.1 |
| PEG-8/Smdi-Copolymer | 0.1 |
| Arctium Lappa-Root Extract | 0.1 |
| Sodium-Polyacrylate | 0.1 |
| Palmitoyl Myristyl Serinate | 0.1 |
| Citral | 0.1 |
| Hexyl Cinnamal | 0.1 |
| Limonene | 0.1 |
| Biotin | 0.1 |
| Pentaerythrityl Tetra-Di-t-Butyl Hydroxyhydrocinnamate | 0.1 |

### Example formulation 250: Repair Shampoo

| Ingredients | % |
|---|---|
| Aqua/Water/Eau | ad 100 |
| Sodium Lauryl Sulfate | 4.5 |
| Sodium Laureth Sulfate | 4.5 |
| Cocamidopropyl Betaine | 3 |
| Glycol Distearate | 3 |
| Dimethicone | 1 |
| Cocamide MEA | 1 |
| Parfum/Fragrance | 0.5 |
| Panthenol | 0.5 |

| Sodium Benzoate | 0.5 |
|---|---|
| Malic Acid | 0.5 |
| Composition A | 1.5 |
| Guar Hydroxypropyltrimonium Chloride | 0.2 |
| Sodium Chloride | 0.2 |
| Salicylic Acid | 0.2 |
| Tocopheryl Acetate | 0.2 |
| Citric Acid | 0.2 |
| Sodium Hydroxide | 0.2 |
| Tetrasodium EDTA | 0.2 |
| Trisodium Ethylenediamine Disuccinate | 0.2 |
| Glycerin | 0.2 |
| Methylparaben | 0.2 |
| Histidine | 0.2 |
| Linalool | 0.2 |
| Citronellol | 0.2 |
| Hydrochloric Acid | 0.2 |
| Oleic Acid | 0.2 |
| Squalane | 0.2 |
| Glyceryl Oleate | 0.2 |
| Magnesium Nitrate | 0.2 |
| Methylchloroisothiazolinone | 0.2 |
| Magnesium Chloride | 0.2 |
| Sodium Citrate | 0.2 |
| Methylisothiazolinone | 0.2 |
| Sodium Xylenesulfonate | 0.2 |
| Lecithin | 0.2 |
| Ascorbyl Palmitate | 0.2 |
| Tocopherol | 0.2 |
| Hydrogenated Palm Glycerides Citrate. | 0.2 |

### Example formulation 251: Shampoo for Glossy Hair

| Ingredients | % |
|---|---|
| Water | ad 100 |
| Sodium Laureth Sulfate | 6 |
| Sodium Lauroyl Sacrospinale | 3 |
| Cocamidopropyl Hydroxysultaine | 3 |
| Glycol Distearate | 1.5 |
| Sodium Lauroyl Glutamate | 1.5 |
| Sodium PCA | 0.5 |
| Panthenol | 0.5 |
| Composition A | 4.5 |
| Glycoproteins | 0.3 |
| Ethylhexyl Methoxycinnamate | 0.3 |
| Tetrasodium Glutamate Diacetate | 0.3 |
| Glycerin | 0.3 |
| Palmitamidopropyltrimonium Chloride | 0.3 |
| Propylene Glycol | 0.3 |
| Punica Granatum Extract | 0.3 |
| Sillicone Quaternium-22 | 0.3 |
| Cocamide MEA | 0.3 |
| Glyoxylic Acid | 0.2 |
| PPG- 3 Myristyl Ether | 0.2 |
| Isotearamide MIPA | 0.2 |
| Hydroxypropyl Guar | 0.2 |
| Hydroxypropyltrimonium Chloride | 0.2 |
| Octyldodecyl PCA | 0.2 |
| Glyceryl Laurate | 0.2 |
| Phenyl Trimethicone | 0.1 |
| Salix Nigra (Willow) Bark Extract | 0.1 |
| Citric Acid | 0.1 |
| Sodium Chloride | 0.1 |
| Sodium Hydroxide | 0.1 |
| Sodium Benzoate | 0.1 |
| Benzoic Acid | 0.1 |
| FragranceV Parfum | 0.1 |
| Limonene | 0.1 |
| Linalool | 0.1 |
| Amyl Cinnamal | 0.1 |
| Benzyl Salicylate | 0.1 |
| Red 33V C.I. 17200 | 0.1 |

### Example formulation 252: Volume Shampoo

| Ingredients | % |
|---|---|
| Aqua/Water | ad 100 |
| Sodium Laureth Sulfate | 4.5 |
| Coco-Betaine | 4 |
| Sodium Lauryl Sulfate | 3.5 |
| Sodium Chloride | 2 |
| Glycol Distearate | 2 |
| Hexylene Glycol | 1 |
| Parfum/Fragrance | 0.5 |
| Aminopropyl Triethoxysilance | 0.5 |
| Acrylates Copolymer | 0.5 |
| Composition A | 0.8 |
| Hydroxypropyl Guar Hydroxypropyltrimonium Chloride | 0.4 |
| Alcohol Denat. | 0.2 |
| Polyquaternium-30 | 0.2 |
| Citric Acid | 0.2 |
| Benzyl Alcohol | 0.2 |
| Linalool | 0.2 |
| Hexyl Cinnamal | 0.2 |
| Magnesium Nitrate | 0.2 |
| Alpha-Isomethyl lonone | 0.2 |
| Limonene | 0.2 |
| Methylchloroisothiazolinone | 0.2 |
| Magnesium Chloride | 0.2 |
| Methylisothiazolinone | 0.2 |
| Sodium Hydroxide. | 0.2 |

### Example formulation 253: Soothing Shampoo

| Ingredients | % |
|---|---|
| Aqua | ad 100 |
| Sodium Laureth Sulfate | 9 |
| Sodium Chloride | 3 |
| Cocamidopropyl Betaine | 3 |
| Dimethicone | 1 |
| Coco-Betaine | 1 |
| Citric Acid | 1 |
| Hexylene Glycol | 1 |
| Glycol Distearate | 1 |
| Sodium Benzoate | 0.5 |
| Sodium Hydroxide | 0.5 |
| Amodimethicone | 0.5 |
| Carbomer | 0.5 |
| Composition A | 6.0 |
| Guar Hydroxypropyltrimonium Chloride | 0.2 |
| Trideceth-10 | 0.2 |
| Glycerin | 0.2 |
| Salicylic Acid | 0.2 |
| Arginine | 0.2 |
| Panthenol | 0.2 |
| Mica | 0.2 |
| Benzyl Salicylate | 0.2 |
| Benzyl Alcohol | 0.2 |
| PEG-100 Stearate | 0.2 |
| Linalool | 0.2 |
| Steareth-6 | 0.2 |
| Phenoxyethanol | 0.2 |
| Limonene | 0.2 |
| Trideceth-3 | 0.2 |
| Hydroxycitronellal | 0.2 |
| Ci 77891 / Titanium Dioxide | 0.1 |
| Citronellol | 0.1 |
| Alpha-Isomethyl lonone | 0.1 |
| Aminopropyl Triethoxysilane | 0.1 |
| Acetic Acid | 0.1 |
| Fumaric Acid | 0.1 |
| Polyquaternium-6 | 0.1 |
| Tetrasodium Edta | 0.1 |
| Parfum / Fragrance | 0.1 |

### Example formulation 254: Classic Anti Dandruff Shampoo

| Ingredients | % |
|---|---|
| Aqua | ad 100 |
| Sodium Laureth Sulfate | 5 |
| Sodium Lauryl Sulfate | 4 |
| Glycol Distearate | 3 |
| Cocamide MEA | 1.5 |
| Cocamidopropyl Betaine | 1 |
| Chamomilla Recutita Flower Extract | 0.5 |
| Climbazole | 0.5 |
| Panthenol | 0.5 |
| Glycerin | 0.5 |
| Composition A | 4.0 |
| Dimethicone | 0.3 |
| Parfum | 0.2 |
| Guar Hydroxypropyltrimonium Chloride | 0.3 |
| Tetrasodium Glutamate Diacetate | 0.2 |
| Sodium Chloride | 0.2 |
| Citric Acid | 0.2 |
| Sodium Citrate | 0.2 |
| Sodium Benzoate | 0.2 |
| Phenoxyethanol | 0.2 |
| Limonene. | 0.2 |

### Example formulation 255: Hydrating Shampoo

| Ingredients | % |
|---|---|
| Aqua | ad 100 |
| Sodium Coco-Sulfate | 4.5 |
| Disodium Lauryl Sulfosuccinate | 3.5 |
| Cocamidopropyl Betaine | 1.5 |
| Sodium Methyl Oleoyl Taurate | 1.5 |
| Cocamide MIPA | 1.5 |
| Glycol Distearate | 1.5 |
| Disodium Cocoamphodiacetate | 1 |
| Hydrogenated Castor Oil | 1 |
| Salix Alba (Willow) Bark Extract | 0.5 |
| Cassia Hydroxypropyltrimonium Chloride | 0.5 |
| Behenoyl PG-Trimonium Chloride | 0.5 |
| Lauryl/Myristyl Polyricinoleate | 0.5 |
| Xylitylglucoside | 0.5 |
| Heptyl Glucoside | 0.5 |
| Composition A | 5.0 |
| Sodium Chloride | 0.3 |
| Sulfated Castor Oil | 0.3 |
| Hexylene Glycol | 0.3 |
| Piroctone Olamine | 0.3 |
| Salicylic Acid | 0.2 |
| Capryloyl Glycine | 0.2 |
| Citric Acid | 0.2 |
| Stearic Acid | 0.2 |
| Palmitic Acid | 0.2 |
| Cetyl Hydroxyethylcellulose | 0.2 |
| Propylene Glycol | 0.2 |
| Zinc Oxide | 0.2 |
| Limonene | 0.2 |
| Sodium Hydroxide | 0.2 |
| Glycerin | 0.2 |
| Sorbitol | 0.1 |
| Parfum | 0.1 |

### Example formulation 256: Hydrating Shampoo with Actives

| Ingredients | % | % | % | % | % | % | % | % | % | % | % | % |
|---|---|---|---|---|---|---|---|---|---|---|---|---|
| Aqua | ad 10 0 | ad 10 0 | ad 10 0 | ad 100 | ad 10 0 | ad 10 0 | ad 10 0 | ad 10 0 | ad 10 0 | ad 10 0 | ad 10 0 | ad 10 0 |
| Sodium Laureth Sulfate | 5 | 5 | 5 | 5 | 5 | 5 | 5 | 5 | 5 | 5 | 5 | 5 |
| Sodium Lauryl Sulfate | 4 | 4 | 4 | 4 | 4 | 4 | 4 | 4 | 4 | 4 | 4 | 4 |
| Glycol Distearate | 3 | 3 | 3 | 3 | 3 | 3 | 3 | 3 | 3 | 3 | 3 | 3 |
| Cocamide MEA | 1.5 | 1.5 | 1.5 | 1.5 | 1.5 | 1.5 | 1.5 | 1.5 | 1.5 | 1.5 | 1.5 | 1.5 |
| Cocamidopropyl Betaine | 1 | 1 | 1 | 1 | 1 | 1 | 1 | 1 | 1 | 1 | 1 | 1 |
| Chamomilla Recutita Flower Extract | 0.5 | 0.5 | 0.5 | 0.5 | 0.5 | 0.5 | 0.5 | 0.5 | 0.5 | 0.5 | 0.5 | 0.5 |
| Climbazole | 0.5 | 0.5 | 0.5 | 0.5 | 0.5 | 0.5 | 0.5 | 0.5 | 0.5 | 0.5 | 0.5 | 0.5 |
| Panthenol | 0.5 | 0.5 | 0.5 | 0.5 | 0.5 | 0.5 | 0.5 | 0.5 | 0.5 | 0.5 | 0.5 | 0.5 |
| Glycerin | 0.5 | 0.5 | 0.5 | 0.5 | 0.5 | 0.5 | 0.5 | 0.5 | 0.5 | 0.5 | 0.5 | 0.5 |
| Composition A | 0.1 | 2.0 | 0.3 | 2.5 | 0.6 | 4.5 | 5.0 | 0.8 | 6.5 | 0.3 | 8.0 | 4.5 |
| Hyaluronic Acid | 1 | 1 | 1 | 1 | 1 | 1 | 1 | 1 | 1 | 1 | 1 | 1 |
| Niacinamide | 0.5 | 1 | | | | | | | | | 1.5 | 2 |
| Retinol | | | 0.5 | 0.7 5 | | | | | | | | |
| Lecithin | | | | | 1.5 | 2 | | | | | | |
| Coffeine | | | | | | | 0.5 | 1 | | | | |
| Peptides | | | | | | | | | 0.5 | 1 | | |
| Dimethicone | 0.3 | 0.3 | 0.3 | 0.3 | 0.3 | 0.3 | 0.3 | 0.3 | 0.3 | 0.3 | 0.3 | 0.3 |
| Parfum | 0.2 | 0.2 | 0.2 | 0.2 | 0.2 | 0.2 | 0.2 | 0.2 | 0.2 | 0.2 | 0.2 | 0.2 |
| Guar Hydroxypropyltrimoniu m Chloride | 0.2 | 0.2 | 0.2 | 0.2 | 0.2 | 0.2 | 0.2 | 0.2 | 0.2 | 0.2 | 0.2 | 0.2 |
| Tetrasodium Glutamate Diacetate | 0.2 | 0.2 | 0.2 | 0.2 | 0.2 | 0.2 | 0.2 | 0.2 | 0.2 | 0.2 | 0.2 | 0.2 |
| Sodium Chloride | 0.2 | 0.2 | 0.2 | 0.2 | 0.2 | 0.2 | 0.2 | 0.2 | 0.2 | 0.2 | 0.2 | 0.2 |
| Citric Acid | 0.2 | 0.2 | 0.2 | 0.2 | 0.2 | 0.2 | 0.2 | 0.2 | 0.2 | 0.2 | 0.2 | 0.2 |
| Sodium Citrate | 0.2 | 0.2 | 0.2 | 0.2 | 0.2 | 0.2 | 0.2 | 0.2 | 0.2 | 0.2 | 0.2 | 0.2 |
| Sodium Benzoate | 0.2 | 0.2 | 0.2 | 0.2 | 0.2 | 0.2 | 0.2 | 0.2 | 0.2 | 0.2 | 0.2 | 0.2 |
| Phenoxyethanol | 0.2 | 0.2 | 0.2 | 0.2 | 0.2 | 0.2 | 0.2 | 0.2 | 0.2 | 0.2 | 0.2 | 0.2 |
| Limonene. | 0.2 | 0.2 | 0.2 | 0.2 | 0.2 | 0.2 | 0.2 | 0.2 | 0.2 | 0.2 | 0.2 | 0.2 |

### Example formulation 257: Sensitive Conditioner

| Ingredients | % |
|---|---|
| Aqua | ad 100 |
| Cetyl Alcohol | 5 |
| Glycerin | 5 |
| Cetrimonium Chloride | 1 |
| Dicaprylyl Ether | 1 |
| Isopropyl Palmitate | 1 |
| Panthenol | 0.5 |
| Composition A | 0.7 |
| Hydroxypropyl Guar Hydroxypropyltrimonium Chloride | 0.2 |
| Sodium Benzoate | 0.2 |
| Citric Acid | 0.2 |
| Niacinamide | 1.0 |
| Potassium Sorbate | 0.2 |

### Example formulation 258: Intense Repair Conditioner

| Ingredients | % |
|---|---|
| Aqua | ad 100 |
| Glycerin | 10 |
| Cetearyl Alcohol | 5 |
| Isopropyl Palmitate | 4 |
| Behentrimonium Chloride | 1 |
| Dimethicone | 0.5 |
| Silicone Quaternium-22 | 0.5 |
| Panthenol | 0.5 |
| Isopropyl Alcohol | 0.5 |
| Dipropylene Glycol | 0.5 |
| Arginine | 0.5 |
| Propylene Glycol | 0.5 |
| Serine | 0.5 |
| Cocos Nucifera Oil | 0.5 |
| Mauritia Flexuosa Fruit Oil | 0.5 |
| Polyglyceryl-3 Caprate | 0.5 |
| Cocamidopropyl Betaine | 0.5 |
| Composition A | 2.5 |
| Parfum | 0.45 |
| PCA | 0.2 |
| Glycine | 0.2 |
| Histidine | 0.2 |
| Sodium PCA | 0.2 |
| Aspartic Acid | 0.2 |
| Palmitamidopropyltrimonium Chloride | 0.2 |
| Alanine | 0.2 |
| Valine | 0.2 |
| Isoleucine | 0.2 |
| Proline | 0.2 |
| Threonine | 0.2 |
| Phenylalanine | 0.2 |
| Sodium Benzoate | 0.2 |
| Potassium Sorbate | 0.2 |
| Citric Acid | 0.2 |
| Sodium Lactate | 0.2 |

### Example formulation 259: Strengthening Conditioner

| Ingredients | % |
|---|---|
| Aqua | ad 100 |
| Cetyl Alcohol | 5 |
| Stearamidopropyl Dimethylamine | 2 |
| Stearyl Alcohol | 1.5 |
| Glutamic Acid | 1 |
| Bis-Aminopropyl Dimethicone | 1 |
| Parfum | 0.5 |
| Polysorbate 20 | 0.5 |
| Composition A | 4.5 |
| Benzyl Alcohol | 0.3 |
| Sodium Benzoate | 0.3 |
| Citric Acid | 0.3 |
| Panthenol | 0.3 |
| Panthenyl Ethyl Ether | 0.2 |
| Disodium EDTA | 0.2 |
| Histidine | 0.2 |
| Benzyl Salicylate | 0.2 |
| Hexyl Cinnamal | 0.2 |
| Alpha-Isomethyl Ionone | 0.2 |
| Biotin | 0.1 |
| Menthol | 0.1 |

### Example formulation 260: Spray Conditioner

| Ingredients | % |
|---|---|
| Aqua | ad 100 |
| Trisiloxane | 6 |
| Dimethicone | 2 |
| Prunus Armeniaca (Apricot) Kernel Oil | 1 |
| Phenyl Trimethicone | 1 |
| Sclerocarya Birrea Seed Oil | 1 |
| Hydrolyzed Keratin | 1 |
| Dimethiconol | 1 |
| Composition A | 1.3 |
| Polyquaternium-16 | 0.2 |
| Parfum (Fragrance) | 0.2 |
| Cetyl PEG/PPG-10/1 Dimethicone | 0.2 |
| Cetrimonium Chloride | 0.2 |
| Lactic Acid | 0.2 |
| Sodium Benzoate | 0.2 |
| Linalool | 0.2 |
| Limonene | 0.2 |
| Alpha-Isomethyl Ionone | 0.2 |
| Geraniol | 0.2 |
| Phenoxyethanol | 0.2 |
| Potassium Sorbate | 0.2 |
| Benzyl Alcohol | 0.2 |

### Example formulation 261: Hydrating Conditioner

| Ingredients | % |
|---|---|
| Aqua | ad 100 |
| Cetearyl Alcohol | 5 |
| Cetyl Esters | 2 |
| Behenamidopropyl Dimethylamine | 2 |
| Triheptanoin | 2 |
| Cetrimonium Chloride | 1 |
| Lactic Acid | 0.5 |
| Benzyl Alcohol | 0.5 |
| Panthenol | 0.5 |
| Ethylhexylglycerin | 0.5 |
| Glycine | 0.5 |
| Hippophae Rhamnoides Fruit Oil | 0.5 |
| Sodium Hyaluronate | 0.5 |
| Composition A | 3.5 |
| Tocopherol | 0.3 |
| Phenoxyethanol | 0.3 |
| Parfum | 0.2 |
| Limonene | 0.2 |
| Linalool. | 0.2 |

### Example formulation 262: Conditioner for Coloured Hair

| Ingredients | % |
|---|---|
| Aqua | ad 100 |
| Cetearyl Alcohol | 5 |
| Behenamidopropyl Dimethylamine | 2 |
| Cetyl Esters | 1.5 |
| Dipropylene Glycol | 1.5 |
| Stearyl Alcohol | 1.5 |
| Dimethicone | 1 |
| Cetrimonium Methosulfate | 1 |
| Parfum | 0.5 |
| Lactic Acid | 0.5 |
| Bis-Cetearyl Amodimethicone | 0.5 |
| Composition A | 0.4 |
| Quaternium-91 | 0.3 |
| Disodium EDTA | 0.3 |
| Glycine | 0.3 |
| Steartrimonium Chloride | 0.2 |
| Glycerin | 0.2 |
| Malic Acid | 0.2 |
| Pearl Powder | 0.2 |
| Prunus Amygdalus Dulcis Oil | 0.2 |
| Stearoxypropyl Dimethylamine | 0.2 |
| C14-28 Isoalkyl Acid | 0.2 |
| Isopropyl Alcohol | 0.2 |
| C14-28 Alkyl Acid | 0.1 |
| Methylchloroisothiazolinone | 0.1 |
| Methylisothiazolinone | 0.1 |
| Hydroxycitronellal | 0.1 |
| Limonene | 0.1 |

### Example formulation 263: Conditioner for Hydration and Shine

| Ingredients | % |
|---|---|
| Aqua | ad 100 |
| Cetearyl Alcohol | 6 |
| Panthenol | 5 |
| Oryza Sativa (Rice) Extract | 5 |
| Isopropyl Myristate | 5 |
| Cetrimonium Chloride | 2 |
| Glyceryl Stearate | 0.5 |
| Glycerin | 0.5 |
| Lauryl Glucoside | 0.5 |
| Composition A | 5.5 |
| Parfum (Fragrance) | 0.3 |
| Polyquaternium-37 | 0.3 |
| Sodium Benzoate | 0.3 |
| Dicaprylyl Carbonate | 0.3 |
| Citric Acid | 0.3 |
| Hexyl Cinnamal | 0.3 |
| Linalool | 0.2 |
| Limonene | 0.2 |
| Benzyl Salicylate | 0.2 |
| Benzyl Alcohol | 0.2 |
| Potassium Sorbate | 0.2 |
| Sorbic Acid | 0.2 |

### Example formulation 264: Oat Conditioner

| Ingredients | % |
|---|---|
| Water (Aqua) | ad 100 |
| Alcohol denat. | 5 |
| Cetearyl Alcohol | 5 |
| Behenyl Alcohol | 2 |
| PCA Glyceryl Oleate | 2 |
| Glyceryl Stearate Citrate | 1 |
| Isoamyl Laurate | 1 |
| Simmondsia Chinensis (Jojoba) Seed Oil+ | 1 |
| Composition A | 2.4 |
| Glyceryl Stearate | 0.5 |
| Avena Sativa (Oat) Flower/Leaf/Stem Extract | 0.3 |
| Fragrance (Parfum)* | 0.2 |
| Althaea Officinalis Root+ Extract | 0.2 |
| Cocos Nucifera (Coconut) Oil+ | 0.2 |
| Carrageenan | 0.2 |
| Citric Acid | 0.2 |
| Arginine | 0.2 |
| Hydrolyzed Wheat Protein | 0.2 |
| Xanthan Gum | 0.2 |
| Limonene* | 0.1 |
| Linalool* | 0.1 |
| Citronellol* | 0.1 |
| Geraniol* | 0.1 |
| Coumarin* | 0.1 |

### Example formulation 265: Deep Repairing Conditioner

| Ingredients | % |
|---|---|
| Aqua | ad 100 |
| Cetyl Alcohol | 6 |
| Malic Acid | 5 |
| Paraffinum Liquidum | 5 |
| Isopropyl Myristate | 5 |
| Ceteareth-25 | 1 |
| Cetrimonium Chloride | 1 |
| Sorbitan Oleate | 1 |
| Sodium Hydroxide | 0.7 |
| Phenoxyethanol | 0.5 |
| Glyceryl Oleate | 0.5 |
| Isopropyl Alcohol | 0.5 |
| Parfum/Fragrance | 0.5 |
| Coco-Glucoside | 0.5 |
| Behentrimonium Chloride | 0.5 |
| Acrylates/Stearyl Methacrylate Copolymer | 0.5 |
| Composition A | 6.0 |
| Amodimethicone | 0.3 |
| Tocopheryl Acetate | 0.3 |
| Polyquaternium-37 | 0.3 |
| Squalane | 0.2 |
| Guar Hydroxypropyltrimonium Chloride | 0.2 |
| Disodium EDTA | 0.2 |
| Oleic Acid | 0.2 |
| Ethylhexylglycerin | 0.2 |
| Propylene Glycol Dicaprylate/Dicaprate | 0.2 |
| Linalool | 0.2 |
| Citric Acid | 0.2 |
| Trideceth-12 | 0.2 |
| PPG-1 Trideceth-6 | 0.2 |
| Citronellol | 0.2 |
| Histidine | 0.2 |
| Sodium Benzoate | 0.2 |
| Tocopherol | 0.2 |
| EDTA | 0.2 |
| Hydrogenated Palm Glycerides Citrate | 0.2 |
| Lecithin | 0.2 |
| Ascorbyl Palmitate | 0.2 |

### Example formulation 266: Deep Repairing Conditioner

| Ingredients | % |
|---|---|
| Aqua | ad 100 |
| Cocos Nucifera Oil | 10 |
| Amodimethicone | 3 |
| Polyquaternium-37 | 2 |
| Phenoxyethanol | 1 |
| Propylene Glycol Dicaprylate/Dicaprate | 1 |
| Sorbitan Oleate | 1 |
| Isopropyl Alcohol | 0.5 |
| Cetrimonium Chloride | 0.5 |
| Xylitylglucoside | 0.5 |
| Sodium Hydroxide | 0.5 |
| Anhydroxylitol | 0.5 |
| Xylitol | 0.5 |
| Dimethicone PEG-7 Phosphate | 0.5 |
| Composition A | 9.0 |
| PPG-1 Trideceth-6 | 0.3 |
| Trideceth-6 | 0.3 |
| Behentrimonium Chloride | 0.3 |
| Glycerin | 0.3 |
| Limonene | 0.2 |
| Xylose | 0.2 |
| Ethylhexylglycerin | 0.2 |
| Linalool | 0.2 |
| Hydrolyzed Vegetable Protein PG-Propyl Silanetriol | 0.2 |
| Benzyl Salicylate | 0.2 |
| Coumarin | 0.2 |
| Zingiber Officinale Root Extract / Ginger Root Extract | 0.2 |
| Citral | 0.2 |
| Benzyl Alcohol | 0.2 |
| Citronellol | 0.2 |
| Benzyl Benzoate | 0.2 |
| Leontopodium Alpinum Callus Culture Extract | 0.2 |
| Potassium Sorbate | 0.2 |
| Tocopherol | 0.2 |
| Citric Acid | 0.2 |
| Xanthan Gum | 0.2 |
| Dipeptide Diaminobutyroyl Benzylamide Diacetate | 0.2 |
| Parfum | 0.2 |

### Example formulation 267: Rich Repair Conditioner

| Ingedients | % |
|---|---|
| Water | ad 100 |
| Cetearyl Alcohol | 5 |
| Isopropyl Myristate | 2 |
| Cetrimonium Chloride | 2 |
| Amodimethicone | 1 |
| Bis-Methoxypropylamido Isodocosane | 0.5 |
| Lanolin Acid | 0.5 |
| Octyldodecyl PCA | 0.5 |
| Sodium PCA | 0.5 |
| Salix Nigra (Willow) Bark Extract | 0.5 |
| Dimethicone | 0.5 |
| Panthenol | 0.5 |
| Glycerin | 0.5 |
| Composition A | 10 |
| Hydroxypropyl Guar Hydroxypropyltrimonium Chloride | 0.2 |
| Ethylhexylglycerin | 0.2 |
| Lactic Acid | 0.2 |
| Dipropylene Glycol | 0.2 |
| C11-15 Pareth-7 | 0.2 |
| Laureth-9 | 0.2 |
| Stearoxypropyl Dimethylamine | 0.2 |
| Glycoproteins | 0.2 |
| Trideceth-12 | 0.2 |
| Acetic Acid | 0.2 |
| Stearyl Alcohol | 0.2 |
| Phenoxyethanol | 0.2 |
| Fragrance / Parfum | 0.2 |
| Linalool | 0.1 |
| Limonene | 0.1 |
| Orange 4 / C.I. 15510 | 0.1 |
| Yellow 5 / C.I. 19140 | 0.1 |
| Blue 1 / C.I. 42090 | 0.1 |
| Red 33 / C.I. 17200. | 0.1 |

### Example formulation 268: Light Conditioner

| Ingredients | % |
|---|---|
| Aqua (Water) | ad 100 |
| Cetyl Alcohol | 5 |
| Coco-Caprylate/Caprate | 3 |
| Isoamyl laurate | 2 |
| Stearyl Alcohol | 2 |
| Behentrimonium Methosulfate | 2 |
| Macadamia Ternifolia Seed oil | 1 |
| Panthenol (Provitamin B5) | 1 |
| Prunus Amygdalus Dulcis (Sweet Almond) Oil | 1 |
| Butyrospermum Parkii (Shea) Butter | 1 |
| Phenoxyethanol | 0.5 |
| Glyceryl Laurate | 0.5 |
| Fragrance | 0.5 |
| Composition A | 9.5 |
| Limonene | 0.2 |
| Hydrolysed Rice Protein | 0.2 |
| Polyquaternium-10 | 0.2 |
| Hydrolysed Vegetable Protein | 0.2 |
| Alpha-Tocopherol (Vitamin E) | 0.2 |
| Glycerin | 0.2 |
| Helianthus Annuus (Sunflower) Seed Oil | 0.2 |
| Davidsonia Pruriens (Davidson's Plum) Fruit Extract | 0.1 |
| Acronychia Acidula (Lemon Aspen) Fruit Extract | 0.1 |
| Syzygium Luehmannii (Lilly Pilly) Fruit Extract. | 0.1 |

### Example formulation 269: Soft Conditioner

| Ingredients | % |
|---|---|
| Aqua | ad 100 |
| Cetearyl Alcohol | 5 |
| Behentrimonium Chloride | 2 |
| Elaeis Guineensis Oil | 1 |
| Cetyl Alcohol | 1 |
| Isopropyl Alcohol | 0.5 |
| Phenoxyethanol | 0.5 |
| Stearamidopropyl Dimethylamine | 0.5 |
| Octyldodecanol | 0.5 |
| Composition A | 8.0 |
| Sodium PCA | 0.3 |
| Parfum / Fragrance | 0.3 |
| Citric Acid | 0.3 |
| Sodium Cocoyl Amino Acids | 0.3 |
| Chlorhexidine Dihydrochloride | 0.3 |
| Potassium Dimethicone PEG-7 Panthenyl Phosphate | 0.3 |
| Sodium Sarcosinate | 0.3 |
| Propylene Glycol | 0.3 |
| Argania Spinosa Kernel Oil | 0.3 |
| Arginine | 0.2 |
| Hydrolyzed Soy Protein | 0.2 |
| Hydrolyzed Vegetable Protein PG-propyl Silanetriol | 0.2 |
| Tetrasodium Edta | 0.2 |
| Potassium Sorbate | 0.2 |

### Example formulation 270: Colour Conditioner

| Ingredients | % |
|---|---|
| Aqua | ad 100 |

| Cetearyl Alcohol | 5 |
|---|---|
| Behentrimonium Chloride | 2 |
| Cetyl Esters | 1.5 |
| Amodimethicone | 1 |
| Isopropyl Alcohol | 0.5 |
| Niacinamide | 0.5 |
| Composition A | 8.5 |
| Citric Acid | 0.3 |
| Trideceth-6 | 0.3 |
| Linalool | 0.2 |
| Potassium Hydroxide | 0.2 |
| Chlorhexidine Digluconate | 0.2 |
| Cetrimonium Chloride | 0.2 |
| Resveratrol | 0.2 |
| Benzyl Alcohol | 0.2 |
| Parfum / Fragrance | 0.2 |

### Example formulation 271: Leave-In Conditioner

| Ingredients | % |
|---|---|
| Aqua | ad 100 |
| Dimethicone | 5 |
| Stearyl Alcohol | 5 |
| Behentrimonium Chloride | 2 |
| Trideceth-9 PG-Amodimethicone | 1 |
| Phenoxyethanol | 1 |
| Polysorbate 20 | 1 |
| Parfum / Fragrance | 0.5 |
| Isopropyl Alcohol | 0.5 |
| PPG-5-Ceteth-20 | 0.5 |
| Laureth-23 | 0.5 |
| Laureth-4 | 0.5 |
| Trideceth-12 | 0.5 |
| Glycine Soja Oil / Soybean Oil | 0.5 |
| Composition A | 6.5 |
| Tocopherol | 0.5 |
| Xylose | 0.3 |
| Arginine | 0.3 |
| Glutamic Acid | 0.3 |
| Limonene | 0.2 |
| Propylene Glycol | 0.2 |
| Serine | 0.2 |
| Salicylic Acid | 0.2 |
| Linalool | 0.2 |
| Benzyl Salicylate | 0.2 |
| PEG/PPG-4/12 Dimethicone | 0.2 |
| Benzyl Alcohol | 0.2 |
| Alpha-Isomethyl Ionone | 0.2 |
| Coumarin | 0.1 |
| Pentaerythrityl Tetra-Di-t-Butyl Hydroxyhydrocinnamate | 0.1 |

### Example formulation 272: Conditioner with Actives

| Ingredients | | | | | | | | | | | | |
|---|---|---|---|---|---|---|---|---|---|---|---|---|
| Aqua | ad 10 0 | ad 10 0 | ad 10 0 | ad 10 0 | ad 10 0 | ad 100 | ad 10 0 | ad 10 0 | ad 10 0 | ad 10 0 | ad 10 0 | ad 100 |
| Cetyl Alcohol | 5 | 5 | 5 | 5 | 5 | 5 | 5 | 5 | 5 | 5 | 5 | 5 |
| Stearamidopropyl Dimethylamine | 2 | 2 | 2 | 2 | 2 | 2 | 2 | 2 | 2 | 2 | 2 | 2 |
| Stearyl Alcohol | 1.5 | 1. 5 | 1. 5 | 1.5 | 1.5 | 1.5 | 1.5 | 1. 5 | 1.5 | 1. 5 | 1. 5 | 1.5 |
| Glutamic Acid | 1 | 1 | 1 | 1 | 1 | 1 | 1 | 1 | 1 | 1 | 1 | 1 |
| Bis-Aminopropyl Dimethicone | 1 | 1 | 1 | 1 | 1 | 1 | 1 | 1 | 1 | 1 | 1 | 1 |
| Parfum | 0.5 | 0. 5 | 0. 5 | 0.5 | 0.5 | 0.5 | 0.5 | 0. 5 | 0.5 | 0. 5 | 0. 5 | 0.5 |
| Composition A | 0.1 | 2. 5 | 3. 0 | 0.8 | 9.5 | 11 | 20 | 0. 8 | 2.5 | 4. 0 | 7. 5 | 0.5 |
| Polysorbate 20 | 0.5 | 0. 5 | 0. 5 | 0.5 | 0.5 | 0.5 | 0.5 | 0. 5 | 0.5 | 0. 5 | 0. 5 | 0.5 |
| Hyaluronic Acid | 1 | 1 | 1 | 1 | 0.5 | 0.5 | 0.5 | 0. 5 | 2 | 2 | 1 | 1 |
| Niacinamide | | 0. 5 | | | | | | | 1.0 | | | |
| Retinol | | | | 0.0 1 | | 0.00 5 | | | | | | 0.00 1 |
| Lecithin | | | | | | | | | | 1. 0 | 2. 0 | |
| Coffeine | 1.7 5 | 2 | 2. 5 | 2.7 5 | | | | | 2.7 5 | 3 | | |
| Peptides | | | | | 0.7 5 | 1.75 | 2.2 5 | 2. 5 | | | 2. 5 | 3 |
| Benzyl Alcohol | 0.3 | 0. 3 | 0. 3 | 0.3 | 0.3 | 0.3 | 0.3 | 0. 3 | 0.3 | 0. 3 | 0. 3 | 0.3 |
| Sodium Benzoate | 0.3 | 0. 3 | 0. 3 | 0.3 | 0.3 | 0.3 | 0.3 | 0. 3 | 0.3 | 0. 3 | 0. 3 | 0.3 |
| Citric Acid | 0.3 | 0. 3 | 0. 3 | 0.3 | 0.3 | 0.3 | 0.3 | 0. 3 | 0.3 | 0. 3 | 0. 3 | 0.3 |
| Panthenol | 0.3 | 0. 3 | 0. 3 | 0.3 | 0.3 | 0.3 | 0.3 | 0. 3 | 0.3 | 0. 3 | 0. 3 | 0.3 |
| Panthenyl Ethyl Ether | 0.2 | 0. 2 | 0. 2 | 0.2 | 0.2 | 0.2 | 0.2 | 0. 2 | 0.2 | 0. 2 | 0. 2 | 0.2 |
| Disodium EDTA | 0.2 | 0. 2 | 0. 2 | 0.2 | 0.2 | 0.2 | 0.2 | 0. 2 | 0.2 | 0. 2 | 0. 2 | 0.2 |
| Histidine | 0.2 | 0. 2 | 0. 2 | 0.2 | 0.2 | 0.2 | 0.2 | 0. 2 | 0.2 | 0. 2 | 0. 2 | 0.2 |
| Benzyl Salicylate | 0.2 | 0. 2 | 0. 2 | 0.2 | 0.2 | 0.2 | 0.2 | 0. 2 | 0.2 | 0. 2 | 0. 2 | 0.2 |
| Hexyl Cinnamal | 0.2 | 0. 2 | 0. 2 | 0.2 | 0.2 | 0.2 | 0.2 | 0. 2 | 0.2 | 0. 2 | 0. 2 | 0.2 |
| Alpha-Isomethyl Ionone | 0.2 | 0. 2 | 0. 2 | 0.2 | 0.2 | 0.2 | 0.2 | 0. 2 | 0.2 | 0. 2 | 0. 2 | 0.2 |
| Biotin | 0.1 | 0. 1 | 0. 1 | 0.1 | 0.1 | 0.1 | 0.1 | 0. 1 | 0.1 | 0. 1 | 0. 1 | 0.1 |
| Menthol | 0.1 | 0. 1 | 0. 1 | 0.1 | 0.1 | 0.1 | 0.1 | 0. 1 | 0.1 | 0. 1 | 0. 1 | 0.1 |

### Example formulation 273: Refreshing Micellar Water

| Ingredients | % |
|---|---|
| Water (Aqua) | ad 100 |
| Glycerin | 5 |
| Alcohol* denat. | 3 |
| Pentylene Glycol | 2 |
| Coco-Glucoside | 1 |
| Sodium Hyaluronate | 0.5 |
| Levulinic Acid | 0.5 |
| Sodium Levulinate | 0.5 |
| Xylitylglucoside | 0.5 |
| Anhydroxylitol | 0.5 |
| Composition A | 3.0 |
| Glycerin* | 0.3 |
| Maltodextrin | 0.3 |
| Vitis Vinifera (Grape) Fruit Extract* | 0.2 |
| Cucumis Sativus (Cucumber) Fruit Extract* | 0.2 |
| Fucus Serratus Extract* | 0.2 |
| Xylitol | 0.2 |
| Biosaccharide Gum-1 | 0.1 |
| Benzyl Salicylate** | 0.1 |
| Linalool** | 0.1 |
| Limonene** | 0.1 |
| Fragrance (Parfum)** | 0.05 |

### Example formulation 274: Micellar Water

| Ingredients | % |
|---|---|
| Aqua | ad 100 |
| Hexylene Glycol | 1.5 |
| Glycerin | 1 |
| Poloxamer 184 | 1 |
| Disodium Cocoamphodiacetate | 1 |
| Composition A | 4.5 |
| Disodium EDTA | 0.5 |
| Myrtrimonium Bromide (F.I.L. Z280823/1). | 0.5 |

### Example formulation 275: Soothing Micellar Water

| Ingredients | % |
|---|---|
| Aqua | ad 100 |
| PEG-40 Hydrogenated Castor Oil | 3 |
| Glycerin | 3 |
| Glycine | 2 |
| Vitis Vinifera Seed Oil | 1 |
| Panthenol | 1 |
| Pantolactone | 1 |
| Sorbitol | 1 |
| Decyl Glucoside | 1 |
| Disodium Cocoyl Glutamate | 1 |
| Propylene Glycol | 0.7 |
| 1.2-Hexanediol | 0.7 |
| Poloxamer 124 | 0.5 |
| Citric Acid | 0.5 |
| Composition A | 3.5 |
| Polyquaternium-10 | 0.2 |
| Trisodium EDTA | 0.2 |
| Phenoxyethanol | 0.2 |
| Cetrimonium Chloride | 0.2 |
| BHT | 0.2 |

### Example formulation 276: Bi-Phasic Aloe Vera Micellar Water

| Ingredients | % |
|---|---|
| Aqua | ad 100 |
| Glycine | 10 |
| Soja Oil | 10 |
| Alcohol | 5 |
| Isoamyl Laurate | 5 |
| Glycerin | 1 |
| Dicaprylyl Ether | 1 |
| Betaine | 1 |
| Levulinic Acid | 0.5 |
| Sodium Chloride | 0.5 |
| Sodium Benzoate | 0.5 |
| Sodium Levulinate | 0.5 |
| Vitis Vinifera Seed Oil | 0.5 |
| Sodium Hydroxide | 0.5 |
| Coco-Glucoside | 0.5 |
| Composition A | 2.5 |
| Aloe Barbadensis Leaf Juice Powder | 0.2 |
| Tocopherol | 0.2 |
| Helianthus Annuus Seed Oil | 0.2 |
| Cucumis Sativus Fruit Extract | 0.2 |

### Example formulation 277: Rose Micellar Water

| Ingredients | % |
|---|---|
| Aqua | ad 100 |
| Pentylene Glycol | 3 |
| Propylene Glycol | 3 |
| Glycerin | 3 |
| Sodium Cocoamphoacetate | 1 |
| Rosa Damascena Flower Water | 1 |
| Aspartic Acid | 1 |
| Fumaric Acid | 1 |
| Maleic Acid | 1 |
| Malic Acid | 1 |
| Sodium Salicylate | 0.5 |
| Sodium Benzoate | 0.5 |
| Citric Acid | 0.5 |
| Sodium Chloride | 0.5 |
| Composition A | 2.5 |
| Tetrasodium Iminodisuccinate | 0.2 |
| Fumaric Acid | 0.2 |
| Maleic Acid | 0.2 |
| Malic Acid | 0.2 |

### Example formulation 278: Deep Cleansing Micellar Water

| Ingredients | |
|---|---|
| Aqua | ad 100 |
| PEG-6 Caprylic/Capric Glycerides | 5 |
| Polysorbate 20 | 2 |
| Butylene Glycol | 2 |
| PEG-8 | 1 |
| Poloxamer 407 | 1 |
| Disodium EDTA | 0.5 |
| Citric Acid | 0.5 |
| BHT | 0.5 |
| Sodium Benzoate | 0.5 |
| Composition A | 20 |

### Example formulation 279: Ficus Micellar Water

| Ingredients | % |
|---|---|
| Aqua | ad 100 |
| Aloe Barbadensis Leaf Juice | 3 |
| Glycerin | 3 |
| Caprylyl/Capryl Glucoside | 2 |
| Phenoxyethanol | 2 |
| Panthenol | 1 |
| Decyl Glucoside | 1 |
| Ethylhexylglycerin | 1 |
| Sodium Hyaluronate | 0.5 |
| Opuntia Ficus-Indica Fruit Extract | 0.5 |
| Benzyl Salicylate | 0.5 |
| Citric Acid | 0.5 |
| Pantolactone | 0.5 |
| Composition A | 4.0 |
| Citronellol | 0.2 |
| Geraniol | 0.2 |
| Hexyl Cinnamal | 0.2 |
| Sodium Benzoate | 0.2 |
| Parfum | 0.1 |

### Example formulation 280: Witch Hazel Micellar Water

| Ingredients | % |
|---|---|
| Aloe Barbadensis (Aloe Vera) Leaf Juice | 3 |
| Butylene Glycol | 3 |
| Glycerin | 3 |
| Caprylyl/Capryl Glucoside | 3 |
| Hamamelis Virginiana (Witch-hazel) Leaf Water | 2 |
| Rosa Damascena (Rose) Flower Water | 2 |
| Aqua/Water | ad 100 |
| Polyglyceryl-4 Caprate | 2 |
| Propanediol | 1 |
| Sodium Levulinate | 0.5 |
| Sodium Benzoate | 0.5 |
| Lactic Acid | 0.5 |
| Paeonia Lactiflora (Peonia) Root Extract | 0.5 |
| Sodium PCA | 0.5 |
| Aroma/Fragrance | 0.5 |
| Xylitylglucoside | 0.5 |
| Anhydroxylitol | 0.5 |
| Xylitol | 0.5 |
| Sodium Hyaluronate | 0.5 |
| Composition A | 6.0 |
| Phragmites Communis Extract | 0.2 |
| Poria Cocos Extract | 0.2 |
| Rhamnose | 0.2 |
| Glucose | 0.2 |
| Glucuronic Acid | 0.2 |

### Example formulation 281: 3 in 1 Micellar Water

| Ingredients | |
|---|---|
| Aqua/Water | ad 100 |
| Poloxamer 184 | 3 |
| Glycerin | 3 |
| Polysorbate 20 | 2.5 |
| Polyglyceryl- 4 Caprate | 2.5 |
| Thymus Vulgaris (Thyme) Leaf Extract | 1 |
| Cucumis Sativus (Cucumber) Fruit Extract | 1 |
| Peg-40 Glyceryl Cocoate | 1 |
| Levulinic Acid | 0.5 |
| Sodium Coceth Sulfate | 0.5 |
| Potassium Sorbate | 0.5 |
| Sodium Levulinate | 0.5 |
| Disodium Edta | 0.5 |
| Caprylyl Glycol | 0.5 |
| Citric Acid | 0.5 |
| Composition A | 7.0 |
| Sodium Benzoate | 0.3 |
| Parfum/Fragrance | 0.1 |

### Example formulation 282: Cleaning Micellar Water

| Ingredients | |
|---|---|
| Aqua | ad 100 |
| Poloxamer 184 | 4 |
| Propanediol | 3 |
| Pentylene Glycol | 3 |
| Glycerin | 3 |
| Caprylyl/Capryl Glucoside | 1 |
| Composition A | 0.2 |
| Parfum / Fragrance | 0.5 |
| Ethylhexylglycerin | 0.5 |
| Sodium Benzoate | 0.5 |
| Maris Aqua | 0.5 |
| Citric Acid | 0.5 |
| Phenethyl Alcohol | 0.5 |
| Laminaria Digitata Extract | 0.5 |
| Chlorella Vulgaris Extract | 0.5 |
| Saccharide Isomerate | 0.2 |
| 4089a | 0.1 |

### Example formulation 283: Micellar Water for Sensitive Skin

| Ingredients | % |
|---|---|
| Aqua | ad 100 |
| Aloe Barbadensis Leaf Juice | 3 |
| Glycerin | 3 |
| Polyglyceryl-4 Caprate | 3 |
| Valeriana Celtica Extract | 0.5 |
| Pelvetia Canaliculata Extract | 0.5 |
| Beta Vulgaris Root Extract | 0.5 |
| Hydrolyzed Corn Starch | 0.5 |
| Composition A | 1.8 |
| Phragmites Kharka Extract | 0.2 |
| Poria Cocos Extract | 0.2 |
| Parfum | 0.2 |
| Sodium Levulinate | 0.2 |
| Sodium Anisate | 0.2 |
| Benzyl Alcohol | 0.2 |
| Citral | 0.2 |
| Limonene | 0.2 |
| Linalool | 0.2 |

### Example formulation 284: Softening Micellar Water

| Ingredients | % |
|---|---|
| Water | ad 100 |
| Propanediol | 3 |
| Poloxamer 184 | 2 |
| Polysorbate 20 | 2 |
| Natrium PCA | 1 |
| Malva Sylvestris (Mallow) flower extract | 1 |
| Glycerine | 1 |
| Natriumbenzoate | 0.5 |
| Citric Acid | 0.5 |
| Composition A | 0.2 |
| Dinatrium EDTA | 0.2 |

### Example formulation 285: Deep Cleansing Micellar Water

| Ingredients | % |
|---|---|
| Aqua | ad 100 |
| Propanediol | 4 |
| Glycerin | 3 |
| Betaine | 1 |
| Coco-Glucoside | 1 |
| Rosa Damascena Flower Water | 0.5 |
| Aloe Barbadensis Leaf Juice Powder | 0.5 |
| Heptyl Glucoside | 0.5 |
| Dipotassium Glycyrrhizate | 0.5 |
| Composition A | 1.5 |
| Sodium Levulinate | 0.2 |
| Sodium Anisate | 0.2 |
| Lactic Acid | 0.2 |
| Sodium Lactate | 0.2 |
| PCA Ethyl Cocoyl Arginate | 0.2 |
| Parfum (Fragrance) | 0.1 |

### Example formulation 286: Micellar Water I

| Ingredients | % |
|---|---|
| Aqua | ad 100 |
| Glycerin | 6 |
| Gossypium Herbaceum Seed Extract | 2 |
| Camellia Sinensis Leaf Extract | 2 |
| Polysorbate 20 | 2 |
| Bisabolol | 1 |
| Helianthus Annuus Seed Oil | 1 |
| Panax Ginseng Root Extract | 0.5 |
| Chamomilla Recutita Flower Extract | 0.5 |
| Psidium Guajava Fruit Extract | 0.5 |
| Gossypium Herbaceum Flower Extract | 0.5 |
| Hypericum Perforatum Flower/Leaf/Stem Extract | 0.5 |
| Decyl Glucoside | 0.5 |
| Ethylhexylglycerin | 0.5 |
| Parfum | 0.5 |
| Disodium Cocoamphodiacetate | 0.5 |
| Sodium Chloride | 0.5 |
| Citric Acid | 0.5 |
| Benzyl Salicylate | 0.5 |
| Butylene Glycol | 0.5 |
| Chlorphenesin | 0.5 |
| Composition A | 1.0 |
| Limonene | 0.2 |
| Sodium Benzoate | 0.2 |
| Potassium Sorbate | 0.2 |
| Benzyl Alcohol | 0.2 |
| Sorbic Acid | 0.2 |
| Tocopherol | 0.2 |
| Phenoxyethanol | 0.2 |

### Example formulation 287: Pure 3-in-1 Micellar Water

| Ingredients | % |
|---|---|
| Aqua | ad 100 |
| Butylene Glycol | 3 |
| Betula Alba (Birch) Juice | 1 |
| Rubus Chamaemorus (Cloudberry) Fruit Juice Extract | 1 |
| Poloxamer 184 | 1 |
| Propanediol | 1 |
| Phenoxyethanol | 0.5 |
| Allantoin | 0.5 |
| Ethylhexylglycerin | 0.5 |
| Composition A | 3.2 |
| Disodium Edta | 0.2 |
| Polysorbate 20 | 0.2 |
| Citric Acid | 0.2 |
| Sodium Hydroxide | 0.2 |

### Example formulation 288: Micellar Water with Actives

| Ingredients | % | % | % | % | % | % | % | % | % | % | % | % |
|---|---|---|---|---|---|---|---|---|---|---|---|---|
| Aqua | ad 100 | ad 100 | ad 100 | ad 100 | ad 100 | ad 100 | ad 100 | ad 100 | ad 100 | ad 100 | ad 100 | ad 100 |
| PEG-6 Caprylic/Capric Glycerides | 5 | 5 | 5 | 5 | 5 | 5 | 5 | 5 | 5 | 5 | 5 | 5 |
| Polysorbate 20 | 2 | 2 | 2 | 2 | 2 | 2 | 2 | 2 | 2 | 2 | 2 | 2 |
| Butylene Glycol | 2 | 2 | 2 | 2 | 2 | 2 | 2 | 2 | 2 | 2 | 2 | 2 |
| PEG-8 | 1 | 1 | 1 | 1 | 1 | 1 | 1 | 1 | 1 | 1 | 1 | 1 |
| Poloxamer 407 | 1 | 1 | 1 | 1 | 1 | 1 | 1 | 1 | 1 | 1 | 1 | 1 |
| Disodium EDTA | 0.5 | 0.5 | 0.5 | 0.5 | 0.5 | 0.5 | 0.5 | 0.5 | 0.5 | 0.5 | 0.5 | 0.5 |
| Citric Acid | 0.5 | 0.5 | 0.5 | 0.5 | 0.5 | 0.5 | 0.5 | 0.5 | 0.5 | 0.5 | 0.5 | 0.5 |
| BHT | 0.5 | 0.5 | 0.5 | 0.5 | 0.5 | 0.5 | 0.5 | 0.5 | 0.5 | 0.5 | 0.5 | 0.5 |
| Sodium Benzoate | 0.5 | 0.5 | 0.5 | 0.5 | 0.5 | 0.5 | 0.5 | 0.5 | 0.5 | 0.5 | 0.5 | 0.5 |
| Composition A | 0.1 | 4.5 | 0.3 | 9.0 | 0.2 | 5.0 | 2.5 | 3.0 | 10 | 0.8 | 7.5 | 8.0 |
| Hyaluronic Acid | 1 | 1 | 1 | 1 | 1 | 1 | 1 | 1 | 2 | 2 | 2 | 2 |
| Niacinamide | 0.5 | 3.0 | | | | 1.5 | | | 0.5 | 3 | | |
| Retinol | | | | | 0.05 | | 0.001 | | | | 0.1 | |
| Lecithin | | | | | | | | | | | | |
| Coffeine | 1.5 | 2.75 | 3.2 | 3.5 | | | | | 1 | 1.75 | | |
| Peptides | | | | | 0.75 | 1 | 1.75 | 2.5 | | | 1.5 | 2 |

### Example formulation 289: Very Sensitive Shower Gel

| Ingredients | % |
|---|---|
| Aqua | ad 100 |
| Sodium Coco-Sulfate | 6 |
| Coco-Glucoside | 3 |
| Glycerin | 2 |
| Maris Sal | 1 |
| Cocamidopropyl Hydroxysultaine | 1 |
| Cocamidopropyl Betaine | 1 |
| Glyceryl Oleate | 1 |
| Composition A | 3.0 |
| Guar Hydroxypropyltrimonium Chloride | 0.2 |
| Sodium Citrate | 0.2 |
| Sodium Chloride | 0.2 |
| Tocopherol | 0.2 |
| Hydrogenated Palm Glycerides Citrate | 0.2 |
| Levulinic Acid | 0.2 |
| Sodium Levulinate | 0.2 |
| Caprylhydroxamic Acid | 0.2 |

### Example formulation 290: Happy Shower Gel

| Ingredients | % |
|---|---|
| Aqua | ad 100 |
| Sodium Laureth Sulfate | 6 |
| Glycerin | 3 |
| Cocamidopropyl Betaine | 1.5 |
| PEG-18 Glyceryl Oleate/Cocoate | 1.5 |
| Coco-Glucoside | 1.5 |
| PEG-40 Sorbitan Peroleate | 1.5 |
| Glyceryl Oleate | 1 |
| Litsea Cubeba Fruit Oil | 0.5 |
| Citrus Limon (Lemon) Peel Oil | 0.5 |
| Cymbopogon Nardus (Citronella) Oil | 0.5 |
| Prunus Amygdalus Dulcis (Sweet Almond) Oil | 0.5 |
| Glycol Distearate | 0.5 |
| Composition A | 0.6 |
| Limonene | 0.2 |
| Citral | 0.2 |
| Geraniol | 0.2 |
| Linalool | 0.2 |
| Citronellol | 0.2 |
| Parfum (Fragrance) | 0.2 |
| Sodium Hydroxide | 0.2 |
| Sodium Chloride | 0.2 |
| Citric Acid | 0.2 |
| Glycine Soja (Soybean) Oil | 0.2 |
| Hydrogenated Palm Glycerides Citrate | 0.2 |
| Tocopherol | 0.2 |
| Cl 19140 | 0.1 |

### Example formulation 291: Lemongrass Shower Oil

| Ingredients | % |
|---|---|
| Aqua | ad 100 |
| Sodium Laureth Sulfate | 9 |
| Cocamidopropyl Betaine | 1.5 |
| Decyl Glucoside | 1.5 |
| Helianthus Annuus Seed Oil | 1 |
| Caprylyl/Capryl Glucoside | 1 |
| Sodium Ascorbyl Phosphate | 0.5 |
| Tocopherol | 0.5 |
| Glycerin | 0.5 |
| Composition A | 6.5 |
| Sodium Chloride | 0.3 |
| Citric Acid | 0.2 |
| Cellulose | 0.2 |
| Cellulose Gum | 0.2 |
| Mannitol | 0.2 |
| Microcrystalline Cellulose | 0.2 |
| Algin | 0.2 |
| Sodium Sulfate | 0.2 |
| Sodium Benzoate | 0.2 |
| Benzoic Acid | 0.2 |
| Parfum | 0.2 |
| Cl 77492 | 0.1 |
| Cl 10316 | 0.1 |
| Cl 42090 | 0.1 |

### Example formulation 292: Shower Oil

| Ingredients | % |
|---|---|
| Glycine soja oil | 20 |
| Lecithin | 20 |
| Caprylic/Capric Triglyceride | 15 |
| MIPA-Laureth Sulfate | 10 |
| Laureth-4 | 8 |
| Cocamide DEA | 3 |
| Poloxamer 101 | 2 |
| Composition A | 0.3 |
| Parfum | 0.5 |
| Persea gratissima oil | 0.5 |
| Lactic Acid | 0.5 |
| Aqua | ad 100 |
| Ascorbyl Palmitate | 0.5 |
| Citric Acid | 0.5 |

### Example formulation 293: Berry Shower Foam

| Ingredients | % |
|---|---|
| Aqua | ad 100 |
| Sodium Laureth Sulfate | 9 |
| Butane | 3 |
| Disodium Cocoamphodiacetate | 3 |
| Propane | 3 |
| Dipropylene Glycol | 1 |
| Prunus Amygdalus Dulcis Oil | 0.5 |
| Persea Gratissima Oil | 0.5 |
| Aloe Barbadensis Leaf Extract | 0.5 |
| Caprylic/Capric Triglyceride | 0.5 |
| Composition A | 4.5 |
| Parfum | 0.2 |
| Sodium Chloride | 0.1 |
| Citric Acid | 0.1 |
| Sodium Benzoate | 0.1 |

### Example formulation 294: Refreshing Shower Foam

| Ingredients | % |
|---|---|
| Aqua (Water) | ad 100 |
| Sodium Laureth Sulfate | 12 |
| Butane | 5 |
| Glycerin | 5 |
| PEG-40 Hydrogenated Castor Oil | 5 |
| Isopropyl Palmitate | 1 |
| Propane | 0.5 |
| Isobutane | 0.5 |
| Parfum (Fragrance) | 0.5 |
| Simmondsia Chinensis (Jojoba) Seed Oil | 0.5 |
| Citrus Aurantium Dulcis (Orange) Flower Extract | 0.5 |
| Citrus Aurantium Dulcis (Orange) Peel Oil | 0.5 |
| Citrus Paradisi (Grapefruit) Peel Oil | 0.5 |
| Citrus Nobilis (Mandarin Orange) Oil | 0.5 |
| Citrus Limon (Lemon) Peel Oil | 0.5 |
| Composition A | 3.0 |
| Limonene | 0.2 |
| Linalool | 0.2 |
| Alpha-Isomethyl Ionone | 0.2 |
| Citronellol | 0.2 |
| Sodium Levulinate | 0.2 |
| Sodium Anisate | 0.2 |
| Hydroxyethylcellulose | 0.2 |
| Sorbitol | 0.2 |
| Citric Acid | 0.2 |
| Sodium Hydroxide | 0.2 |

### Example formulation 295: Brilliance Shower Oil

| Ingredients | % |
|---|---|
| Canola Oil | ad 100 |
| Ricinus Communis Seed Oil | 20 |
| Laureth-4 | 20 |
| MIPA-Laureth Sulfate | 16 |
| Parfum | 0.5 |
| Propylene Glycol | 0.5 |
| Tocopheryl Acetate | 0.5 |
| Laureth-2 | 0.5 |
| Tocopherol | 0.5 |
| Composition A | 3.7 |
| Linalool | 0.2 |
| Benzyl Alcohol | 0.2 |
| Citronellol | 0.2 |

### Example formulation 296: Shower Gel II

| Ingredients | |
|---|---|
| Aqua/Water | ad 100 |
| Sodium Laureth Sulfate | 9 |
| Coco-Glucoside | 3 |
| Coco-Betaine | 3 |
| Glycerin | 2 |
| Sodium Chloride | 0.5 |
| Sodium Benzoate | 0.5 |
| Citric Acid | 0.5 |
| Composition A | 0.5 |
| Polyquaternium-10 | 0.2 |
| Parfum/Fragrance | 0.2 |
| Hexyl Cinnamal | 0.2 |
| Alpha-Isomethyl Ionone | 0.2 |
| Coumarin | 0.2 |
| Eugenol | 0.2 |
| Limonene | 0.2 |

### Example formulation 297: Energy Shower Gel

| Ingredients | % |
|---|---|
| Water (Aqua) | ad 100 |
| Alcohol | 5 |
| Disodium Cocoyl Glutamate | 5 |
| Coco-Glucoside | 3 |
| Sodium Cocoyl Glutamate | 3 |
| Glycerin | 3 |
| Xanthan Gum | 0.7 |
| Fragrance (Parfum) | 0.5 |
| Lactic Acid | 0.5 |
| Citric Acid | 0.5 |
| Tocopherol | 0.5 |
| Lecithin | 0.5 |
| Glyceryl Oleate | 0.5 |
| Hydrogenated Palm Glycerides Citrate | 0.5 |
| Composition A | 6.0 |
| Ascorbyl Palmitate | 0.2 |
| Limonene | 0.2 |
| Linalool | 0.2 |
| Citronellol | 0.1 |
| Geraniol | 0.1 |
| Citral | 0.1 |

### Example formulation 298: Natural Shower Gel

| Ingredients | % |
|---|---|
| Aqua | ad 100 |
| Sodium C14-16 Olefin Sulfonate | 6 |
| Cocamidopropyl Betaine | 4.5 |
| Sodium Sulfate | 1.5 |
| Sodium Chloride | 0.7 |
| Glycerin | 0.5 |
| Sucrose Cocoate | 0.5 |
| C12-13 Alkyl Lactate | 0.5 |
| Composition A | 5.0 |
| Hydrolyzed Wheat Protein | 0.2 |
| Hydrolyzed Wheat Gluten Panthenol | 0.2 |
| Valeriana Celtica Extract | 0.2 |
| Salvia Officinali Leaf Extract | 0.2 |
| Citrus Aurantium Dulcis Oil Parfum | 0.2 |
| Sodium Benzoate | 0.2 |
| Potassium Sorbate | 0.2 |
| Citric Acid | 0.2 |
| Alcohol | 0.2 |
| Cl 75120 | 0.1 |
| Limonene | 0.1 |
| Linalool | 0.1 |

### Example formulation 299: Luxurious Shower Oil

| Ingredients | % |
|---|---|
| Aqua | ad 100 |
| Sodium Lauroyl Sarcosinate | 5 |
| Coco-Glucoside | 3 |
| Glycerin | 3 |
| Cocamidopropyl Betaine | 3 |
| Prunus Amygdalus Dulcis (Sweet Almond) Oil | 2 |
| Parfum/Fragrance | 1 |
| Glyceryl Oleate | 1 |
| Acrylates/C10-30 Alkyl Acrylate Crosspolymer | 1 |
| PEG-7 Glyceryl Cocoate | 0.7 |
| Benzyl Alcohol | 0.5 |
| Phenoxyethanol | 0.5 |
| Glyceryl Laurate | 0.5 |
| Sodium Chloride | 0.5 |
| Sodium Gluconate | 0.5 |
| Sodium Hydroxide | 0.5 |
| Citric Acid | 0.5 |
| Mica | 0.5 |
| Composition A | 5.5 |
| Cl 77891/Titanium Dioxide | 0.2 |
| Dehydroacetic Acid | 0.2 |
| Sodium Benzoate | 0.2 |
| CI 77491/Iron Oxides | 0.2 |
| Hydrogenated Palm Glycerides Citrate | 0.2 |
| Tocopherol | 0.2 |
| Benzyl Salicylate | 0.2 |
| Linalool | 0.1 |
| Limonene | 0.1 |
| Citronellol | 0.1 |
| Geraniol [N2210/A] | 0.1 |

### Example formulation 300: Rosemary Shower Oil

| Ingredients | % |
|---|---|
| Prunus Armeniaca Kernel Oil | ad 100 |
| MIPA-Laureth Sulfate | 25 |
| Laureth-4 | 10 |
| Cocamide DEA | 10 |
| Octyldodecanol | 20 |
| Vegetable Oil | 10 |
| Silica | 5 |
| Lavandula Angustifolia Oil | 5 |
| Rosmarinus Officinalis Leaf Oil | 1 |
| Helianthus Annuus Seed Oil | 1 |
| Parfum | 1 |
| Composition A | 3.0 |
| Linalool | 0.2 |
| Limonene | 0.2 |
| BHT | 0.2 |
| Lecithin | 0.2 |
| Geraniol | 0.2 |
| Ascorbyl Palmitate | 0.2 |
| Tocopherol | 0.2 |
| Citral | 0.2 |
| Eugenol | 0.2 |
| Luffa Cylindrica Fruit Powder | 0.1 |
| Cl 77288 | 0.1 |

### Example formulation 301: Sesame Shower Oil

| Ingredients | % |
|---|---|
| Aqua | ad 100 |
| Sodium Laureth Sulfate | 9 |
| PEG-7 Glyceryl Cocoate | 5 |
| Cocamidopropyl Betaine | 3 |
| Helianthus Annuus Seed Oil | 3 |
| Sesamum Indicum Seed Oil | 3 |
| Coco-Glucoside | 1.5 |
| Sodium Chloride | 1 |
| Glyceryl Oleate | 1 |
| PEG-6 Caprylic/Capric Glycerides | 1 |
| PEG-200 Hydrogenated Glyceryl Palmate | 1 |
| Parfum | 0.5 |
| Glycerin | 0.5 |
| Phyllanthus Emblica Fruit Extract | 0.5 |
| Tocopherol | 0.5 |
| Tetrasodium Glutamate Diacetate | 0.5 |
| Citric Acid | 0.5 |
| Phenoxyethanol | 0.5 |
| Composition A | 0.5 |
| Sodium Benzoate | 0.2 |
| Benzyl Salicylate | 0.2 |
| Citronellol | 0.2 |
| Limonene | 0.2 |
| Linalool | 0.2 |
| Caramel | 0.2 |

### Example formulation 302: Happy Shower Oil

| Ingredients | % |
|---|---|
| Aqua | ad 100 |
| Sodium Laureth Sulfate | 12 |
| Laureth-4 | 3 |
| Isopentane | 3 |
| Isopropyl Palmitate | 3 |
| Isobutane | 3 |
| Glycerin | 1 |
| Parfum | 0.5 |
| Sorbitol | 0.5 |
| Composition A | 1.5 |
| Limonene | 0.2 |
| Linalool | 0.2 |
| Citric Acid | 0.2 |
| Hydroxypropyl Guar Hydroxypropyltrimonium Chloride | 0.15 |
| Sodium Benzoate | 0.2 |
| Citral | 0.2 |

### Example formulation 303: Vitality Bubble Bath

| Ingredients | % |
|---|---|
| Aqua | ad 100 |
| Disodium Laureth Sulfosuccinate | 6 |
| Sodium C14-16 Olefin Sulfonate | 4.5 |
| Sodium Laureth Sulfate | 3.5 |
| Laureth-2 | 1.5 |
| Glycine | 0.7 |
| Parfum | 0.5 |
| Hydrolyzed Pea Protein | 0.5 |
| Allantoin | 0.5 |
| Composition A | 8.0 |
| Niacinamide | 0.3 |
| Alanine | 0.2 |
| Lysine | 0.2 |
| Leucine | 0.2 |
| Magnesium Aspartate | 0.2 |
| Sodium Lactate | 0.2 |
| Propylene Glycol | 0.2 |
| Butylene Glycol | 0.2 |
| Sodium Chloride | 0.2 |
| PEG-55 Propylene Glycol Oleate | 0.2 |
| Benzyl Alcohol | 0.2 |
| Phenoxyethanol | 0.2 |
| Sodium Benzoate | 0.2 |
| Cl 47005 | 0.1 |
| Cl 42090 | 0.1 |

### Example formulation 304: Rich Shower Cream

| Ingredients | % |
|---|---|
| Aqua | ad 100 |
| Cocamidopropyl Betaine | 6 |
| Sodium Hydroxypropyl Starch Phosphate | 3 |
| Lauric Acid | 1.5 |
| Sodium Lauroyl Glycinate | 1.5 |
| Sodium Lauroyl Isethionate | 1.5 |
| Sodium Chloride | 1 |
| Glycerin | 1 |
| Hydrogenated Soybean Oil | 1 |
| Helianthus Annuus Seed Oil | 1 |
| Parfum | 0.5 |
| Sodium Benzoate | 0.5 |
| Caprylyl Glycol | 0.5 |
| Sodium Hydroxide | 0.5 |
| Citric Acid | 0.5 |
| Sodium Isethionate | 0.5 |
| Composition A | 2.0 |
| Stearic Acid | 0.3 |
| Palmitic Acid | 0.3 |
| Sodium Gluconate | 0.3 |
| Argania Spinosa Kernel Oil | 0.3 |
| Caprylic Acid | 0.3 |
| Guar Hydroxypropyltrimonium Chloride | 0.2 |
| Hydroxystearic Acid | 0.2 |
| Hydrogenated Vegetable Glycerides | 0.2 |
| Sodium Methyl Lauroyl Taurate | 0.2 |
| Hexyl Cinnamal | 0.1 |
| Limonene | 0.1 |
| Linalool | 0.1 |

### Example formulation 305: Wash Cream for Babies

| Ingredients | % |
|---|---|
| Aqua | ad 100 |
| Glycerin | 6 |
| Cocamidopropyl Betaine | 6 |
| PEG-80 Sorbitan Laurate | 2 |
| PEG-150 Pentaerythrityl Tetrastearate | 2 |
| Ethylhexylglycerin | 1.5 |
| Coconut Acid | 1.5 |
| Glycol Distearate | 1.5 |
| PPG-2 Hydroxyethyl Cocamide | 1.5 |
| Decyl Glucoside | 1.5 |
| Sodium Methyl Cocoyl Taurate | 1.5 |
| Laureth-4 | 1 |
| Sodium Chloride | 0.75 |
| Citric Acid | 0.5 |
| Formic Acid | 0.5 |
| Composition A | 0.7 |
| Phenoxyethanol | 0.2 |
| Sodium Benzoate | 0.2 |
| Parfum | 0.2 |

### Example formulation 306: Natural Shower Cream

| Ingredients | % |
|---|---|
| Aqua | ad 100 |
| Helianthus Annuus Seed Oil | 5 |
| Coco-Glucoside | 4 |
| Glycerin | 4 |
| Decyl Glucoside | 3 |
| Alcohol | 3 |
| Persea Gratissima Oil | 1.5 |
| Pyrus Cydonia Seed Extract | 1.5 |
| Sodium Cocoyl Glutamate | 1.5 |
| Disodium Cocoyl Glutamate | 1.5 |
| Xanthan Gum | 0.5 |
| Citrus Limon Peel Oil | 0.5 |
| Cymbopogon Flexuosus Oil | 0.5 |
| Composition A | 8.0 |
| Parfum | 0.3 |
| Limonene | 0.2 |
| Citral | 0.2 |
| Geraniol | 0.1 |
| Linalool | 0.1 |
| Citronellol | 0.1 |
| Farnesol | 0.1 |
| Citric Acid | 0.1 |
| Hectorite | 0.1 |

### Example formulation 307: Dreamy Shower Cream

| Ingredients | % |
|---|---|
| Aqua | ad 100 |
| Vitis Vinifera (Grape) Seed Oil | 8 |
| Coco-Glucoside | 6 |
| Glycerin | 5 |
| Butyrospermum Parkii (Shea) Butter | 2.5 |
| Glyceryl Oleate | 2 |
| Disodium Cocoyl Glutamate | 1.5 |
| Sodium Cocoyl Glutamate | 1.5 |
| Carrageenan | 1 |
| Vanilla Planifolia Fruit Extract | 1 |
| Cocos Nucifera (Coconut) Oil | 1 |
| Prunus Domestica Seed Extract | 1 |
| Glyceryl Caprylate | 1 |
| Xanthan Gum | 0.5 |
| Tocopherol | 0.5 |
| Helianthus Annuus (Sunflower) | 0.5 |
| Seed Oil | 0.5 |
| Composition A | 3.5 |
| Lactic Acid | 0.2 |
| Citric Acid | 0.2 |
| Phytic Acid | 0.2 |
| Hydrogenated Palm Glycerides Citrate | 0.2 |
| Potassium Sorbate | 0.2 |
| Parfum (Fragrance) | 0.1 |
| Coumarin | 0.1 |
| Limonene | 0.1 |

### Example formulation 308: Shower Gels with Actives

| Ingredients | % | % | % | % | % | % | % | % | % | % | % | % |
|---|---|---|---|---|---|---|---|---|---|---|---|---|
| Canola Oil | ad 10 0 | ad 10 0 | ad 100 | ad 10 0 | ad 10 0 | ad 10 0 | ad 10 0 | ad 100 | ad 10 0 | ad 100 | ad 100 | ad 10 0 |
| Ricinus Communis Seed Oil | 20 | 20 | 20 | 20 | 20 | 20 | 20 | 20 | 20 | 20 | 20 | 20 |
| Laureth-4 | 20 | 20 | 20 | 20 | 20 | 20 | 20 | 20 | 20 | 20 | 20 | 20 |
| MIPA-Laureth Sulfate | 16 | 16 | 16 | 16 | 16 | 16 | 16 | 16 | 16 | 16 | 16 | 16 |
| Parfum | 0.5 | 0.5 | 0.5 | 0.5 | 0.5 | 0.5 | 0.5 | 0.5 | 0.5 | 0.5 | 0.5 | 0.5 |
| Propylene Glycol | 0.5 | 0.5 | 0.5 | 0.5 | 0.5 | 0.5 | 0.5 | 0.5 | 0.5 | 0.5 | 0.5 | 0.5 |
| Tocopheryl Acetate | 0.5 | 0.5 | 0.5 | 0.5 | 0.5 | 0.5 | 0.5 | 0.5 | 0.5 | 0.5 | 0.5 | 0.5 |
| Laureth-2 | 0.5 | 0.5 | 0.5 | 0.5 | 0.5 | 0.5 | 0.5 | 0.5 | 0.5 | 0.5 | 0.5 | 0.5 |
| 9.Tocopherol | 0.5 | 0.5 | 0.5 | 0.5 | 0.5 | 0.5 | 0.5 | 0.5 | 0.5 | 0.5 | 0.5 | 0.5 |
| Composition A | 0.2 | 8.0 | 1.4 | 3.0 | 3.5 | 6.0 | 11 | 9.0 | 2.5 | 8.0 | 7.5 | 1.0 |
| Hyaluronic Acid | 1 | 2 | 1 | 2 | 1 | 2 | 1 | 1 | 2 | 1 | 1 | 2 |
| Niacinamide | 0.5 | 1 | | | | | | | | | | |
| Retinol | | | 0.7 5 | 1.5 | | | | 0.0 1 | | | | |
| Lecithin | | | | | 1 | 1.5 | | | | | 0.3 | |
| Coffeine | | | | | | | 1.5 | 2.7 5 | 3.5 | | | |
| Peptides | | | 0.2 | | 0.5 | | | | | 0.7 5 | 1.2 5 | 2 |
| Linalool | 0.2 | 0.2 | 0.2 | 0.2 | 0.2 | 0.2 | 0.2 | 0.2 | 0.2 | 0.2 | 0.2 | 0.2 |
| Benzyl Alcohol | 0.2 | 0.2 | 0.2 | 0.2 | 0.2 | 0.2 | 0.2 | 0.2 | 0.2 | 0.2 | 0.2 | 0.2 |
| Citronellol | 0.2 | 0.2 | 0.2 | 0.2 | 0.2 | 0.2 | 0.2 | 0.2 | 0.2 | 0.2 | 0.2 | 0.2 |

### Example formulation 309: Hydro Cleansing Gel

| Ingredients | % |
|---|---|
| Aqua | ad 100 |
| Glycerin | 5 |
| Cocamidopropyl Hydroxysultaine | 5 |
| Sodium Cocoyl Isethionate | 3 |
| Sodium Methyl Cocoyl Taurate | 2 |
| Sodium Chloride | 1 |
| Citric Acid | 0.8 |
| Sodium Hydrolyzed Potato Starch Dodecenylsuccinate | 0.5 |
| Potassium Acrylates Copolymer | 0.5 |
| Hydroxyacetophenone | 0.5 |
| Phenoxyethanol | 0.5 |
| Sodium Hydroxide | 0.5 |
| Composition A | 6.5 |
| Linoleamidopropyl PG-dimonium Chloride Phosphate | 0.3 |
| Propylene Glycol | 0.3 |
| Coconut Acid | 0.3 |
| Polyquaternium-10 | 0.2 |
| Disodium EDTA | 0.2 |
| Ethylhexylglycerin | 0.2 |
| Disodium Tetrapropenyl Succinate | 0.2 |
| Hydrolyzed Hyaluronic Acid | 0.2 |
| Tocopheryl Acetate | 0.2 |
| Tocopherol | 0.2 |

### Example formulation 310: Grape Cleansing Oil

| Ingredients | |
|---|---|
| Aqua (Water) | ad 100 |
| Coco-Glucoside | 4 |
| Lauryl Glucoside | 4 |
| Vitis Vinifera (Grape) Seed Oil | 1.5 |
| Glycerin | 1.5 |
| Dehydroxanthan Gum | 1.5 |
| Glyceryl Oleate | 1 |
| Carrageenan | 0.5 |
| Levulinic Acid | 0.5 |
| p-Anisic Acid | 0.5 |
| Sodium Levulinate | 0.5 |
| Sodium Lactate | 0.5 |
| Plukenetia Volubilis Seed Oil | 0.5 |
| Composition A | 2.0 |
| Lactococcus Ferment Lysate | 0.2 |
| Lactic Acid | 0.2 |
| PCA Ethyl Cocoyl Arginate | 0.2 |
| Citric Acid | 0.2 |
| Sodium Chloride | 0.2 |
| Tocopherol | 0.2 |
| Phenylpropanol | 0.2 |
| Hydrogenated Palm Glycerides Citrate | 0.2 |
| Lecithin | 0.2 |
| Ascorbyl Palmitate | 0.2 |
| Parfum (Fragrance) | 0.1 |
| Linalool | 0.1 |
| Limonene | 0.1 |

### Example formulation 311: Ultra Sensitive Cleansing Gel

| Ingredients | % |
|---|---|
| Aqua | ad 100 |
| Glycerin | 5 |
| Pentylene Glycol | 5 |
| Cocamidopropyl Betaine | 3 |
| Decyl Glucoside | 2 |
| Xanthan Gum | 1 |
| Citric Acid | 1 |
| Sodium Chloride | 0.5 |
| Composition A | 4.5 |
| Panthenol | 0.3 |
| Bisabolol | 0.2 |

### Example formulation 312: Vitamin C Cleansing Gel

| Ingredients | % |
|---|---|
| Aqua | ad 100 |
| Glycerin | 6 |
| Cocamidopropyl Betaine | 4 |
| Sodium C14-16 Olefin Sulfonate | 2 |
| Glycol Distearate | 1.5 |
| Triethyl Citrate | 1 |
| PEG-55 Propylene Glycol Oleate | 1 |
| Sodium Chloride | 0.5 |
| Sodium Hydroxide | 0.5 |
| Coco-Betaine | 0.5 |
| Ascorbyl Glucoside | 0.5 |
| Propylene Glycol | 0.5 |
| Composition A | 2.5 |
| Citric Acid | 0.3 |
| Acrylates Copolymer | 0.2 |
| Sodium Benzoate | 0.2 |
| Benzoic Acid | 0.2 |
| Linalool | 0.2 |
| Limonene | 0.2 |
| Parfum / Fragrance (F.I.L. Z70034188/1) | 0.1 |

### Example formulation 313: Cleansing Milk

| Ingredients | % |
|---|---|
| Aqua | ad 100 |
| Isopropyl Palmitate | 5 |
| Glycerin | 4 |
| Dicaprylyl Ether | 3 |
| Glyceryl Stearate SE | 2 |
| Cetearyl Alcohol | 2 |
| Nelumbo Nucifera Flower Extract | 1 |
| Sodium Cetearyl Sulfate | 1 |
| Tocopheryl Acetate | 0.5 |
| Tocopherol | 0.5 |
| Xanthan Gum | 0.5 |
| Sodium Hydroxide | 0.5 |
| Composition A | 1.0 |
| Carbomer | 0.3 |
| Phenoxyethanol | 0.2 |
| Methylparaben | 0.2 |
| Geraniol | 0.2 |
| Benzyl Alcohol | 0.2 |
| Linalool | 0.2 |
| Alpha-Isomethyl Ionone | 0.2 |
| Limonene | 0.1 |
| Citronellol | 0.1 |
| Triethanolamine | 0.1 |
| Parfum | 0.1 |

### Example formulation 314: Wild Roses Cleansing Milk

| Ingredients | % |
|---|---|
| Aqua | ad 100 |
| Glycine Soja Oil | 10 |
| Glycerin | 5 |
| Alcohol | 5 |
| Glyceryl Stearate Citrate | 4 |
| Myristyl Myristate | 4 |
| Cetearyl Alcohol | 4 |
| Helianthus Annuus Seed Cera | 2 |
| Butyrospermum Parkii Butter | 1 |
| Helianthus Annuus Seed Oil | 1 |
| Rosa Canina Fruit Extract | 0.5 |
| Rosa Canina Fruit Oil | 0.5 |
| Macadamia Ternifolia Seed Oil | 0.5 |
| Simmondsia Chinensis Seed Oil | 0.5 |
| Vitis Vinifera Seed Oil | 0.5 |
| Tocopherol | 0.5 |
| Xanthan Gum | 0.5 |
| Composition A | 3.0 |
| Sodium Citrate | 0.2 |
| Levulinic Acid | 0.2 |
| Sodium Levulinate | 0.2 |
| p-Anisic Acid | 0.2 |
| Sodium Hydroxide | 0.2 |
| Alcohol | 0.2 |
| Parfum | 0.1 |
| Linalool | 0.1 |
| Geraniol | 0.1 |
| Limonene | 0.1 |
| Citronellol | 0.1 |
| Eugenol | 0.1 |

### Example formulation 315: Vitamin C Cleansing Foam

| Ingredients | % |
|---|---|
| Aqua | ad 100 |
| Sodium Laureth Sulfate | 4 |
| Coco-Betaine | 3 |
| Sodium Chloride | 2 |
| Glycerin | 2 |
| PPG-5-Ceteth-20 | 1.5 |
| Sodium Hydroxide | 0.5 |
| Ascorbyl Glucoside | 0.5 |
| Citric Acid | 0.5 |
| Panthenol | 0.5 |
| Composition A | 2.0 |
| Polyquaternium-7 | 0.3 |
| Tocopheryl Acetate | 0.3 |
| Sodium Benzoate | 0.2 |
| Salicylic Acid | 0.2 |
| Cl 15510 / Orange 4 | 0.1 |
| Parfum / Fragrance (F.I.L. Z70027052/1) | 0.1 |

### Example formulation 316: Cleansing Foam for Kids

| Ingredients | % |
|---|---|
| Aqua | ad 100 |
| Coco-Glucoside | 5 |
| Glycerin | 5 |
| Sodium Cocoyl Glutamate | 3 |
| Glyceryl Oleate | 2 |
| Citric Acid | 1 |
| Glycol Distearate | 1 |
| Sodium Benzoate | 0.5 |
| Parfum | 0.5 |
| Sodium Laureth Sulfate | 0.5 |
| Sodium Chloride | 0.5 |
| Tetrasodium Glutamate Diacetate | 0.5 |
| Cocamidopropyl Betaine | 0.5 |
| Sodium Sulfate | 0.5 |
| Composition A | 4.0 |
| Denatonium Benzoate | 0.2 |
| Sodium Hydroxide | 0.2 |
| Potassium Sorbate | 0.2 |
| Tocopherol | 0.2 |
| Hydrogenated Vegetable Glycerides Citrate | 0.2 |
| Lecithin | 0.1 |
| Ascorbyl Palmitate | 0.1 |
| Benzyl Alcohol | 0.1 |
| Cl 10316 | 0.1 |
| Cl 42051 | 0.1 |

### Example formulation 317: Soft Cleansing Gel

| Ingredients | % |
|---|---|
| Aqua | ad 100 |
| Mipa-Laureth Sulfate | 5 |
| Cocamidopropyl Betaine | 3 |
| Sodium Lauroyl Glutamate | 1 |
| Sodium Lauroyl Sarcosinate | 1 |
| Sodium Levulinate | 0.5 |
| Glycol Stearate | 0.5 |
| PEG-15 Glyceryl Isostearate | 0.5 |
| PEG-90 Glyceryl Isostearate | 0.5 |
| Composition A | 7.0 |
| Parfum (Fragrance) | 0.2 |
| Lactic Acid | 0.2 |
| Panisic Acid | 0.2 |
| Alcohol | 0.2 |
| Laureth-2 | 0.2 |
| Tilia Cordata Flower Water | 0.2 |
| Sodium Benzoate | 0.2 |
| Ethylparaben | 0.2 |

### Example formulation 318: Honey Cleansing Gel

| Ingredients | % |
|---|---|
| Aqua/Water | ad 100 |
| Glycerin | 6 |
| Sodium Cocoamphoacetate | 4.5 |
| Sodium Lauroyl Sarcosinate | 2.5 |
| Lauryl Glucoside | 1.5 |
| Parfum/Fragrance | 1 |
| Mel/Honey | 1 |
| Sunflower Seed Oil PEG-8 Esters | 1 |
| Sodium Cocoyl Glutamate | 1 |
| Sodium Lauryl Glucose Carboxylate | 1 |
| Acrylates/C10-30 Alkyl Acrylate Crosspolymer | 1 |
| Phenoxyethanol | 0.5 |
| Gluconolactone | 0.5 |
| Sodium Hydroxide | 0.5 |
| Coco-Glucoside | 0.5 |
| Glyceryl Oleate | 0.5 |
| Composition A | 2.5 |
| Citric Acid | 0.3 |
| Allantoin | 0.3 |
| Sodium Benzoate | 0.2 |
| Tetrasodium Glutamate Diacetate | 0.2 |
| 1.2-Hexanediol | 0.2 |
| Caprylyl Glycol | 0.2 |
| Caramel | 0.1 |
| Calcium Gluconate | 0.1 |
| Tropolone | 0.1 |
| Hydrogenated Palm Glycerides Citrate | 0.1 |
| Tocopherol [N2002/A] | 0.1 |

### Example formulation 319: Clean Cleansing Gel

| Ingredients | % |
|---|---|
| Aqua | ad 100 |
| Caprylic/Capric Triglyceride | 6 |
| Sorbitol | 5 |
| Phenoxyethanol | 1 |
| Peg-40 Hydrogenated Castor Oil | 1 |
| Glycerin | 1 |
| Peg-6 Stearate | 0.5 |
| Carbomer | 0.5 |
| Trideceth-9 | 0.5 |
| Sodium Hydroxide | 0.5 |
| Ethylhexylglycerin | 0.5 |
| Composition A | 1.8 |
| Fragrance (Parfum) | 0.2 |
| Aloe Barbadensis Leaf Juice Powder | 0.2 |
| Tetrasodium Glutamate Diacetate | 0.2 |
| Butylene Glycol | 0.2 |
| Propylene Glycol | 0.2 |
| Sodium Benzoate | 0.2 |
| Glucose | 0.2 |
| Urtica Dioica (Nettle) Leaf Extract | 0.2 |
| Potassium Sorbate | 0.2 |

### Example formulation 320: Soft Cleansing Milk

| Ingredients | % |
|---|---|
| Aqua | ad 100 |
| C15-19 Alkane | 5 |
| Cetearyl Alcohol | 5 |
| Poloxamer 184 | 2.5 |
| Caprylic/Capric Triglyceride | 2.5 |
| Propanediol | 1 |
| Dicaprylyl Ether | 1 |
| Arachidyl Alcohol | 1 |
| Ethylhexyl Stearate | 1 |
| Helianthus Annuus (Sunflower) Seed Oil | 1 |
| Behenyl Alcohol | 0.5 |
| Glycerin | 0.5 |
| Arachidyl Glucoside | 0.5 |
| Ethylhexylglycerin | 0.5 |
| Parfum / Fragrance | 0.5 |
| Sodium Benzoate | 0.5 |
| Composition A | 3.0 |
| Xanthan Gum | 0.2 |
| Maris Aqua / Sea Water / Eau De Mer | 0.2 |
| Tocopherol | 0.2 |
| Citric Acid | 0.2 |
| Phenethyl Alcohol | 0.2 |
| Laminaria Digitata Extract | 0.2 |
| Chlorella Vulgaris Extract | 0.2 |
| Saccharide Isomerate | 0.1 |
| 4090A | 0.1 |

### Example formulation 321: Natural Cleansing Milk

| Ingredients | % |
|---|---|
| Aqua | ad 100 |
| Alcohol | 8 |
| Anthyllis Vulneraria Extract | 2 |
| Glycerin | 2 |
| Simmondsia Chinensis Seed Oil | 1 |
| Prunus Amygdalus Dulcis Oil | 1 |
| Prunus Armeniaca Kernel Oil | 1 |
| Cetearyl Alcohol | 1 |
| Lactobacillus/Oat/Rye/Wheat Seed Extract Ferment | 0.5 |
| Composition A | 4.0 |
| Bentonite | 0.2 |
| Lecithin | 0.2 |
| Xanthan Gum | 0.2 |
| Hydrolyzed Wheat Gluten | 0.2 |
| Parfum | 0.1 |
| Limonene | 0.1 |
| Linalool | 0.1 |
| Geraniol | 0.1 |
| Citronellol | 0.1 |

### Example formulation 322: Cleansing Foam for Kids

| Ingredients | % |
|---|---|
| Aloe Barbadensis (Aloe) Leaf Juice | ad 100 |
| Caprylyl/Capryl Glucoside | 5 |
| Cocamidopropyl Betaine | 4 |
| Glycerin | 3 |
| Butylene Glycol | 1.5 |
| Pentylene Glycol | 1.5 |
| Sodium PCA | 0.5 |
| Sucrose Cocoate | 0.5 |
| Composition A | 1.5 |
| Chamomilla Recutita (Camomile) Flower Extract | 0.3 |
| Linum Usitatissimum (Flax/Linseed) Seed Extract | 0.3 |
| Lactic Acid | 0.2 |
| Vanillin | 0.2 |

### Example formulation 323: Thermal Cleansing Foam

| Ingredients | % |
|---|---|
| Aqua | ad 100 |
| Alcohol | 6 |
| Aloe Barbadensis Leaf Juice | 5 |
| Glycerin | 5 |
| Coco-Glucoside | 3 |
| Glyceryl Oleate | 2 |
| Disodium Cocoyl Glutamate | 2 |
| Sodium Cocoyl Glutamate | 1 |
| Betaine | 1 |
| Composition A | 0.2 |
| Hydrolyzed Soy Protein | 0.3 |
| Valeriana Celtica Extract | 0.3 |
| Pelvetia Canaliculata Extract | 0.3 |
| Linoleic Acid | 0.2 |
| Linolenic Acid | 0.2 |
| Beta Vulgaris Root Extract | 0.2 |
| Hydrolyzed Corn Starch | 0.2 |
| Poria Cocos Extract | 0.2 |
| Phragmites Kharka Extract | 0.2 |
| Parfum | 0.2 |
| Citric Acid Sodium Benzoate | 0.2 |
| Benzyl Alcohol | 0.2 |
| Citral | 0.1 |
| Coumarin | 0.1 |
| Limonene | 0.1 |
| Linalool | 0.1 |

### Example formulation 324: Cleansing Gel with Actives

| Ingredients | % | % | % | % | % | % | % | % | % | % | % | % |
|---|---|---|---|---|---|---|---|---|---|---|---|---|
| AQUA | ad 100 | ad 100 | ad 100 | ad 10 0 | ad 10 0 | ad 100 | ad 100 | ad 10 0 | ad 100 | ad 10 0 | ad 10 0 | ad 100 |
| GLYCERIN | 5 | 5 | 5 | 5 | 5 | 5 | 5 | 5 | 5 | 5 | 5 | 5 |
| PENTYLENE GLYCOL | 5 | 5 | 5 | 5 | 5 | 5 | 5 | 5 | 5 | 5 | 5 | 5 |
| COCAMIDOPROP YL BETAINE | 3 | 3 | 3 | 3 | 3 | 3 | 3 | 3 | 3 | 3 | 3 | 3 |
| DECYL GLUCOSIDE | 2 | 2 | 2 | 2 | 2 | 2 | 2 | 2 | 2 | 2 | 2 | 2 |
| XANTHAN GUM | 1 | 1 | 1 | 1 | 1 | 1 | 1 | 1 | 1 | 1 | 1 | 1 |
| CITRIC ACID | 1 | 1 | 1 | 1 | 1 | 1 | 1 | 1 | 1 | 1 | 1 | 1 |
| SODIUM CHLORIDE | 0.5 | 0.5 | 0.5 | 0.5 | 0.5 | 0.5 | 0.5 | 0.5 | 0.5 | 0.5 | 0.5 | 0.5 |
| Composition A | 0.2 | 1.5 | 3.0 | 2.5 | 6.0 | 7.6 | 0.0 5 | 10 | 4.5 | 5.0 | 3.0 | 1.0 |
| Hyaluronic Acid | 1 | 2 | 1 | 2 | 1 | 2 | 1 | 2 | 1 | 2 | 1 | 2 |
| Niacinamide | 4.5 | 4.7 5 | | | | | | | | | 2.5 | 3.2 5 |
| Retinol | 0.00 1 | | 0.7 5 | 1.5 | | | 0.0 5 | | | | | |
| Lecithin | | | | | 1.5 | 2.7 5 | | | | | | |
| Coffeine | | | | | | | 1.7 5 | 2.5 | | | | |
| Peptides | 0.5 | | | 0.1 | | | | 1.5 | 0.7 5 | 1.5 | | |
| PANTHENOL | 0.3 | 0.3 | 0.3 | 0.3 | 0.3 | 0.3 | 0.3 | 0.3 | 0.3 | 0.3 | 0.3 | 0.3 |
| BISABOLOL | 0.2 | 0.2 | 0.2 | 0.2 | 0.2 | 0.2 | 0.2 | 0.2 | 0.2 | 0.2 | 0.2 | 0.2 |

### Example formulation 325: Bi Phasic Make-Up Remover

| Ingredients | % |
|---|---|
| Aqua / Water | ad 100 |
| Cyclopentasiloxane | 8 |
| Isohexadecane | 5 |
| Isopropyl Palmitate | 3 |
| Sodium Chloride | 0.5 |
| Dipotassium Phosphate | 0.5 |
| Poloxamer 184 | 0.5 |
| Composition A | 9.0 |
| Potassium Phosphate | 0.2 |
| Panthenol | 0.2 |
| Polyaminopropyl Biguanide | 0.2 |
| Cl 61565 / Green 6 (F.I.L. B4885/3) | 0.1 |

### Example formulation 326: Waterproof Make-Up Entferner

| Ingredients | % |
|---|---|
| Aqua | ad 100 |
| Cyclopentasiloxane | 10 |
| Isohexadecane | 10 |
| Decyl Glucoside | 2 |
| CI 61565 / Green 6 | 0.5 |
| Dipotassium Phosphate | 0.5 |
| Composition A | 0.9 |
| Disodium Edta | 0.2 |
| Hexylene Glycol | 0.2 |
| Polyaminopropyl Biguanide | 0.2 |
| Potassium Phosphate | 0.2 |
| Sodium Chloride (F.I.L. B4989/5) | 0.2 |

### Example formulation 327: Effective Make Up Remover

| Ingredients | % |
|---|---|
| Aqua | ad 100 |
| Isododecane | 5 |
| C15-19 Alkane | 5 |
| Isopropyl Palmitate | 4 |
| Ethylhexyl Cocoate | 3 |
| Glycerin | 3 |
| Dicaprylyl Ether | 2 |
| Helianthus Annuus Seed Oil | 1 |
| Caprylyl/Capryl Glucoside | 1 |
| Biotin | 0.5 |
| Centaurea Cyanus Flower Extract | 0.5 |
| Tocopherol | 0.5 |
| Sodium Chloride | 0.5 |
| Sodium Hydroxide | 0.5 |
| Composition A | 0.7 |
| Trisodium EDTA | 0.2 |
| Phenoxyethanol | 0.2 |
| Benzethonium Chloride | 0.2 |
| Cl 60725 | 0.1 |
| Cl 61565 | 0.1 |

### Example formulation 328: Calendula Make Up Remover

| Ingredients | % |
|---|---|
| Aqua | ad 100 |
| Glycine Soja Oil | 2 |
| Glycerin | 2 |
| Cetearyl Alcohol | 1 |
| Glyceryl Stearate Citrate | 1 |
| Caprylic/Capric Triglyceride | 1 |
| Glyceryl Caprylate | 1 |
| Butyrospermum Parkii Butter | 0.5 |
| Calendula Officinalis Flower Extract | 0.5 |
| Simmondsia Chinensis Seed Oil | 0.5 |
| Helianthus Annuus Seed Oil | 0.5 |
| Alcohol | 0.5 |
| Composition A | 4.5 |
| Sodium Levulinate | 0.2 |
| Tocopherol | 0.2 |
| Xanthan Gum | 0.2 |
| Sodium Stearoyl Glutamate | 0.2 |
| Sodium Anisate | 0.2 |
| Citric Acid | 0.2 |

### Example formulation 329: BiPhasic Make Up Remover

| Ingredients | % |
|---|---|
| Aqua | ad 100 |
| Ethylhexyl Stearate | 20 |
| Caprylyl Caprylate / Caprate | 3 |
| Helianthus Annuus Hybrid Oil | 1 |
| Panthenol | 0.5 |
| Sodium Chloride | 0.5 |
| Composition A | 2.3 |
| Potassium Phosphate | 0.2 |
| Citric Acid | 0.2 |
| Tocopherol | 0.2 |
| Sodium Benzoate | 0.2 |
| Cl 61565 | 0.1 |

### Example formulation 330: Eye Make-Up Remover

| Ingredients | % |
|---|---|
| Water | ad 100 |
| Aloe Barbadensis Leaf Juice | 10 |
| Glycerin | 5 |
| Alcohol* denat. | 5 |
| Pentylene Glycol | 3 |
| Polyglyceryl-10 Laurate | 3 |
| Tocopherol | 0.5 |
| Helianthus Annuus (Sunflower) Seed Oil | 0.5 |
| Composition A | 0.45 |
| Glycyrrhiza Glabra (Licorice) Root Extract | 0.3 |
| Hippophae Rhamnoides Fruit Extract | 0.3 |
| Chamomilla Recutita (Matricaria) Flower Extract | 0.3 |
| Levulinic Acid | 0.2 |
| Arginine | 0.2 |
| Sodium Levulinate | 0.2 |
| Tridecane | 0.2 |
| Xanthan Gum | 0.2 |
| Undecane | 0.2 |
| Sodium Anisate | 0.2 |
| Sodium Hydroxide | 0.2 |
| Dipotassium Glycyrrhizate | 0.2 |
| Lactic Acid | 0.2 |
| Fragrance (Parfum) | 0.1 |
| Limonene | 0.1 |
| Linalool | 0.1 |
| Citral | 0.1 |
| Geraniol | 0.1 |

### Example formulation 331: Micellar Make-Up Remover

| Ingredients | % |
|---|---|
| Water | ad 100 |
| Dodecane | 8 |
| Caprylic/Capric Triglyceride | 5 |
| Glycerin | 5 |
| Alcohol* denat. | 4 |
| Pentylene Glycol | 2 |
| Aloe Barbadensis Leaf Juice | 2 |
| Cucumis Sativus (Cucumber) Fruit Extract | 1 |
| Chamomilla Recutita (Matricaria) Flower Extract | 0.5 |
| Vitis Vinifera (Grape) Fruit Extract | 0.5 |
| Levulinic Acid | 0.5 |
| Sodium Levulinate | 0.5 |
| Sodium Hydroxide | 0.5 |
| Sodium Anisate | 0.5 |
| Arginine | 0.5 |
| Tocopherol | 0.5 |
| Helianthus Annuus (Sunflower) Seed Oil | 0.5 |
| Disodium Cocoyl Glutamate | 0.5 |
| Sodium Cocoyl Glutamate | 0.5 |
| Composition A | 0.35 |
| Lactic Acid | 0.2 |
| Fragrance (Parfum) | 0.2 |
| Limonene | 0.2 |
| Benzyl Salicylate | 0.2 |
| Linalool | 0.1 |
| Citral | 0.1 |
| Citronellol | 0.1 |
| Geraniol | 0.1 |

### Example formulation 332: Oil free Make Up Remover

| Ingredients | % |
|---|---|
| Aqua | ad 100 |
| Aloe Barbadensis Leaf Juice | 15 |
| Glycerin | 10 |
| Decyl Glucoside | 2 |
| Composition A | 1.3 |
| Sodium Benzoate | 0.2 |
| Levulinic Acid | 0.2 |
| Lactic Acid | 0.2 |
| Sodium Levulinate | 0.2 |

### Example formulation 333: 2in1 Make Up Remover

| Ingredients | % |
|---|---|
| Aqua / Water | ad 100 |
| Isododecane | 15 |
| Isopropyl Palmitate | 10 |
| Undecane | 5 |
| Arginine | 2 |
| Cl 60725 / Violet 2 | 0.5 |
| Citric Acid | 0.5 |
| Dipotassium Phosphate | 0.5 |
| Disodium EDTA | 0.5 |
| Hexylene Glycol | 0.5 |
| Octyldodecanol | 0.5 |
| Composition A | 3.3 |
| Octyldodecyl Xyloside | 0.3 |
| Polyaminopropyl Biguanide | 0.2 |
| Potassium Phosphate | 0.2 |
| Sodium Chloride | 0.2 |
| Tridecane (F.I.L. B162099/2) | 0.2 |

### Example formulation 334: 2 Phase Make Up Remover

| Ingredients | % |
|---|---|
| Aqua | ad 100 |
| Glycine Soja Oil | 10 |
| Glycerin | 5 |
| Pentylene Glycol | 3 |
| Helianthus Annuus Seed Oil | 2 |
| Helianthus Annuus Hybrid Oil | 1 |
| Persea Gratissima Oil | 1 |
| Coco-Glucoside | 0.8 |
| Sodium Lactate | 0.5 |
| Levulinic Acid | 0.5 |
| Sodium Levulinate | 0.5 |
| Simmondsia Chinensis Seed Oil | 0.5 |
| Olea Europaea Fruit Oil | 0.5 |
| Tocopherol | 0.5 |
| Composition A | 7.5 |
| Cl 75810 | 0.2 |
| Prunus Amygdalus Dulcis Seed Extract | 0.2 |
| Aloe Barbadensis Leaf Extract | 0.2 |
| Lactic Acid | 0.2 |
| Sodium Hydroxide | 0.2 |
| Parfum | 0.1 |
| Limonene | 0.1 |

### Example formulation 335: Cleansing Gel and Make Up Remover

| Ingredients | % |
|---|---|
| Caprylic/Capric Triglyceride | 20 |
| Rizinus Communis Seed Oil | 15 |
| Aqua | ad 100 |
| Glycerin | 5 |
| Sesamum Indicum Seed Oil | 4 |
| Alcohol | 4 |
| Prunus Armeniaca Kernel Oil | 4 |
| Squalane | 1 |
| Quillaja Saponaria Wood Extract | 0.5 |
| Ficus Carica Leaf Extract | 0.5 |
| Hippophae Rhamnoides Fruit Extract | 0.5 |
| Composition A | 5.0 |
| Parfum | 0.2 |
| Rosmarinus Officinalis Leaf Extract | 0.2 |
| Eugenol | 0.1 |
| Geraniol | 0.1 |
| Citronellol | 0.1 |

### Example formulation 336: Bi Phasic Make Up Remover

| Ingredients | % |
|---|---|
| Aqua | ad 100 |
| Isododecane | 15 |
| Isopropyl Palmitate | 10 |
| Ethylhexyl Stearate | 5 |
| Propanediol | 5 |
| Poloxamer 184 | 1 |
| Sodium Benzoate | 0.5 |
| Citric Acid | 0.5 |
| Composition A | 4.2 |
| Sodium Phytate | 0.2 |
| Phenoxyethanol | 0.2 |
| Cl 42090 | 0.1 |

### Example formulation 337: Eye Make-Up Remover

| Ingredients | % |
|---|---|
| Aqua | ad 100 |
| Butylene Glycol | 5 |
| PEG-6 Caprylic/Capric Triglyceride | 3 |
| Decyl Glucoside | 3 |
| Acrylates/C10-30 Alkyl Acrylate Crosspolymer | 1 |
| Panthenol | 0.5 |
| Composition A | 3.8 |
| Xanthan Gum | 0.2 |
| Sodium Hydroxide | 0.2 |
| Disodium EDTA | 0.2 |
| Phenoxyethanol | 0.2 |
| Methylparaben | 0.2 |
| Propylparaben | 0.2 |

### Example formulation 338: Eye Make Up Remover

| Ingredients | % |
|---|---|
| Aqua (Mineral Spring Water) | 45 |
| Cyclomethicone | 15 |
| Isohexadecane | 10 |
| Glycerin | 6 |
| Aloe Barbadensis Leaf Juice | 5 |
| Butylene Glycol | 1.5 |
| Calendula Officinalis Flower Extract | 1 |
| Propylene Glycol | 1 |
| Phenoxyethanol | 0.5 |
| Ethylhexylglycerin | 0.5 |
| Allantoin | 0.5 |
| Cucumis Sativus (Cucumber) Fruit Extract | 0.5 |
| Aqua (Water) | ad 100 |
| Maris Aqua (Dead Sea Water) | 0.5 |
| Composition A | 6.8 |
| Tetrasodium EDTA | 0.2 |
| Bisabolol | 0.2 |
| Cocamine Oxide | 0.2 |
| Blue 1 (Cl 42090) | 0.1 |
| Acid Red 18 (Cl 16255) | 0.1 |
| Yellow 6 (Cl 15985) | 0.1 |

### Example formulation 339: Very Efficient Make Up Remover

| Ingredients | % |
|---|---|
| Aqua | ad 100 |
| Prunus Amygdalus Dulcis Oil | 15 |
| Ricinus Communis Seed Oil | 10 |
| Coco-Caprylate/Caprate | 8 |
| Octyldodecanol | 5 |
| Olea Europaea Fruit Oil | 5 |
| Aloe Barbadensis Leaf Juice | 5 |
| Sodium Chloride | 0.7 |
| Sclerocarya Birrea Seed Oil | 0.5 |
| Oenothera Biennis Oil | 0.5 |
| Panthenol | 0.5 |
| Phenoxyethanol | 0.5 |
| Tocopherol | 0.5 |
| Helianthus Annuus Seed Oil | 0.5 |
| Ethylhexylglycerin | 0.5 |
| Composition A | 2.8 |
| Xanthan Gum | 0.2 |
| Pantolactone | 0.2 |
| Citric Acid | 0.2 |

### Example formulation 340: Make Up Remover with Actives

| Ingredients | % | % | % | % | % | % | % | % | % | % | % | % |
|---|---|---|---|---|---|---|---|---|---|---|---|---|
| Aqua | ad 100 | ad 100 | ad 100 | ad 100 | ad 100 | ad 10 0 | ad 10 0 | ad 10 0 | ad 100 | ad 10 0 | ad 100 | ad 100 |
| Isododecane | 15 | 15 | 15 | 15 | 15 | 15 | 15 | 15 | 15 | 15 | 15 | 15 |
| Isopropyl Palmitate | 10 | 10 | 10 | 10 | 10 | 10 | 10 | 10 | 10 | 10 | 10 | 10 |
| Ethylhexyl Stearate | 5 | 5 | 5 | 5 | 5 | 5 | 5 | 5 | 5 | 5 | 5 | 5 |
| Propanediol | 5 | 5 | 5 | 5 | 5 | 5 | 5 | 5 | 5 | 5 | 5 | 5 |
| Poloxamer 184 | 1 | 1 | 1 | 1 | 1 | 1 | 1 | 1 | 1 | 1 | 1 | 1 |
| Sodium Benzoate | 0.5 | 0.5 | 0.5 | 0.5 | 0.5 | 0.5 | 0.5 | 0.5 | 0.5 | 0.5 | 0.5 | 0.5 |
| Citric Acid | 0.5 | 0.5 | 0.5 | 0.5 | 0.5 | 0.5 | 0.5 | 0.5 | 0.5 | 0.5 | 0.5 | 0.5 |
| Composition A | 0.1 | 0.3 | 3.5 | 1.8 | 1.0 | 6.6 | 7.0 | 4.6 | 9.0 | 4.6 | 10 | 4.5 |
| Hyaluronic Acid | 1 | 1 | 1 | 1 | 1 | 1 | 1 | 1 | 1 | 1 | 1 | 1 |
| Niacinamide | 1.5 | 2 | 3.2 5 | 4.5 | | | | | | | | |
| Retinol | | 0.00 1 | | | 0.1 5 | 0.5 | | | 0.0 5 | | | 0.0 2 |
| Lecithin | | | | | | | 0.8 | 1.5 | | | | |
| Coffeine | | 0.3 | | | | | | | 0.7 5 | 1.5 | | |
| Peptides | 0.0 1 | | | 0.0 6 | | | 0.1 | | 0.0 2 | | 0.2 5 | 0.7 5 |
| Sodium Phytate | 0.2 | 0.2 | 0.2 | 0.2 | 0.2 | 0.2 | 0.2 | 0.2 | 0.2 | 0.2 | 0.2 | 0.2 |
| Phenoxyethanol | 0.2 | 0.2 | 0.2 | 0.2 | 0.2 | 0.2 | 0.2 | 0.2 | 0.2 | 0.2 | 0.2 | 0.2 |
| Cl 42090 | 0.1 | 0.1 | 0.1 | 0.1 | 0.1 | 0.1 | 0.1 | 0.1 | 0.1 | 0.1 | 0.1 | 0.1 |

### Example formulation 341: Simple Cleansing Oil

| Ingredients | % |
|---|---|
| Helianthus Annuus Hybrid Oil | ad 100 |
| Isopropyl Palmitate | 15 |
| Isopropyl Myristate | 10 |
| PEG-20 Glyceryl Triisostearate | 1.5 |
| Argania Spinosa Kernel Oil | 1 |
| Ethylhexyl Stearate | 0.5 |
| Tocopherol | 0.5 |
| Composition A | 2.8 |

### Example formulation 342: Soft Cleansing Oil

| Ingredients | % |
|---|---|
| Helianthus Annuus Seed Oil | ad 100 |
| Polyglyceryl-4 Oleate | 20 |
| Caprylic/Capric Triglyceride | 15 |
| Squalane | 3 |
| Ethylhexyl Palmitate | 2 |
| Helianthus Annuus Hybrid Oil | 1.5 |
| Tocopherol | 0.5 |
| Centella Asiatica Extract | 0.5 |
| Composition A | 3.5 |

### Example formulation 343: Soothing Cleansing Oil for Sensitive Skin

| Ingredients | % |
|---|---|
| Isopropyl Palmitate | ad 100 |
| Ethylhexyl Stearate | 17 |
| Ricinus Communis Seed Oil | 12 |
| Polyglyceryl-2 Sesquioleate | 10 |
| Polyglyceryl-4 Oleate | 7 |
| Tocopherol | 0.5 |
| Centella Asiatica Extract | 0.5 |
| Helianthus Annuus Hybrid Oil | 0.5 |
| Composition A | 4.0 |
| Squalane | 0.3 |

### Example formulation 344: Camomile Cleansing Oil

| Ingredients | % |
|---|---|
| Helianthus Annuus Hybrid Oil | ad 100 |
| Polyglyceryl-2 Sesquioleate | 12 |
| Simmondsia Chinensis Seed Oil | 10 |
| Tocopherol | 1 |
| Vitis Vinifera Seed Oil | 1 |
| Caprylic/Capric Triglyceride | 1 |
| Helianthus Annuus Seed Oil | 1 |
| Bisabolol | 0.5 |
| Chamomilla Recutita Flower Extract | 0.5 |
| Composition A | 0.4 |

### Example formulation 345: Deep Cleansing Cleansing Oil

| Ingredients | % |
|---|---|
| Coco-Caprylate/Caprate | ad 100 |
| Isopropyl Myristate | 30 |
| Triethylhexanoin | 10 |
| Isohexadecane | 5 |
| PEG-20 Glyceryl Triisostearate | 5 |
| Olea Europaea (Olive) Fruit Oil | 3 |
| Prunus Amygdalus Dulcis (Sweet Almond) Oil | 3 |
| Ethylhexyl Palmitate | 2 |
| Cocos Nucifera (Coconut) Oil | 1 |
| Tocopheryl Acetate | 0.5 |
| Glycine Soja (Soybean) Oil | 0.5 |
| Tocopherol | 0.5 |
| Composition A | 0.7 |
| Squalane | 0.3 |
| Beta-Sitosterol | 0.2 |
| Squalene | 0.2 |

### Example formulation 346: Olive Oil Cleansing Oil

| Ingredients | % |
|---|---|
| Caprylic/Capric Triglyceride | ad 100 |
| Polyglyceryl-4 Oleate | 12 |
| Olea Europaea (Olive) Fruit Oil | 10 |
| Polyglyceryl-2 Caprate | 6 |
| Argania Spinosa Kernel Oil | 5 |
| Aroma | 2 |
| Helianthus Annuus Seed Oil | 1 |
| Tocopherol | 0.5 |
| Composition A | 1.0 |
| Linalool | 0.2 |
| Bisabolol | 0.2 |

### Example formulation 347: Hydroxytyrosol Cleansing Oil

| Ingredients | % |
|---|---|
| Olea Europaea (Olive)Fruit Oil | ad 100 |
| Polyglyceryl-4 Caprate | 15 |
| Hydroxytyrosol | 3 |
| Lavandula Angustifolia Flower Oil | 2 |
| Composition A | 2.5 |
| Linalool | 0.2 |
| Aqua | 0.2 |
| Aroma | 0.2 |

### Example formulation 348: Derma Cleansing Oil

| Ingredients | % |
|---|---|
| Ethylhexyl Palmitate | ad 100 |
| Sorbeth-30 Tetraoleate | 8 |
| Citrus Aurantium Bergamia (Bergamot) Fruit Oil | 7 |
| Water | 5 |
| Limnanthes Alba (Meadowfoam) Seed Oil | 3 |
| Simmondsia Chinensis (Jojoba) Seed Oil | 3 |
| Helianthus Annuus (Sunflower) Seed Oil | 3 |
| Prunus Amygdalus Dulcis (Sweet Almond) Oil | 2 |
| Olea Europaea (Olive) Fruit Oil | 2 |
| Vitis Vinifera (Grape) Seed Oil | 2 |
| Prunus Armeniaca (Apricot) Kernel Oil | 2 |
| Camellia Sinensis Leaf Extract | 1 |
| Alcohol | 1 |
| Zanthoxylum Piperitum Fruit Extract | 0.5 |
| Pulsatilla Koreana Extract | 0.5 |
| Usnea Barbata (Lichen) Extract | 0.5 |
| 1.2-Hexanediol | 0.5 |
| Composition A | 1.2 |

### Example formulation 349: Vitamin E Cleansing Oil

| Ingredients | % |
|---|---|
| Oryza Sativa (Rice) Bran Oil | ad 100 |
| Caprylic/Capric Triglyceride | 10 |
| PEG-20 Glyceryl Triisostearate | 8 |
| Adansonia Digitata (Baobab) Seed Oil | 8 |
| Olea Europaea (Olive) Fruit Oil | 1 |
| Tocopheryl Acetate | 0.5 |
| Calendula Officinalis Flower Extract | 0.5 |
| Composition A | 2.5 |

### Example formulation 350: Mushroom Cleansing Oil

| Ingredients | % |
|---|---|
| Caprylic/Capric Triglyceride | ad 100 |
| Ricinus Communis (Castor) Seed Oil | 15 |
| Polyglyceryl-3 Caprate | 10 |
| Polyglyceryl-3 Polyricinoleate | 8 |
| Glyceryl Citrate/Lactate/Linoleate/Oleate | 7 |
| Ganoderma Lucidum (Reishi Mushroom) Extract | 2 |
| Inonotus Obliquus (Chaga Mushroom) Extract | 2 |
| Tremella Fuciformis (Snow Mushroom) Extract | 2 |
| Citrus Aurantium Bergamia (Bergamot) Fruit Oil | 1 |
| Citrus Limon (Lemon) Peel Oil | 1 |
| Citrus Aurantifolia (Lime) Oil | 1 |
| Pelargonium Graveolens (Geranium) Flower Oil | 1 |
| Lavandula Angustifolia (Lavender) Oil | 1 |
| Composition A | 2.5 |
| Limonene | 0.2 |
| Linalool | 0.2 |
| Geraniol | 0.2 |
| Citronellol | 0.2 |

### Example formulation 351: Micellar Cleansing Oil

| Ingredients | % |
|---|---|
| PEG-20 Glyceryl Triisostearate | ad 100 |
| C13-15 Alkane | 17 |
| Butyrospermum Parkii Oil | 12 |
| Propanediol | 8 |
| Simmondsia Chinensis (Jojoba) Seed Oil | 8 |
| Decyl Oleate | 8 |
| Aqua (Water) | 6 |
| Oleyl Erucate | 3 |
| Coco-Glucoside | 2 |
| Ethylhexylglycerin | 1 |
| Tocopherol | 0.5 |
| Hydrogenated Palm Glycerides Citrate | 0.5 |
| Citric Acid | 0.5 |
| Phenoxyethanol | 0.5 |
| Composition A | 1.5 |
| Parfum (Fragrance) | 0.2 |

### Example formulation 352: Fresh Cleansing Oil

| Ingredients | % |
|---|---|
| Helianthus Annuus (Sunflower) Seed Oil | ad 100 |
| Polyglyceryl-4 Oleate | 25 |
| Triticum Vulgare (Wheat) Germ Oil | 12 |
| Prunus Mume Fruit | 8 |
| Simmondsia Chinensis (Jojoba) Seed Oil | 5 |
| Adansonia Digitata Seed Oil | 5 |
| Rosmarinus Officinalis (Rosemary) Leaf Extract | 1 |
| Composition A | 0.8 |

### Example formulation 353: Resetting Cleansing Oil

| Ingredients | % |
|---|---|
| Ethylhexyl Palmitate | ad 100 |
| Isopropyl Palmitate | 13 |
| Sorbeth-30 Tetraoleate | 10 |
| Isohexadecane | 10 |
| Caprylic/Capric Triglyceride | 8 |
| Propylene Glycol Dicaprylate/Dicaprate | 7 |
| Isododecane | 7 |
| Ethylhexyl Pelargonate | 6 |
| Moringa Oleifera Seed Oil | 5 |
| Helianthus Annuus (Sunflower) Seed Oil | 5 |
| Hippophae Rhamnoides Fruit Oil | 5 |
| Calendula Officinalis Flower Oil | 5 |
| Glycine Soja (Soybean) Oil | 5 |
| Retinol | 1 |
| Galactomyces Ferment Filtrate | 0.5 |
| Lactobacillus Ferment | 0.5 |
| Bifida Ferment Lysate | 0.5 |
| Saccharomyces/Xylinum/Black Tea Ferment | 0.5 |
| Saccharomyces Ferment Filtrate | 0.5 |
| Pentaerythrityl Tetra-di-t-butyl Hydroxyhydrocinnamate | 0.5 |
| Pentylene Glycol | 0.5 |
| Water | 0.5 |
| Composition A | 0.55 |
| Tocopherol | 0.5 |
| Tocopheryl Linoleate | 0.3 |
| Caprylyl Glycol | 0.3 |
| 1.2-Hexanediol | 0.2 |
| Juniperus Virginiana Oil | 0.2 |
| Cedrus Atlantica Bark Oil | 0.2 |
| Amyris Balsamifera Bark Oil | 0.2 |
| Copaifera Officinalis (Balsam Copaiba) Resin | 0.2 |
| Pogostemon Cablin Oil | 0.2 |

### Example formulation 354: Cleansing Oil to Milk

| Ingredients | % |
|---|---|
| Prunus Amygdalus Dulcis Oil | ad 100 |
| Ethylhexyl Stearate | 15 |
| PEG-20 Glyceryl Triisostearate | 15 |
| Parfum | 1.5 |
| Helianthus Annuus Seed Oil | 1.5 |
| Stevia Rebaudiana Extract | 1 |
| Aqua | 1 |
| Panax Ginseng Root Extract | 0.5 |
| Psidium Guajava Fruit Extract | 0.5 |
| Glycerin | 0.5 |
| Tocopheryl Acetate | 0.5 |
| Benzyl Salicylate | 0.5 |
| Composition A | 0.7 |
| Pentaerythrityl Tetra-Di-T-Butyl Hydroxyhydrocinnamate | 0.3 |
| Limonene | 0.2 |
| Benzyl Alcohol | 0.2 |
| Sodium Benzoate | 0.2 |
| Potassium Sorbate | 0.2 |

### Example formulation 355: Superfood Cleansing Face Oil

| Ingredients | % |
|---|---|
| Helianthus Annuus Seed Oil | ad 100 |
| Caprylic/Capric Triglyceride | 20 |
| Isoamyl Laurate | 5 |
| Polyglyceryl-3 Polyricinoleate | 5 |
| Laureth-3 | 3 |
| Parfum | 1 |
| Simmondsia Chinensis Seed Oil | 1 |
| Brassica Campestris/Aleurites Fordi Oil Copolymer | 1 |
| Macadamia Integrifolia Seed Oil | 1 |
| Isoamyl Cocoate | 0.7 |
| Polyglyceryl-3 Ricinoleate | 0.7 |
| Camellia Sinensis Leaf Extract | 0.5 |
| Squalane | 0.5 |
| Solanum Lycopersicum Fruit Extract | 0.5 |
| Tocopherol | 0.5 |
| Composition A | 1.7 |

### Example formulation 356: Cleansing Oil with Actives

| Ingredients | % | % | % | % | % | % | % | % | % | % | % | % |
|---|---|---|---|---|---|---|---|---|---|---|---|---|
| Caprylic/Capric Triglyceride | ad 10 0 | ad 10 0 | ad 100 | ad 10 0 | ad 10 0 | ad 100 | ad 10 0 | ad 10 0 | ad 100 | ad 100 | ad 10 0 | ad 100 |
| Polyglyceryl-4 Oleate | 12 | 12 | 12 | 12 | 12 | 12 | 12 | 12 | 12 | 12 | 12 | 12 |
| Olea Europaea (Olive) Fruit Oil | 10 | 10 | 10 | 10 | 10 | 10 | 10 | 10 | 10 | 10 | 10 | 10 |
| Polyglyceryl-2 Caprate | 6 | 6 | 6 | 6 | 6 | 6 | 6 | 6 | 6 | 6 | 6 | 6 |
| Argania Spinosa Kernel Oil | 5 | 5 | 5 | 5 | 5 | 5 | 5 | 5 | 5 | 5 | 5 | 5 |
| Aroma | 2 | 2 | 2 | 2 | 2 | 2 | 2 | 2 | 2 | 2 | 2 | 2 |
| Helianthus Annuus Seed Oil | 1 | 1 | 1 | 1 | 1 | 1 | 1 | 1 | 1 | 1 | 1 | 1 |
| Tocopherol | 0.5 | 0.5 | 0.5 | 0.5 | 0.5 | 0.5 | 0.5 | 0.5 | 0.5 | 0.5 | 0.5 | 0.5 |
| Composition A | 1.5 | 0.1 | 5.5 | 8.0 | 10 | 15 | 4.5 | 1.2 | 3.0 | 0.8 | 4.6 | 6.0 |
| Hyaluronic Acid | 1 | 1 | 1.5 | 1.5 | 1 | 1.5 | 1 | 1.5 | 1 | 1.5 | 1 | 1.5 |
| Niacinamide | 1 | 2.5 | 4 | 4.5 | | | | | | | | |
| Retinol | | | 0.00 1 | | 0.2 | 0.3 5 | | | 0.0 5 | | | 0.00 1 |
| Lecithin | | | | | | | 1 | 2.5 | | | | |
| Coffeine | | | | 0.5 | | 0.8 | | | 1 | 1.7 5 | | |
| Peptides | 5 | | 0.01 | | | | 0.5 | 0.8 | | | 0.1 | 0.35 |
| Linalool | 0.2 | 0.2 | 0.2 | 0.2 | 0.2 | 0.2 | 0.2 | 0.2 | 0.2 | 0.2 | 0.2 | 0.2 |
| Bisabolol | 0.2 | 0.2 | 0.2 | 0.2 | 0.2 | 0.2 | 0.2 | 0.2 | 0.2 | 0.2 | 0.2 | 0.2 |

### Example formulation 357: Essential Toner

| Ingredients | % |
|---|---|
| Aqua | ad 100 |
| Glycerin | 5 |
| PEG-8 | 3 |
| PEG-40 Hydrogenated Castor Oil | 3 |
| Prunus Amygdalus Dulcis Oil | 1 |
| Polyquaternium-10 | 0.5 |
| Citric Acid | 0.5 |
| Phenoxyethanol | 0.5 |
| Composition A | 1.5 |
| 1.2-Hexanediol | 0.3 |
| Trisodium EDTA | 0.3 |
| Geraniol | 0.2 |
| Benzyl Alcohol | 0.2 |
| Linalool | 0.2 |
| Alpha-Isomethyl Ionone | 0.2 |
| Limonene | 0.1 |
| Parfum | 0.1 |

### Example formulation 358: Ultra Sensitive Facial Toner

| Ingredients | % |
|---|---|
| Aqua | ad 100 |
| Glycerin | 6 |
| Pentylene Glycol | 3 |
| Hydrogenated Starch Hydrolysate | 0.5 |
| Panthenol | 0.5 |
| Composition A | 0.9 |
| Cucumis Sativus Fruit Extract | 0.3 |
| Sodium Carboxymethyl Betaglucan | 0.2 |
| Citric Acid | 0.2 |

### Example formulation 359: Wildrose Facial Toner

| Ingredients | % |
|---|---|
| Aqua | ad 100 |
| Rosa Damascena Flower Water | 4 |
| Glycerin | 4 |
| Sodium Benzoate | 1 |
| Potassium Sorbate | 1 |
| Citric Acid | 1 |
| Sodium PCA | 0.5 |
| Sodium Lactate | 0.5 |
| Lactic Acid | 0.5 |
| Composition A | 1.1 |
| Citronellol | 0.2 |
| Rosa Canina Fruit Extract | 0.2 |

### Example formulation 360: Age Perfect Facial Toner

| Ingredients | % |
|---|---|
| Aqua | ad 100 |
| Glycerin | 7 |
| Calcium Pantetheine Sulfonate | 3.5 |
| Disodium EDTA | 1 |
| Magnesium Sulfate | 0.7 |
| Sodium Citrate | 0.7 |
| Ascorbyl Glucoside | 0.7 |
| Panthenol | 0.5 |
| Caprylyl Glycol | 0.5 |
| PEG-60 Hydrogenated Castor Oil | 0.5 |
| Benzyl Alcohol | 0.5 |
| Composition A | 4.5 |
| Citronellol | 0.2 |
| Geraniol | 0.2 |
| Limonene | 0.2 |
| Linalool | 0.2 |
| Phenoxyethanol | 0.2 |
| Parfum / Fragrance (F.I.L. B236100/1) | 0.2 |

### Example formulation 361: Peeling Toner

| Ingredients | % |
|---|---|
| Aqua (Water) | ad 100 |
| Propylene Glycol | 5 |
| Pentylene Glycol | 3 |
| Salicylic Acid | 2 |
| Succinic Acid | 1 |
| Sodium PCA | 1 |
| Resveratrol | 0.5 |
| Composition A | 3.5 |
| Epigallocatechin Gallate | 0.2 |
| Phospholipids | 0.2 |
| Leuconostoc/Radish Root Ferment Filtrate | 0.2 |
| Superoxide Dismutase | 0.2 |
| Xanthan Gum | 0.2 |
| Phytic Acid | 0.2 |
| Sodium Hydroxide | 0.2 |

### Example formulation 362: Beauty Toner

| Ingredients | % |
|---|---|
| Aqua | ad 100 |
| Glycerin | 7 |
| Panthenol | 3.5 |
| Betaine | 1.5 |
| Allantoin | 1 |
| Urea | 1 |
| Sodium PCA | 1 |
| Sodium Lactate | 0.5 |
| PCA | 0.5 |
| Magnesium Aspartate | 0.5 |
| Zinc Gluconate | 0.5 |
| Composition A | 0.9 |
| Phragmites Karka Extract | 0.3 |
| Poria Cocos Extract | 0.3 |
| Pantolactone | 0.2 |
| Serine | 0.2 |
| Alanine | 0.2 |
| Glycine | 0.2 |
| Biosaccharide Gum-1 | 0.2 |
| Glutamic Acid | 0.2 |
| Lysine Hcl | 0.2 |
| Threonin | 0.2 |
| Arginine | 0.2 |
| Copper Gluconate | 0.1 |
| Proline | 0.1 |
| Disodium Phosphate | 0.1 |
| Phenoxyethanol | 0.1 |
| Ethylhexylglycerin | 0.1 |
| Sodium Benzoate | 0.1 |
| Citric Acid | 0.1 |

### Example formulation 363: BHA Toner

| Ingredients | % |
|---|---|
| Aqua (Water) | ad 100 |
| Alcohol | 8 |
| Glycerin | 5 |
| Bellis Perennis (Daisy) Flower Extract | 3 |
| Butylene Glycol | 1.5 |
| Betaine | 1.5 |
| Levulinic Acid | 0.5 |
| Sodium Levulinate | 0.5 |
| Salicylic Acid | 0.5 |
| Sodium Hydroxide | 0.5 |
| Composition A | 6.0 |
| Enantia Chlorantha Bark Extract | 0.3 |
| Sodium Lactate | 0.3 |
| Camellia Sinensis Leaf Extract | 0.3 |
| Sodium Chloride | 0.3 |
| Oleanolic Acid | 0.2 |
| Disodium Phosphate | 0.2 |
| Potassium Chloride | 0.2 |
| Potassium Phosphate | 0.2 |
| Parfum (Fragrance) | 0.2 |
| Linalool | 0.1 |
| Limonene | 0.1 |
| Citral | 0.1 |
| Citronellol | 0.1 |

### Example formulation 364: Moisture Boost Toner

| Ingredients | % |
|---|---|
| Aqua (Water) | ad 100 |
| Pentylene Glycol | 8.5 |
| Glycerin | 5.5 |
| Betaine | 1 |
| Zinc PCA | 0.5 |
| Benzyl Alcohol | 0.5 |
| Citric Acid | 0.5 |
| Composition A | 3.5 |
| Sodium Hyaluronate | 0.1 |

### Example formulation 365: Clearing Facial Toner

| Ingredients | % |
|---|---|
| Aqua/Water | ad 100 |
| Rosa Damascena (Rose) Flower Water | 10 |
| Alcohol | 3.5 |
| Glycerin | 3 |
| Acorus Calamus (Sweet flag) Root Extract | 0.5 |
| Cucumis Sativus (Cucumber) Fruit Extract | 0.5 |
| Betaine | 0.5 |
| Quercus Robur (Oak) Bark Extract | 0.5 |
| Salvia Officinalis (Salvia) Leaf Extract | 0.5 |
| Composition A | 1.4 |
| Salicylic Acid | 0.2 |

### Example formulation 366: Revitalizing Facial Toner

| Ingredients | % |
|---|---|
| Water (Aqua) | ad 100 |
| Alcohol | 5 |
| Rosa Moschata Leaf Extract | 1.5 |
| Hamamelis Virginiana (Witch Hazel) Water | 1.5 |
| Citrus Limon (Lemon) Juice | 1 |
| Composition A | 4.0 |
| Fragrance (Parfum) | 0.2 |
| Limonene | 0.1 |
| Linalool | 0.1 |
| Geraniol | 0.1 |
| Citral | 0.1 |

### Example formulation 367: Hydrating Toner

| Ingredients | % |
|---|---|
| Aqua (Water) | ad 100 |
| Rosa Damascena Flower Water | 5 |
| Glycerin | 5 |
| Alcohol | 2.5 |
| Hamamelis Virginiana (Witch Hazel) Leaf Extract | 1 |
| Levulinic Acid | 0.5 |
| Salix Alba (Willow) Bark Extract | 0.5 |
| Sodium Levulinate | 0.5 |
| Calendula Officinalis Flower Extract | 0.5 |
| Composition A | 3.0 |
| Phytic Acid | 0.2 |
| Sodium Hydroxide | 0.2 |
| PCA Ethyl Cocoyl Arginate | 0.2 |

### Example formulation 368: Soft Facial Toner

| Ingredients | % |
|---|---|
| Aqua | ad 100 |
| Butylene Glycol | 5 |
| Dipropylene Glycol | 5 |
| Methylpropanediol | 3 |
| Propylene Glycol | 2 |
| Propanediol | 2 |
| Panthenol | 2 |
| Sodium Benzoate | 2 |
| Caprylyl/Capryl Glucoside | 2 |
| Glycerin | 2 |
| Sodium Cocoyl Glutamate | 1.5 |
| Cocamidopropyl Betaine | 1.5 |
| Citric Acid | 0.5 |
| Composition A | 5.0 |
| Parfum | 0.2 |
| Sodium Formate | 0.2 |
| Tetrasodium Glutamate Diacetate | 0.2 |
| Sodium Chloride | 0.2 |
| Hexyl Cinnamal | 0.2 |
| Aloe Barbadensis Leaf Juice | 0.2 |
| Linalool | 0.1 |
| Limonene | 0.1 |
| Benzyl Salicylate | 0.1 |
| Citronellol | 0.1 |

### Example formulation 369: Classic Toner

| Ingredients | % |
|---|---|
| Aloe Barbadensis Leaf Juice | ad 100 |
| Nardostachys Jatamansi Rhizome/Root Extract | 3 |
| Alcohol | 2.5 |
| Salix Nigra Bark Extract | 2 |
| Glycerin | 1 |
| Levulinic Acid | 0.5 |
| Sodium Levulinate | 0.5 |
| Composition A | 6.0 |

### Example formulation 370: Calming Toner

| Ingredients | % |
|---|---|
| Rosa Damascena (Rose) Flower Water | 5 |
| Lavandula Angustifolia (Lavender) Flower Water | 5 |
| Polyglyceryl-4 Laurate/Sebacate | 3 |
| Polyglyceryl-6 Caprylate/Caprate | 3 |
| Aqua | ad 100 |
| Propanediol | 2 |
| Aloe Barbadensis (Barbados Aloe) Leaf Juice Powder | 1 |
| Triticum Vulgare (Wheat) Germ Extract | 1 |
| Citrus Bergamia (Bergamot) Peel Oil Expressed | 1 |
| Ocimum Basilicum (Basil) Herb Oil | 1 |
| Composition A | 2.5 |
| Lactobacillus/Soybean Ferment Extract | 0.2 |
| Centella Asiatica (Hydrocotyl) Extract | 0.2 |
| Ginkgo Biloba (Ginkgo) Leaf Extract | 0.2 |
| Imperata Cylindrica (Alang-Alang) Root Extract | 0.2 |
| Panax Ginseng (Ginseng) Root Extract | 0.2 |
| Faex (Yeast/Levure) Extract | 0.2 |
| Viscum Album (Mistletoe) Leaf Extract | 0.2 |
| Vaccinium Myrtillus (Bilberry) Leaf Extract | 0.2 |
| Chamomilla Recutita (Matricaria) Extract | 0.2 |
| Citrus Aurantium Bergamia (Bergamot) Fruit Extract | 0.2 |
| Citrus Aurantium Dulcis (Orange) Fruit Extract | 0.2 |
| Citrus Grandis (Grapefruit) Fruit Extract | 0.2 |
| Citrus Nobilis (Mandarin Orange) Fruit Extract | 0.2 |
| Glycerin | 0.2 |
| Gluconolactone | 0.2 |
| Calcium Gluconate | 0.2 |
| Potassium Sorbate | 0.2 |
| Sodium Benzoate | 0.2 |
| Caprylhydroxamic Acid | 0.2 |
| Caprylyl Glycol | 0.2 |
| Linalool | 0.1 |
| Limonene | 0.1 |
| Citral | 0.1 |
| Eugenol | 0.1 |
| Flavor (Aroma) | 0.1 |

### Example formulation 371: Balancing Toner

| Ingredients | % |
|---|---|
| Aloe Barbadensis Leaf Juice | 30 |
| Niacinamide | 5 |
| Glycerin | 5 |
| Hydrolyzed Yeast Protein | 3 |
| Polyglyceryl10 Laurate | 3 |
| Pistacia Lentiscus (Mastic) Gum | 2 |
| Rosmarinus Officinalis(Rosemary) Leaf Oil | 2 |
| Citrus Aurantium Dulcis(Orange) Flower Oil | 2 |
| Salvia Officinalis (Sage) Leaf Extract | 1 |
| Hamamelis Virginiana (Witch Hazel) Leaf Extract | 1 |
| Butylene Glycol | 1 |
| Pentyleneglycol | 1 |
| Aqua/Water/Eau | ad 100 |
| Lecithin | 0.5 |
| Lactic Acid | 0.5 |
| Ethylhexylglycerin | 0.5 |
| Phenoxyethanol | 0.5 |
| Alcohol | 0.5 |
| Composition A | 3.0 |
| Farnesol | 0.2 |
| Linalool | 0.2 |
| Limonene | 0.2 |
| Geraniol | 0.2 |
| Sodium Benzoate | 0.2 |
| Potassium Sorbate | 0.2 |

### Example formulation 372: Toner with Actives

| Ingredients | % | % | % | % | % | % | % | % | % | % | % | % |
|---|---|---|---|---|---|---|---|---|---|---|---|---|
| Aqua | ad 100 | ad 100 | ad 100 | ad 10 0 | ad 100 | ad 10 0 | ad 10 0 | ad 100 | ad 10 0 | ad 10 0 | ad 10 0 | ad 10 0 |
| Glycerin | 6 | 6 | 6 | 6 | 6 | 6 | 6 | 6 | 6 | 6 | 6 | 6 |
| Pentylene Glycol | 3 | 3 | 3 | 3 | 3 | 3 | 3 | 3 | 3 | 3 | 3 | 3 |
| Hydrogenated Starch Hydrolysate | 0.5 | 0.5 | 0.5 | 0.5 | 0.5 | 0.5 | 0.5 | 0.5 | 0.5 | 0.5 | 0.5 | 0.5 |
| Panthenol | 0.5 | 0.5 | 0.5 | 0.5 | 0.5 | 0.5 | 0.5 | 0.5 | 0.5 | 0.5 | 0.5 | 0.5 |
| Composition A | 0.9 | 1.6 | 7.0 | 9.0 | 4.5 | 3.9 | 2.9 | 3.5 | 7.2 | 11 | 0.2 | 2.8 |
| Hyaluronic Acid | 0.8 | 0.8 | 0.8 | 0.8 | 0.8 | 0.8 | 0.8 | 0.8 | 0.8 | 0.8 | 0.8 | 0.8 |
| Niacinamide | 1.2 5 | 1.7 | 2 | 3.5 | | | | | 1.2 | | 1.5 | |
| Retinol | | 0.00 1 | 0.0 2 | | 0.1 | 0.3 | | | 0.6 | | | |
| Lecithin | | | 0.5 | | | | 0.8 | 1.2 5 | | | | |
| Coffeine | | | | | | | | | 0.3 | 0.5 | | |
| Peptides | 0.0 1 | | 0.3 | | 0.0 5 | | | | | | 0.1 | 0.3 |
| Cucumis Sativus Fruit Extract | 0.3 | 0.3 | 0.3 | 0.3 | 0.3 | 0.3 | 0.3 | 0.3 | 0.3 | 0.3 | 0.3 | 0.3 |
| Sodium Carboxymethyl Betaglucan | 0.2 | 0.2 | 0.2 | 0.2 | 0.2 | 0.2 | 0.2 | 0.2 | 0.2 | 0.2 | 0.2 | 0.2 |

### Example formulation 373: Soft Cleansing Wipes

| Ingredients | % |
|---|---|
| Aqua | ad 100 |
| Propylene Glycol | 5 |
| Brassica Campestris Seed Oil | 1 |
| Caprylyl/Capryl Glucoside | 0.7 |
| Composition A | 0.9 |
| Phenoxyethanol | 0.3 |
| Salicylic Acid | 0.3 |
| Sodium Benzoate | 0.2 |
| Parfum | 0.2 |
| Benzoic Acid | 0.2 |
| Xanthan Gum | 0.2 |
| Dehydroacetic Acid | 0.2 |
| Tocopherol | 0.2 |
| Glycine Soja Oil | 0.2 |
| Sodium Hydroxide | 0.2 |

### Example formulation 374: 3in1 Cleansing Wipes

| Ingredients | % |
|---|---|
| Aqua | ad 100 |
| Isopropyl Stearate | 5 |
| Glycerin | 3 |
| Dicaprylyl Ether | 1.5 |
| Panthenol | 1.5 |
| Prunus Amygdalus Dulcis Oil | 1 |
| Tocopherol | 0.5 |
| Polyglyceryl-3 Methylglucose Distearate | 0.5 |
| Composition A | 1.9 |
| Xanthan Gum | 0.2 |
| Ethylhexylglycerin | 0.2 |
| Hydroxyacetophenone | 0.2 |
| Phenoxyethanol | 0.2 |
| Citric Acid | 0.2 |
| Pantolactone | 0.2 |
| Linalyl Acetate | 0.2 |
| Limonene | 0.2 |
| Linalool | 0.2 |
| Citronellol | 0.2 |
| Alpha-Isomethyl Ionone | 0.2 |
| Benzyl Alcohol | 0.2 |
| Geraniol | 0.2 |
| Citrus Aurantium Peel Oil | 0.2 |
| Acetyl Cedrene | 0.2 |
| Benzyl Salicylate | 0.2 |
| Hexyl Cinnamal | 0.2 |
| Terpineol | 0.2 |
| Cananga Odorata Oil/Extract | 0.2 |
| Geranyl Acetate | 0.2 |
| Parfum | 0.2 |

### Example formulation 375: Soothing Cleansing Wipes

| Ingredients | % |
|---|---|
| Aqua | ad 100 |
| Decyl Isostearate | 3.5 |
| Isopropyl Isostearate | 3.5 |
| Glycerin | 3 |
| Dimethicone | 1 |
| Isohexadecane | 1 |
| Polysorbate 20 | 1 |
| Hexylene Glycol | 0.5 |
| Cocos Nucifera Fruit Juice | 0.5 |
| Trisiloxane | 0.5 |
| PEG-6 Caprylic/Capric Glycerides | 0.5 |
| Sucrose Cocoate | 0.5 |
| Acrylates/C10-30 Alkyl Acrylate Crosspolymer | 0.5 |
| Composition A | 2.1 |
| Sodium Hydroxide | 0.2 |
| Citric Acid | 0.2 |
| Sodium Benzoate | 0.2 |
| Potassium Sorbate | 0.2 |
| Phenoxyethanol | 0.2 |
| Chlorphenesin | 0.2 |
| Parfum | 0.2 |

### Example formulation 376: Wildrose Cleansing Wipes

| Ingredients | % |
|---|---|
| Aqua | ad 100 |
| Aloe Barbadensis Leaf Jucie | 7 |
| Glycerin | 3.5 |
| Sodium Levulinate | 1.5 |
| Glyceryl Caprylate | 1 |
| Sodium Anisate | 0.5 |
| Prunus Armeniaca Kernel Oil | 0.5 |
| Alcohol | 0.5 |
| Helianthus Annuus Seed Oil | 0.5 |
| Composition A | 3.2 |
| Parfum | 0.2 |
| Sodium Citrate | 0.2 |
| Hydrolyzed Vegetable Protein | 0.2 |
| Tocopheryl Acetate | 0.2 |
| Xanthan Gum | 0.2 |
| Cyamopsis Tetragonoloba Gum | 0.2 |
| Citric Acid | 0.2 |
| Tocopherol | 0.2 |
| Rosa Canina Fruit Extract | 0.2 |
| Citronellol | 0.1 |
| Geraniol | 0.1 |
| Linalool | 0.1 |
| Glycine Soja Oil | 0.1 |

### Example formulation 377: Aloe Wet Wipes

| Ingredients | % |
|---|---|
| Aqua | ad 100 |
| Glycerin | 6 |
| Aloe Barbadensis Leaf Extract | 4.5 |
| Phenoxyethanol | 2.5 |
| Sodium Benzoate | 1 |
| Caprylic / Capric Triglyceride | 1 |
| Composition A | 2.8 |
| Cetyl Palmitate | 0.4 |
| Parfum | 0.3 |
| C12-15 Alkyl Benzoate | 0.2 |
| Triisostearin | 0.2 |
| Benzoic Acid | 0.2 |
| Dehydroacetic Acid | 0.2 |
| Diglycerin | 0.2 |
| Glyceryl Stearate | 0.2 |
| Potassium Cetyl Phosphate | 0.2 |
| Caprylyl Glycol | 0.2 |
| Ethylhexylglycerin | 0.2 |

### Example formulation 378: Wet Wipes for Face & Hands

| Ingredients | |
|---|---|
| Aqua | ad 100 |
| Caprylyl/Capryl Glucoside | 2.5 |
| Citric Acid | 0.5 |
| Composition A | 1.2 |
| Sodium Citrate | 0.25 |
| Sodium Benzoate | 0.25 |

### Example formulation 379: Water Wet Wipes

| Ingredients | % |
|---|---|
| Aqua | ad 100 |
| Glycerin | 0.5 |
| Composition A | 3.5 |
| Cocamidopropyl PG-Dimonium Chloride | 0.15 |
| Sodium Benzoate | 0.15 |
| Levulinic Acid | 0.1 |
| Sodium Levulinate | 0.1 |

### Example formulation 380: Micellar Cleansing Wipes

| Ingredients | % |
|---|---|
| Aqua | ad 100 |
| Alcohol Denat. | 4.5 |
| PEG-40 Hydrogenated Castor Oil | 2.5 |
| Tocopheryl Acetate | 1.5 |
| Tocopherol | 1.5 |
| Glycerin | 1.5 |
| Sorbitol | 1 |
| Poloxamer 124 | 1 |
| Decyl Glucoside | 1 |
| Disodium Cocoyl Glutamate | 0.7 |
| Sodium Sulfate | 0.7 |
| Polyquaternium-10 | 0.5 |
| Sodium Chloride | 0.5 |
| Propylene Glycol | 0.5 |
| Ethylhexylglycerin | 0.5 |
| 1.2-Hexanediol | 0.5 |
| Composition A | 1.5 |
| Trisodium EDTA | 0.2 |
| Citric Acid | 0.2 |
| Phenoxyethanol | 0.2 |
| Linalool | 0.1 |
| Citronellol | 0.1 |
| Geraniol | 0.1 |
| Benzyl Alcohol | 0.1 |
| Alpha-Isomethyl Ionone | 0.1 |
| Citral | 0.1 |
| BHT | 0.1 |
| Parfum | 0.1 |

### Example formulation 381: Make Up Remover Wipes

| Ingredients | % |
|---|---|
| Aqua (Water) | ad 100 |
| Methylpropanediol | 10 |
| Betaine | 4 |
| C12-13-Pareth-9 | 3 |
| Ceteareth-25 | 1 |
| Laureth-23 | 1 |
| Propylene Glycol | 1 |
| Pentylene Glycol | 1 |
| Simethicone | 1 |
| Disodium Phosphate | 0.5 |
| Malva sylvestris (Mallow) Flower/Leaf/Stem Extract | 0.5 |
| Cetrimonium Bromide | 0.5 |
| Phenoxyethanol | 0.5 |
| Composition A | 4.0 |
| Citric Acid | 0.3 |
| Sodium Carboxymethyl Beta-Glucan | 0.2 |
| Ethylhexylglycerin | 0.2 |
| Polysorbate 65 | 0.2 |
| Methylcellulose | 0.2 |
| Sorbic Acid | 0.2 |
| Benzoic Acid | 0.2 |
| Parfum (Fragrance) | 0.2 |

### Example formulation 382: Detox Cleansing Wipes

| Ingredients | % |
|---|---|
| Aqua/Water/Eau | ad 100 |
| Aloe Barbadensis Leaf Extract | 8 |
| Bioflavonoids | 4 |
| Brassica Oleracea Italica Broccoli Extract | 3 |
| Calendula Officinalis Flower Extract | 3 |
| Caprylic/Capric Triglyceride | 3 |
| Chamomilla Recutita Matricaria Flower Extract | 3 |
| Citric Acid | 1.5 |
| Dicaprylyl Carbonate | 1.5 |
| Ethylhexylglycerin | 1.5 |
| Glycerin | 1.5 |
| Glyceryl Oleate | 1 |
| Lactic Acid | 0.5 |
| Lauryl Glucoside | 0.5 |
| Macadamia Ternifolia Seed Oil | 0.5 |
| Olive Oil PEG-7 Esters | 0.5 |
| Olus Oil/Vegetable Oil/ Huile Végétale | 0.5 |
| Polyglyceryl-2 Dipolyhydroxystearate | 0.5 |
| Propanediol | 0.5 |
| Retinyl Palmitate | 0.5 |
| Sideritis Scardica Flower/Leaf/Stem Extract | 0.5 |
| Simmondsia Chinensis Jojoba Seed Oil | 0.5 |
| Composition A | 3.5 |
| Tetrasodium EDTA | 0.3 |
| Tocopherol | 0.3 |
| Triticum Vulgare Wheat Germ Oil | 0.3 |
| Zea Mays Corn Germ Oil | 0.3 |
| Phenoxyethanol | 0.2 |
| Potassium Sorbate | 0.2 |
| Sodium Benzoate | 0.2 |
| Parfum/Fragrance | 0.2 |

### Example formulation 383: Beard Cleansing Wipes

| Ingredients | % |
|---|---|
| Aqua (Water) | ad 100 |
| Aloe Barbadensis (Aloe Vera) Leaf Juice | 10 |
| Commiphora Myrrha Resin Extract | 1.5 |
| Angelica Archangelica (Rose Of 225Ericho) Root Extract | 1.5 |
| Acorus Calamus Root Extract | 1.5 |
| Eugenia Caryophyllus (Clove) Bud Extract | 1.5 |
| Myristica Fragrans (Nutmeg) Fruit Extract | 1.5 |
| Piper Nigrum (Pepper) Fruit Extract | 1.5 |
| Curcuma Zedoaria Root Extract | 1.5 |
| Zingiber Officinale (Ginger) Rhizome Extract | 1.5 |
| Elettaria Cardamomum Seed Extract | 1.5 |
| Alcohol | 1 |
| PEG-40 Hydrogenated Castor Oil | 1 |
| Didecyldimonium Chloride | 0.5 |
| Composition A | 2.0 |
| Parfum (Fragrance) | 0.2 |
| Limonene | 0.1 |

### Example formulation 384: Cleansing Wipes with Actives

| Ingredients | % | % | % | % | % | % | % | % | % | % | % | % |
|---|---|---|---|---|---|---|---|---|---|---|---|---|
| Aqua | ad 100 | ad 100 | ad 100 | ad 100 | ad 100 | ad 100 | ad 100 | ad 100 | ad 100 | ad 100 | ad 100 | ad 100 |
| Caprylyl/Capryl Glucoside | 2.5 | 2.5 | 2.5 | 2.5 | 2.5 | 2.5 | 2.5 | 2.5 | 2.5 | 2.5 | 2.5 | 2.5 |
| Citric Acid | 0.5 | 0.5 | 0.5 | 0.5 | 0.5 | 0.5 | 0.5 | 0.5 | 0.5 | 0.5 | 0.5 | 0.5 |
| Composition A | 1.2 | 0.9 | 2.0 | 6.0 | 6.5 | 3.0 | 8.0 | 10 | 2.5 | 5.0 | 7.5 | 0.2 |
| Hyaluronic Acid | 1 | 1 | 1 | 1 | 1 | 1 | 1 | 1 | 1 | 1 | 1 | 1 |
| Niacinamide | 0.3 5 | 0.5 | 1 | | | | | | | | | |
| Retinol | | | | 0.1 | 0.3 | | | 0.0 1 | 0.2 | 0.0 4 | | |
| Lecithin | | 0.4 | | | | 0.2 5 | 0.5 | | | | | |
| Coffeine | | | | | | | | 0.1 | 0.3 | 0.5 | | |
| Peptides | | 0.1 | 1.0 | | 0.3 | | 0.3 | | | | 0.1 | 0.3 |
| Sodium Citrate | 0.2 5 | 0.2 5 | 0.2 5 | 0.2 5 | 0.2 5 | 0.2 5 | 0.2 5 | 0.2 5 | 0.2 5 | 0.2 5 | 0.2 5 | 0.2 5 |
| Sodium Benzoate | 0.2 5 | 0.2 5 | 0.2 5 | 0.2 5 | 0.2 5 | 0.2 5 | 0.2 5 | 0.2 5 | 0.2 5 | 0.2 5 | 0.2 5 | 0.2 5 |

### Example formulation 385: Vitamin C Serum

| Ingredients | % |
|---|---|
| Aqua | ad 100 |
| Dicaprylyl Ether | 5 |
| Pentylene Glycol | 4 |
| Polyglyceryl-6 Stearate | 3 |
| Glycerin | 3 |
| Prunus Armeniaca Kernel Oil | 1.5 |
| Ascorbyl Tetraisopalmitate | 1 |
| Phenoxyethanol | 1 |
| Composition A | 3.0 |
| Parfum | 0.2 |
| Sclerotium Gum | 0.2 |
| Stearyl Alcohol | 0.2 |
| Ascorbyl Palmitate | 0.2 |
| Xanthan Gum | 0.2 |
| Limonene | 0.2 |
| Polyglyceryl-6 Behenate | 0.2 |
| Potassium Cetyl Phosphate | 0.2 |
| 1.2-Hexanediol | 0.2 |
| Caprylyl Glycol | 0.2 |
| Malpighia Glabra Fruit Juice | 0.2 |
| Maltodextrin | 0.2 |
| Sodium Hydroxide | 0.2 |
| Linalool | 0.1 |
| Tocopherol | 0.1 |
| Geraniol | 0.1 |

### Example formulation 386: Vit C Serum

| Ingredients | % |
|---|---|
| Aqua | ad 100 |
| Ascorbic Acid | 4 |
| Pentylene Glycol | 4 |
| Ethoxydiglycol | 2 |
| Prunus Amygdalus Dulcis Oil | 1 |
| Sodium Hydroxide | 0.5 |
| Composition A | 1.2 |
| Sclerotium Gum | 0.35 |
| Polyglyceryl-3 Cocoate | 0.35 |
| Polyglyceryl- 4 Caprate | 0.35 |
| Polyglyceryl-6 Caprylate | 0.35 |
| Polyglyceryl-6 Ricinoleate | 0.35 |
| Xanthan Gum | 0.1 |
| Mannitol | 0.1 |
| Ergothioneine | 0.1 |

### Example formulation 387: Glow Serum

| Ingredients | % |
|---|---|
| Water | ad 100 |
| (L-)Ascorbic Acid | 6 |
| Dimethyl Isosorbide | 4 |
| Butylene Glycol | 4 |
| PEG/PPG/Polybutylene Glycol-8/5/3 Glycerin | 3 |
| Ferulic Acid | 0.5 |
| Tocopherol | 0.5 |
| Phenoxyethanol | 0.5 |
| Composition A | 4.0 |
| Ethylhexylglycerin | 0.2 |
| Sodium Hydroxide | 0.2 |

### Example formulation 388: Niacinamide Serum

| Ingredients | % |
|---|---|
| Aqua | ad 100 |
| Niacinamide | 5 |
| Glycerin | 4 |
| Microcrystalline Cellulose | 2 |
| Diglycerin | 2 |
| Amylopectin | 2 |
| Lithothamnion Calcareum Extract | 1.5 |
| Butylene Glycol | 1.5 |
| Vitis Vinifera (Grape) Fruit Extract | 1 |
| Composition A | 1.8 |
| Acacia Senegal Gum | 0.3 |
| Xanthan Gum | 0.2 |
| Phenoxyethanol | 0.2 |
| Ethylhexylglycerin | 0.2 |
| Potassium Sorbate | 0.2 |
| Sodium Benzoate | 0.2 |
| Pentylene Glycol | 0.2 |
| Lactic Acid | 0.2 |
| Citric Acid | 0.2 |

### Example formulation 389: Anti Wrinkles Serum

| Ingredients | % |
|---|---|
| Aqua | ad 100 |
| Glycerin | 6 |
| Caprylic/Capric Triglyceride | 5 |
| Alcohol Denat. | 5 |
| Cocoglycerides | 3 |
| Glyceryl Stearate Citrate | 2 |
| Butyrospermum Parkii Butter | 1 |
| Distarch Phosphate | 0.5 |
| Acteyl Hydroxyproline | 0.5 |
| Ubiquinone | 0.5 |
| 1-Methylhydantoin-2-Imide | 0.5 |
| Creatine | 0.5 |
| Hydroxyacetophenone | 0.5 |
| Xanthan Gum | 0.5 |
| Composition A | 3.0 |
| Sodium Hydroxide | 0.2 |
| Phenoxyethanol | 0.2 |
| Pogostemon Cablin Oil | 0.2 |
| Parfum | 0.2 |

### Example formulation 390: Hyaluronic Acid Serum

| Ingredients | % |
|---|---|
| Aqua / Water | ad 100 |
| Glycerin | 6 |
| Hydroxyethylpiperazine Ethane Sulfonic Acid | 3 |
| Sodium Hyaluronate | 1 |
| Citric Acid | 1 |
| Dipeptide Diaminobutyroyl Benzylamide Diacetate | 0.5 |
| Secale Cereale Seed Extract / Rye Seed Extract | 0.5 |
| Sodium Acetylated Hyaluronate | 0.5 |
| Composition A | 7.0 |
| Trisodium Ethylenediamine Disuccinate | 0.3 |
| Ascorbyl Glucoside | 0.3 |
| PEG-60 Hydrogenated Castor Oil | 0.3 |
| Pentylene Glycol | 0.2 |
| Benzyl Alcohol | 0.2 |
| Benzyl Benzoate | 0.2 |
| Benzyl Salicylate | 0.2 |
| Citronellol | 0.1 |
| Linalool | 0.1 |
| Chlorphenesin | 0.1 |
| Phenoxyethanol | 0.1 |
| Parfum / Fragrance (F.I.L. Z70022624/1) | 0.1 |

### Example formulation 391: Olive Serum

| Ingredients | % |
|---|---|
| Aqua | ad 100 |
| Olea Europaea Fruit Oil | 3.5 |
| Pentylene Glycol | 3 |
| Glycerin | 3 |
| Glycine Soja Oil | 2 |
| C14-22 Alcohols | 2 |
| Olea Europaea Fruit Extract | 2 |
| Butyrospermum Parkii Butter | 1 |
| Glyceryl Undecylenate | 1 |
| Coco-Glucoside | 1 |
| Maltodextrin | 0.5 |
| Polyglyceryl-4 Oleate | 0.5 |
| C12-20 Alkyl Glucoside | 0.5 |
| Glyceryl Olivate | 0.5 |
| Olea Europaea Leaf Extract | 0.5 |
| Xanthan Gum | 0.5 |
| Coconut Alcohol | 0.5 |
| Hydrogenated Rapeseed Alcohol | 0.5 |
| Composition A | 6.0 |
| Parfum | 0.2 |
| Potassium Cetyl Phosphate | 0.2 |
| Limonene | 0.2 |
| Linalool | 0.2 |
| Geraniol | 0.2 |
| Citric Acid | 0.2 |
| Citral | 0.2 |
| Citronellol | 0.2 |

### Example formulation 392: Pore Minimizer Serum

| Ingredients | % |
|---|---|
| Aqua (Water) | ad 100 |
| Azelaic Acid | 5 |
| Glycerin | 5 |
| Caprylic/Capric Triglyceride | 3 |
| Ethylhexyl Stearate | 2 |
| Dicaprylyl Carbonate | 2 |
| Glyceryl Stearate Citrate | 2 |
| Cetearyl Alcohol | 2 |
| Tocopherol | 1 |
| Propylene Glycol | 0.5 |
| Xanthan Gum | 0.5 |
| Ethylhexylglycerin | 0.5 |
| Sodium Stearoyl Glutamate | 0.5 |
| Althaea Officinalis Root Extract | 0.5 |
| Composition A | 6.0 |
| Sodium Chloride | 0.2 |
| Sodium Sulfate | 0.2 |
| Sodium Hydroxide | 0.2 |
| Phenoxyethanol | 0.2 |
| Parfum (Fragrance) | 0.2 |
| Ci 14700 (Red 4) | 0.2 |

### Example formulation 393: Ceramide Serum

| Ingredients | % |
|---|---|
| Cyclopentasiloxane | ad 100 |
| Dimethicone Crosspolymer | 5 |
| C12-15 Alkyl Benzoate | 5 |
| BHT | 5 |
| C18-36 Acid Triglyceride | 4 |
| Camellia Senensis Leaf Extract | 3 |
| Ceramide NP | 2 |
| Ceamide NS | 2 |
| Cyclohexasiloxane | 1 |
| Dimethiconol | 1 |
| Ethylhexyl Cocoate | 1 |
| Helianthus Annuus (Sunflower) Seed Oil | 1 |
| Laurylmethacrylate/Glycol Dimethacrylate Crosspolymer | 1 |
| Lecithin | 0.5 |
| Olea Europaea (Olive) Fruit Oil | 0.5 |
| Palmitoyl Tetrapeptide-7 | 0.5 |
| Palmitoyl Tripeptide-1 | 0.5 |
| Persea Gratissima (Avocado) Oil | 0.5 |
| Composition A | 1.5 |
| Phytosphingosine | 0.3 |
| Phytosterols | 0.3 |
| Polysorbate 20 | 0.3 |
| Propylene Glycol Dicaprylate/Dicaprate | 0.3 |
| Retinol | 0.2 |
| Sorbitan Laurate | 0.2 |
| Tetrahydrodiferuloylmethane | 0.2 |
| Phenoxyethanol. | 0.2 |

### Example formulation 394: Glowing Serum

| Ingredients | % |
|---|---|
| Aqua/Water | ad 100 |
| Glycerin | 5 |
| Pentylene Glycol | 5 |
| Dimethicone | 3 |
| Ascorbyl Glucoside | 3 |
| C14-22 Alcohols | 2 |
| Hydroxyethyl Acrylate/Sodium Acryloyldimethyl Taurate Copolymer | 1.5 |
| Oenothera Biennis (Evening Primrose) Oil | 1.5 |
| Corn Starch Modified | 1.5 |
| Camelina Sativa Seed Oil | 1 |
| Borago Officinalis Seed Oil | 1 |
| Echium Plantagineum Seed Oil | 1 |
| Helichrysum Italicum Flower Oil | 1 |
| Myrtus Communis Oil | 1 |
| Helianthus Annuus (Sunflower) Seed Oil | 1 |
| Bellis Perennis (Daisy) Flower Extract | 0.5 |
| Menyanthes Trifoliata Leaf Extract | 0.5 |
| Helichrysum Italicum Extract | 0.5 |
| Helichrysum Italicum Flower/Stem Extract | 0.5 |
| Rosmarinus Officinalis (Rosemary) Leaf Extract | 0.5 |
| Adenosine | 0.5 |
| Dicaprylyl Carbonate | 0.5 |
| Sucrose Palmitate | 0.5 |
| Tocopheryl Acetate | 0.5 |
| C12-20 Alkyl Glucoside | 0.5 |
| Sodium Citrate | 0.5 |
| Glyceryl Stearate | 0.5 |
| Sodium Hydroxide | 0.5 |
| PEG-100 Stearate | 0.5 |
| Glyceryl Linoleate | 0.5 |
| Ethylhexylglycerin | 0.5 |
| Sclerotium Gum | 0.5 |
| Xanthan Gum | 0.5 |
| Caprylic/Capric Triglyceride | 0.5 |
| Polysorbate 60 | 0.5 |
| Sorbitan Isostearate | 0.5 |
| Composition A | 0.8 |
| Disodium EDTA | 0.3 |
| Zinc Gluconate | 0.3 |
| Magnesium Aspartate | 0.2 |
| Sodium Hyaluronate | 0.2 |
| Citric Acid | 0.2 |
| Copper Gluconate | 0.2 |
| Tocopherol | 0.2 |
| Phenoxyethanol | 0.2 |
| Potassium Sorbate | 0.2 |
| Sodium Benzoate | 0.2 |
| Parfum/Fragrance | 0.2 |
| Benzyl Salicylate | 0.2 |
| Limonene | 0.2 |
| Linalool | 0.2 |
| Geraniol | 0.2 |
| Citral | 0.2 |
| Coumarin | 0.2 |

### Example formulation 395: Lifting Serum

| Ingredients | % |
|---|---|
| Water (Aqua) | ad 100 |
| Alcohol denat. | 8 |
| Glycerin | 5 |
| Aloe Barbadensis Leaf Juice | 5 |
| Squalane | 3 |
| Octyldodecanol | 3 |
| Lauroyl Lysine | 2 |
| Caprylic/Capric Triglyceride | 2 |
| Lavandula Hybrida Flower Extract | 1 |
| Gossypium Herbaceum (Cotton) Extract | 1 |
| Helianthus Annuus (Sunflower) Seed Oil | 1 |
| Sodium Lactate | 0.5 |
| Dehydroxanthan Gum | 0.5 |
| Glyceryl Undecylenate | 0.5 |
| Glyceryl Stearate Citrate | 0.5 |
| Levulinic Acid | 0.5 |
| Tocopheryl Acetate | 0.5 |
| Sodium Levulinate | 0.5 |
| Composition A | 0.9 |
| Sodium Hyaluronate | 0.3 |
| Pongamia Glabra Seed Oil | 0.3 |
| Glycine Soja (Soybean) Oil | 0.3 |
| Bakuchiol | 0.2 |
| Gellan Gum | 0.2 |
| Dipalmitoyl Hydroxyproline | 0.2 |
| Tocopherol | 0.2 |
| Leontopodium Alpinum Callus Culture Extract | 0.2 |
| Ascorbyl Palmitate | 0.2 |
| Hydrogenated Palm Glycerides | 0.2 |
| Hydrogenated Lecithin | 0.2 |
| Butyrospermum Parkii (Shea) Butter | 0.2 |
| Brassica Campestris (Rapeseed) Sterols | 0.2 |
| Xanthan Gum | 0.2 |
| Citric Acid | 0.2 |
| Fragrance (Parfum) | 0.1 |
| Linalool | 0.1 |
| Limonene | 0.1 |
| Geraniol | 0.1 |
| Citronellol | 0.1 |
| Citral | 0.1 |
| Benzyl Salicylate | 0.1 |

### Example formulation 396: Hydra Serum

| Ingredients | % |
|---|---|
| Aqua | ad 100 |
| Triethyl Citrate | 8 |
| Isopentyldiol | 5 |
| Glycerin | 5 |
| Propanediol | 4 |
| Pentylene Glycol | 3 |
| Algin | 0.8 |
| Gluconolactone | 0.8 |
| Trehalose | 0.8 |
| Urea | 0.8 |
| Composition A | 0.7 |
| Sodium Hyaluronate | 0.3 |
| Sodium Benzoate | 0.3 |
| Maltodextrin | 0.2 |
| Calcium Citrate | 0.2 |
| Parfum / Fragrance | 0.2 |
| Sodium Hydroxide | 0.2 |
| Citric Acid | 0.2 |
| Serine | 0.2 |
| Biosaccharide Gum-1 | 0.2 |
| Caprylyl Glycol | 0.2 |
| Disodium Phosphate | 0.2 |
| Glyceryl Polyacrylate | 0.2 |
| Pullulan | 0.2 |
| Calcium Gluconate | 0.2 |
| Bacillus Ferment | 0.1 |
| Potassium Phosphate | 0.1 |
| 4106a | 0.1 |

### Example formulation 397: Almond Serum

| Ingredients | % |
|---|---|
| Aqua | ad 100 |
| Glycerin | 6 |
| Prunus Amygdalus Dulcis (Sweet Almond) Oil | 5 |
| Caprylic/Capric Triglyceride | 4 |
| Dicaprylyl Ether | 3 |
| C14-22 Alcohols | 3 |
| Hydroxyethyl Urea | 2 |
| Tocopherol | 1.5 |
| Phenoxyethanol | 1 |
| C12-20 Alkyl Glucoside | 1 |
| Ethylhexylglycerin | 1 |
| Acrylates/C10-30 Alkyl Acrylate Crosspolymer | 1 |
| Dehydroacetic Acid | 0.5 |
| Bougainvillea Glabra Leaf Cell Extract | 0.5 |
| Sodium Gluconate | 0.5 |
| Sodium Stearoyl Glutamate | 0.5 |
| Xanthan Gum | 0.5 |
| Butylene Glycol | 0.5 |
| Hyaluronic Acid | 0.5 |
| Sodium Hydroxide | 0.5 |
| Ammonium Lactate | 0.5 |
| Ascorbyl Glucoside | 0.5 |
| Olea Europaea (Olive) Leaf Extract | 0.5 |
| Composition A | 1.3 |
| Zinc PCA | 0.3 |
| Undaria Pinnatifida Extract | 0.2 |
| Alteromonas Ferment Extract | 0.2 |
| Theobroma Cacao (Cocoa) Leaf Cell Extract | 0.2 |
| Crocus Sativus Flower Extract | 0.2 |
| Fragrance / Perfume | 0.2 |
| Benzyl Alcohol | 0.2 |
| Ascorbic Acid | 0.2 |
| Benzyl Salicylate | 0.2 |
| Linalool | 0.1 |
| Coumarin | 0.1 |
| Geraniol | 0.1 |
| Citronellol [N3201/B] | 0.1 |

### Example formulation 398: Brilliance Serum

| Ingredients | % |
|---|---|
| Aqua | ad 100 |
| Caprylic/ Capric Triglyceride | 5 |
| Palmitic Acid | 3 |
| Stearic Acid | 3 |
| Prunus Amygdalus Dulcis (Sweet Almond) Oil | 2 |
| Dipropylene Glycol | 2 |
| Tocopherol Acetate | 1.5 |
| Glycine Soja (Soybean) Oil | 1 |
| Sodium Hyaluronate | 1 |
| Alcohol | 0.5 |
| Ethylhexylglycerin | 0.5 |
| Phenoxyethanol | 0.5 |
| Composition A | 9.0 |
| Sodium Pca | 0.2 |
| Ammonium Polyacryloyldimethyl Taurate | 0.2 |
| Sodium Hyaluronate | 0.2 |
| Disodium EDTA | 0.2 |
| Xanthan Gum | 0.2 |
| Pentaerythrityl Tetra-Di-t-Butyl Hydroxyhydrocinnamate | 0.2 |
| Benzotriazolyl Dodecyl p-Cresol | 0.2 |
| Acmella Oleracea Extract | 0.2 |
| Maltodextrin | 0.2 |
| Diamond Powder | 0.2 |
| Acetyl SH-Pentapeptide-35 | 0.2 |
| Potassium Sorbate | 0.2 |
| Fragrance (Parfum) | 0.2 |
| Benzyl Salicylate | 0.2 |
| Geraniol | 0.1 |
| Citronellol | 0.1 |
| Hexyl Cinnamal | 0.1 |
| D-Limonene | 0.1 |
| Linalool | 0.1 |
| Alpha-Isomethyl Ionone | 0.1 |

### Example formulation 399: Scalp Serum

| Ingredients | % |
|---|---|
| Aqua | ad 100 |
| Glycerin | 5 |
| Citric Acid | 3 |
| Polyquaternium-37 | 3 |
| Paraffinumliquidum / Mineral Oil | 3 |
| Amodimethicone | 2 |
| Phenoxyethanol | 2 |
| Niacinamide | 2 |
| Polysorbate 20 | 1 |
| Quaternium-87 | 1 |
| Stearyl Alcohol | 1 |
| Behentrimonium Chloride | 1 |
| Panthenol | 1 |
| Ascorbyl Glucoside | 1 |
| Butylene Glycol | 0.8 |
| Propylene Glycol | 0.8 |
| Candelilla Cera / Candelilla Wax | 0.5 |
| PPG-1 Trideceth-6 | 0.5 |
| Chlorhexidine Digluconate | 0.5 |
| Isopropyl Alcohol | 0.5 |
| Trideceth-6 | 0.5 |
| Acrylates Copolymer | 0.5 |
| Sorbitan Oleate | 0.5 |
| Polyquaternium-11 | 0.5 |
| Composition A | 2.5 |
| Linalool | 0.3 |
| Hydrolyzed Wheat Protein | 0.3 |
| 2-Oleamido-1.3-Octadecanediol | 0.3 |
| Hydroxycitronellal | 0.3 |
| Benzyl Salicylate | 0.3 |
| Hydrolyzed Corn Protein | 0.3 |
| Hydrolyzed Soy Protein | 0.3 |
| Cetrimonium Chloride | 0.3 |
| Benzyl Alcohol | 0.2 |
| Hexyl Cinnamal | 0.2 |
| Citronellol | 0.2 |
| Iris Florentina Root Extract | 0.2 |
| Alpha-Isomethyl Ionone | 0.2 |
| Coumarin | 0.2 |
| Limonene | 0.2 |
| Parfum / Fragrance | 0.2 |

### Example formulation 400: Serum with Actives

| Ingredients | % | % | % | % | % | % | % | % | % | % | % | % |
|---|---|---|---|---|---|---|---|---|---|---|---|---|
| Aqua | ad 10 0 | ad 100 | ad 10 0 | ad 100 | ad 10 0 | ad 10 0 | ad 10 0 | ad 10 0 | ad 100 | ad 100 | ad 10 0 | ad 10 0 |
| Glycerin | 6 | 6 | 6 | 6 | 6 | 6 | 6 | 6 | 6 | 6 | 6 | 6 |
| Caprylic/Capric Triglyceride | 5 | 5 | 5 | 5 | 5 | 5 | 5 | 5 | 5 | 5 | 5 | 5 |
| Alcohol Denat. | 5 | 5 | 5 | 5 | 5 | 5 | 5 | 5 | 5 | 5 | 5 | 5 |
| Cocoglycerides | 3 | 3 | 3 | 3 | 3 | 3 | 3 | 3 | 3 | 3 | 3 | 3 |
| Glyceryl Stearate Citrate | 2 | 2 | 2 | 2 | 2 | 2 | 2 | 2 | 2 | 2 | 2 | 2 |
| Butyrospermum Parkii Butter | 1 | 1 | 1 | 1 | 1 | 1 | 1 | 1 | 1 | 1 | 1 | 1 |
| Distarch Phosphate | 0.5 | 0.5 | 0.5 | 0.5 | 0.5 | 0.5 | 0.5 | 0.5 | 0.5 | 0.5 | 0.5 | 0.5 |
| Acteyl Hydroxyproline | 0.5 | 0.5 | 0.5 | 0.5 | 0.5 | 0.5 | 0.5 | 0.5 | 0.5 | 0.5 | 0.5 | 0.5 |
| Ubiquinone | 0.5 | 0.5 | 0.5 | 0.5 | 0.5 | 0.5 | 0.5 | 0.5 | 0.5 | 0.5 | 0.5 | 0.5 |
| 1-Methylhydantoin-2-Imide | 0.5 | 0.5 | 0.5 | 0.5 | 0.5 | 0.5 | 0.5 | 0.5 | 0.5 | 0.5 | 0.5 | 0.5 |
| Creatine | 0.5 | 0.5 | 0.5 | 0.5 | 0.5 | 0.5 | 0.5 | 0.5 | 0.5 | 0.5 | 0.5 | 0.5 |
| Hydroxyacetopheno ne | 0.5 | 0.5 | 0.5 | 0.5 | 0.5 | 0.5 | 0.5 | 0.5 | 0.5 | 0.5 | 0.5 | 0.5 |
| Xanthan Gum | 0.5 | 0.5 | 0.5 | 0.5 | 0.5 | 0.5 | 0.5 | 0.5 | 0.5 | 0.5 | 0.5 | 0.5 |
| Composition A | 2.0 | 0.7 | 1.5 | 5.0 | 8.0 | 6.5 | 10 | 9.0 | 3.0 | 3.5 | 2.5 | 0.1 |
| Hyaluronic Acid | 2 | 2 | 2 | 2 | 2 | 2 | 2 | 2 | 2 | 2 | 2 | 2 |
| Niacinamide | 3 | 3.75 | 4 | 4.75 | | | | | | | | |
| Retinol | | 0.00 1 | | 0.1 | 0.2 | 0.4 | | | 0.00 5 | 0.0 5 | | |
| Lecithin | | | | | | | 2 | 3 | | | | |
| Coffeine | | | | | | | | | 1 | 1.5 | | |
| Peptides | | 0.3 | 0.1 | 0.00 5 | | 0.5 | | | | | 0.5 | 1.5 |
| Sodium Hydroxide | 0.2 | 0.2 | 0.2 | 0.2 | 0.2 | 0.2 | 0.2 | 0.2 | 0.2 | 0.2 | 0.2 | 0.2 |
| Phenoxyethanol | 0.2 | 0.2 | 0.2 | 0.2 | 0.2 | 0.2 | 0.2 | 0.2 | 0.2 | 0.2 | 0.2 | 0.2 |
| Pogostemon Cablin Oil | 0.2 | 0.2 | 0.2 | 0.2 | 0.2 | 0.2 | 0.2 | 0.2 | 0.2 | 0.2 | 0.2 | 0.2 |
| Parfum | 0.2 | 0.2 | 0.2 | 0.2 | 0.2 | 0.2 | 0.2 | 0.2 | 0.2 | 0.2 | 0.2 | 0.2 |

### Further example formulations:

| | I | II | III | IV | V | VI | VII | VIII | IX | X |
|---|---|---|---|---|---|---|---|---|---|---|
| Water | ad 100 | | | | | | | | | |
| Composition A | 9.0 | 15.0 | 5.5 | 5.0 | 7.0 | 3.0 | 6.0 | 4.0 | 3.5 | 3.0 |
| Sodium Laureth Sulfate | - | - | - | 7.0 | - | - | - | 4.5 | - | - |
| Sodium Lauryl Sulfate | - | - | - | - | 5.0 | - | - | - | 3.5 | 1.0 |
| Cocamidopropyl Betaine | - | - | - | - | - | 6.0 | - | 3.0 | 2.0 | 4.5 |
| Sodium Cocoamphoacetate | - | - | - | - | - | - | 2.0 | - | 3.5 | - |
| Coco-Glucoside | - | - | - | - | - | - | 5.5 | 1.5 | - | - |
| Sodium Cocoyl Glutamate | - | - | - | - | - | - | 0.7 | - | - | - |
| Stearic Acid | - | - | - | - | - | - | | 1.0 | - | 3.5 |
| Glyceryl Glucoside | - | 0.5 | - | - | 0.3 | - | 0.2 | - | - | - |
| Sucrose Cocoate | 0.5 | - | 1.0 | 1.0 | 1.0 | 0.5 | | 1.0 | 1.0 | 1.0 |
| Glycerin | - | 1.0 | 0.5 | - | - | 0.3 | 1.5 | 1.0 | - | 1.0 |
| PEG-7 Glyceryl Cocoate | - | 0.7 | - | 0.5 | - | - | - | - | - | 0.5 |
| Trideceth-9 | - | - | - | 0.2 | - | - | - | 0.3 | - | - |
| Polyglyceryl-4 Caprate | - | - | 1.0 | - | 0.4 | - | 0.5 | - | - | - |
| Polyquaternium-10 | - | 0.2 | - | 0.1 | - | - | - | 0.2 | - | - |
| Hydroxypropyl Guar Hydroxypropyltrimonium Chloride | 0.2 | - | 0.3 | 0.2 | 0.2 | 0.2 | 0.3 | 0.1 | 0.2 | - |
| Silicone Quaternium-22 | - | - | - | 0.5 | - | - | - | 0.5 | - | - |
| Dimethicone | - | 0.5 | - | - | - | - | - | 1.0 | - | - |
| Amodimethicone | - | 0.1 | - | - | 1.0 | 0.3 | - | - | 0.5 | - |
| Argania Spinosa Oil | - | - | 0.2 | 0.1 | - | 0.1 | 0.2 | - | - | - |
| Glycol Distearate | 0.5 | 0.5 | - | - | 0.5 | | 0.3 | 0.5 | 0.5 | 0.5 |
| Acrylates/C10-30 Alkyl Acrylate Crosspolymer | 1.0 | - | - | - | 0.5 | - | - | - | 0.5 | - |
| Sodium Hydroxide. 25% | 1.5 | - | - | - | 0.8 | - | - | - | 0.7 | - |
| Isostearamide MIPA; Glyceryl Laurate | - | - | - | - | - | 2.5 | - | - | 1.0 | 0.5 |
| Cocamide DEA | - | - | - | 1.0 | 0.5 | - | - | - | - | - |
| Sodium Chloride | 0.1 | 1.2 | - | 0.5 | - | 1.5 | - | 2.0 | | 1.2 |
| PEG-120 Methyl Glucose Dioleate | - | 3.5 | - | 0.2 | - | - | - | 0.6 | - | - |
| Xanthan Gum | - | - | 1.2 | - | - | 0.2 | 1.5 | - | - | - |
| Cellulose | - | - | - | 0.1 | - | 0.1 | 0.1 | - | 0.1 | - |
| Zinc Pyrithione | - | 0.1 | - | 1.1 | - | - | - | 0.5 | - | - |
| Benzophenone-4 | - | 0.1 | - | 0.2 | - | - | - | 0.2 | 0.1 | - |
| Tetrasodium EDTA | 0.1 | 0.1 | - | 0.1 | - | - | - | 0.1 | 0.1 | - |
| Caffeine | - | - | 0.1 | - | - | - | - | 0.1 | 0.1 | - |
| Hydrolyzed Keratin | - | - | 0.1 | 0.1 | - | 0.1 | 0.2 | - | 0.1 | - |
| Panthenol | 0.1 | 0.1 | 0.1 | 0.1 | 0.1 | 0.1 | - | 0.1 | 0.1 | 0.1 |
| Citric Acid | ad pH 6.0 | | | | | | | | | |
| Parfums. Dyes. Preservatives | q. s. | | | | | | | | | |

| | XI | XII | XIII | XIV | XV | XVI | XVII | XVIII | XIX | XX |
|---|---|---|---|---|---|---|---|---|---|---|
| Water | ad 100 | | | | | | | | | |
| Composition A | 10.0 | 7.0 | 5.0 | 4.0 | 7.0 | 2.5 | 6.0 | 4.0 | 2.0 | 1.0 |
| Sodium Laureth Sulfate | - | - | 8.0 | - | - | - | - | 3.5 | - | - |
| Coco-Betaine | - | - | - | 5.5 | 5.0 | - | - | 3.0 | - | - |
| Cocamidopropyl Betaine | - | - | - | - | - | 6.0 | - | - | 3.0 | 4.0 |
| Sodium Cocoamphoacetate | - | - | - | - | 2.0 | - | - | - | - | - |
| Coco Glucoside | - | - | - | - | 2.0 | - | 4.0 | 1.5 | 6.0 | - |
| Glycolipids | - | - | - | - | - | - | 2.5 | 0.5 | 2.5 | 0.3 |
| Stearic Acid | - | 8.0 | - | - | - | - | - | - | - | 4.5 |
| Sodium Cocoyl Glycinate | - | - | - | - | - | 3.0 | 0.5 | - | - | 0.3 |
| Sodium Lauroyl Methyl Isethionate | - | - | - | - | - | 1.0 | - | 1.0 | 2.0 | 0.5 |
| Sucrose Cocoate | 0.5 | 1.1 | - | 1.0 | 1.0 | - | - | 0.3 | 1.0 | 0.3 |
| Glycerin | 1.5 | 0.3 | 0.5 | - | 0.3 | 0.5 | 1.0 | 0.5 | - | 1.0 |
| PEG-7 Glyceryl Cocoate | - | - | 0.5 | - | - | 0.8 | - | 0.5 | - | 0.5 |
| PEG-40 Hydrogenated Castor Oil | - | - | 0.5 | - | - | 1.5 | - | 0.3 | - | - |
| Polyglyceryl-4 Caprate | 0.5 | - | - | - | 0.4 | - | 0.5 | | 1.1 | - |
| Polyquaternium-11 | - | 0.2 | - | - | 0.1 | 0.3 | - | 0.2 | - | - |
| Guar Hydroxypropyltrimonium Chloride | 0.3 | - | 0.3 | 0.2 | 0.2 | - | 0.3 | 0.1 | 0.4 | 0.2 |
| Silicone Quaternium-22 | - | - | 0.5 | - | - | - | - | 1.5 | - | - |
| PEG-12 Dimethicone | - | - | - | - | - | 1.5 | - | - | - | - |
| Dimethicone | - | - | 1.0 | - | 0.5 | - | - | - | - | - |
| Aminopropyl Dimethicone | - | - | - | - | 0.3 | - | - | - | - | - |
| Helianthus Annuus Oil | 0.1 | - | 0.2 | 0.1 | - | 0.1 | 0.3 | 0.2 | - | 0.1 |
| PEG-3 Distearate | - | - | 0.5 | - | - | 0.8 | - | 0.5 | - | - |
| Acrylates/C10-30 Alkyl Acrylate Crosspolymer | - | - | 0.2 | - | 0.5 | - | - | - | - | - |
| Sodium Hydroxide. 25% | - | - | 0.3 | - | 0.8 | - | - | - | - | - |
| Cocamide DEA | - | - | - | - | - | 2.5 | - | | - | 0.5 |
| Cocamide MEA | - | - | - | 1.0 | - | - | - | 1.0 | - | - |
| Sodium Chloride | 0.4 | 0.7 | 0.1 | - | - | 1.5 | - | 2.0 | - | 1.2 |
| Propylene Glycol (and) PEG-55 Propylene Glycol Oleate | - | 2.5 | 2.0 | - | - | 0.3 | - | 0.6 | - | 0.2 |
| Xanthan Gum | 1.2 | - | - | 1.0 | 0.2 | - | 1.5 | 0.1 | 1.1 | - |
| Hydroxyethyl Cellulose | 0.1 | - | - | 0.1 | - | 0.1 | 0.1 | - | - | - |
| Benzophenone-4 | - | 0.1 | 0.2 | - | - | 0.2 | - | 0.2 | - | 0.1 |
| Menthol | 0.1 | - | - | - | - | - | - | - | - | 0.1 |
| Tetrasodium Glutamate Diacetate | - | 0.1 | - | 0.1 | 0.1 | - | 0.1 | 0.1 | - | 0.1 |
| Caffeine | 0.1 | - | 0.1 | - | - | - | - | 0.1 | - | - |
| Coumarin | 0.1 | - | - | 0.1 | 0.1 | - | 0.1 | 0.1 | - | 0.1 |
| Hydrolyzed Wheat Protein | 0.1 | - | 0.1 | 0.1 | - | - | 0.2 | 0.1 | 0.1 | 0.1 |
| Panthenol | 0.1 | 0.1 | - | 0.1 | 0.1 | 0.1 | 0.1 | - | 0.1 | 0.1 |
| Citric Acid | ad pH 5.5 | | | | | | | | | |
| Parfums. Dyes. Preservatives | q. s. | | | | | | | | | |

| | XXI | XXII | XXIII | XIV | XXV | XXVI | XXVII | XXVIII | XXIX | XXX |
|---|---|---|---|---|---|---|---|---|---|---|
| Water | ad 100 | | | | | | | | | |
| Composition A | 6.0 | 4.0 | 3.5 | 1.0 | 3.0 | 3.0 | 4.0 | 2.0 | 2.5 | 5.0 |
| Sodium Coco-Sulfate | 3.0 | 1.0 | - | 5.0 | 3.0 | 3.0 | 2.0 | - | 2.5 | 3.0 |
| Glycolipids | 1.0 | - | 1.5 | 1.0 | - | 2.0 | - | - | 2.0 | - |
| Lauryl Glucoside | - | 2.0 | - | - | - | - | - | 2.0 | 3.5 | 2.0 |
| Sodium Cocoyl Glutamate | 1.0 | - | - | - | 3.0 | - | - | - | - | - |
| Sodium Cocoyl Glycinate | - | - | 1.5 | - | - | - | - | 2.5 | 1.5 | - |
| Sodium C14-16 Olefin Sulfonate | - | - | - | - | 3.0 | - | 2.0 | - | - | 2.0 |
| Lauroyl/Myristoyl Methyl Glucamide | - | 1.0 | - | 2.0 | - | - | - | - | - | - |
| Cocamidopropyl Betaine | - | 2.0 | 1.5 | 4.0 | - | 2.0 | - | 7.5 | - | - |
| Sodium Cocoamphoacetate | - | 2.0 | 5.0 | - | 2.0 | 2.0 | 4.0 | - | - | - |
| Sucrose Cocoate | 1.0 | - | 1.5 | - | 1.5 | 1.0 | - | 1.0 | - | 3.5 |
| Glycerin | 0.5 | 0.5 | - | - | 0.3 | - | 0.2 | - | 0.5 | - |
| Alcohol Denat. | - | - | 3.0 | - | - | 2.0 | - | - | - | - |
| Sorbitan Sesquicaprylate | 1.0 | - | 0.7 | 1.0 | - | 0.5 | - | 0.5 | - | 1.0 |
| Isostearamide MIPA; Glyceryl Laurate | - | 1.0 | - | - | - | 2.0 | - | - | - | 0.5 |
| Xanthan Gum | 1.0 | 0.7 | 0.5 | - | 0.7 | 0.3 | 0.7 | 0.5 | - | 0.5 |
| Chondrus Crispus | - | - | 1.5 | - | 2.0 | 1.5 | 0.7 | - | - | 0.5 |
| Carrageenan | - | - | - | 1.0 | - | - | - | 0.3 | - | 0.5 |
| Hydroxypropyl Starch Phosphate | - | - | - | - | - | - | - | 4.5 | 5.5 | - |
| Guar Hydroxpropyltrimonium Chloride | 0.2 | - | 0.5 | 0.2 | 0.2 | 0.5 | 0.3 | 0.1 | - | - |
| Bis-(Isostearoyl/Oleyl Isopropyl) Dimonium Methosulfate | 0.5 | 0.5 | - | - | 0.5 | - | - | - | 0.5 | - |
| Prunus Amygdalus Dulcis (Almond) Oil | - | 0.1 | - | 0.1 | - | 0.05 | - | - | - | - |
| Lemon Oil | - | - | 0.2 | - | 0.1 | - | 0.2 | | | |
| Cellulose | 0.5 | - | - | - | 0.7 | - | 0.3 | 0.1 | - | 0.5 |
| Charcoal Powder | 1.0 | - | - | - | - | - | - | - | - | - |
| Saccharum Officinarum (Sugar Cane) Extract | - | - | - | 0.1 | - | 0.2 | - | - | - | - |
| Sodium Chloride | 0.7 | - | - | 0.5 | 1.0 | 0.7 | - | 2.0 | - | 1.2 |
| Polyglyceryl-6 Caprylate; Polyglyceryl-3 Cocoate; Polyglyceryl-4 Caprate; Polyglyceryl-6 Ricinoleate | - | 3.0 | - | 4.0 | - | 2.5 | - | - | | - |
| Caprylyl/Capryl Glucoside; Aqua. Sodium Cocoyl Glutamate; Glyceryl Caprylate;Ccitric Acid; Polyglyceryl-6 Oleate. Sodium Surfactin | - | - | 3.0 | - | 1.0 | - | 2.0 | - | - | - |
| Tocopherol | - | 0.1 | - | 0.1 | - | 0.1 | 0.1 | - | 0.1 | - |
| Phytic Acid | 0.1 | 0.1 | - | - | - | - | - | 0.1 | 0.1 | - |
| Citric Acid | ad pH 6.0 | | | | | | | | | |
| Parfums. Dyes. Preservatives | q. s. | | | | | | | | | |

| | XXXI | XXXII | XXXIII | XXXIV | XXXV | XXXVI | XXXVII | XXXVIII |
|---|---|---|---|---|---|---|---|---|
| Water | ad 100 | | | | | | | |
| Composition A | 1.0 | 1.5 | 2.0 | 1.5 | 3.0 | 2.5 | 4.0 | 5.0 |
| Capryl/Capramidopropyl Betaine | - | 2.0 | - | - | 1.0 | - | 2.0 | - |
| Glycolipids | 1.0 | - | - | - | 1.0 | - | - | - |
| Decyl Glucoside | 1.0 | - | - | - | - | 2.0 | - | - |
| Disodium Cocoamphodiacetate | - | - | 3.0 | 2.0 | - | - | 2.0 | - |
| PEG-6 Caprylic/Capric Glycerides | 3.0 | 1.5 | - | - | - | - | - | 2.0 |
| Polyglyceryl-6 Caprylate; Polyglyceryl-4 Caprate | - | - | 2.0 | - | - | 3.5 | - | - |
| Polyglyceryl-6 Caprylate; Polyglyceryl-3 Cocoate; Polyglyceryl-4 Caprate; Polyglyceryl-6 Ricinoleate | - | 3.0 | - | - | 4.0 | - | - | - |
| Polyglyceryl-4 Caprate | - | - | - | 2.5 | - | - | - | - |
| Persea Gratissima (Avocado) Oil | - | 0.1 | - | - | 0.2 | - | 0.1 | 0.05 |
| Rosemary Oil | 0.1 | - | 0.05 | - | 0.1 | - | - | - |
| Hydroxypropyl Methylcellulose | - | 0.5 | - | - | - | - | 0.5 | - |
| Sorbitan Sesquicaprylate | 0.3 | - | - | 0.5 | - | 0.3 | 0.5 | 0.1 |
| Betaine | - | 0.5 | - | 1.0 | - | 1.0 | - | 1.0 |
| Allantoin | - | - | 0.1 | - | 0.1 | - | - | - |
| Starch | - | - | - | 0.1 | 0.05 | | - | 0.1 |
| Glycerin | 3.0 | 1.0 | - | - | - | 1.0 | 1.0 | - |
| Hexylene Glycol | 1.0 | - | 1.0 | - | 1.0 | 1.0 | - | - |
| Methylpropanediol; Caprylyl Glycol; Benzoic Acid | 1.2 | - | - | - | - | - | - | - |
| Benzyl Alcohol. Caprylyl Glycol. Benzoic Acid | - | 1.0 | 1.0 | 1.0 | 1.0 | - | - | - |
| Aqua. Sodium Levulinate. Sodium Benzoate | - | - | - | - | - | 1.0 | 1.0 | 1. |
| pH adjuster (Citric Acid or NaOH) | ad pH 5,0 | | | | | | | |
| Preservatives. Parfum | q. s. | | | | | | | |

## Claims

1. Composition comprising
A) at least one ceramide, and
B) at least one rhamnolipid, **characterized in that** component B) comprises at least 50 wt.-% mono-rhamnolipids, wherein the weight percentages refer to all rhamnolipids comprised in the total composition.

2. Composition according to claim 1, **characterized in that** said at least one ceramide is selected from the group comprising ceramide NP, ceramide AP, ceramide EOP, ceramide NG, ceramide ADS, ceramide EODS, ceramide NS, ceramide AS, ceramide EOS, ceramide NH, ceramide AH and ceramide EOH, preferably selected from the group comprising ceramide NP, ceramide NG, ceramide AP, most preferably ceramide NP.

3. Composition according to claim 1 or 2, **characterised in that** component A) is comprised in an amount of from 0.0005 wt.-% to 5.0 wt.-%, more preferably from 0.001 wt.-% to 2.0 wt.-%, more preferably from 0.002 wt.-% to 1.5 wt.-%, even more preferably from 0.05 wt.-% to 1.0 wt.-%, where the percentages by weight refer to the total composition.

4. Composition according to at least one of the preceding claims, **characterised in that** component B) comprises
12 wt.-% to 32 wt.-% monoRL-C8C10,
51 wt.-% to 81 wt.-% monoRL-C10C10,
1 wt.-% to 9 wt.-% monoRL-C10C12,
1 wt.-% to 9 wt.-% monoRL-C10C12:1, wherein the weight percentages refer to all mono-rhamnolipids comprised in the composition.

5. Composition according to at least one of the preceding claims, **characterised in that** component B) is comprised in an amount of from 0.1 wt.-% to 15.0 wt.-%, preferably from 0.5 wt.-% to 10.0 wt.-%, more preferably from 1.0 wt.-% to 8.0 wt.-%, wherein the percentages by weight refer to the total composition.

6. Composition according to at least one of the preceding claims, **characterized in that** it comprises
C) cholesterol and/or at least one cholesterol derivative selected from the group comprising cholesterol sulfate, cholesterol hydrogen succinate and 7-dehydrocholesterol,
wherein preferably component C) is comprised in an amount of from 0.01 wt.-% to 3.0 wt.-%, preferably from 0.02 wt.-% to 2.0 wt.-%, more preferably from 1.0 wt.-% to 0.05 wt.-%, where the percentages by weight refer to the total composition.

7. Composition according to at least one of the preceding claims, **characterized in that** it comprises
D) at least one sphingoid base wherein preferably component D) is comprised in an amount of from 0.001 wt.-% to 2.0 wt.-%, preferably from 0.002 wt.-% to 1.5 wt.-%, more preferably from 0.05 wt.-% to 1.0 wt.-%, where the percentages by weight refer to the total composition.

8. Composition according to at least one of the preceding claims, **characterized in that** it comprises instead of or additionally to component D)
E) at least one fatty acid, preferably selected from the group of fatty acids comprising 12 to 28 carbon atoms, especially palmitic acid, stearic acid, octadecanoic acid, arachidic acid, behenic acid, docosanoic acid and lignoceric acid.
wherein preferably component E) is comprised in an amount of from 0.01 wt.-% to 3.0 wt.-%, preferably from 0.02 wt.-% to 2.0 wt.-%, more preferably from 1.0 wt.-% to 0.05 wt.-%, where the percentages by weight refer to the total composition.

9. Composition according to at least one of the preceding claims, **characterized in that** said composition has a pH in the range of 4.0 to 8.0, preferably 4.5 to 7.4 particularly preferably 5.0 to 7.2.

10. Composition according to at least one of the preceding claims, **characterized in that** said composition comprises water, preferably in the range of 75.0 wt.-% to 99.5 wt.-%, preferably from 80.0 wt.-% to 98.5 wt.-%, more preferably from 85.0 wt.-% to 98.0 wt.-%, based on the total composition.

11. Process for producing a cosmetic or pharmaceutical formulation comprising at least one ceramide, comprising the process steps of:
a) providing a composition according to at least one of the claims 1 to 10;
b) adding a cosmetic or pharmaceutical acceptable carrier, and preferably one further cosmetical and pharmaceutical formulation ingredient.

12. Process according to claim 11, **characterized in that** process comprises
c) solubilization of the formulation.

13. Process according to claim 11 or 12, **characterized in that** process comprises
d) adjusting the pH of the formulation to a range of 4.0 to 8.0, preferably 4.5 to 7.4 particularly preferably 5.0 to 7.2.

14. Use of mono-rhamnolipid, preferably in an amount of from 0.1 wt.-% to 15.0 wt.-%, preferably from 0.5 wt.-% to 10.0 wt.-%, more preferably from 1.0 wt.-% to 8.0 wt.-%, wherein the percentages refer to the total composition, for solubilizing and/or physical stabilization of a ceramide-containing composition, in particular with regard to homogeneity, and/or for preventing crystallization of at least one ceramide in a composition.
